# EUROPEAN PATENT SPECIFICATION

(11) **EP 0 597 540 B1**
(45) Date of publication and mention of the grant of the patent: **05.06.1996**
(21) Application number: 93203115.6
(22) Date of filing: 06.11.1993
(51) Int. Cl.: C07D 243/04, A61K 31/55, C07D 409/04, C07D 405/12, C07D 413/06, C07D 401/04, C07D 405/06, C07D 495/04

(54) **Aryl-fused and hetaryl-fused-2,4-diazocine antiarrhythmic agents**
Aryl und Hetaryl annelierte 2,4-Diazocine als anti-arryhthmische Wirkstoffe
Diazocines-2,4 condensées avec des aryles et des hétéroarnyles comme agents antiarrhytmiques

(30) Priority: 10.11.1992 US 974396; 24.02.1993 US 21926
(43) Date of publication of application: 18.05.1994
(73) Proprietor: STERLING WINTHROP INC., New York, NY 10016 (US)
(72) Inventor: Johnson, Robert Ed., c/o Sterling Winthrop Inc., New York, New York 10016 (US); Schlegel, Donald Charles., c/o Sterling Win. Inc., New York, New York 10016 (US); Ezrin, Alan Mark., c/o Sterling Winthrop Inc., New York, New York 10016 (US)
(74) Representative: Le Guen, Gérard

(56) References cited:
- EP-A- 0 389 765
- EP-A- 0 431 945
- EP-A- 0 475 527
- GB-A- 1 183 135
- US-A- 3 696 093
- US-A- 5 215 989

## Description

The present invention relates to novel 4,5-dihydro-1H-2,4-aryl fused diazepines, and to methods and compositions for treating cardiac arrhythmias in mammals utilizing said 4,5-dihydro-1H-2,4-benzodiazepines.

U.S. Patent 3,696,093 to Rodriguez et al. discloses a single 3,4-disubstituted benzodiazepine: 3,4-dimethyl-4,5-dihydro-1H-2,4-benzodiazepine hydrochloride. 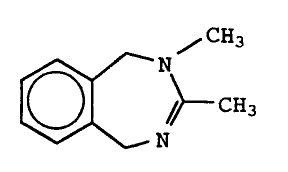
Also disclosed are 4,5-dihydro-1H-2,4-benzodiazepines monosubstituted in the 3 position with benzyl, dimethylaminoethyl, amino, 1-piperidinylmethyl, and phenyl. The compounds are said to be useful as cardiovascular agents, for example, in the treatment or management of the various forms of hypertension or of congestive heart failure. The patent does not disclose antiarrhythmic properties for the genus, and the single example of a disubstituted benzodiazepine was found to be inactive as an antiarrhythmic when tested in the protocol used to evaluate the compounds of the present invention.

Japanese application 59/013766 (CA 101:23612m) discloses a series of 1,2,4-tri-substituted-tetrahydrobenzodiazepines of general structure 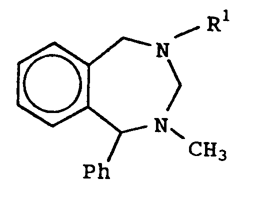
wherein R¹ is lower alkyl or phenethyl (opt. substd. with lower alkoxy). The compounds are said to be analgesics.

Elslager et al [J. Het. Chem. 5, 609-613 (1968)] describe the synthesis of a series of tetrahydrothiazolo-[3,2-b][2,4] benzodiazepines. The authors state that "None of the compounds possessed appreciable biological activity." As intermediates in the synthesis, they disclose 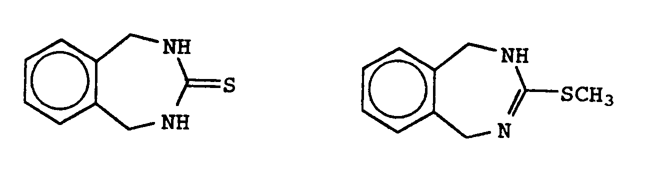
1,2,4,5-tetrahydro-3H-2,4-benzodiazepine-3-thione and 2,5,dihydro-3-(methylthio)-1H-2,4-benzodiazepine hydroiodide.

U.S. Patent 4,840,948 to Lang et al. discloses a series of 1-(hydroxystyryl)-5H-2,3-benzodiazepines of general formula: 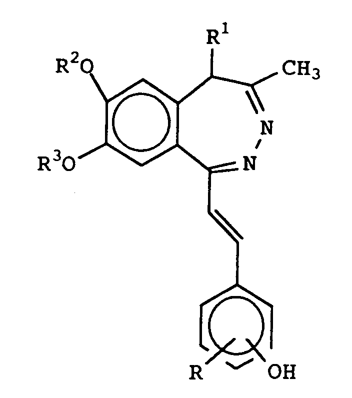
wherein
R stands for a hydrogen or halogen atom, or a C₁₋₄ alkoxy group,
R¹ represents a hydrogen atom or a C₁₋₄ alkyl group,
R² and R³ are identical and denote a C₁₋₄ alkyl group,
or combined they denote a methylene group.
The compounds are said to be positive inotropes and therefore useful as cardiotonics.

Carr et al., European Patent Application 389765 disclose compounds of the formula: 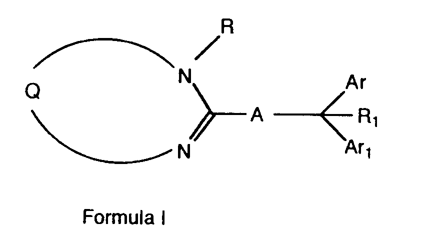
in which Q is represented by a substituent selected from the group consisting of (CH₂)ₙ in which n is an integer from 2-10, 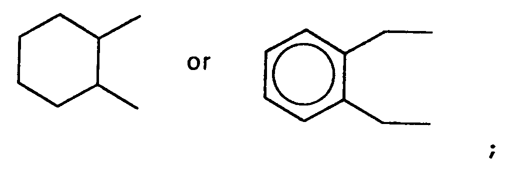
A is a substituent selected from the group consisting of -NH-(CH₂)ₘ in which m is an integer from 0-5, a piperidino substituent, or a piperazino substituent; both Ar and Ar₁ are each independently represented by a phenyl ring each of which may be optionally substituted with up to 3 substituents, each selected from the group consisting of halogen, C₁₋₄ alkyl, C₁₋₄ alkoxy, hydroxy, and trifluoromethyl; and R is represented by hydrogen or C₁₋₄ alkyl; R₁ is represented by hydrogen or C₁₋₄ alkyl; the optical isomers and tautomers thereof; and the pharmaceutically acceptable acid addition salts thereof; with the provisos that:
1) when Q is represented by (CH₂)_{2,3} or ₄, then A is not represented by NH-(CH₂)ₒ; and
2) when Q is represented by (CH₂)₂ and R is a C₁₋₄ alkyl; then A is not NH-(CH₂).
The compounds are said to be useful as calcium antagonists and thus to be useful in the treatment of a variety of disease states, for example, cardiac arrhythmias, angina, depression, hypertension, epilepsy and mania.

Rodriquez and Stevens, GB 1183135 disclose 4,5-dihydro-1H-2,4-benzodiazepines of the formula: 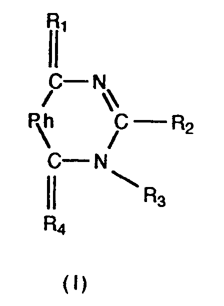
in which Ph represents an optionally substituted 1,2-phenylene group, R₁ and R₄ (which may be the same or different) each represents two hydrogen atoms or two aliphatic groups, or a hydrogen atom together with an aliphatic group, an araliphatic group, an aromatic group, a heterocyclic group of aromatic characteristics or a heterocyclic-aliphatic group, in which the heterocyclic portion has aromatic characteristics; R₂ represents a hydrogen atom, a hydroxy group, a mercapto group, an optionally substituted amino group, an aliphatic group, an araliphatic group, an aromatic group, a heterocyclic group of aromatic characteristics or a heterocyclic-aliphatic group, in which the heterocyclic portion has aromatic characteristics; and R₃ represents a hydrogen atom, an aliphatic group, an araliphatic group, an aromatic group, a heterocyclic group of aromatic characteristics or a heterocyclic-aliphatic group, in which the heterocyclic portion has aromatic characteristics, or the acyl group of a carboxylic acid; N-oxides, quaternary derivatives and salts thereof. The compounds are said to be useful as cardiovascular agents, for example, in the treatment of congestive heart failure; and as coccidiostatic or CNS-stimulating agents. A substantially similar disclosure can be found in Rodriquez and Stevens, DE 11770135.

Zeugner et al., European Patent Application 66303 disclose compounds of the formula 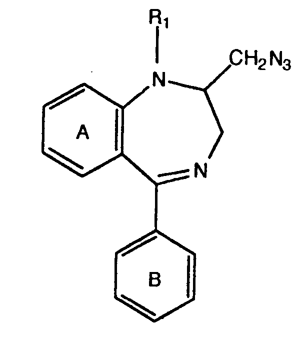
wherein R₁ is H or lower-alkyl; rings A and B have 0-3 substituents selected from halogen, lower-alkyl-thio, lower-alkoxy, lower-alkyl, OH, NO₂ and CF₃, or a methylenedioxy or ethylenedioxy group attached to 2 adjacent C atoms. The compounds are said to be useful as intermediates and to have sedative, broncholytic and antiarrhythmic activity.

Zeugner et al., U.S. Patent 4,325,957, , disclose compounds of the formula: 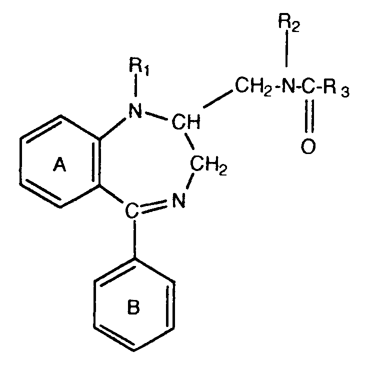
wherein
R₁ represents hydrogen, lower alkyl, lower alkenyl or cyclopropylmethyl,
R₂ represents hydrogen, lower alkyl or lower alkenyl,
R₃ represents a group of the formula a, b, c, or d 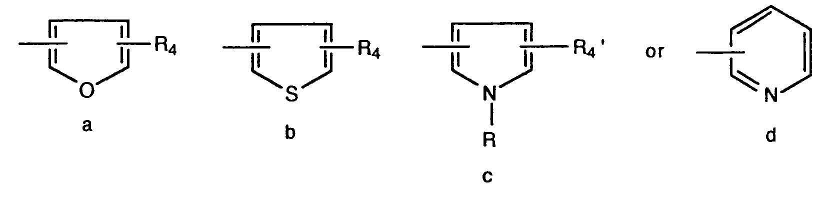
wherein R is hydrogen or C₁-C₃-alkyl, R₄ is hydrogen, lower alkyl, lower alkoxy, nitro or halogen, in particular chlorine or bromine, and R₄' is hydrogen or C₁-C₄-alkyl, and the aromatic groups A and B independently from each other each may be unsubstituted or be substituted by 1 to 3 substituents selected from the group consisting of halogen, lower alkythio, lower alkoxy, lower alkyl, hydroxy, nitro and trifluoromethyl, or be substituted at two adjacent carbon atoms by methylenedioxy or ethylenedioxy, and optical isomers and pharmaceutically-acceptable acid addition salts thereof. The compounds are said to exhibit pscho pharmacological, diuretic, antiarrhythmic and analgesic activities. A substantially similar disclosure can be found in Zeugner et al., U.S. Patent 4,382,030, which is a divisional of U.S. Patent 4,325,957.

EP 0475527 concerns anti-arrhythmic agents of the formula 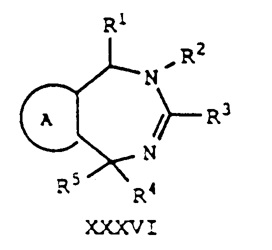
wherein
- A: is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
- R¹: is hydrogen, lower-alkyl, benzyl, naphthyl, thienyl, pyridinyl, phenyl, or phenyl having one or two substituents chosen from the group consisting of lower-alkyl and lower-alkoxy;
- R²: is hydrogen; lower-alkyl; benzyl; phenyl; phenyl substituted with halogen, lower-alkyl or lower-alkoxy; or
- R²: is -CH₂CH₂R⁷ where R⁷ is lower-alkoxy; benzyl; di(lower-alkyl)amino, pyrrolidino; piperidino; morpholino; phenyl; or phenyl substituted with amino, nitro or lower-alkyl sulfonamido;
- R³: is Yₚ-(CH₂)ₘ-Xₙ-R⁸ wherein
- Y: is -NH-, -O-, -S-, 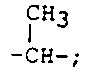
- p: is zero or one:
- m: is an integer from zero to seven;
- X: is -S-, -O-, -SO₂-, 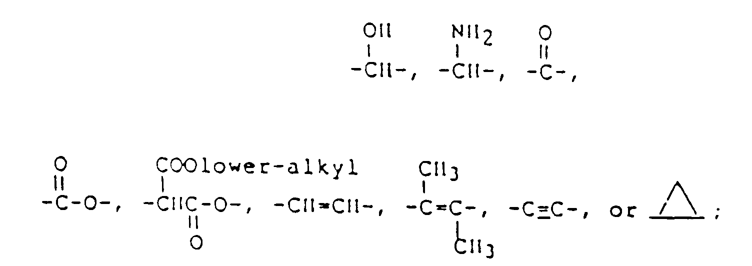
- n: is zero or one: and
- R⁸: is hydrogen:lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino: or with a 5-membered heterocycle containing one or more oxygen atoms; or when n is zero and m is other than zero. R⁸ is additionally halogen; benzyl(lower-alkyl)- amino; di(lower-alkyl)amino: or a 5- or 6- membered heterocycle containing one or two nitrogens, said heterocycle being unsubstituted or substituted with one lower-alkyl group: or X and R⁸ taken together are cyclohexylidine;
- R⁴: is hydrogen; lower-alkyl: allyl: lower-alkoxy-lower-alkyl; acetyl; lower-alkoxy-carbonyl, lower-alkyl-carboxy-alkyl; or a-hydroxy-lower -alkyl; and
- R⁵: is hydrogen: lower-alkyl; naphthyl: thienyl; pyridinyl: benzyl; phenyl; or phenyl having one or two substituents chosen independently from the group consisting of lower-alkyl,lower-alkoxy, halogen, hydroxyl, amino, di(lower-alkyl)amino, lower-alkylsulfonamido and lower-acylamino;
with the proviso that the total number of carbon atoms in R¹ plus R² plus R⁴ plus R⁵ must be five or greater.

This document does not the least suggest the possibility of using the above anti-arrhythmic agents in the treatment of cardiac arrhythmia in patients with impaired ventricular function or congestive heart failure.

According to the present invention there is provided a compound of the formula XXXVI: 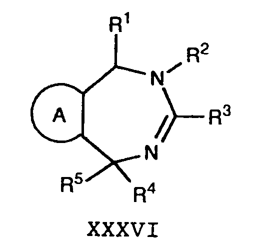
wherein
1) A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
   R¹ is hydrogen, lower-alkyl, benzyl, naphthyl, thienyl, pyridinyl, phenyl, or phenyl having one or two substituents chosen from the group consisting of lower-alkyl and lower-alkoxy;
   R² is -CH₂CH₂R⁷ where R⁷ is pyridinyl;
   R³ is Yₚ-(CH₂)ₘ-Xₙ-R⁸ wherein
   Y is -NH-, -O-, -S-, or 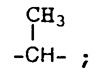 p is zero or one;
   m is an integer from zero to seven;
   X is -S-, -O-, -SO₂-, 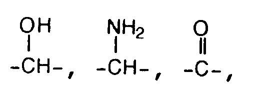 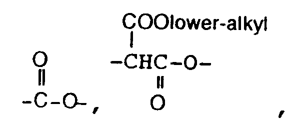 -CH=CH-, 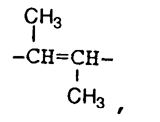 -C≡C-, 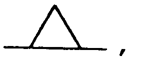 or -NHSO₂-;
   n is zero or one; and
   R⁸ is phenyl having one or two substituents chosen independently from the group consisting of lower-alkylsulfonamido, polyfluorolower-alkylsulfonamido and lower-alkylaminosulfonyl,
   R⁴ is hydrogen; lower-alkyl; allyl; lower-alkoxy-lower-alkyl; acetyl; lower-alkylaceto; lower-alkyl carboxyl; or α-hydroxy-lower-alkyl; and
   R⁵ is hydrogen; lower-alkyl; naphthyl; thienyl; pyridinyl; benzyl; phenyl; or phenyl having one or two substituents chosen independently from the group consisting of lower-alkyl, lower-alkoxy, halogen, hydroxyl, amino, di(lower-alkyl)amino, lower-alkyl-sulfonamido, lower-acylamino, lower-alkylthio, and lower-alkylsulfonyl;
   or an acid-addition salt thereof; with the proviso that the total number of carbon atoms in R¹ plus R² plus R⁴ plus R⁵ must be five or greater; or
2) R² is hydrogen; lower-alkyl; benzyl; phenyl; phenyl substituted with halogen, lower-alkyl or lower-alkoxy; or
   R² is -CH₂CH₂R⁷ where R⁷ is lower-alkoxy; benzyl; di(lower-alkyl)amino, pyrrolidino; piperidino; morpholino; pyridinyl; phenyl; or phenyl substituted with amino, nitro or lower-alkylsulfonamido;
   n is one;
   X is -NHSO₂-; and
   A, R¹, R³, Y, p, m, R⁸, R⁴ and R⁵ are as defined hereinabove in part 1); or
3) R⁸ is phenyl having one or two substituents chosen independently from the group consisting of polyfluoro-loweralkylsulfonamido and lower-alkylaminosulfonyl;
   R² is as defined in part 2) above; and A, R¹, R³, Y, p, m, X, n, R⁴ and R⁵ are defined in part 1) above; or
4) R⁵ is phenyl having one or two substituents chosen independently from the group consisting of lower-alkylthio and lower-alkylsulfonyl;
   R² is as defined in part 2) above; and A, R¹, R³, Y, p, m, X, n, R⁸, and R⁴ are as defined in part 1) above.

The compounds of formula XXXVI are useful as antiarrhythmic agents.

Preferred compounds within part 1) above are those wherein A is phenyl; R¹ is hydrogen or lower-alkyl; R³ is Yₚ-(CH₂)ₘ-Xₙ-R⁸ wherein p is zero, m is an integer from one to three, X is -O-, n is one, and R⁸ is phenyl having one or two lower-alkylsulfonamido substituents; R⁴ is hydrogen or lower-alkyl; and R⁵ is phenyl having one or two halogen substituents. More preferably, R¹ is hydrogen; R³ is Yₚ-(CH₂)ₘ-Xₙ-R⁸ wherein m is one, R⁸ is phenyl having one lower-alkyl-sulfonamido substituent; R⁴ is hydrogen; and R⁵ is phenyl having one halogen substituent. The compound 4,5-dihydro-3-[(4-methanesulfonamidophenoxy)methyl]-4-(4-pyridinylethyl)-1-(4-chlorophenyl)-1H-2,4-benzodiazepine is particularly preferred.

Preferred compounds within part 3) above are those wherein A is phenyl; R¹ is hydrogen or lower-alkyl; R² is hydrogen or lower-alkyl; R³ is Yₚ-(CH₂)ₘ-Xₙ-R⁸ wherein p is zero, m is an integer from one to three, n is zero, and R⁸ is phenyl having one polyfluorolower-alkylsulfonamido or lower-alkylaminosulfonyl substituent; R⁴ is hydrogen or lower-alkyl; and R⁵ is phenyl. Particularly preferred compounds are those wherein R¹ is hydrogen; R² is lower-alkyl; R³ is Yₚ-(CH₂)ₘ-Xₙ-R⁸ wherein m is 2; and R⁴ is hydrogen.

Preferred compounds within part 4) above are those wherein A is phenyl; R¹ is hydrogen or lower-alkyl; R² is hydrogen or lower-alkyl; R³ is Yₚ-(CH₂)ₘ-Xₙ-R⁸ wherein p is zero, m is an integer from one to three, X is -O-, n is zero, and R⁸ is phenyl having one lower-alkylsulfonamido substituent; and R⁴ is hydrogen or lower-alkyl. More preferably, R¹ is hydrogen; R² is lower-alkyl; R³ is Yₚ-(CH₂)ₘ-Xₙ-R⁸ wherein m is one; and R⁴ is hydrogen. Particularly preferred are 4,5-dihydro-3-{(4-methanesulfonamidophenoxy)methyl]-4-methyl-1-(4-methylthiophenyl)-1H-2,4-benzodiazepine or 4,5-dihydro-3-{(4-methanesulfonamidophenoxy)methyl]-4-methyl-1-(4-methylsulfonylphenyl)-1H-2,4-benzodiazepine

Lower-alkyl as used herein describes linear, branched, or cyclic saturated carbon chains of eight or fewer carbon atoms; lower-alkoxy as used herein describes linear or branched alkyloxy substituents containing eight or fewer carbon atoms; halogen describes bromine, chlorine or fluorine.

In the text that follows, the substituents R are defined when initially presented and maintain that definition whenever they occur subsequently.

The invention further comprises methods of using the benzodiazepines of the invention for the treatment of cardiac arrhythmia in patients and compositions for the treatment of cardiac arrhythmia containing those compounds.

It has been further found that certain compounds (illustrated hereinbelow) do not reduce (depress) cardiac function and they are thus particularly useful in the treatment of cardiac arrhythmia in a patient with impaired ventricular function or congestive heart failure as is described more fully hereinbelow in the Biological Test Result section. Accordingly, the invention further comprises the use of the compounds disclosed hereinbelow for the preparation of a medicament for the treatment of cardiac arrhythmia in a patient with impaired ventricular function or congestive heart failure which comprises the administration of an antiarrhythmically effective amount of a compound of the formula: 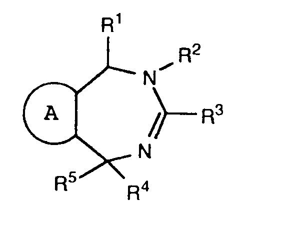
wherein:
A is a ring chosen from the group consisting of phenyl, thienyl and phenyl substituted at any available carbon atom by one or two lower-alkylsulfonamido groups;
R¹ is hydrogen or phenyl;
R² is lower-alkyl or -CH₂CH₂R^{7'} wherein R^{7'} is lower-alxoxy, pyridinyl, phenyl or phenyl having one or two lower-alkylsulfonamido substituents;
R³ is - (CH₂)ₘ-Xₙ-R⁸ wherein
m is an integer from zero to seven;
X is -S-, -O-, -SO₂-, -CH=CH-, or -NHSO₂-;
n is zero or one;
R⁸ is having one or two substituents chosen independently from the group consisting of lower-alkylsulfonamido, and polyfluorolower-alkylsulfonamido;
R⁴ is hydrogen or lower-alkyl; and
R⁵ is hydrogen, pyridinyl, benzyl, phenyl or phenyl having one or two substituents chosen independently from the group consisting of halogen, lower-alkyl, lower-alkoxy, lower-alkylthio, lower-alkylsulfonyl, lower-alkylsulfonamido, and hydroxy;
or an acid-addition salt thereof; with the proviso that the total number of carbon atoms in R¹ plus R² plus R⁴ plus R⁵ must be five or greater; further provided that at least one of R², R³, R⁵ or ring A must contain a pyridinyl group, an -NHSO₂- group, or a lower-alkylsulfonamido, dilower-alkylaminosulfonyl, or polyfluorolower-alkylsulfonamido substituent.

The invention further provides a composition for the treatment of cardiac arrhythmia in a patient with impaired ventricular function or congestive heart failure which comprises an antiarrhythmically effective amount of a compound of the formula: 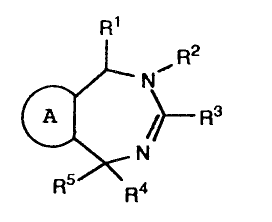
wherein: A, R¹, R², R^{7'}, R³, m, X, n, R⁸, R⁴ and R^{5,} and the provisos in connection therewith, are as defined with the reduced scope for the use of the compounds for the preparation of a medicament for the treatment of cardiac arrhythmia in a patient with impaired ventricular function or congestive heart failure as last mentioned,
or an acid-addition salt thereof; together with a pharmaceutically acceptable vehicle, adjuvant or excipient.

Preferred compounds within this reduced scope are those wherein A is a phenyl group, optionally substituted with one or two lower-alkylsulfonamido groups substituted at any available phenyl ring carbon atom, preferably unsubstituted or substituted with one lower-alkyl-sulfonamido group, especially when R² is lower-alkyl or CH₂CH₂R^{7'}, wherein R^{7'} is lower-alkoxy, pyridinyl, or phenyl having one lower-alkylsulfonamido substituent; R³ is -(CH₂)ₘ-Xₙ-R⁸ wherein m is an integer from zero to three; and R⁸ is phenyl having one or two substituents chosen independently from the group consisting of lower-alkylsulfonamido, and trifluoromethylsulfonamido;.

More preferably R² is methyl or CH₂CH₂R^{7'} wherein R^{7'} is methoxy, 4-pyridinyl, or phenyl having one methylsulfonamido substituent; R⁸ is phenyl having one or two substituents chosen independently from the group consisting of lower-alkylsulfonamido, and trifluoromethylsulfonamido; R⁴ is hydrogen or methyl; and R⁵ is hydrogen, 4-pyridinyl, benzyl, phenyl or phenyl having one or two substituents chosen independently from the group consisting of halogen, lower-alkyl, lower-alkoxy, lower-alkylthio, lower-alkylsulfonyl, lower-alkylsulfonamido, and hydroxy; and R⁶ is hydrogen or a 6- or 8-methylsulfonamido group. Paricularly preferred compounds are
4,5-dihydro-3[(4-methanesulfonamidophenoxy)methyl]-4-methyl-1-phenyl-1H-2,4-benzodiazepine; especially the (+)- isomer thereof;
4,5-dihydro-3-[(4-methanesulfonamidophenoxy)methyl]-4-methyl-1-(4-chlorophenyl)-1H-2,4-benzodiazepine;
4,5-dihydro-3-[(4-methanesulfonamidophenoxy)methyl]-4-methyl-1-(4-methoxyphenyl)-1H-2,4-benzodiazepine;
4,5-dihydro-3[(4-methanesulfonamidophenylsulfonyl)methyl]-4-methyl-1-(4-chlorophenyl)-1H-2,4-benzodiazepine;
4,5-dihydro-3-[(4-methanesulfonamidophenoxy)methyl]-4-methyl-1-(4-methylthiophenyl)-1H-2,4-benzodiazepine;
4,5-dihydro-3-[2-(4-methanesulfonamidophenyl)ethyl]-4-methyl-1-(2,4-difluorophenyl)-1H-2,4-benzodiazepine;
Further preferred compounds within this reduced scope are those wherein A is a thienyl group, in particular wherein R¹ is hydrogen; R² is lower-alkyl; R³ is -(CH₂)ₘ-Xₙ-R⁸ wherein m is one or two; n is zero; R⁸ is phenyl having one lower-alkylsulfonamido substituent; R⁴ is hydrogen; and R⁵ is phenyl. More preferably R² is methyl and R⁸ is 4-methyl-sulfonamidophenyl. Paricularly preferred compounds are 4,5-dihydro-4-methyl-1-phenyl-3-[(4-methanesulfonamidophenoxy)methyl]-1H thieno[2,3-e]-2,4-diazepine and 4,5-dihydro-4-methyl-1-phenyl-3-[2-(4-methanesulfonamidophenyl)ethyl]-1H thieno[2,3-e]-2,4-diazepine.

A general synthesis of compounds of the invention sharing general formula XXXVI may be outlined as shown in Scheme A. 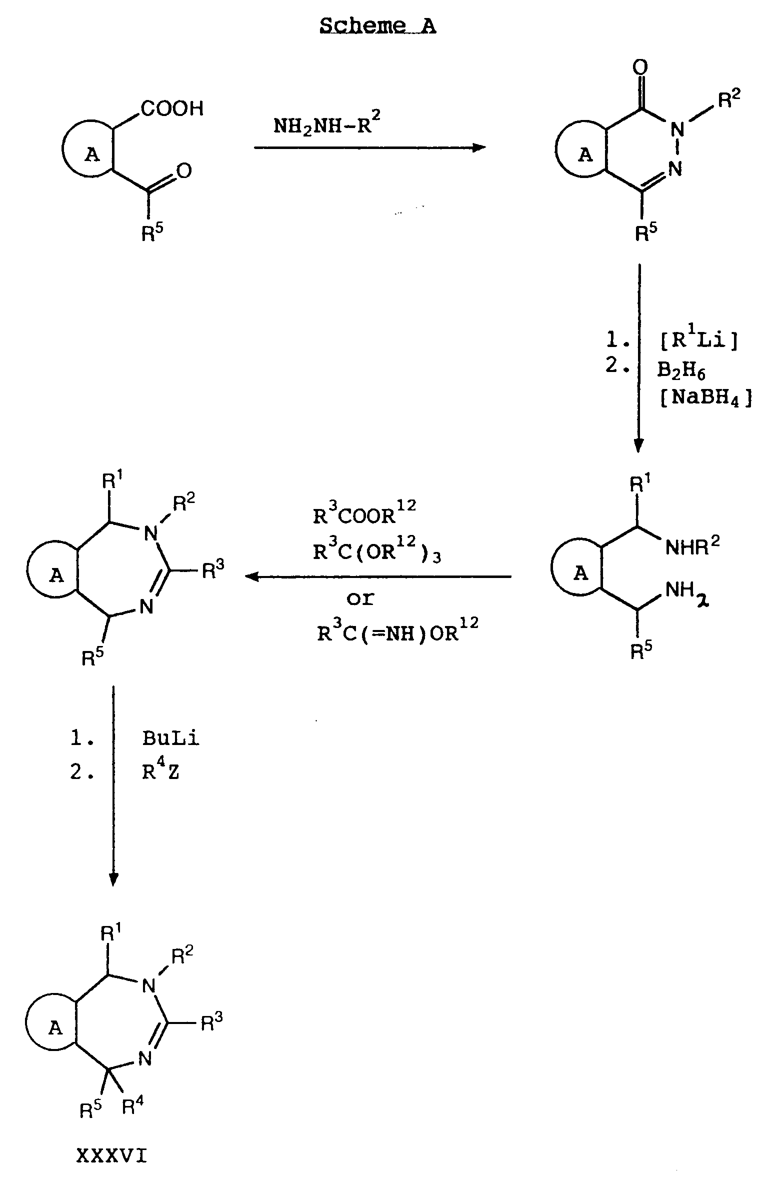
This is more particularly illustrated in Scheme B wherein A is phenyl or R⁶-substituted phenyl, wherein R⁶ is one or two substituents chosen from the group consisting of hydrogen, amino, lower-alkyl, lower-alkoxy, alkoxy, halogen, nitro, and lower-alkylsulfonamido, more preferably R₆ being hydrogen or one or two lower-alkylsulfonamido groups, giving an end-product of formula I : 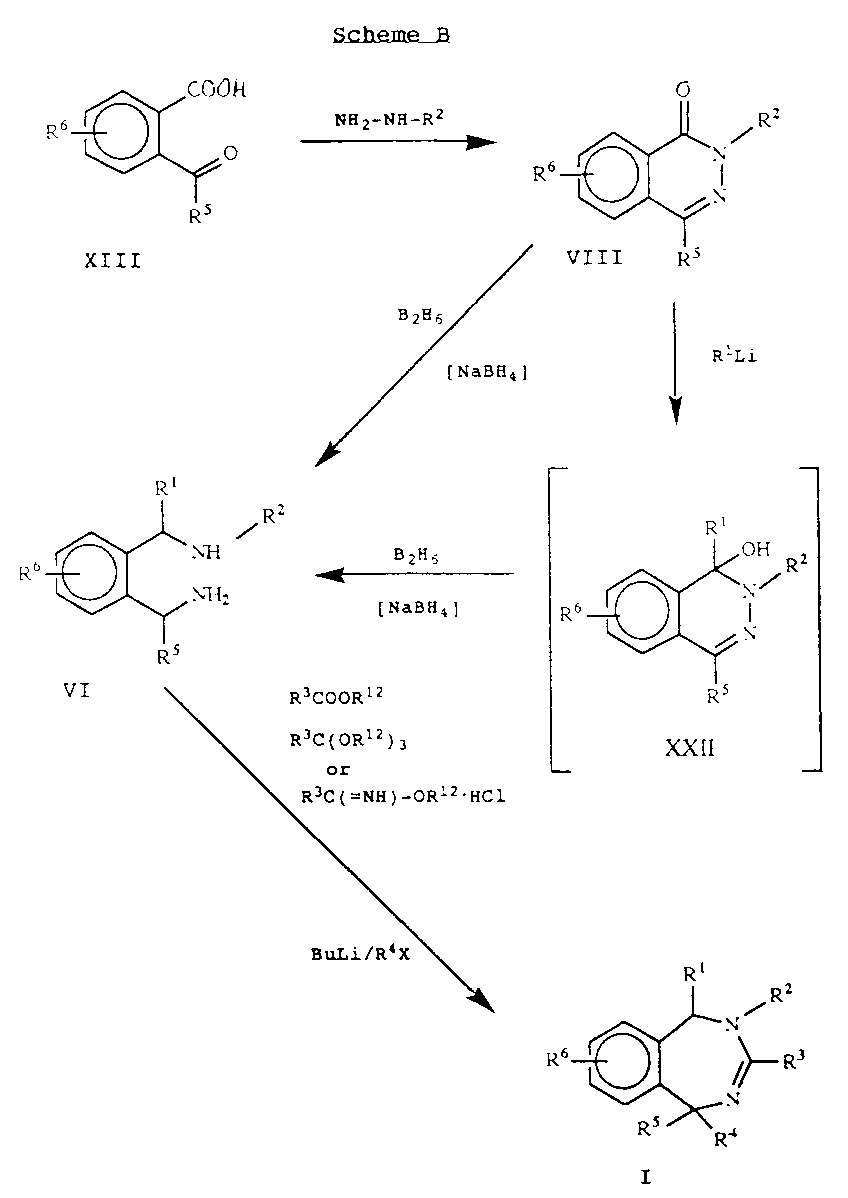
A suitably substituted γ-oxo-acid of formula XIII is reacted with a suitably substituted hydrazine to form a phthalazinone (VIII). In the case where it is desired that R¹ be other than hydrogen, the phthalazinone is reacted with a slight excess of a suitable alkyl or aryllithium compound in an inert solvent, preferably THF, at -78° to 0°C, preferably about -65°C, and the resulting adduct is reduced as described below without isolation. In the case where R¹ is hydrogen, the phthalazinone (VIII) is reduced directly to the diamine (VI) with 3.5 to 9.0 equivalents of diborane in an inert solvent, preferably THF, at 20° to 100°, preferably 67°C. A catalytic amount of sodium borohydride and some diglyme may be added.

The diamine (VI) may be condensed in one of three ways to produce the benzodiazepine (I, R₄=H):
(1) the free base of the diamine in acetic acid is treated with five to seven equivalents of the appropriate orthoester R³C(OR¹²)₃ at 0-50°C, preferably 25°C, or the diacid salt of the diamine, preferably the dihydrochloride salt, in an inert solvent is treated with one to seven equivalents of an appropriate orthoester plus one to two equivalents of a weak base, preferably sodium or potassium acetate;
(2) a diacid salt of the diamine (VI), preferably the dihydrochloride salt, in an inert solvent, preferably methanol, is treated with two to three equivalents of the appropriate iminoether hydrochloride and about two equivalents of a weak base, preferably sodium acetate, at 0 to 60°C, preferably 25°C, or the free base of the diamine (VI) in an inert solvent, preferably methanol, is treated with two to three equivalents of the appropriate iminoether hydrochloride and two to three equivalents of a weak acid, preferably acetic acid, at 0°to 60°C, preferably 25°C, or
(3) a diamine or a diacid salt of the diamine, preferably the dihydrochloride salt, in an inert solvent, preferably toluene or sulfolane or a mixture thereof, is treated with slightly more than two equivalents of trimethylaluminum to 4.5 equivalents of trimethylaluminum at -30°C up to the boiling point of the solvent used, preferably at -30° to +110°C, or with 2.5-3.5 equivalents of triisobutylaluminum at a temperature in the range of about room temperature up to the boiling point of the solvent used, followed by treatment with 1 to 1.5 equivalents of a lower-alkyl ester of the appropriate acid (R³COOR¹²); wherein R¹² given above is methyl or ethyl.

In the case where it is desired that R⁴ be other than hydrogen, the diazepine (I, R₄=H) may be reacted with a strong base such as butyllithium and the resulting anion reacted with an appropriate electrophile.

It will, of course, be appreciated that all of the reactions described for the compounds of formula XXXVI wherein A is a phenyl ring are equally applicable to the compounds wherein A is other than phenyl. The starting materials are likewise commercially available or known in the art.

Compounds of formula V, a subset of benzodiazepines of formula I, may be prepared by the ring closure of aminoamides. A monosalt of the aminoamide XXVII or XXVIII, preferably the monohydrochloride in an inert solvent, preferably toluene, is treated with a slight excess, preferably about 1.1 equivalents, of trimethylaluminum at 0° to 150°, preferably about 110°, to produce a benzodiazepine of formula V, wherein
R^{3a} is (Y^{a})ₚ-(CH₂)ₘ-(X^{a})ₙ-R⁸ wherein
Y^{a} is -O-, -S-, or 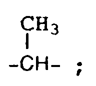
and
X^{a} is -S-, -O-, -SO₂- or -CH=CH-; and
R^{5b} is hydrogen; lower-alkyl; naphthyl; thienyl; pyridinyl; benzyl; phenyl; or phenyl having one or two substituents chosen independently from the group consisting of lower-alkyl, lower-alkoxy and halogen. 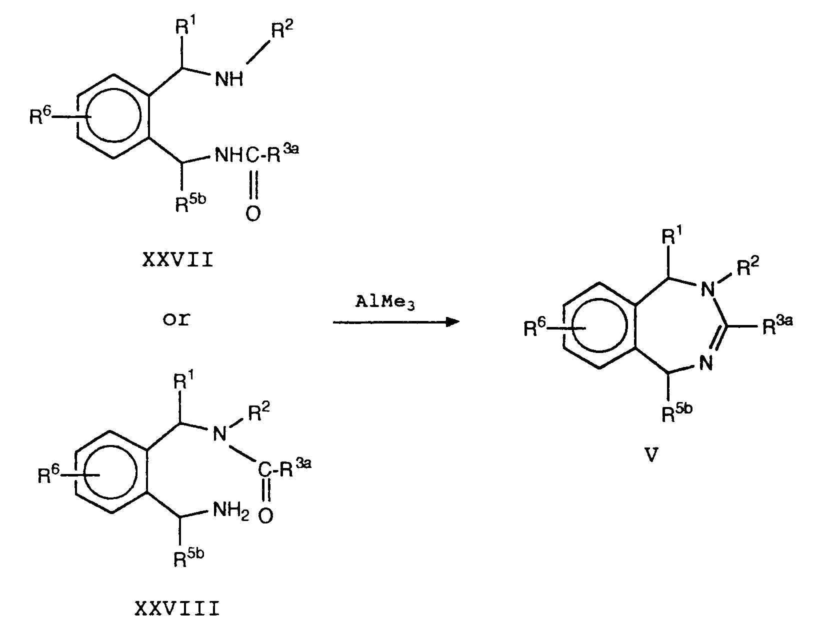
Aminoamides of formula XXVII may be obtained as described in Examples 177-184 by incomplete cyclization or, as described in General Method U, by hydrolytic cleavage of benzodiazepines. Aminoamides XXVII or XXVIII or mixtures of the two may also be obtained by procedures well known in the art for condensing acids of formula R^{3a}COOH with amines of formula VId. 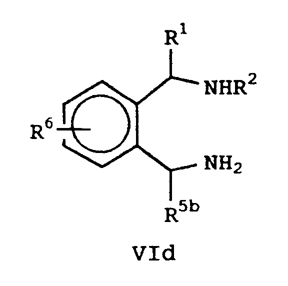
In the case of compounds of formula I where p (in R³) is one and Y^{b} is -NH-, -S-, or -O-, the compounds may be made by an alternate route from the diamine VI shown in Scheme C: 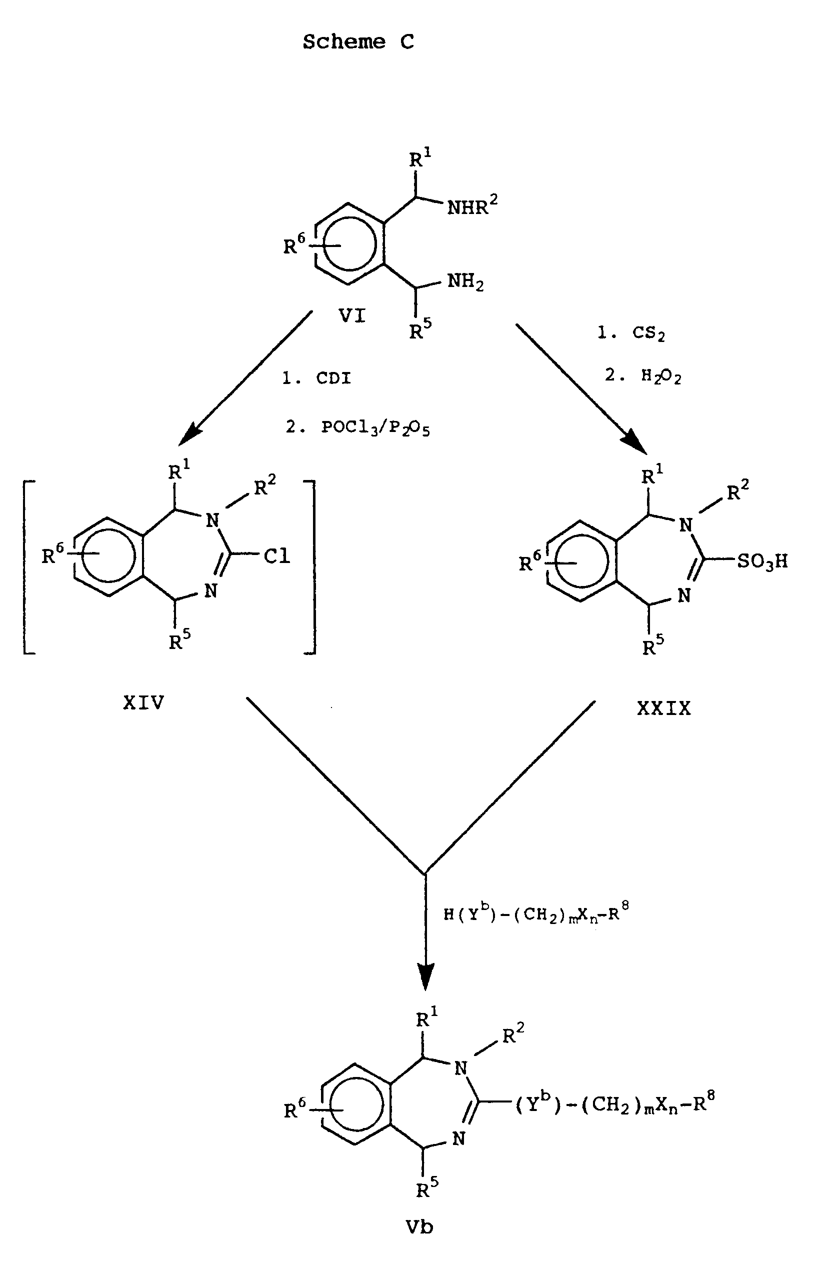
The diamine (VI) is reacted with carbonyl diimidazole (CDI) in an inert solvent, preferably chloroform, at ambient temperature to produce a benzodiazepin-3-one, which is reacted with a large excess, preferably about 13 equivalents, of phosphorus oxychloride and preferably about 0.25 equivalents of phosphorus pentoxide to produce a 3-chlorobenzodiazepine (XIV). The 3-chlorobenzodiazepine is then reacted, usually without isolation, with the appropriate nucleophile, R⁸Xₙ(CH₂)ₘ(Y^{b})H, to produce the benzodiazepines of structure Vb.

Alternatively the diamine VI is reacted with preferably about one equivalent of carbon disulfide in an inert solvent, preferably 2-propanol, at 0°to 100°C, preferably at about 20° to 85°C and the resulting carbamodithioic acid is treated with a catalytic amount of an acid, preferably hydrochloric acid, in an inert solvent, preferably ethanol, at 0 to 100°C, preferably at about 78°C, to produce a tetrahydrobenzodiazepine-3-thione. The thione is oxidized with slightly more than three equivalents of 30% hydrogen peroxide according to the procedure of Maryanoff et al. [J. Org. Chem. 51, 1882 (1986)] to produce the sulfonic acid XXIX. The sulfonate may then be displaced by an appropriate nucleophile, as before, optionally in an inert solvent at 0°-100°C. Example 153 illustrates an alternative, lower-yield conversion of the thione to the compounds of formula Vb.

In the case where all of R², R⁴ and R⁵ are other than hydrogen, the subgenus of compounds of formula VII wherein R³ is attached to the benzodiazepine ring through a carbon (i.e. p in R³ is zero and R³ is -CH₂(CH₂)ₘ₋₁XₙR⁸ or p is one and Y is 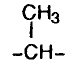
may be made alternately by treatment of a compound of formula VIIa wherein R¹⁴ is hydrogen or methyl in an inert solvent, preferably THF, at -78° to +25°, with a slight excess, preferably about 10 to 20%, of a strong base, preferably n-butyllithium followed by a slight excess, preferably 10-50%, of an appropriate electrophile. The electrophile can be of the form R⁸-(X^{a})ₙ-(CH₂)ₘ₋₁-Z wherein Z is a group that is readily displaced by an anion, such as a halogen, sulfide, sulfonate, ester, etc. or in the case where m-1 is zero it can take the form of an aldehyde, ketone or imine so that addition of the anion to the electrophile followed by quenching with a proton source results in the overall addition of the elements of VIIa to the electrophile. 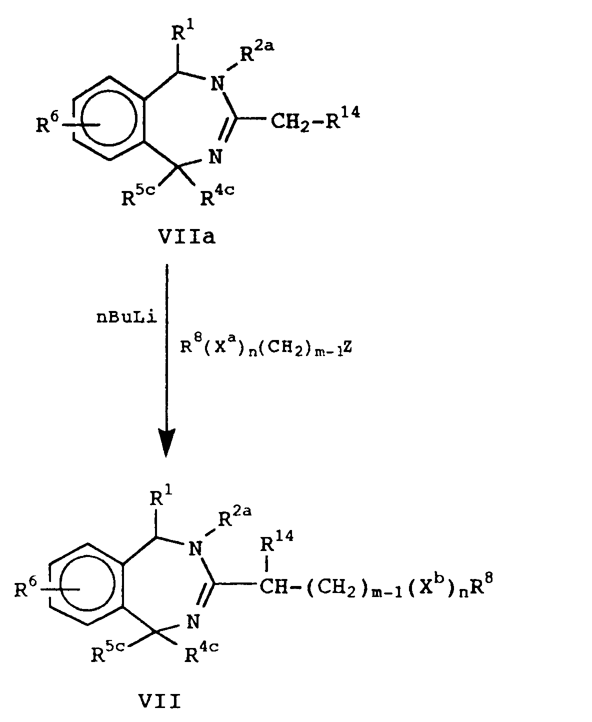
In the formulae above,
R^{2a} is lower-alkyl; benzyl; phenyl or phenyl substituted with halogen, lower-alkyl or lower-alkoxy;
R^{4c} is lower-alkyl; allyl or lower-alkoxy-lower-alkyl;
R^{5c} is phenyl or phenyl having one or two substituents chosen independently from the group consisting of lower-alkyl, lower-alkoxy and halogen, and
X^{b} is -S-, -O-, -SO₂-, 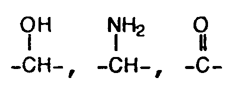
or -CH=CH-.

In cases where R³ is attached to the benzodiazepine via a heteroatom-methylene link (XV), that is R³ is of the form -CH₂(X^{c})ₙR⁸ wherein X^{c} is -S-, -O- or -SO₂- (i.e. in formula XXXVI, X is -S-, -O- or -SO₂-, p is zero, m is one and n is one when R⁸ is lower-alkyl, phenyl or substituted phenyl or n is zero when R⁸ is amino or an N-attached heterocycle), the compounds may conveniently be synthesized from the corresponding chloromethyl species (XVI). 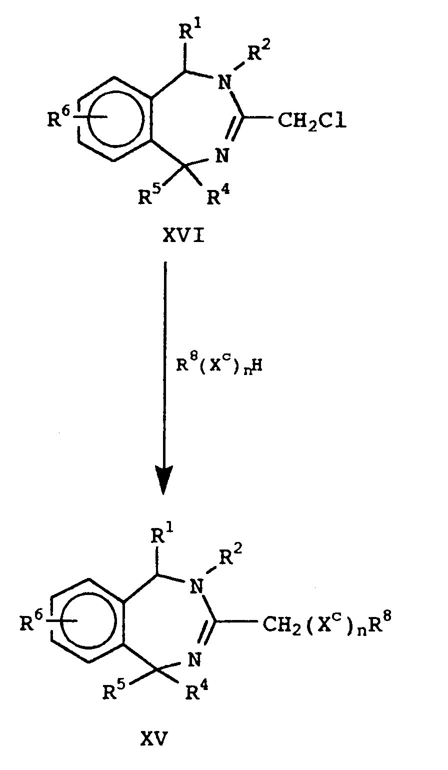
The 3-chloromethyl-2,4-benzodiazepine (XVI) in an inert solvent, preferably chloroform when the heteroatom is nitrogen and methanol or acetonitrile when the heteroatom is sulfur, is treated with one to three equivalents of the R⁸(X^{c})ₙH species at 0° to 100°C, preferably at 25° to 65°C. The chloromethyl benzodiazepine (XVI) can be synthesized directly from the diamine VI by condensation with ethyl 2-chloroethanimidate or in the case where all of R², R⁴ and R⁵ are other than hydrogen, the chloromethyl benzodiazepine (XVI) may be synthesized from the corresponding 3-methylbenzodiazepine by anion formation as described in the foregoing paragraph and quenching with about 1.1 equivalents of hexachloroethane.

The γ-oxo-acids of formula XIII, the phthalazinones of formula VIII, the diamines of formula VI, aminoamides of formula XXVII, aminoamides of formula XXVIII, amines of formula VI d, and phthalazines of formula XXII are commercially available, or they are known (see e.g. Johnson and Schlegel, in published European Patent Application 475527) and thus can be prepared by procedures known in the art, or they can be prepared as described hereinbelow.

Compounds of formula VI may be prepared by the reduction and cleavage of phthalazinones and phthalazines as shown in Scheme F. 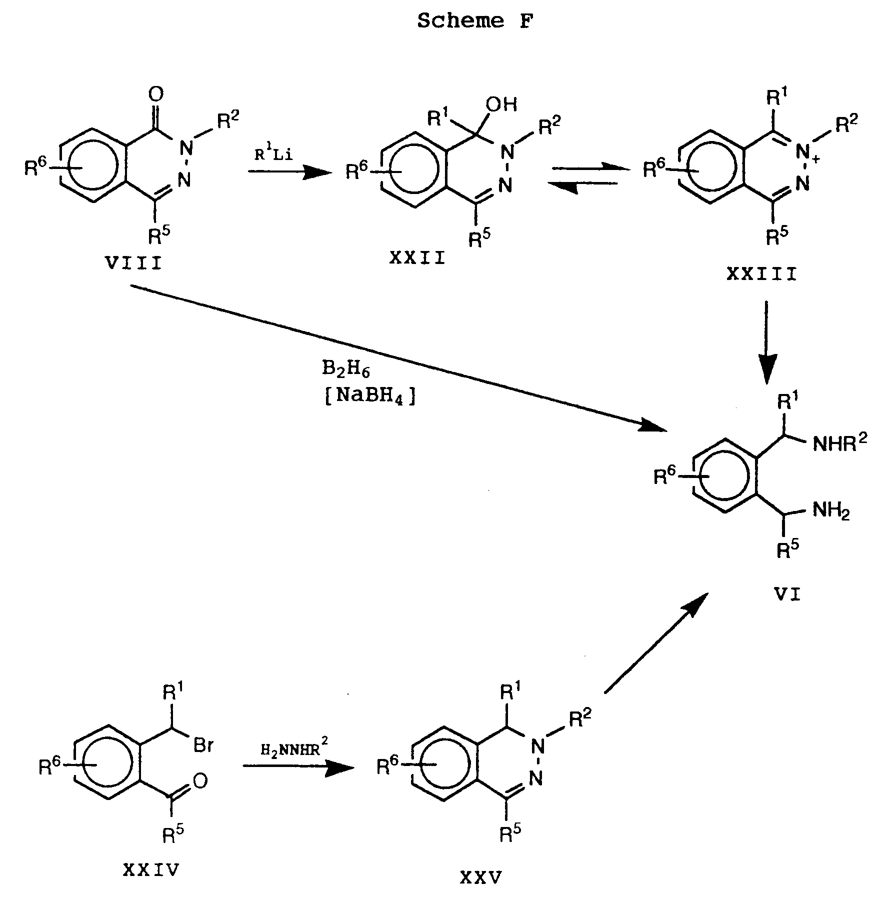
A phthalazinone of formula VIII is reacted with 3.5 to 9.0 equivalents of diborane in an inert solvent, preferably THF, at 20° to 100°, preferably about 67°, to produce diamines of the formula VI wherein R¹ is hydrogen. A catalytic amount of sodium borohydride in diglyme may be added. If it is desired that R¹ be other than hydrogen, phthalazines of formulae XXII, XXIII and XXV may be reduced in like fashion. The phthalazines XXII and XXIII may be obtained from the corresponding phthalzinones by reaction with a suitable alkyllithium or aryllithium in an inert solvent, preferably THF, at -78° to 0°, preferably about -65°. The resulting phthalazine may exist as the hydroxyphthalazine XXII or may spontaneously eliminate the elements of water to form the phthalazinium species XXIII. The phthalazines of formula XXV may be synthesized by the condensation of the appropriate hydrazine with a γ-haloketone, preferably a γ-bromoketone.

The diamines of the formula VI wherein R¹ is hydrogen, can also be prepared by
(a) the treatment of an aminoamide of the formula XXVII, wherein R^{5b}=R⁵, with an excess of an appropriate acid, such as sulfuric acid or hydrochloric acid, or a mixture of said acids, at a temperature of about room temperature up to the boiling point of the acid or acid mixture; or
(b) the treatment of a benzodiazepine of formula I, wherein R⁴=hydrogen, with an excess of ethylenediamine, in an inert solvent, such as toluene, or neat, at a temperature in the range of about room temperature up to the boiling point of the solvent used.

In the case wherein in formula VIII R² is hydrogen, the simple reduction with diborane described above is so slow as to be of less practical use than a three-step reduction, wherein in the formulae
R^{6a} is one or two substituents chosen from the group consisting of hydrogen, lower-alkyl, lower-alkoxy and halogen: 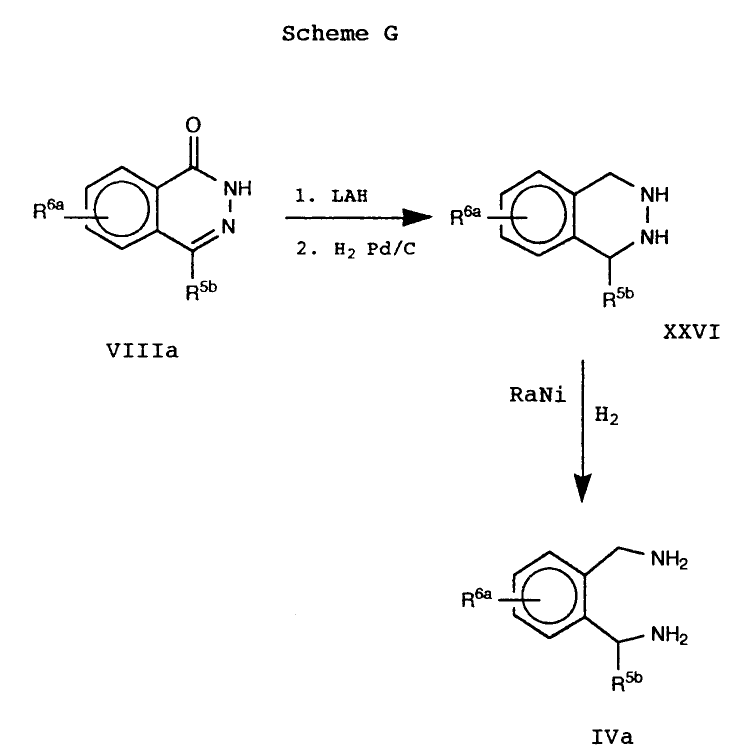
The phthalazinone VIIIa is treated with about two equivalents of an aluminum hydride reducing agent, preferably lithium aluminum hydride, in an inert solvent, preferably THF, at 20° to 120°, preferably about 65°. The resulting dihydrophthalazine is reacted with hydrogen in an inert solvent, preferably a lower alkanol, most preferably ethanol, in the presence of a palladium catalyst at 20° to 60°, preferably 40°-50°, preferably at about 3 atmospheres pressure. The resulting tetrahydrophthalazine (XXVI) is reacted with hydrogen in an inert solvent, preferably methanol, in the presence of a Raney nickel catalyst at 20° to 80°, preferably about 65°, preferably at about 3 atmospheres pressure.

It will be noted that many of the compounds of the invention are asymmetric at C-1 of the benzodiazepine or benzodiazocine. In some cases there may be an advantage to using one or the other enantiomer for the treatment of arrhythmia. Single enantiomers may be synthesized from chiral starting materials or the racemates may be resolved by methods well known in the art, such as chromatography on chiral media or recrystallization of diastereomeric salts.

Simple chemical transformations which are conventional and well known to those skilled in the art of chemistry can be used for effecting change in functional groups in the compounds of the invention. For example, catalytic reduction of arylnitro compounds to afford the corresponding arylamines; dealkylation of aryl ethers to afford the corresponding phenols; and treatment of arylamines with lower-alkylsulfonyl halides to afford the corresponding sulfonamides.

The compounds of the invention are useful both in the free base form and the form of acid-addition salts, and both forms are within the purview of the invention. The acid-addition salts are in some cases a more convenient form for use, and in practice the use of the salt form inherently amounts to the use of the base form. The acids which can be used to prepare the acid-addition salts include preferably those which produce, when combined with the free base, medicinally acceptable salts, that is, salts whose anions are relatively innocuous to the animal organism in medicinal doses of the salts so that the beneficial properties inherent in the free base are not vitiated by side effects ascribable to the anions. In practising the present invention, it is convenient to form the hydrochloride, fumarate, toluenesulfonate, hydrogen sulfate, methanesulfonate, or maleate salts. However, other appropriate medicinally acceptable salts within the scope of the invention are those derived from other mineral acids and organic acids. The acid-addition salts of the basic compounds are prepared either by dissolving the free base in aqueous alcohol solution containing the appropriate acid and isolating the salt by evaporating the solution, or by reacting the free base and an acid in an organic solvent, in which case the salt separates directly, is precipitated with a second organic solvent, or can be obtained by concentration of the solution. Although medicinally acceptable salts of the basic compounds are preferred, all acid-addition salts are within the scope of the present invention. All acid-addition salts are useful as sources of the free base form even if the particular salt per se is desired only as an intermediate product, as, for example, when the salt is formed only for purposes of purification or identification, or when it is used as an intermediate in preparing a medicinally acceptable salt by ion exchange procedures.

The structures of the compounds of the invention were established by the mode of synthesis, by elemental analysis, and by infrared, nuclear magnetic resonance, and mass spectroscopy. The course of the reactions and the identity and homogeneity of the products were assessed by thin layer chromatography (TLC) and high-pressure liquid chromatography (HPLC). The starting materials are either commercially available or may be prepared by procedures well known in the art.

In the following procedures, melting points are given in degrees C and are uncorrected.

In the examples which follow, Me is methyl, Et is ethyl, Ph is phenyl, Bzl is benzyl, iPr is isopropyl, tBu is t-butyl, OAc is acetyl, THF is tetrahydrofuran, hex is hexane, IPA is isopropylamine, DMF is dimethylformamide, and TMS is trimethylsilyl.

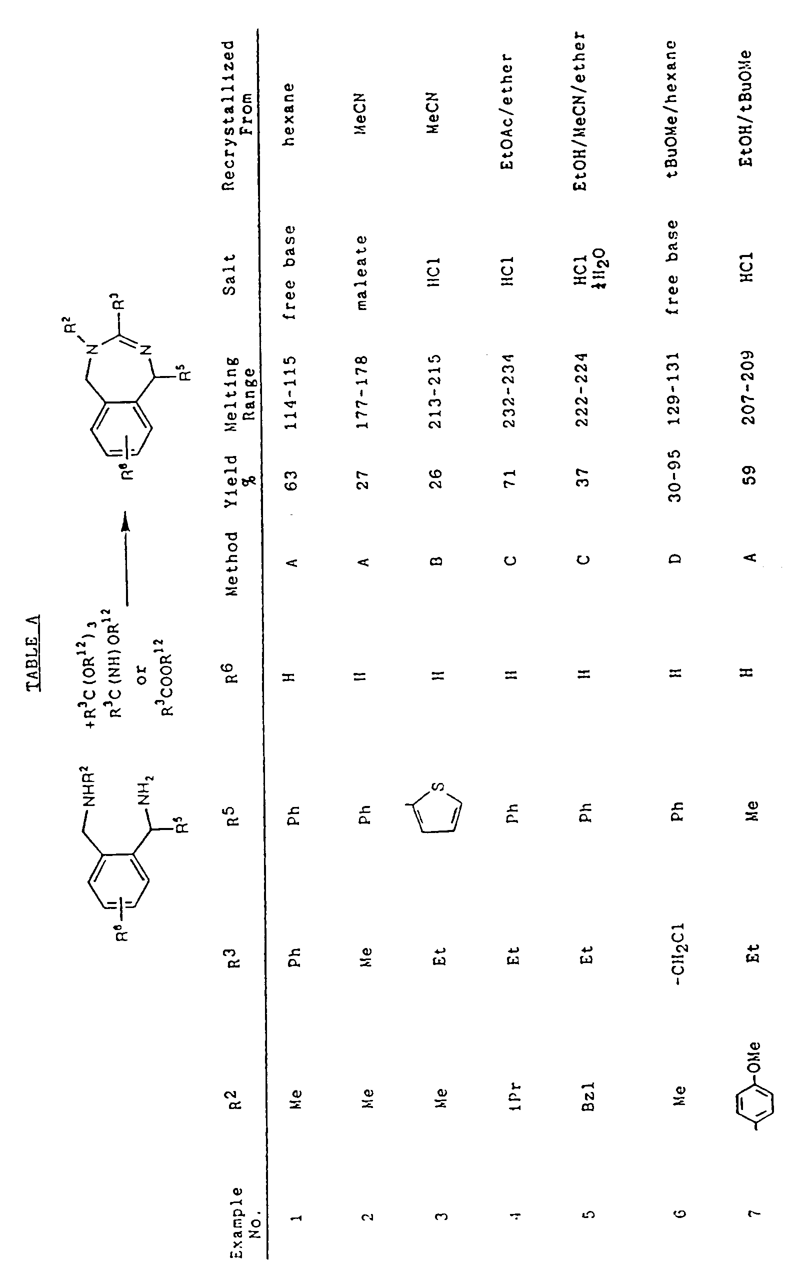

In the following specification compounds of examples 1 to 160, 167 to 204, 207 to 264, 266 to 279, 287 and 289 are identical to those described in EP 475527.

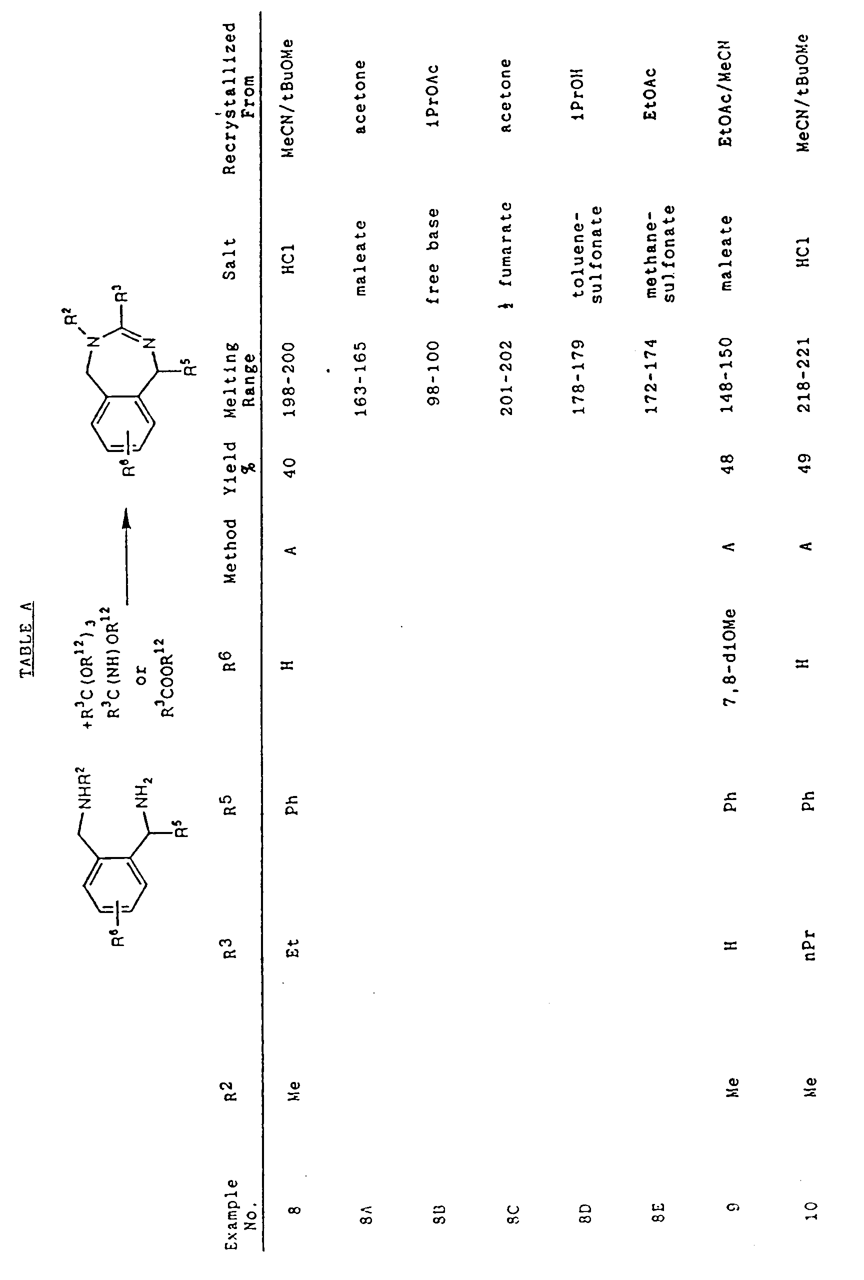

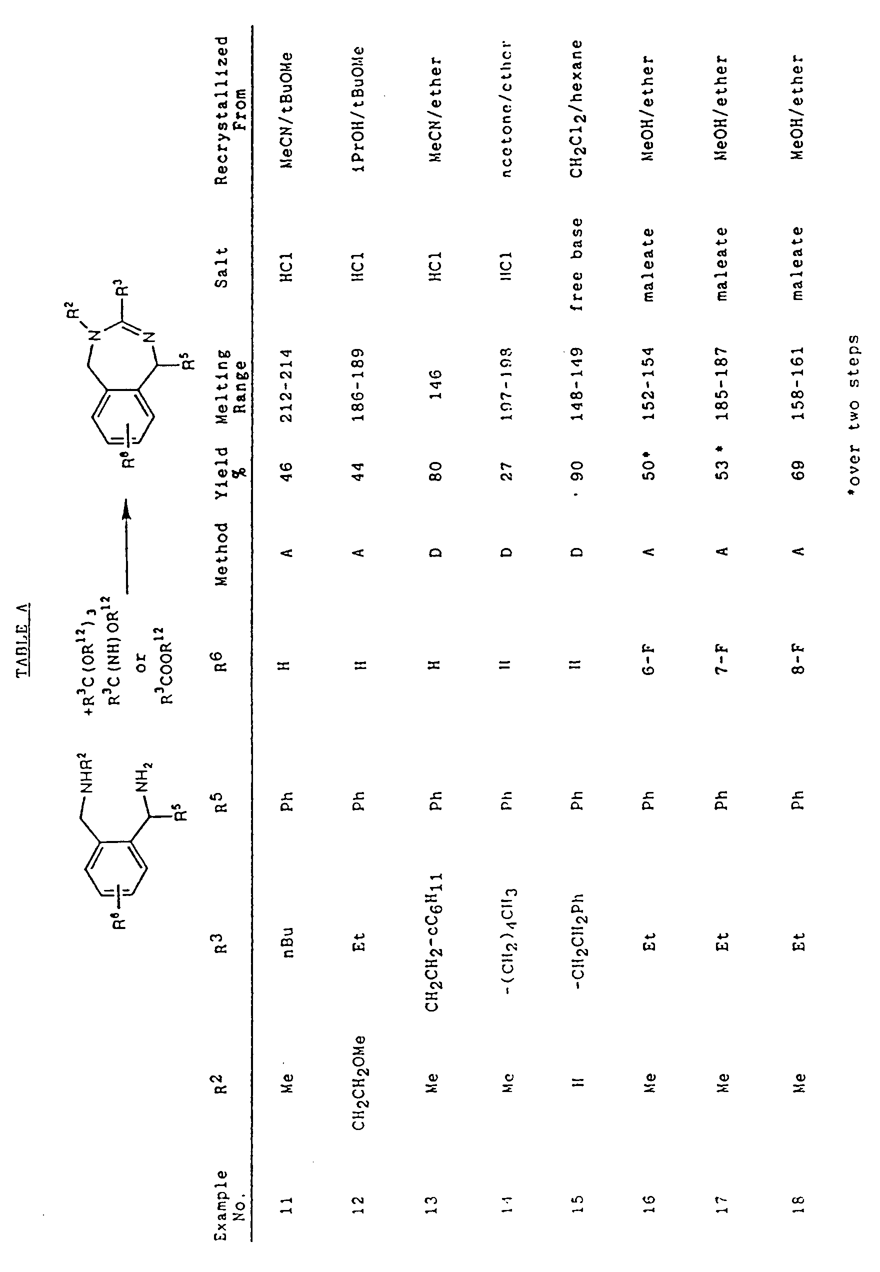

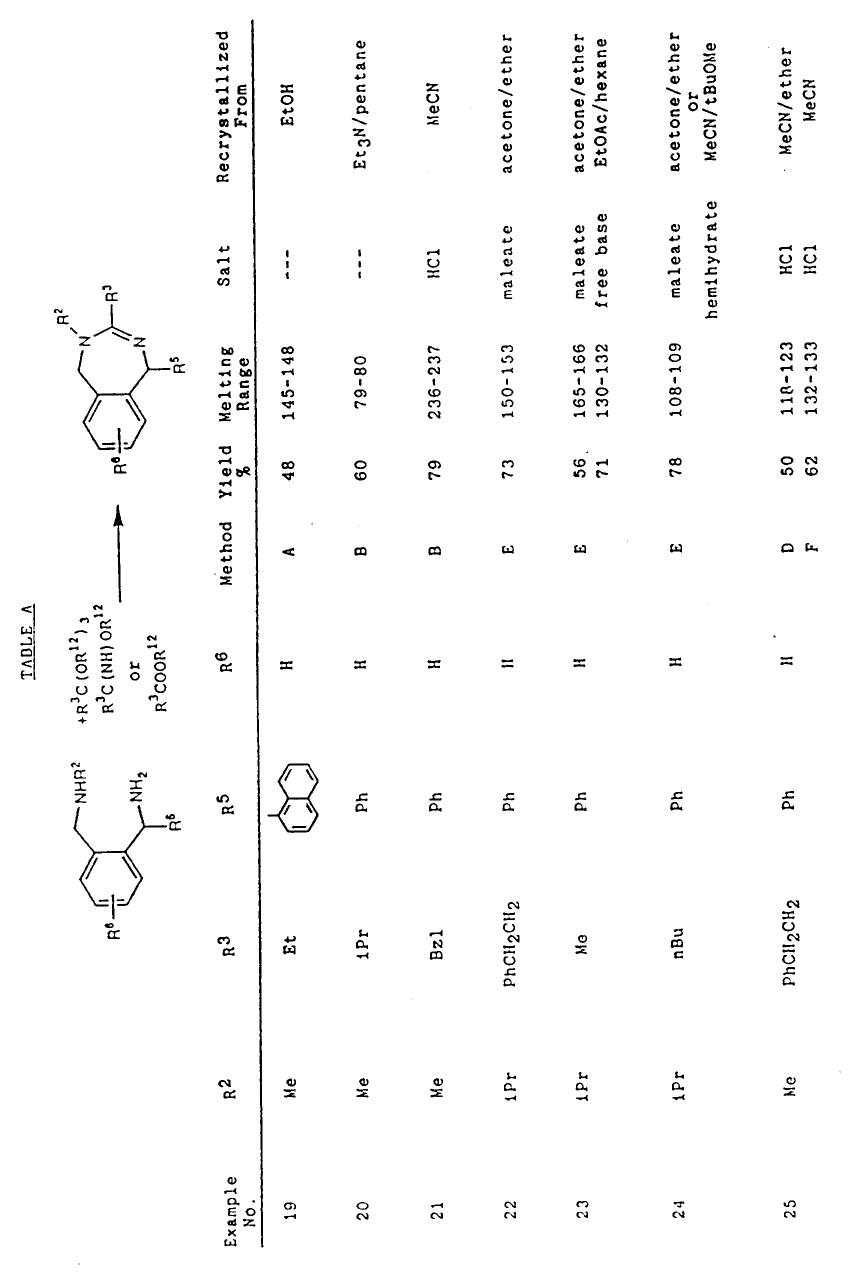

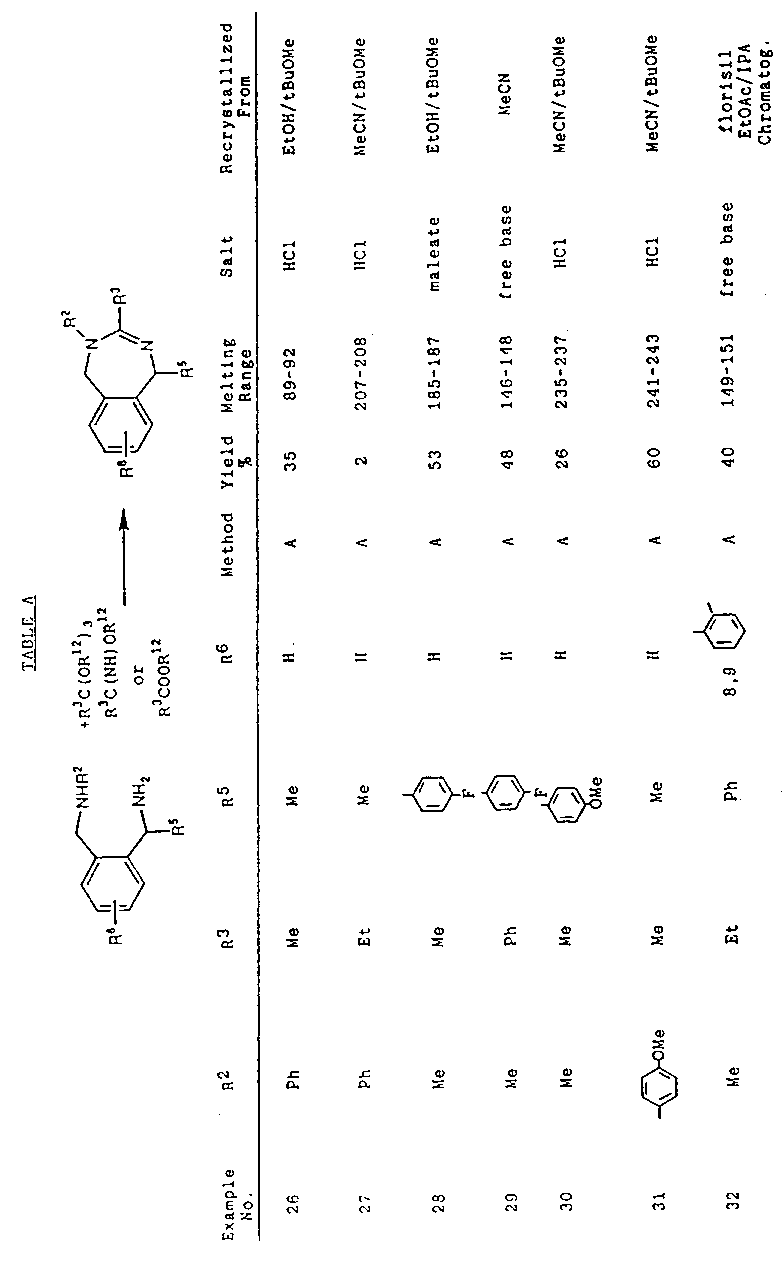

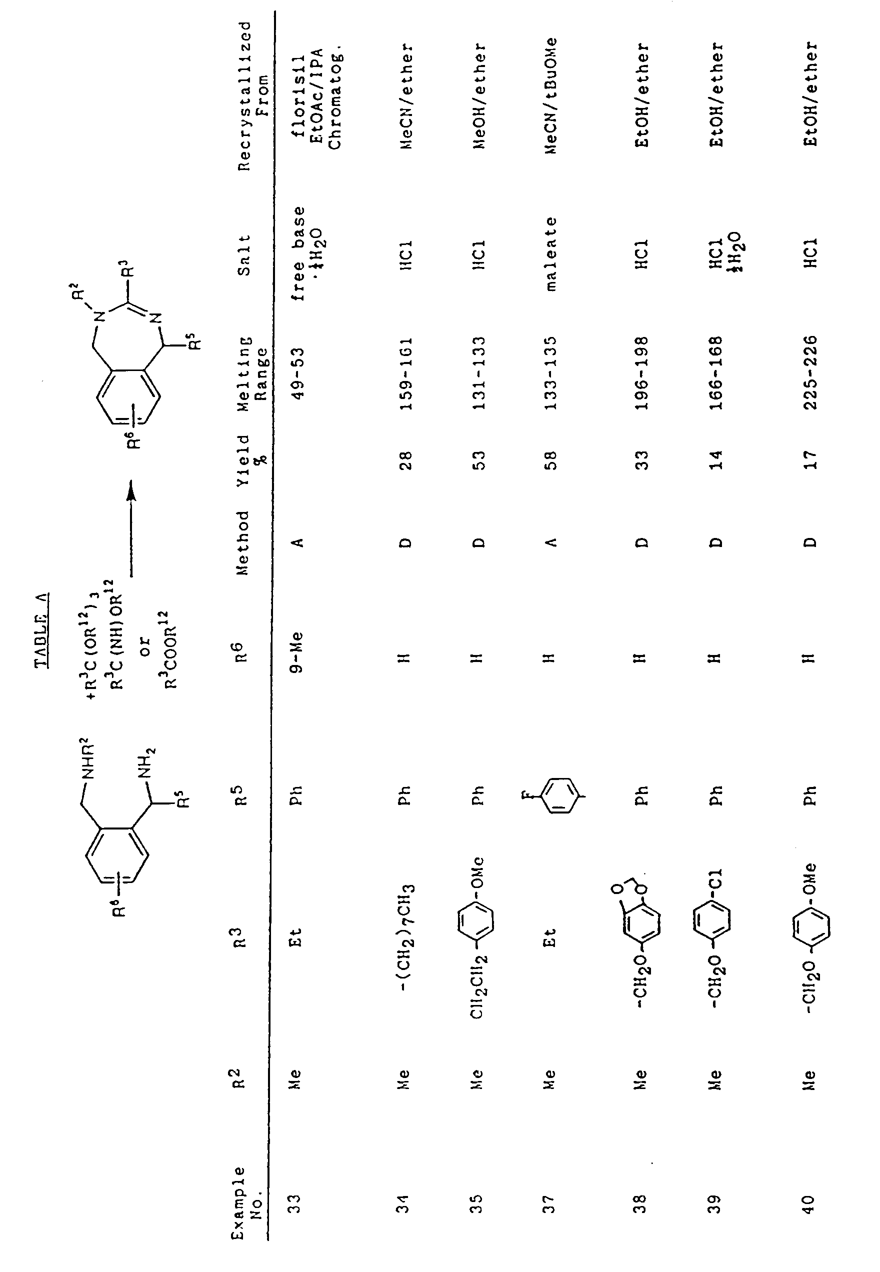

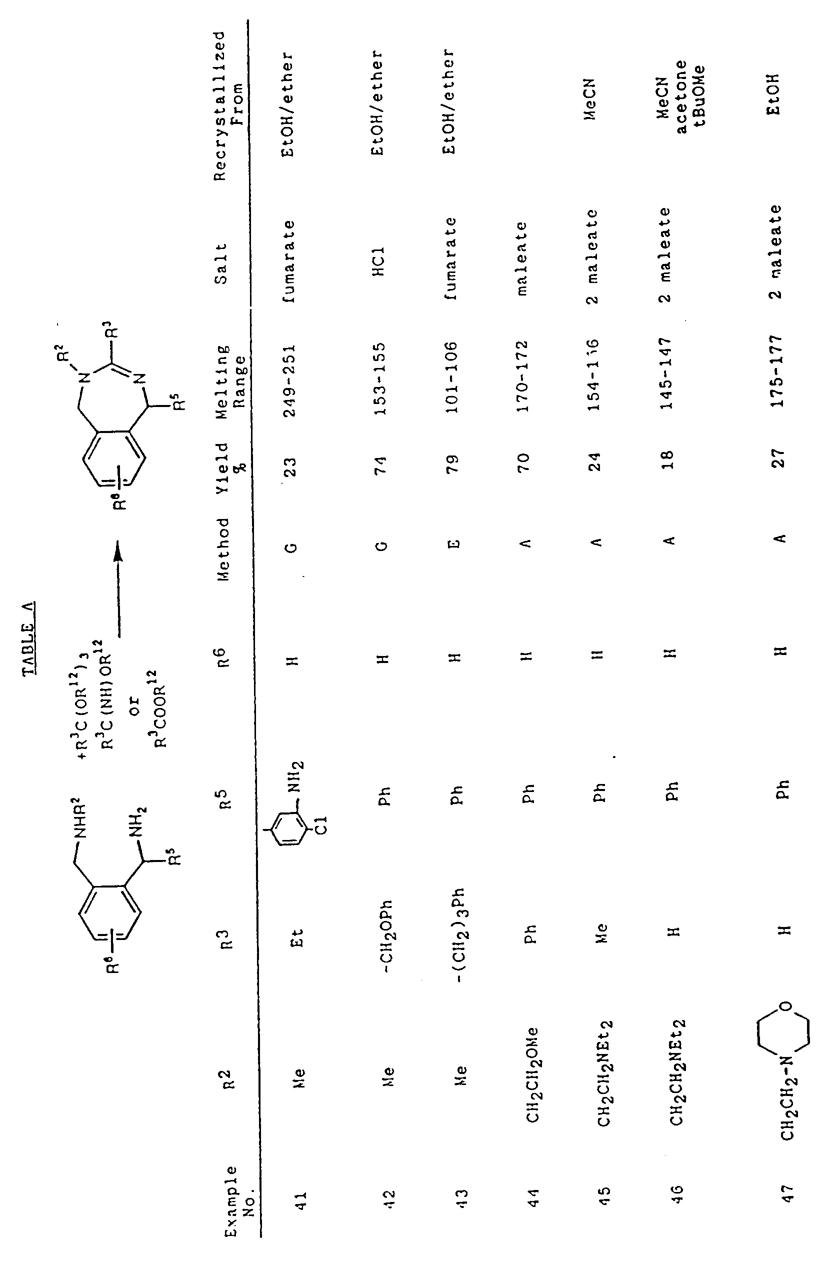

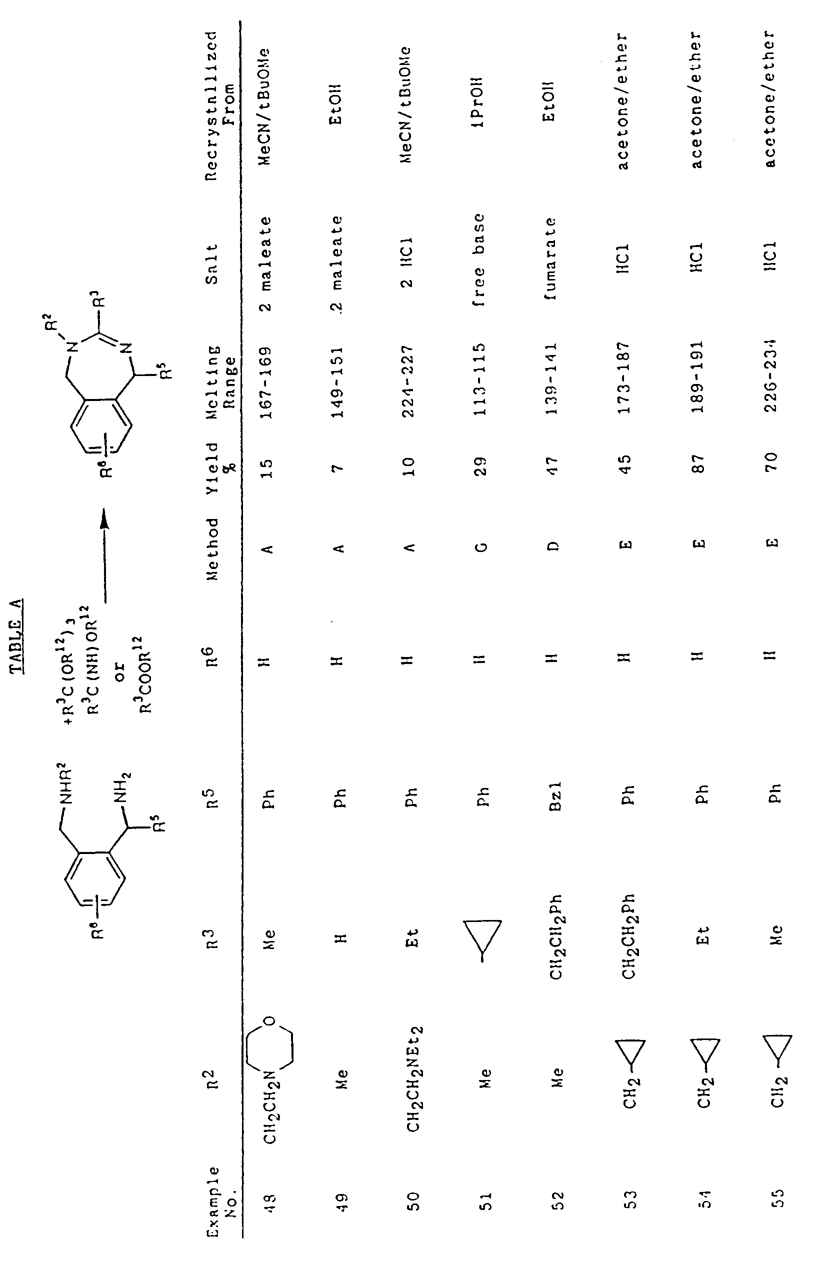

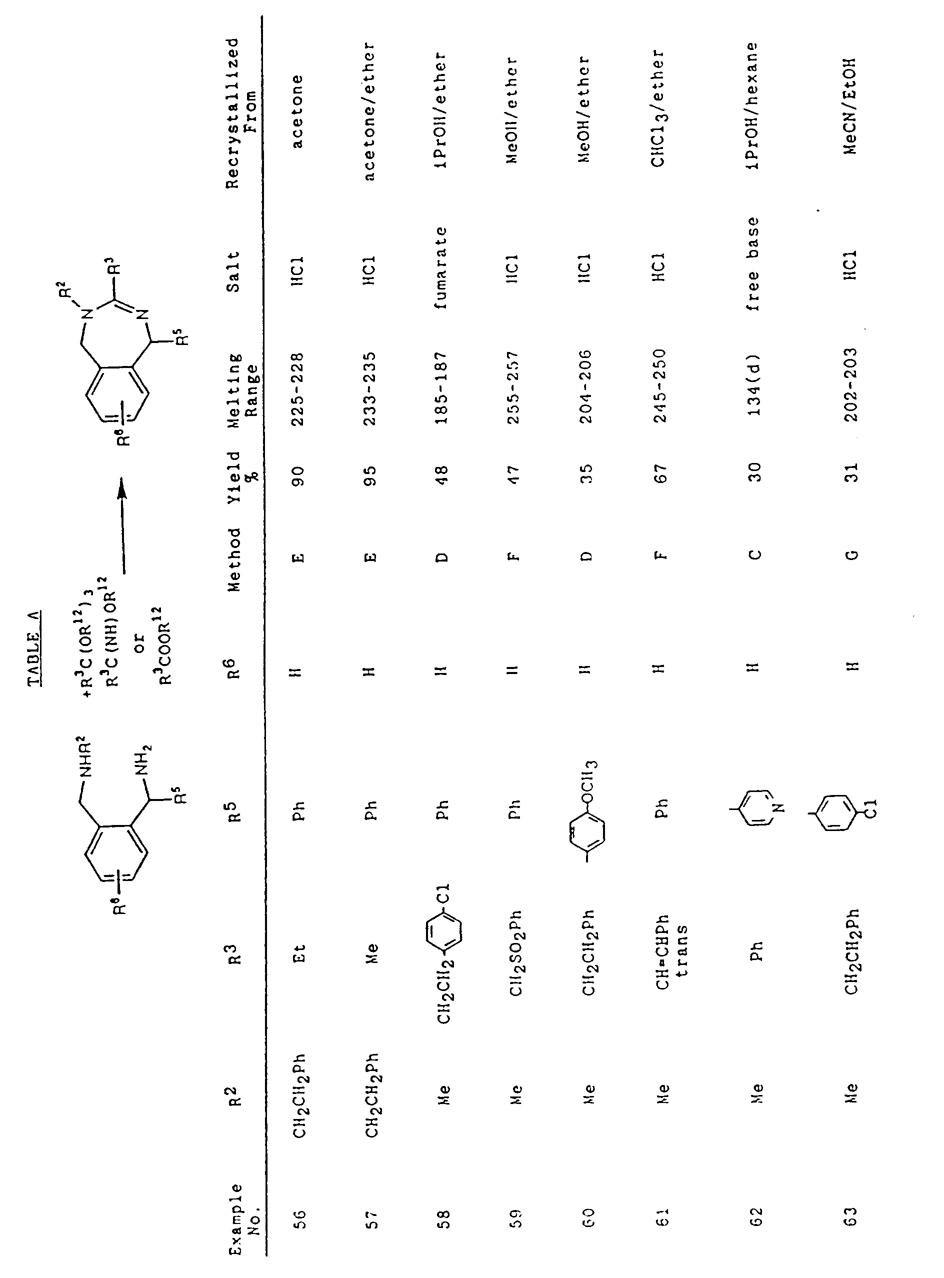

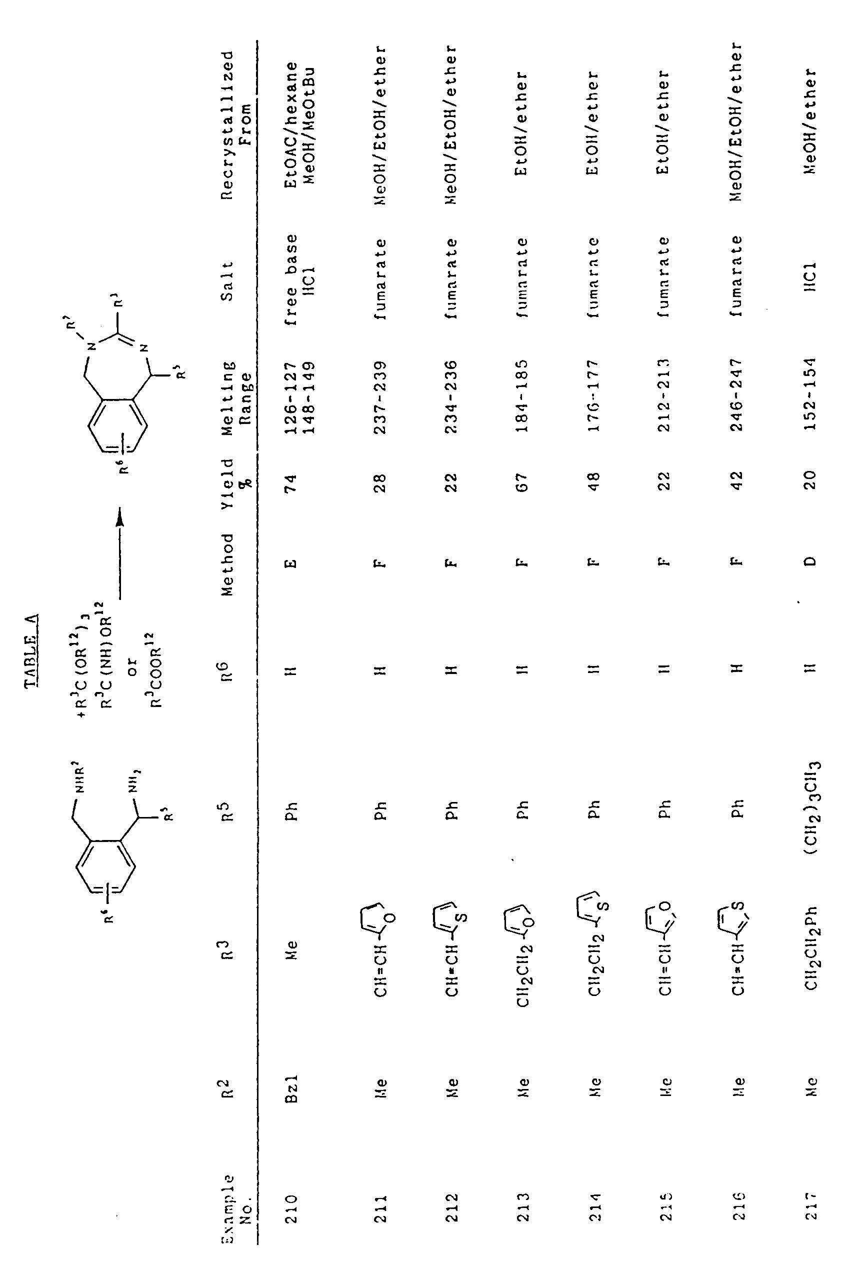

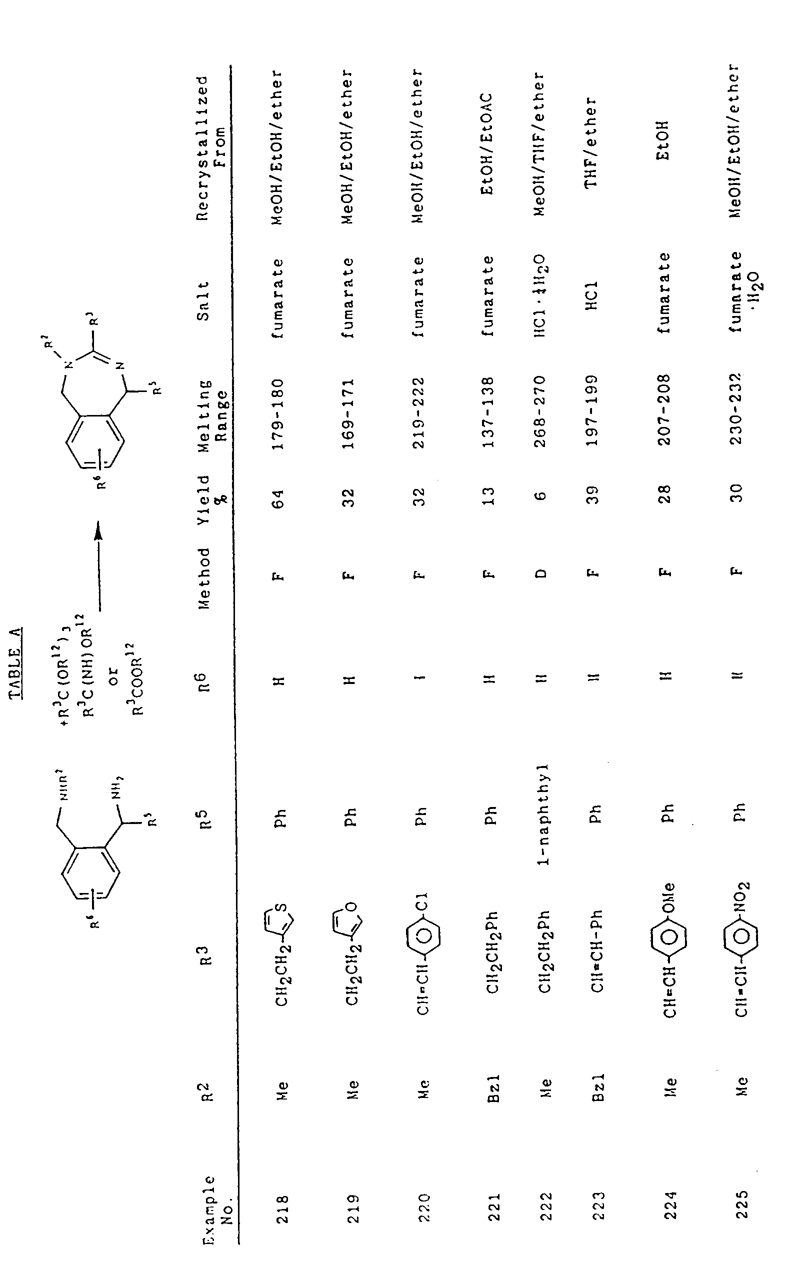

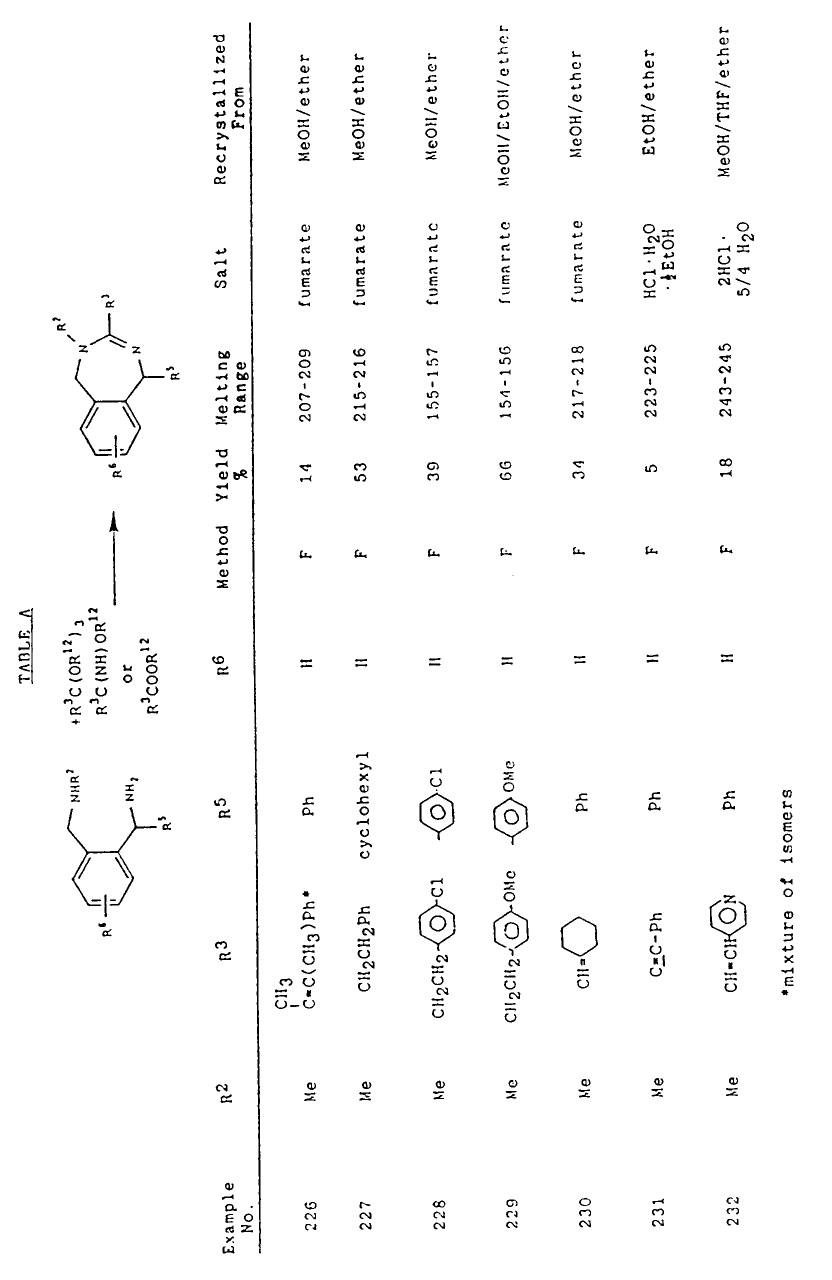

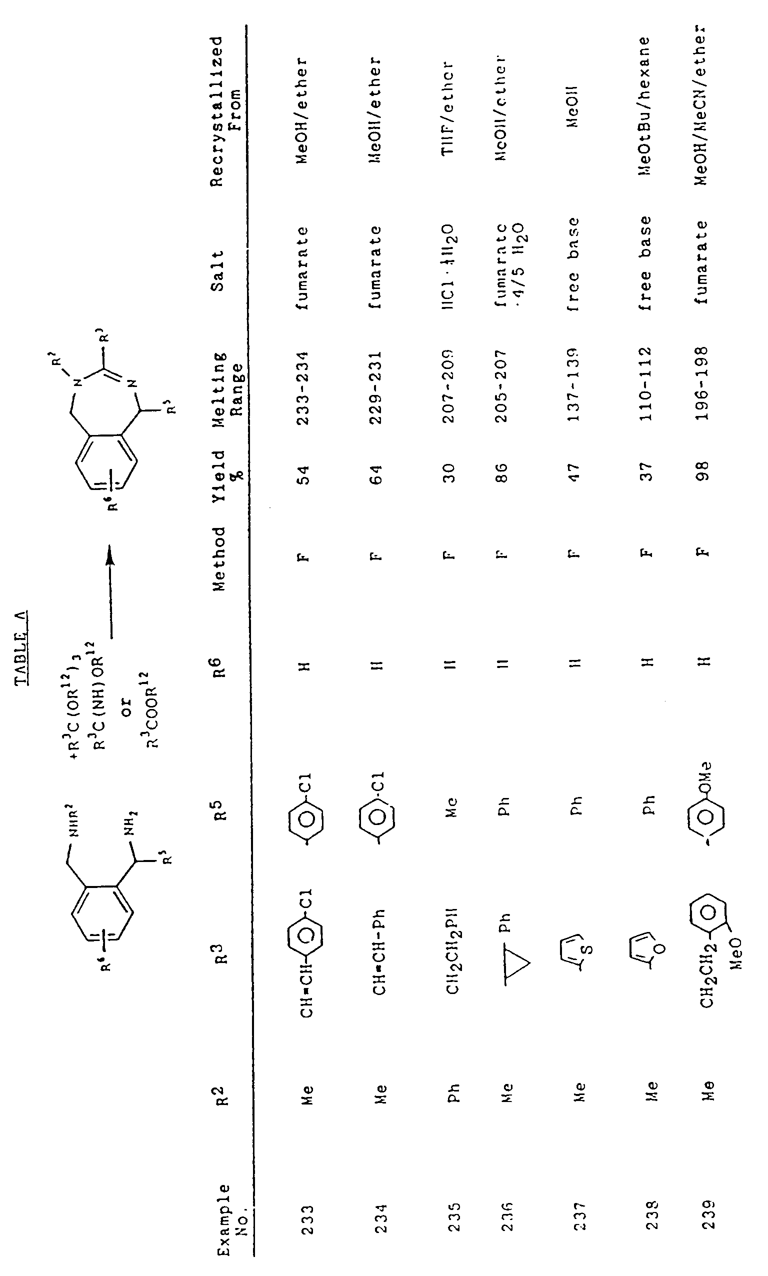

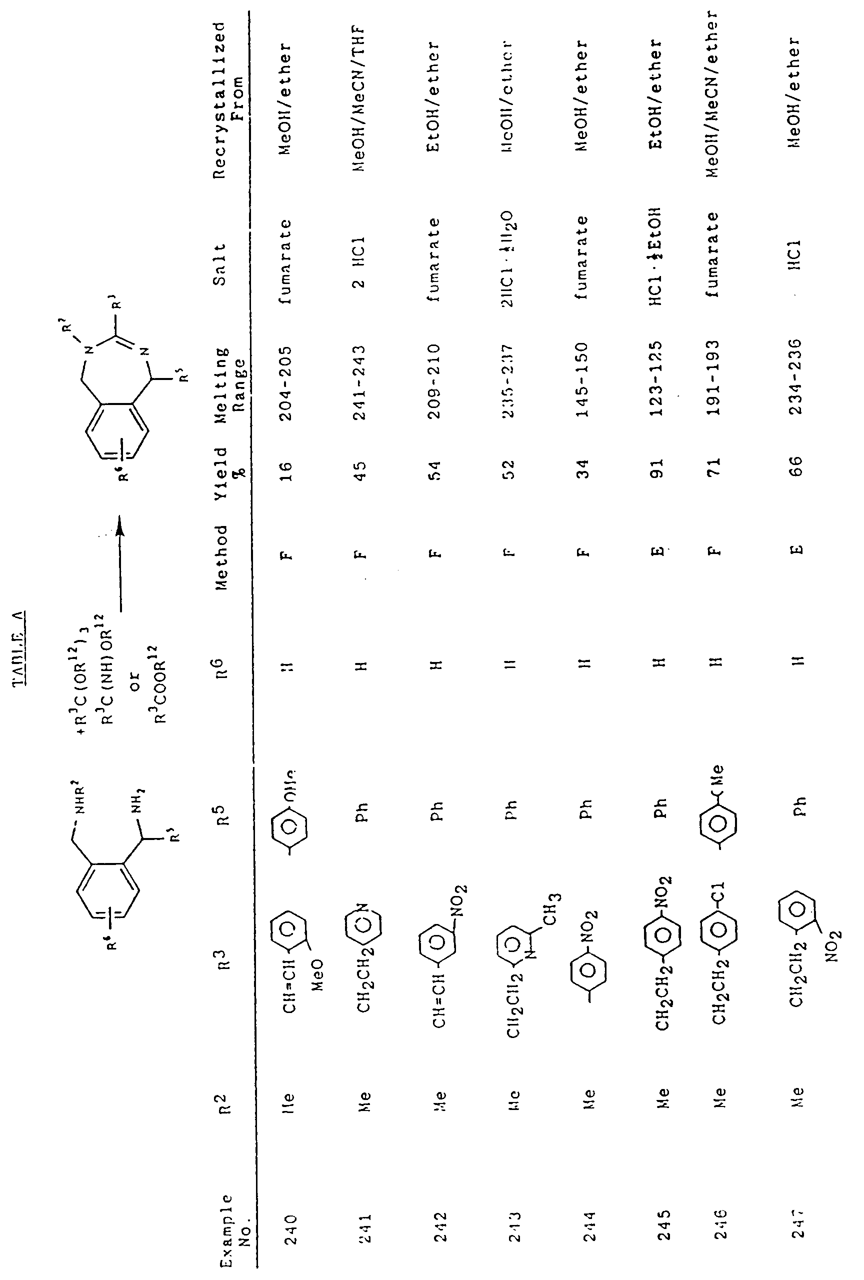

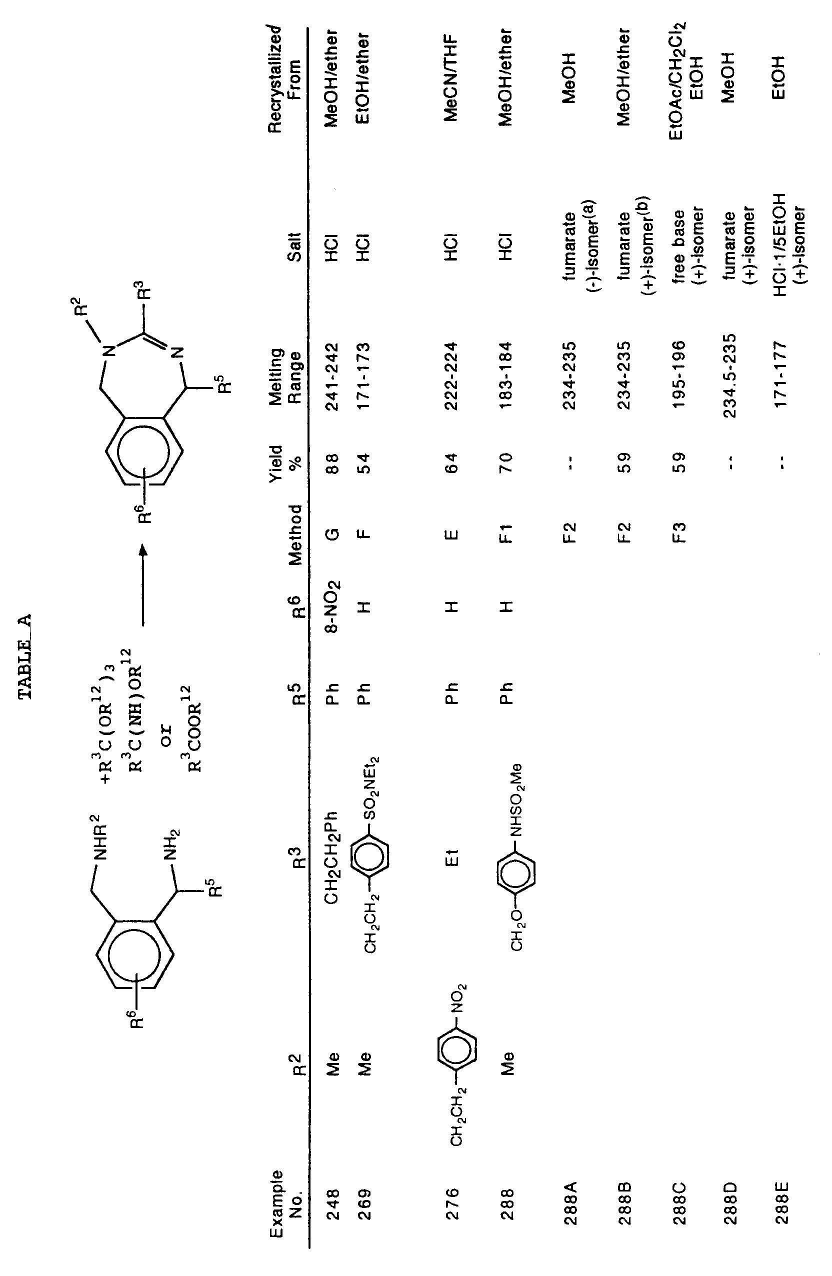

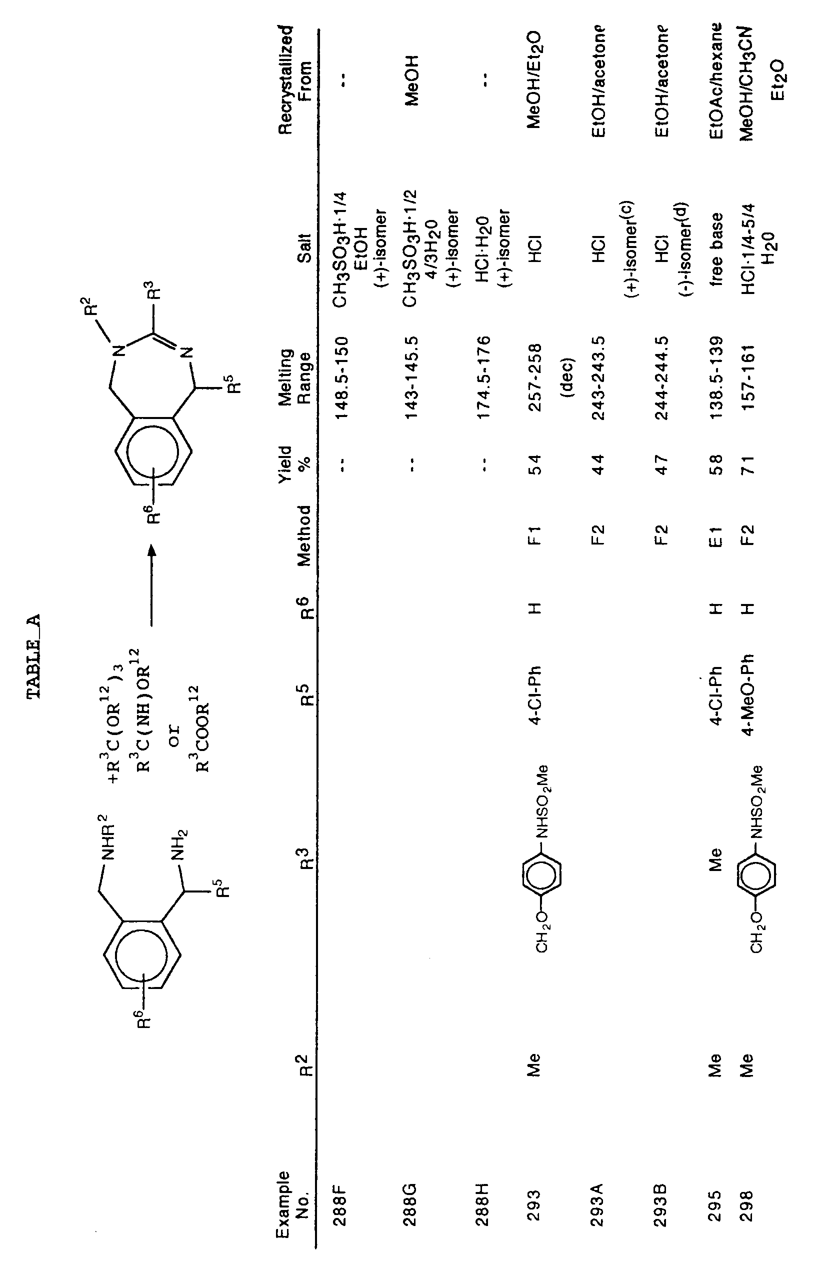

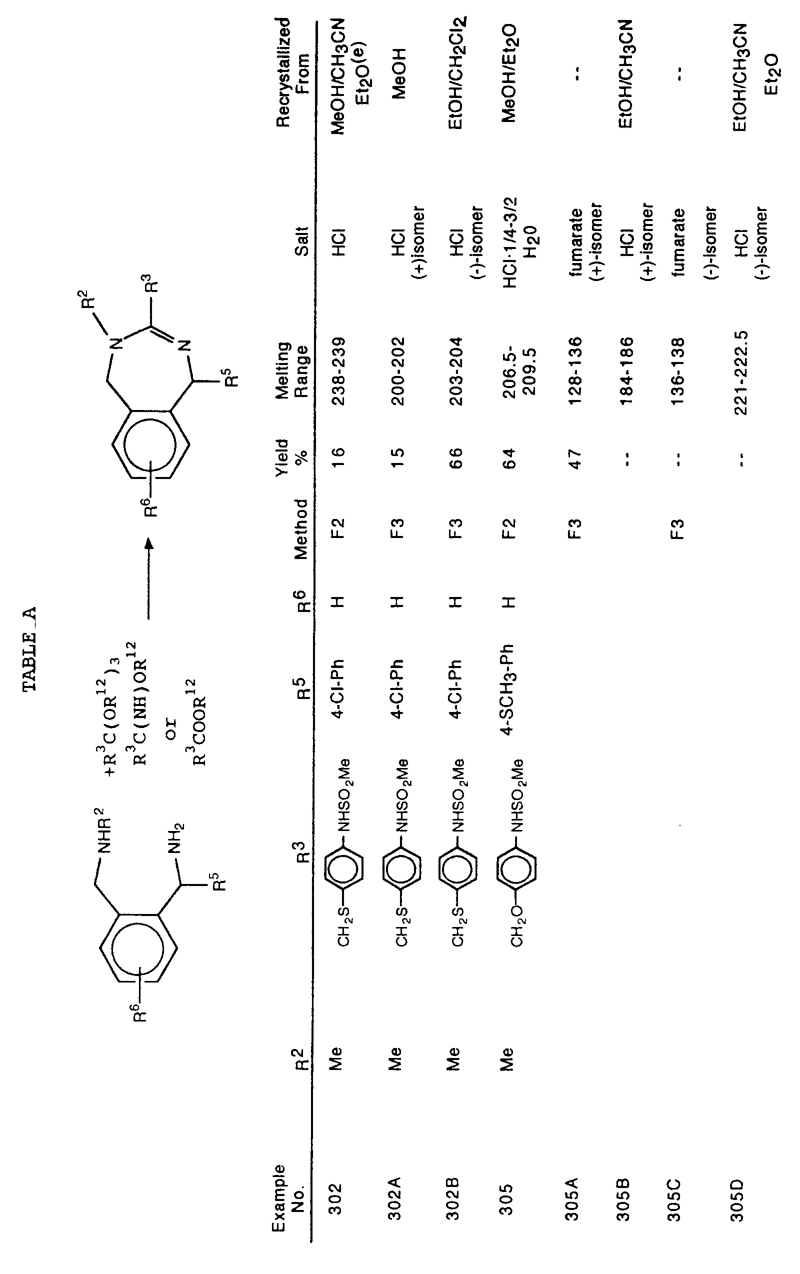

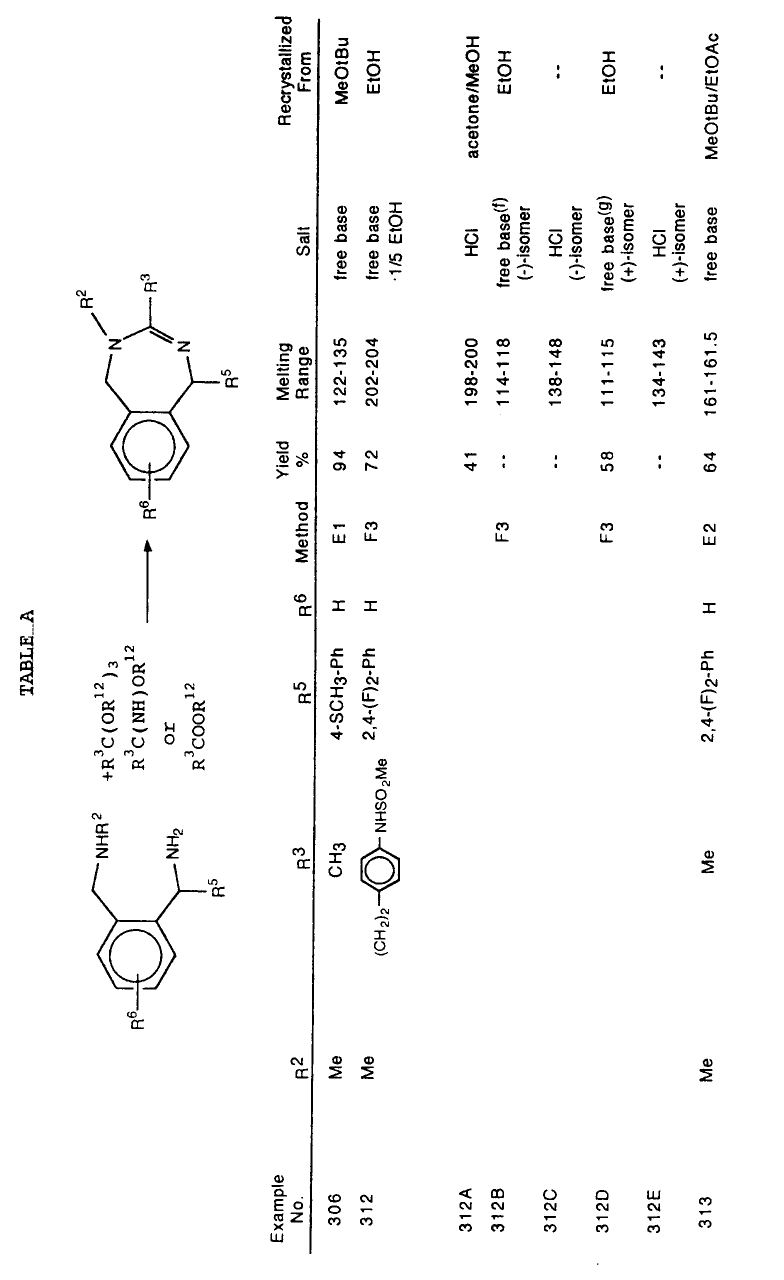

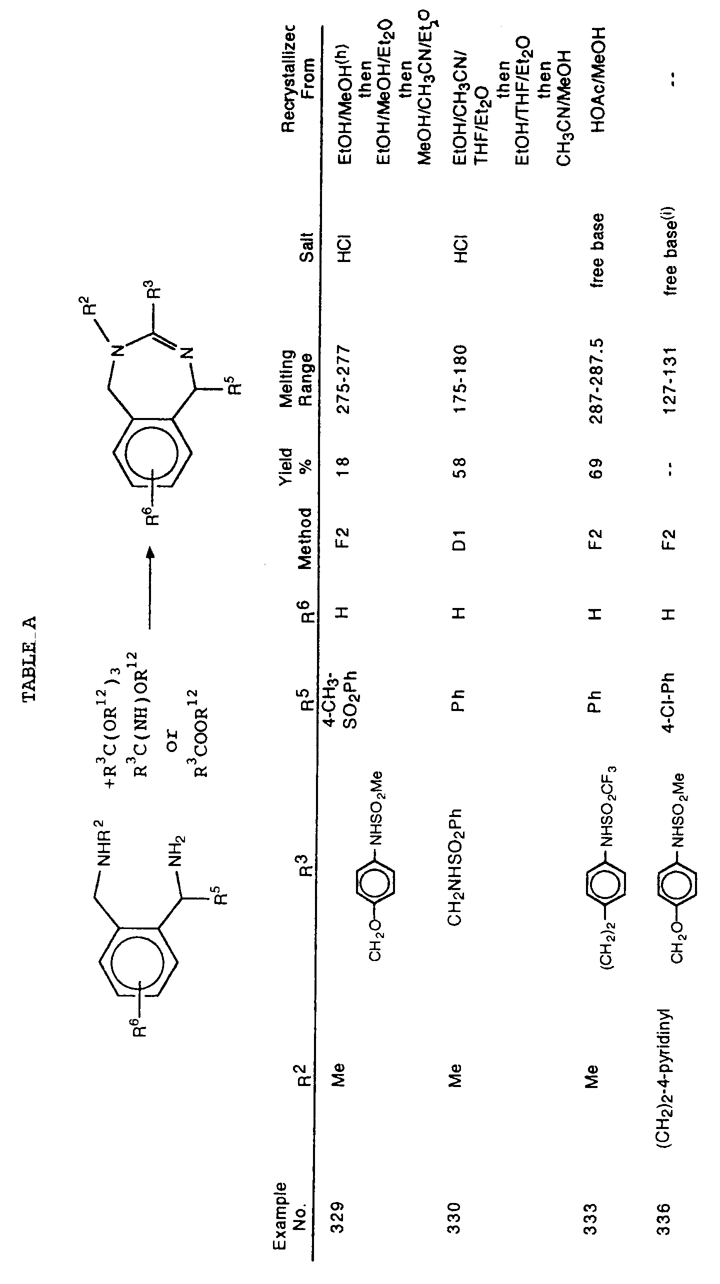

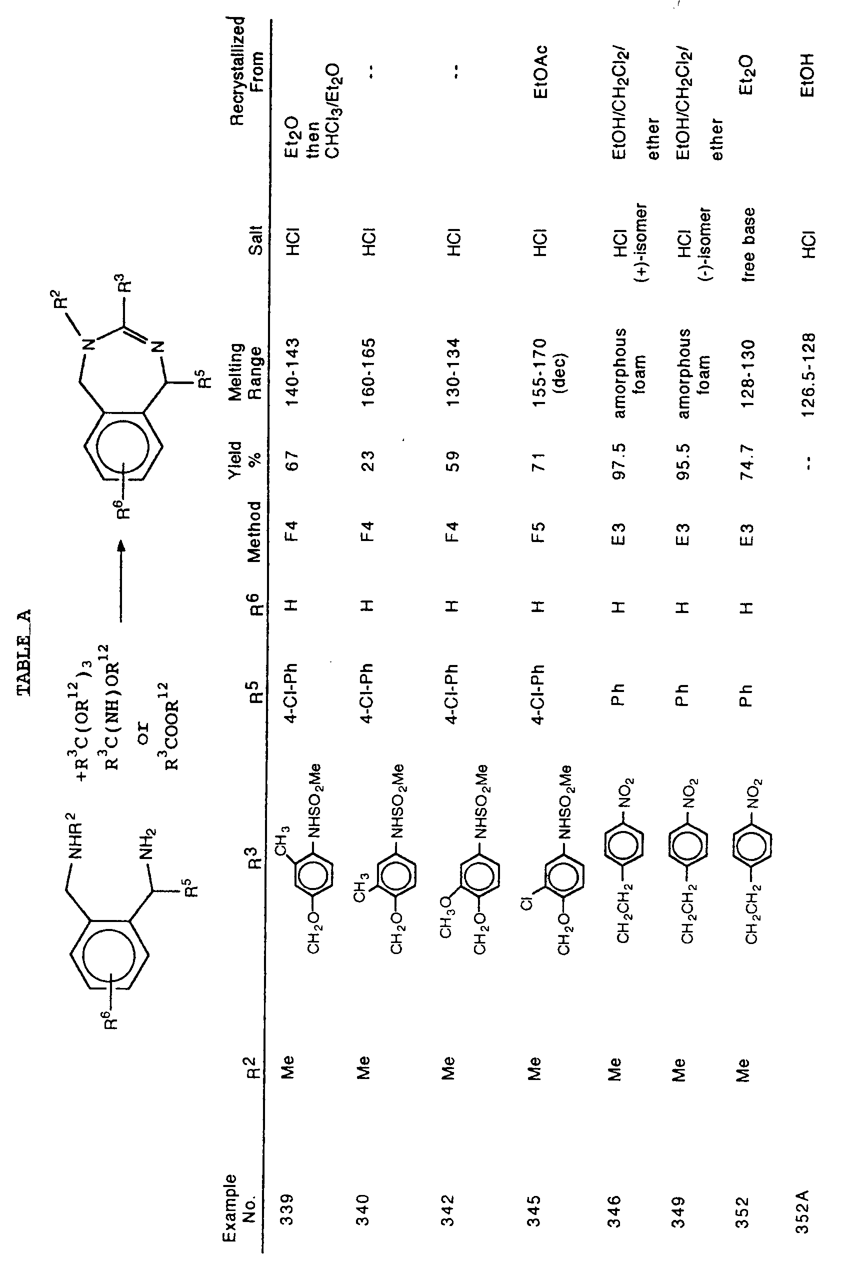

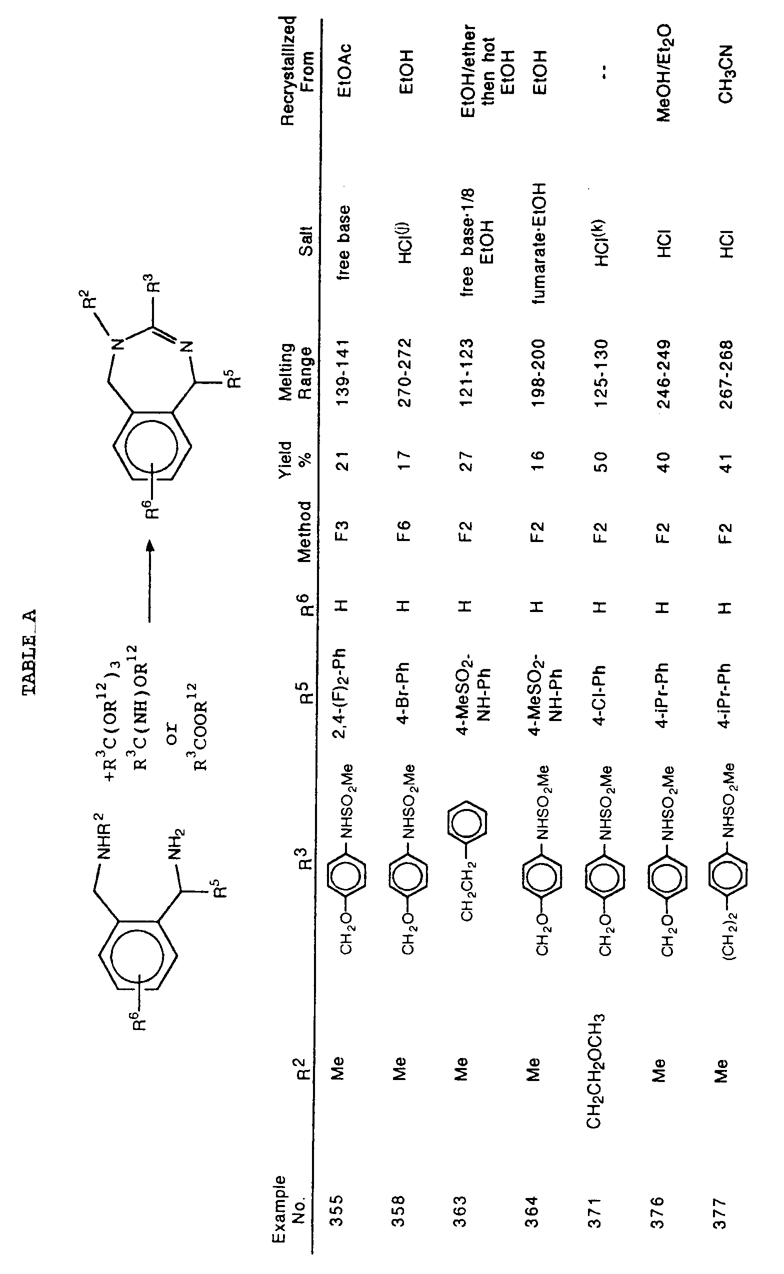

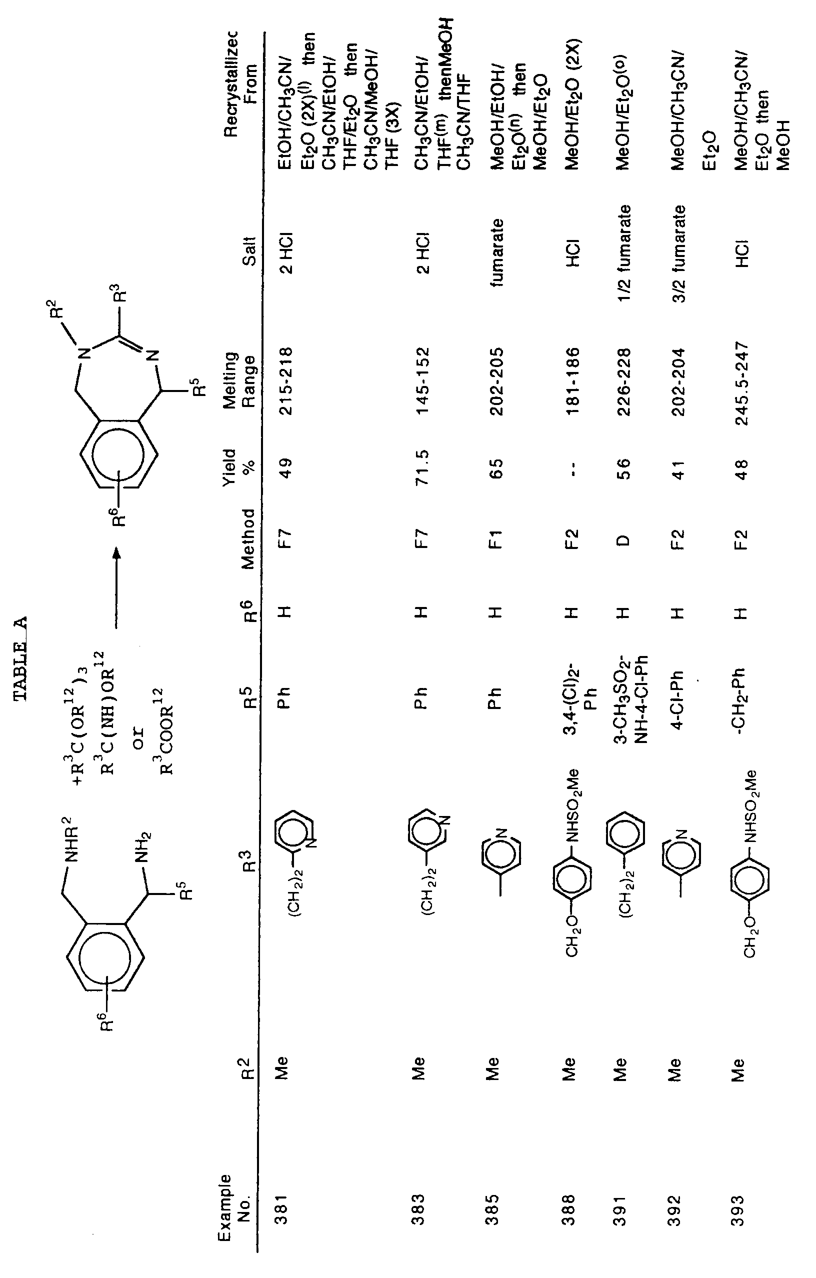

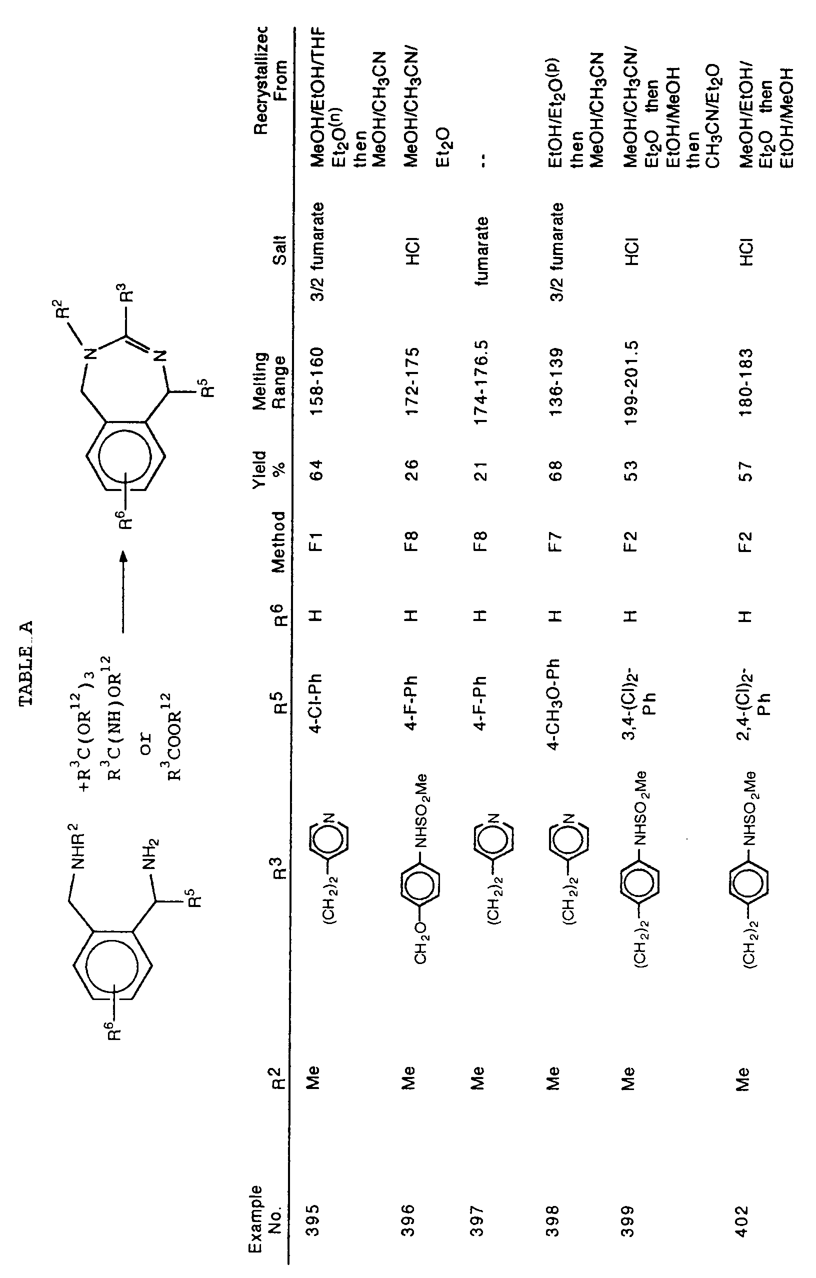

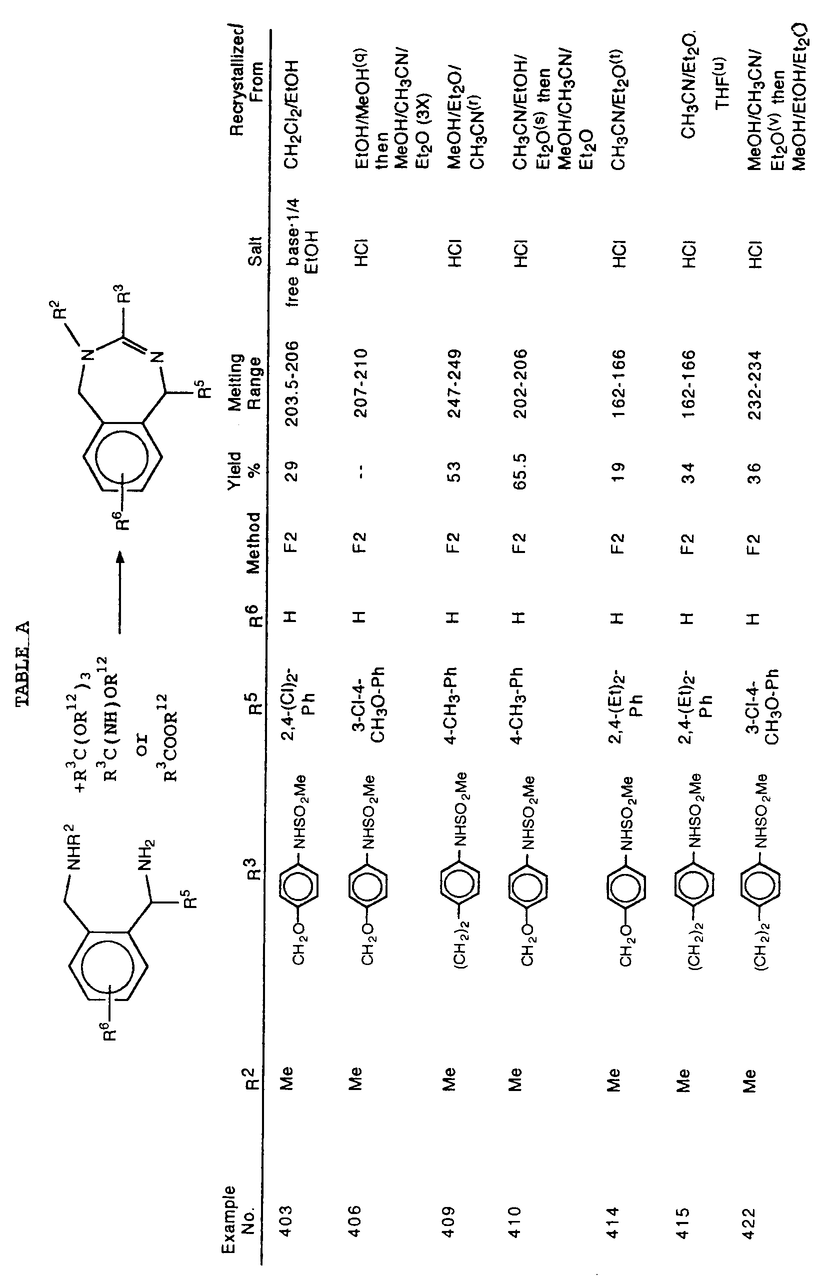

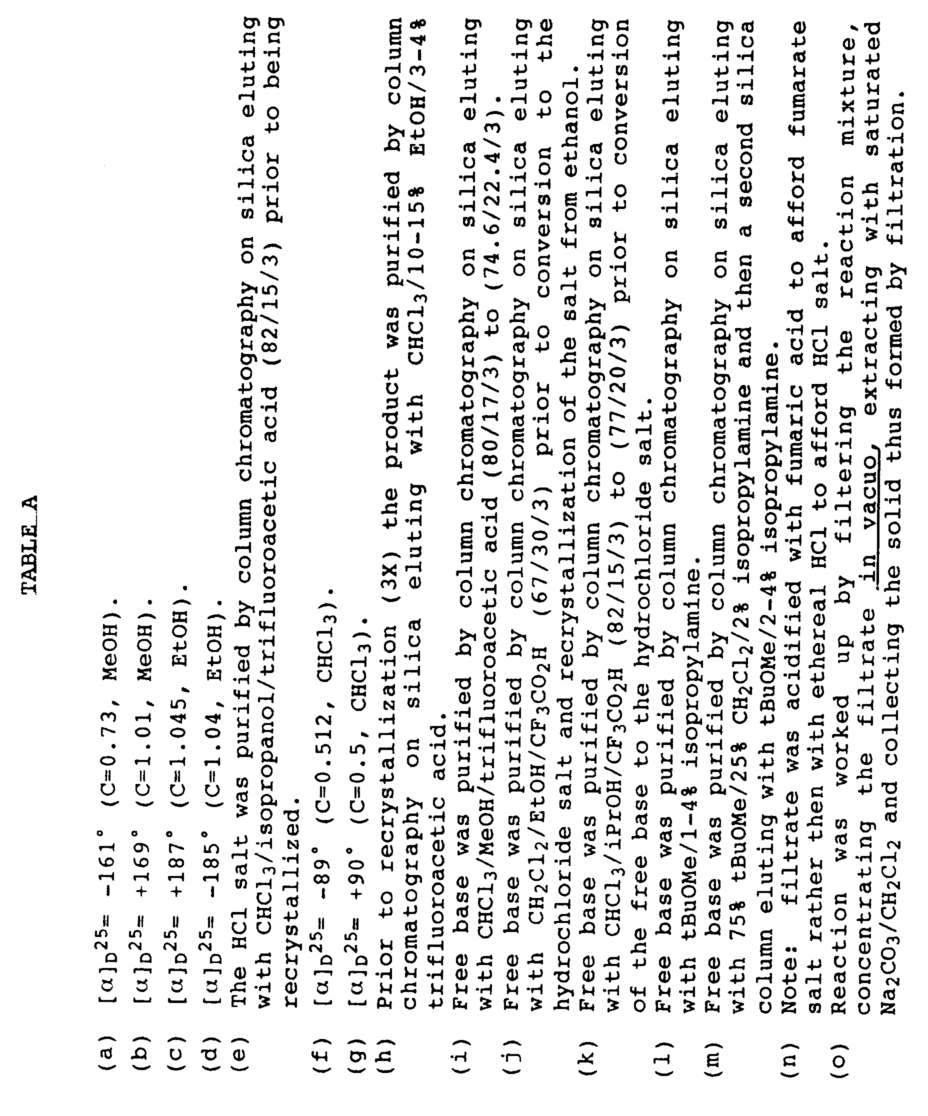

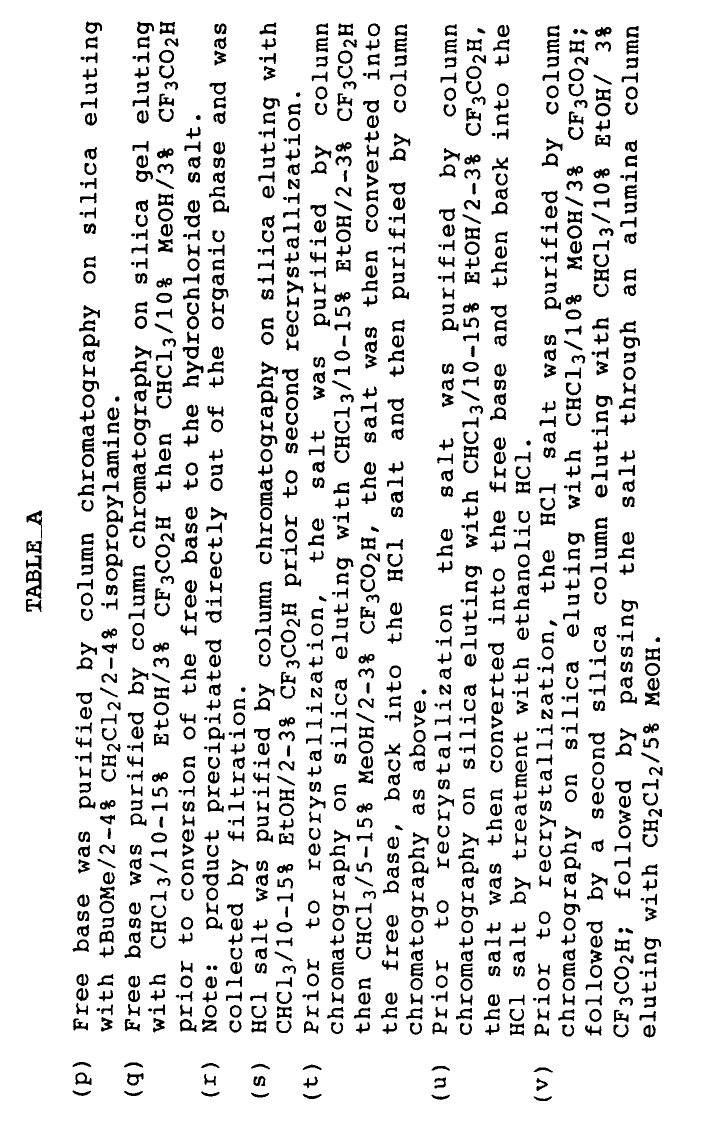

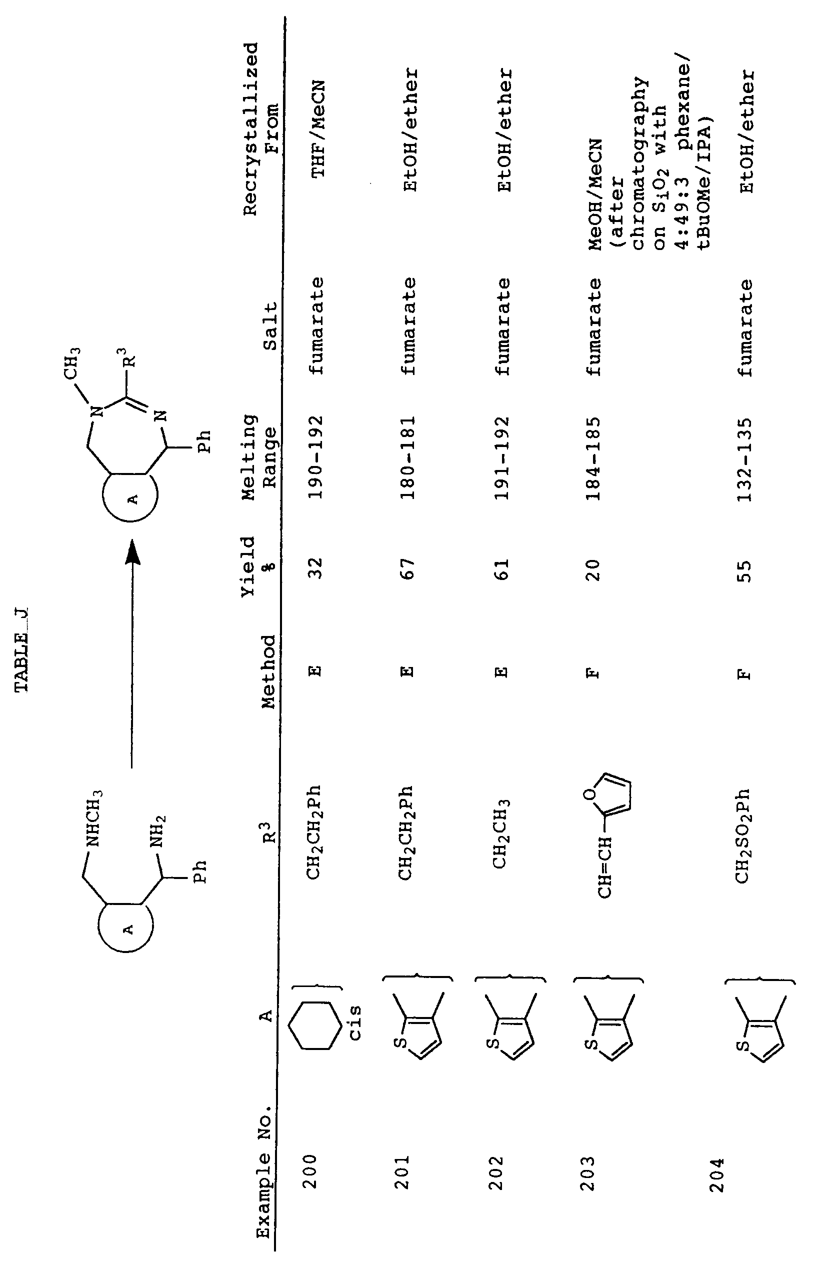

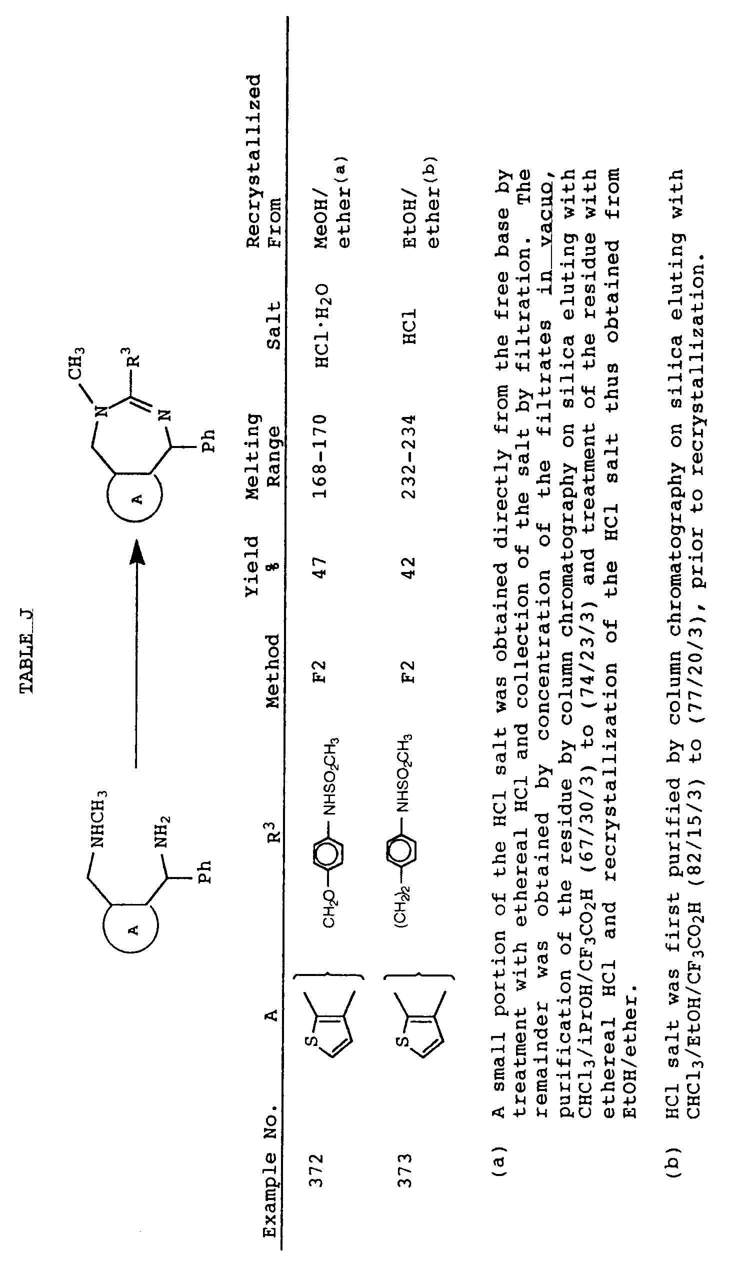

### General Method A

The appropriate diamine and five to seven equivalents of the corresponding triethylorthoester were stirred at room temperature while 0.4-0.5 ml acetic acid/mmol diamine were added in one portion. The mixture was stirred at reflux for 2-12 hours or stirred at room temperature for 2-72 hours. The reaction was diluted with ethyl acetate, washed with 2N sodium hydroxide and extracted into three portions of 2N HCl. The HCl extracts were combined, washed twice with ether, made basic with excess 35% sodium hydroxide and extracted into three portions of ether. The ether extracts were combined, dried over magnesium sulfate and the solvent removed in vacuo. The free base or the salt was recrystallized as shown in Table A.

### General Method B

The diamine was added to two equivalents of potassium acetate or a catalytic amount of potassium acetate in 0.8-1.2 ml acetic acid/mmol diamine. The mixture was stirred at room temperature and from two to five equivalents of the appropriate triethylorthoester were added. The reaction was stirred at room temperature for 18-72 hours and stripped in vacuo. The product was worked up as described for General Method A.

### General Method C

The dihydrochloride of the diamine was dissolved in 1-3 ml acetic acid/mmol diamine and about 2.0 to 2.5 equivalents of sodium acetate were added. The mixture was stirred for about ten minutes at room temperature, and three to five equivalents of the appropriate triethylorthoester were added. The mixture was stirred at room temperature for 2-48 hours and stripped in vacuo. The reaction was worked up as described in General Method A.

### General Method D

The diamine dihydrochloride and two to three equivalents of the appropriate methoxyimine hydrochloride were dissolved in 2-6 ml methanol/mmol diamine. The mixture was stirred, and two equivalents of sodium acetate were added. After 2-18 hours the solvent was removed in vacuo and the product worked up as described in General Method E.

### General Method D1

The procedure was the same as that described hereinabove in General Method D except that the appropriate ethoxyimine hydrochloride was used and the reaction mixture was worked up as follows. The reaction mixture was filtered, washed with methanol, concentrated in vacuo and extracted with aqueous Na₂CO₃/CH₂Cl₂. The organic layer was separated, washed with water, then aqueous Na₂CO₃ and then brine and the solvent was dried over Na₂SO₄ and concentrated in vacuo. The residue was converted into an appropriate salt which was purified as shown in Table A.

### General Method E

The diamine dihydrochloride, 1.3-3.0 equivalents of the appropriate trimethyl- or triethylorthoester and 1.0-1.8 equivalents of sodium acetate were combined in about 3 to 6 ml isopropyl acetate/mmol diamine. The mixture was refluxed for 3-18 hours. The reaction was cooled, washed with two portions of 2N sodium hydroxide and dried over sodium sulfate. The solvent was removed in vacuo and either the salt or the free base was purified from the residue as shown in Table A.

### General Method E1

The diamine dihydrochloride, methanol, 2.0 equivalents of the appropriate trimethyl or triethylorthoester and 1.75-1.9 equivalents sodium acetate were combined and stirred at about 25°C to reflux for 3-24 hours. The solvent was removed in vacuo, and the residue was extracted with CH₂Cl₂ or a mixture of t-butylmethyl ether/CH₂Cl₂ (1/1) and the extract was washed with 1-2N NaOH. The mixture was dried over Na₂SO₄, filtered and the filtrate was concentrated in vacuo to afford the free base which was purified by recrystallization as shown in Table A.

### General Method E2

The procedure was substantially similar to that described in General Method E1 except that the reaction mixture was worked-up as follows. The reaction mixture was cooled, filtered, and the filtrate was concentrated in vacuo. The residue was partitioned between dilute HCl (1N) and ether. The aqueous layer was separated, basified with 35% NaOH and extracted with t-butylmethyl ether/CH₂Cl₂. The aqueous layer was saturated with brine and again extracted with t-butylmethyl ether/CH₂Cl₂ and the combined organic extracts were washed with brine, dried over Na₂SO₄ and concentrated in vacuo. The residue thus obtained was then purified by recrystallization as shown in Table A.

### General Method E3

The procedure was similar to that described in General Method E except that the reaction mixture was worked up as follows. The reaction mixture was treated with water, a sufficient amount of 2N NaOH or 35% NaOH to basify the solution, ether, and CH₂Cl₂. The organic layer was separated and the aqueous layer was saturated with sodium chloride and extracted with ether. The organic layers were combined, washed with brine and dried over Na₂SO₄ and concentrated in vacuo and either the salt or the free base was purified from the residue thus obtained as illustrated in Table A.

### General Method F

To the diamine dihydrochloride, slurried in about 3 ml toluene/mmol diamine, was added dropwise at 0° under nitrogen 2.1 equivalents 2M trimethylaluminum in toluene. The reaction was allowed to come to room temperature and stirred for 2 hours, then 1.25 to 1.50 equivalents of the appropriate methyl or ethyl ester were added. The reaction was refluxed 2 hours, cooled and quenched by the sequential addition of ice, methanol, dichloromethane and 2N NaOH. The aluminum salts were filtered off, the layers separated, washed with more dichloromethane, dried over sodium sulfate, stripped and crystallized as shown in Table A. Occasionally flash chromatography on silica gel with MeOtBu, optionally containing up to 2% isopropylamine, was necessary before crystallization.

### General Method F1

To a slurry of the diamine dihydrochloride in about 3.5-5 ml sulfolane/mmol of diamine was added dropwise at room temperature under nitrogen 3.1-4.5 equivalents of 2M trimethylaluminum in toluene. The reaction mixture was stirred at room temperature to 35°C for 15-60 minutes and then 1.07-1.25 equivalents of the appropriate ethyl ester was added in one portion. The resulting solution was heated at about 95-110°C for 50 minutes to 2.5 hours, cooled and was poured into a mixture of water, Rochelle salt, CH₂Cl₂, 2N NaOH and ice. The organic layer was separated, and the aqueous layer was extracted with CH₂Cl₂. The organic layers were combined, washed with water containing Rochelle salt, and then with a Na₂CO₃ solution. The organic layer was dried over Na₂SO₄, filtered, and the filtrate was acidified with ethereal·HCl and evaporated to afford the product as the acid salt which was recrystallized as shown in Table A.

### General Method F2

The procedure was the same as that described hereinabove in General Method F1 except that the reaction mixture was worked up by diluting the cooled reaction mixture with CH₂Cl₂ and then quenching with a saturated Rochelle salt solution, water and a saturated Na₂CO₃ solution. After filtering the mixture through solka Flok, if necessary, the organic phase was separated, dried over Na₂SO₄ and concentrated in vacuo. The residue was then purified as such, or was converted into an appropriate salt by standard procedures which are well known in the art, and the salt was purified by recrystallization as shown in Table A.

### General Method F3

The diamine dihydrochloride was stirred with about 6-12 ml toluene/mmol of diamine under nitrogen, and 3.0-3.5 equivalents of 1M trisobutylaluminum in toluene was added while maintaining the reaction temperature at about room temperature. The mixture was heated from about 60°C to reflux for 15-20 minutes and then 1.0-1.2 equivalents of the appropriate ethyl ester was added. The mixture was heated to reflux for 1-4 hours and then was allowed to stand at room temperature overnight or was worked-up directly. The reaction mixture was poured into a mixture of a saturated Rochelle salt solution, ethyl acetate, CH₂Cl₂ (optional), saturated Na₂CO₃ solution and ice-water and the organic layer was separated. The aqueous layer was extracted with CH₂Cl₂ or ethyl acetate and the combined organic layers were washed with brine (optional), and dried over Na₂SO₄. The solvent was concentrated in vacuo and either the appropriate salt or the free base was purified from the residue as shown in Table A.

### General Method F4

The diamine hydrochloride was stirred with about 5-6 ml toluene/mmol diamine, optionally in the presence of about 2 ml sulfolane/mmol diamine and 2.5-2.6 equivalents 1M triisobutylaluminum in toluene was added. The mixture was heated to reflux for 10-15 minutes and then about 1.3 equivalents of the appropriate ethyl ester was added. The reaction mixture was refluxed for 3-4 hours and then was cooled and diluted with CH₂Cl₂. The mixture was poured into a Rochelle salt solution, water was added and the mixture was stirred for 15-30 minutes. The mixture was filtered and the filtrate was concentrated in vacuo to afford the free base of the product which was purified by recrystallization as shown in Table A, or which was converted into an appropriate salt which was purified, where applicable, as shown in Table A.

### General Method F5

The procedure was similar to that described in General Method F3, except that the reaction mixture was worked up by pouring the reaction mixture into a saturated Rochelle salt solution, adding water and ethyl acetate and then separating the organic layer. The organic layer was then washed with a saturated Rochelle salt solution (2X), dried over Na₂SO₄ and concentrated in vacuo. The residue thus obtained was then purified, where necessary, as shown in Table A or it was converted into an appropriate salt by standard procedures which are well known in the art, and the salt was purified, where necessary, by recrystallization as shown in Table A.

### General Method F6

The procedure was similar to that described in General Method F1, except the reaction was washed up as follows. The reaction mixture was quenched with a saturated Rochelle salt solution, then CH₂Cl₂ and finally saturated Na₂CO₃ was added. The phases were separated, the aqueous phase was extracted with CH₂Cl₂ and the organic layers were combined and dried over Na₂SO₄. The solvent was removed in vacuo and the residue was then purified as the free base as shown in Table A or was converted into an appropriate salt by standard procedures and the salt was purified as shown in Table A.

### General Method F7

To the diamine dihydrochloride in about 3-5 ml toluene/mmol of diamine, was added dropwise at 0°C under nitrogen 2.1 equivalents of 2M trimethylaluminum in toluene. The reaction mixture was brought to room temperature, stirred for about 2 hours and then 1.5 equivalents of the appropriate methyl ester was added. The reaction mixture was refluxed for about 45 minutes to 2.5 hours, cooled in an ice bath and then treated with 2N NaOH and Et₂O/CH₂Cl₂/MeOH (2/1/1) to (3.5/1.5/1). The mixture was stirred for about 1/2 - 1 hour, and was filtered through solka floc. The organic layer was separated, washed with brine and then 2N NaOH (optional) and the solvent was dried over Na₂SO₄ and concentrated in vacuo. The residue was purified by column chromatography on silica if necessary, and the residue was converted into an appropriate salt and recrystallized as illustrated in Table A.

### General Method F8

The procedure was similar to that described in General Method F1 except that the reaction was worked up as follows. The reaction mixture was poured into a water/Rochelle salt/CH₂Cl₂ solution, and the mixture was filtered through solka folc. The filtrate layers were separated, the aqueous layer was extracted with CH₂Cl₂ and the combined organic layers were washed with water, a Rochelle salt solution and saturated Na₂CO₃. The solvent was dried over Na₂SO₄ and the solvent was removed in vacuo. The residue was passed through a plug of neutral alumina eluting with tBuOMe/CH₂Cl₂ (1/1) and the residue thus obtained was converted into an appropriate salt by standard procedures which are well known in the art and the salt was purified by recrystallization as shown in Table A.

### General Method G

The free base of the diamine, three equivalents of acetic acid and three equivalents of either the appropriate triethylorthoester or the hydrochloride of the appropriate ethoxyimine in 2-4 m methanol/mmol of diamine were stirred at room temperature for 18 hours. The solvent was removed in vacuo and the product treated as described in General Method A.

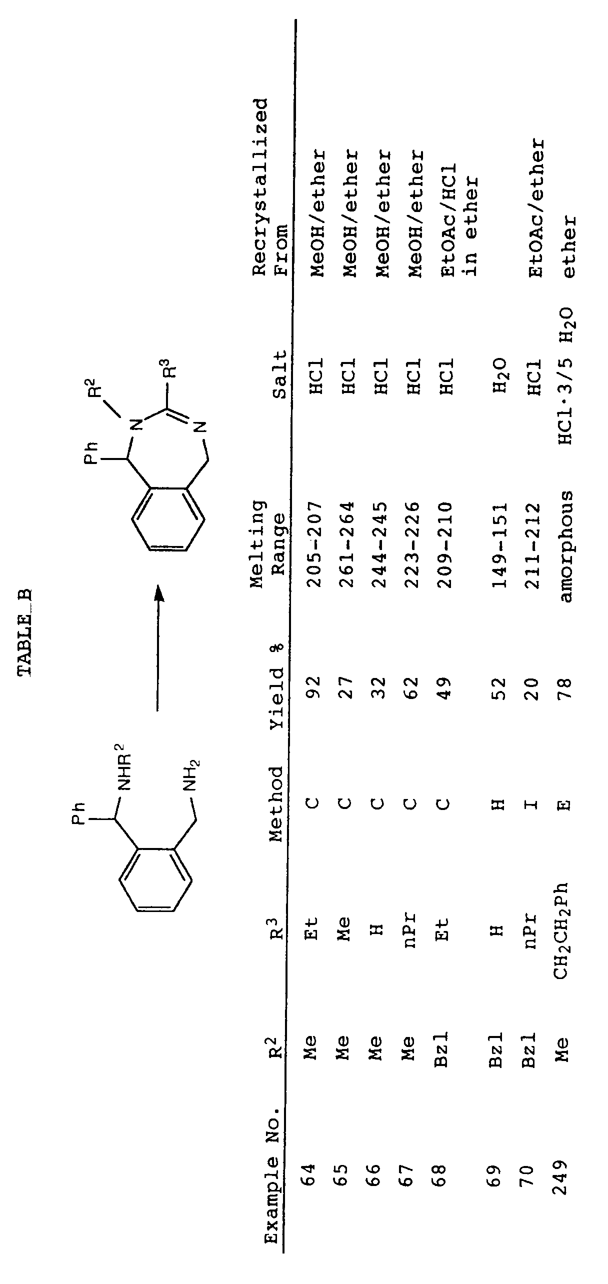

### General Method H

The diamine dihydrochloride, 2.2 equivalents of sodium acetate and 1.5 equivalents of triethylorthoester were combined in 1.2 ml isopropyl acetate/mmol diamine and refluxed for four days. The solvent was removed in vacuo, the residue taken up in dichloromethane, the dichloromethane was washed two times with 2N sodium hydroxide and dried over magnesium sulfate. The solvent was removed in vacuo and the product chromatographed on silica gel eluting with 49:49:2 ethyl acetate/dichloromethane/diethylamine. The hydrochloride of the purified free base was formed by dissolving the free base in ethyl acetate and adding HCl in ether.

### General Method I

The diamine dihydrochloride, 2.1 equivalents of sodium acetate, 3 equivalents of trimethylorthoester, and 5 equivalents of acetic acid were stirred together for seven days at room temperature. The workup was the same as that described for General Method H.

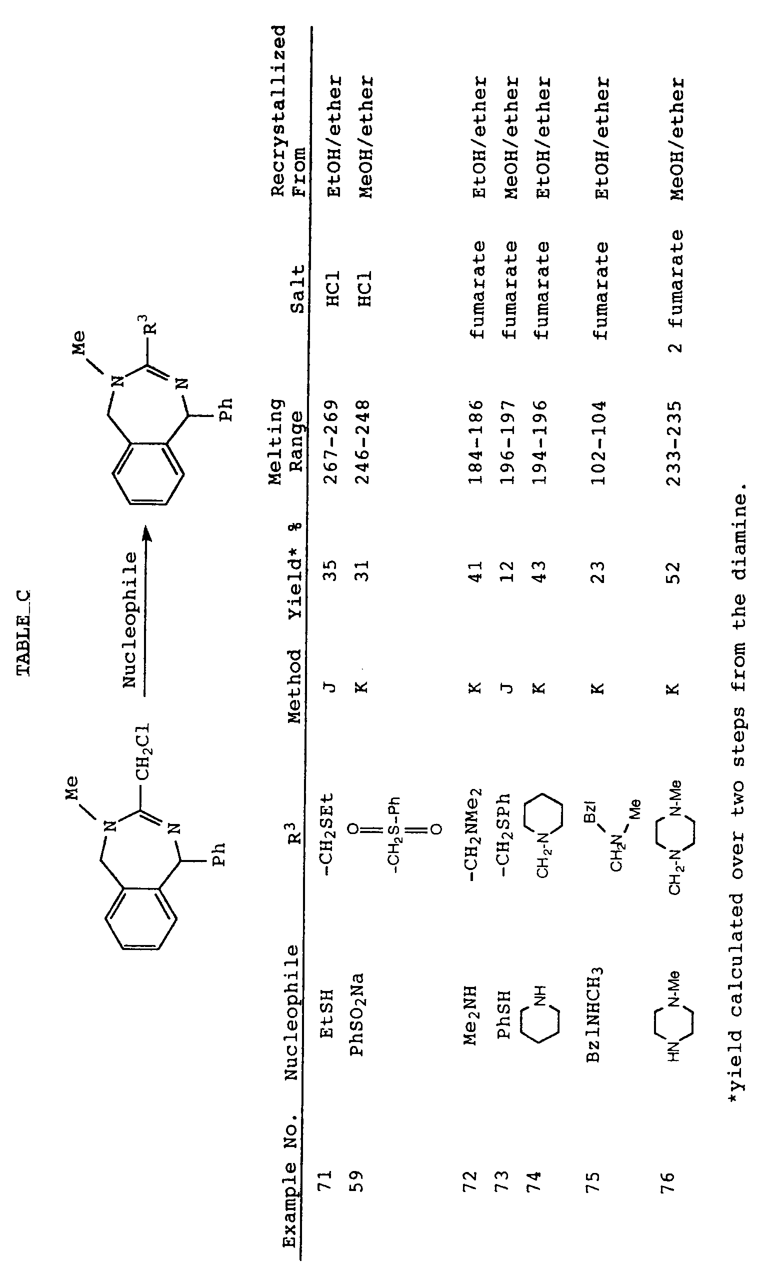

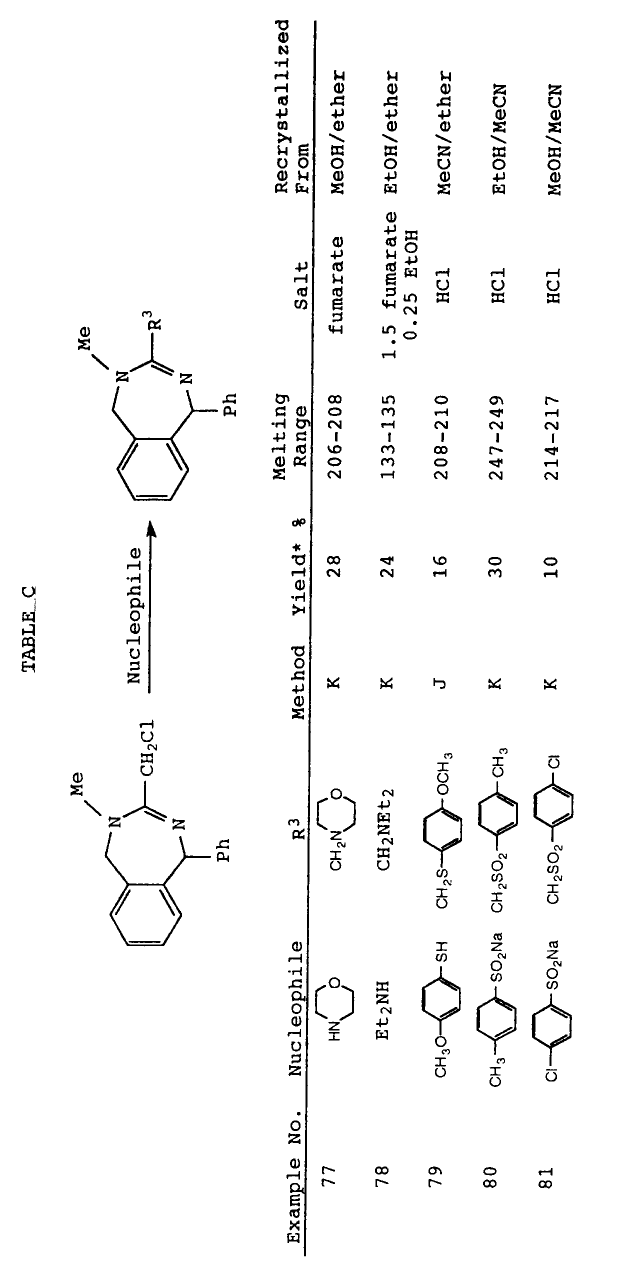

### General Method J

The 3-chloromethylbenzodiazepine was dissolved in three ml acetonitrile/mmol benzodiazepine and added to 1.0-1.7 equivalents of the thiol plus 2.3 equivalents of milled potassium carbonate in 3 ml acetonitrile/mmol benzodiazepine. The reaction was stirred at room temperature for 18 hours and filtered. The acetonitrile was removed in vacuo and the product worked up as described in General Method A.

### General Method K

The 3-chloromethylbenzodiazepine and three equivalents of the appropriate amine or sodium sulfinate were combined in 1-5 ml solvent/mmol of benzodiazepine and refluxed for 3-5 hours. The reactions with amines were run in chloroform; the reactions with sulfinate were run in methanol. The product was worked up as described in General Method A.

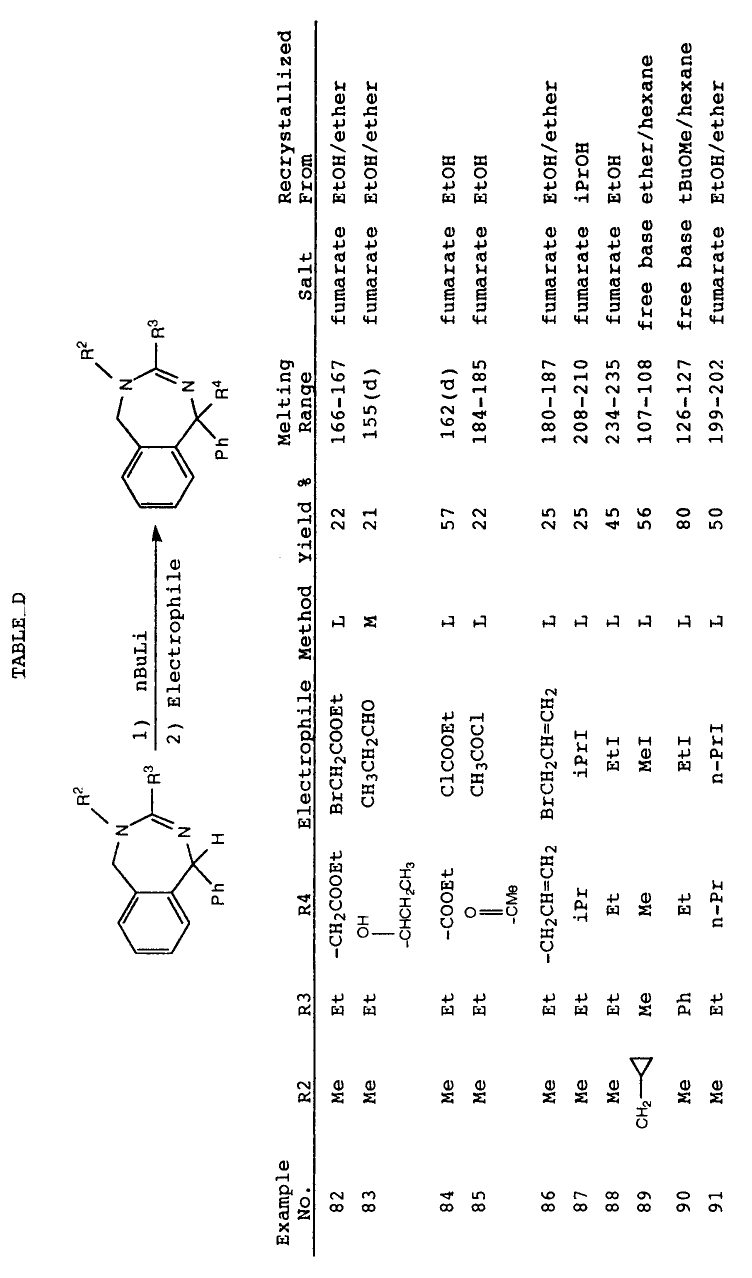

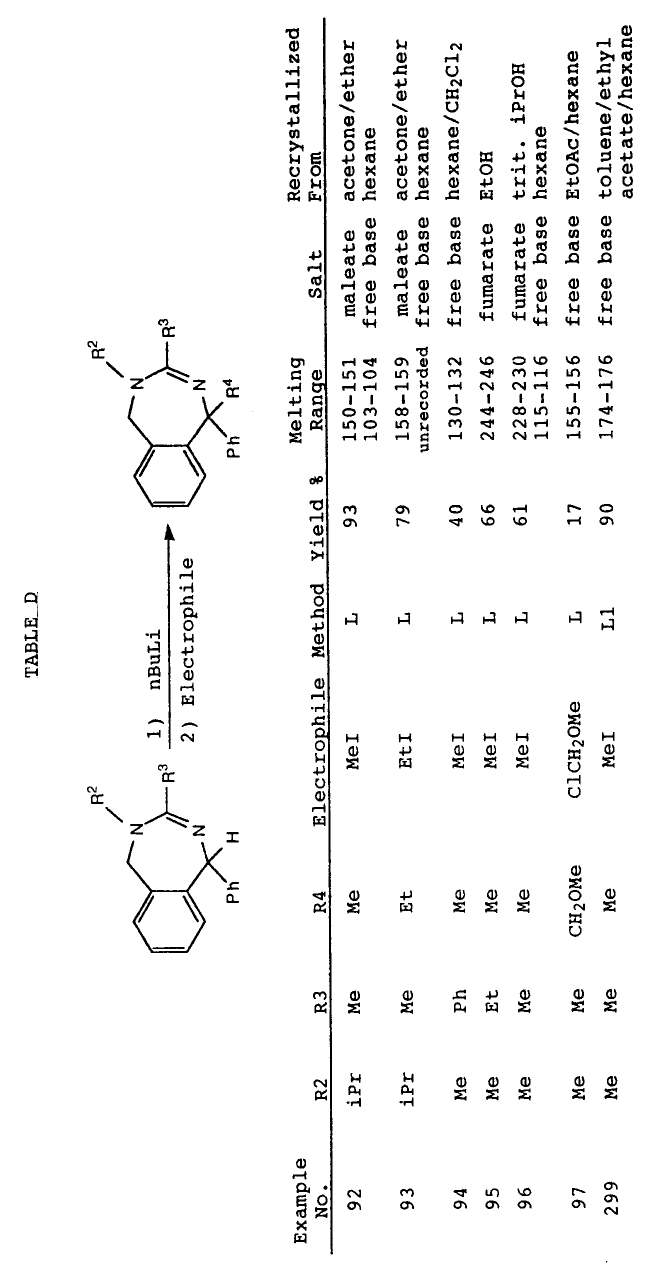

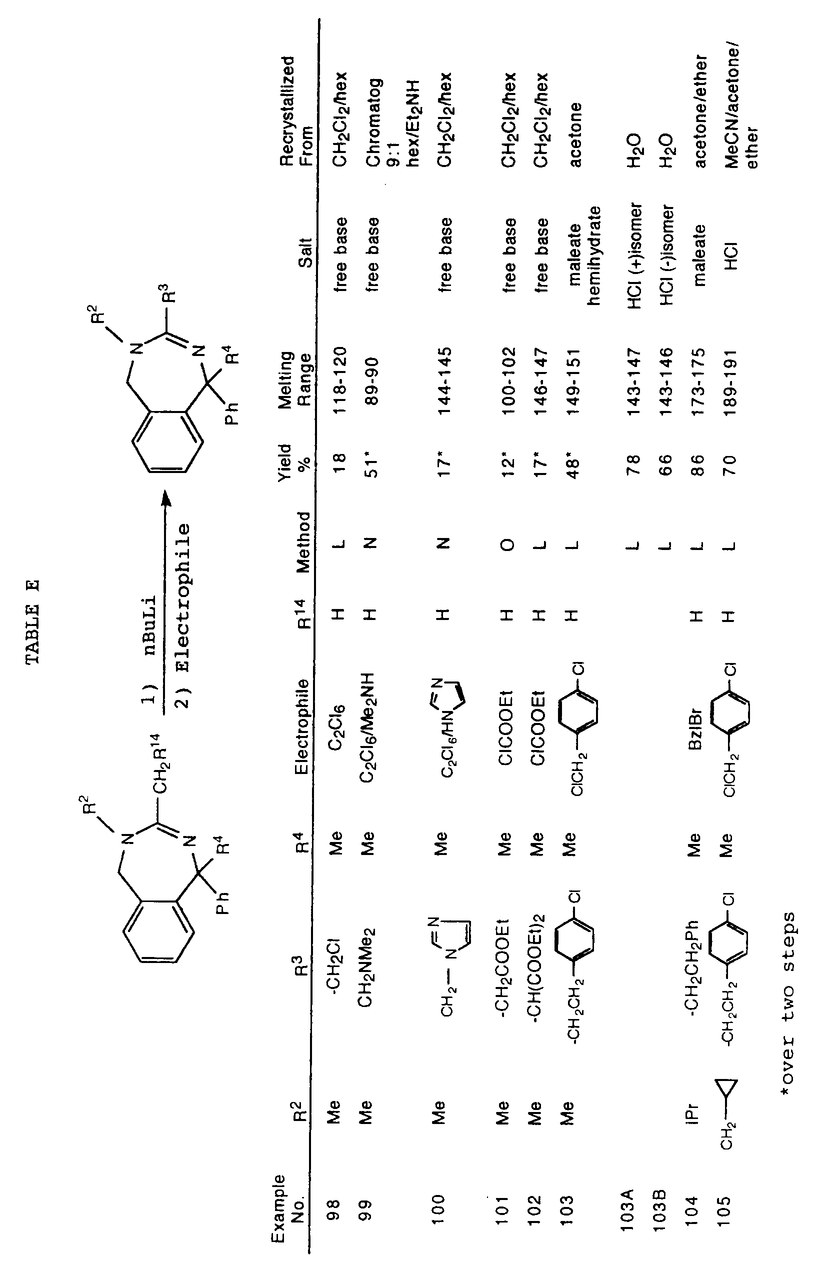

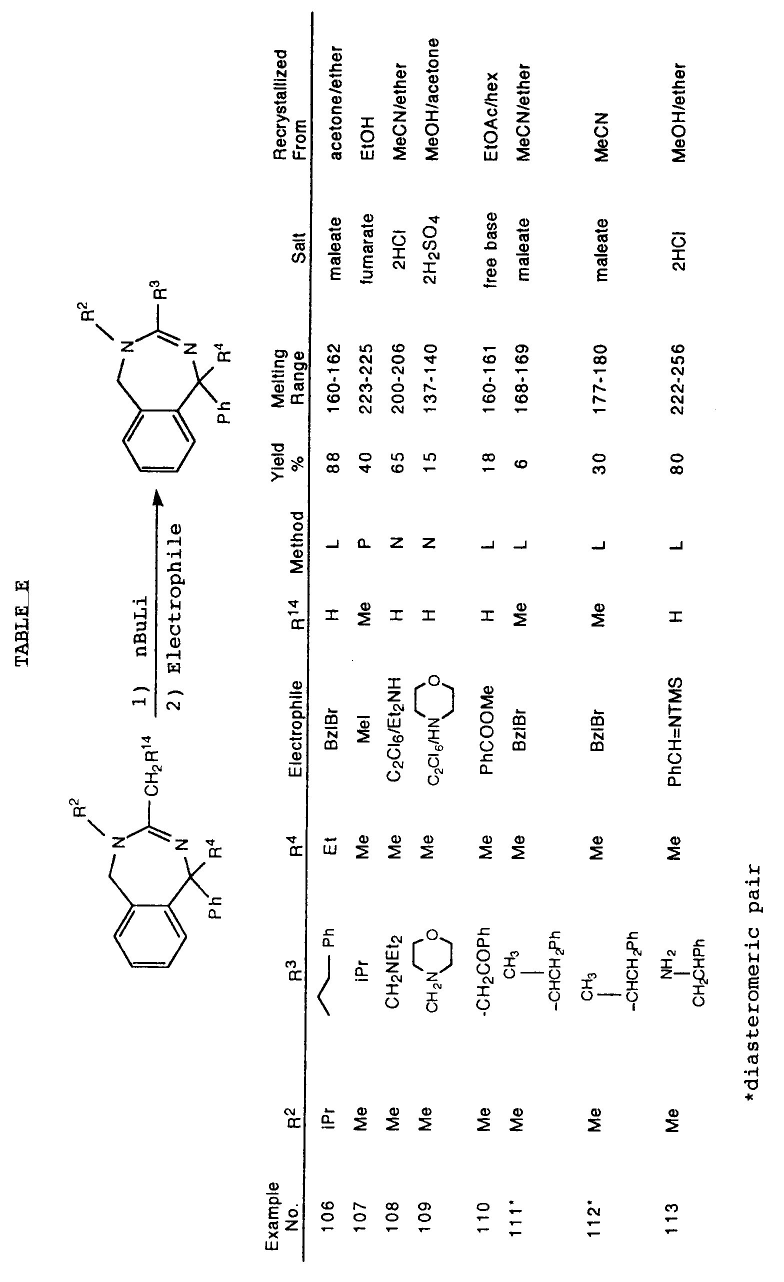

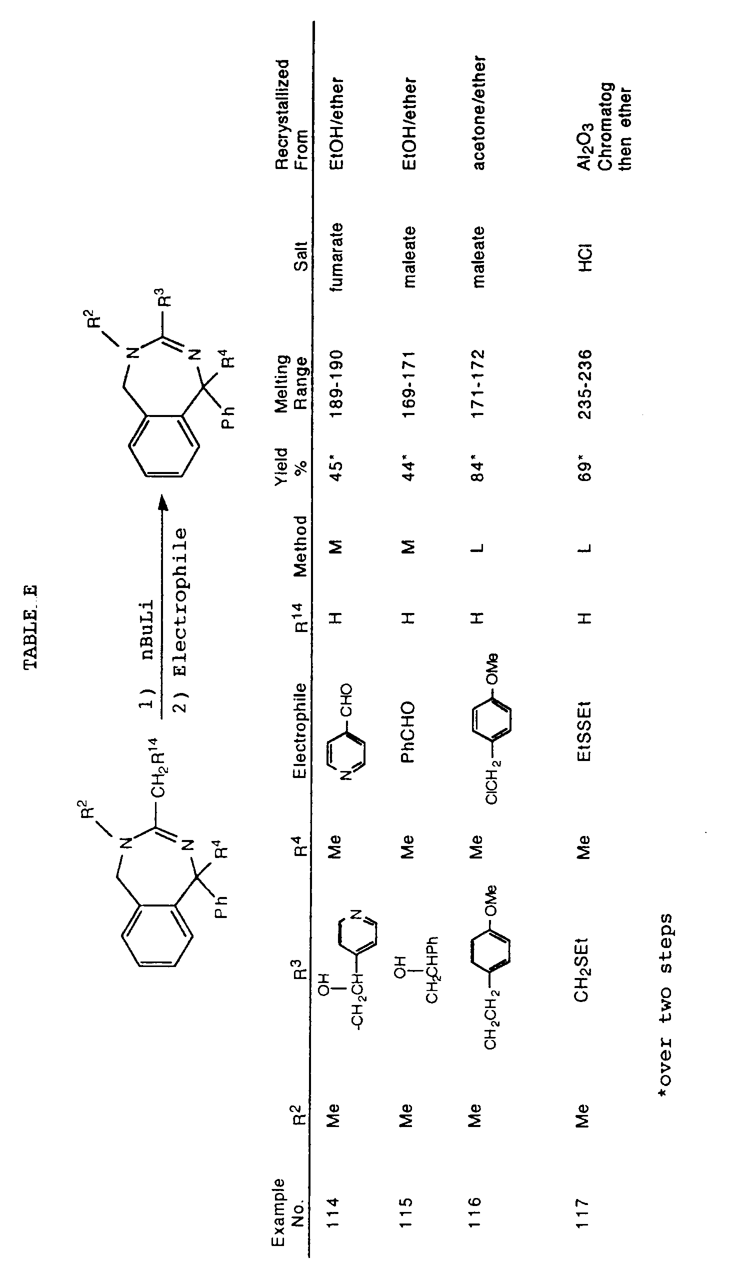

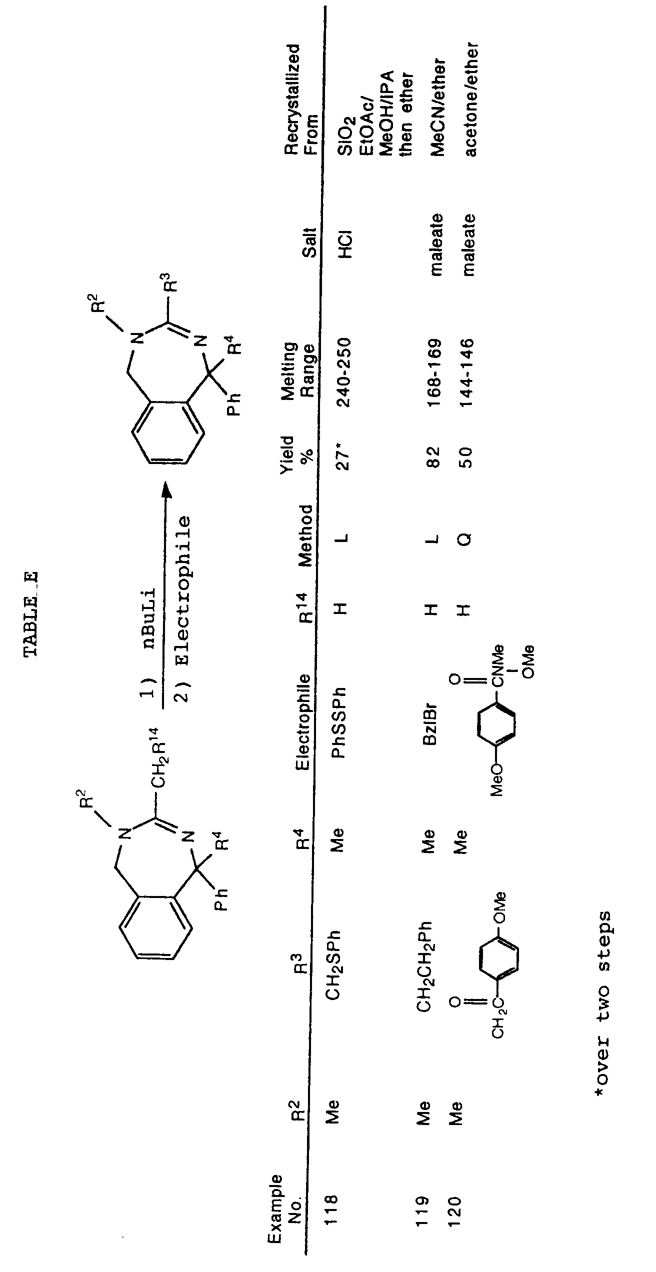

### General Method L

The benzodiazepine in 3-7 ml THF/mmol benzodiazepine was stirred at -78°C to -42°C while 1.1 equivalent of N-butyllithium was added under nitrogen. The solution was stirred for one hour at -78°C, 1.1-1.3 equivalents of the appropriate electrophile were added, and the reaction was allowed to come to room temperature. The reaction was poured into 1N HCl, washed with ether, made basic, and extracted into ether. The combined ether layers were dried over potassium carbonate, filtered and the solvent removed in vacuo. The free base was recrystallized or a salt was prepared as shown in Table D.

### General Method L1

The benzodiazepine in about 5.2 ml THF/mmol of benzodiazepine was stirred at -78°C while 1.05 equivalents of n-butyllithium was added under nitrogen. The mixture was stirred for 30 minutes at -78°C, then 1.25 equivalents of the appropriate electrophile was added and the reaction mixture was stirred at -78°C for 30 minutes. The reaction was quenched at -78°C with saturated ammonium chloride, warmed to room temperature and poured into a separatory funnel containing CH₂Cl₂/t-butylmethyl ether (2/1), brine and 2N NaOH. The organic layer was removed, washed with brine, dried over Na₂SO₄, filtered and concentrated in vacuo. The free base was purified by recrystallization as shown in Table D.

### Example 265

### 1-(4-Chlorophenyl)-3-[2-(4-chlorophenyl)ethyl]-4,5-dihydro-1,4-dimethyl-1H-2,4-benzodiazepine

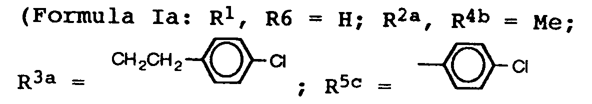

According to General Method L, 14.5 g 1-(4-chlorophenyl)-3-[2-(4-chlorophenyl)ethyl]-4,5-dihydro-1,4-dimethyl-1H-2,4-benzodiazepine as its fumarate salt was prepared from 17.3 g of the compound of Example 228 and 7.6 g methyl iodide. The salt was recrystallized from EtOH/ether, mp 173-175.

### General Method M

The procedure described under General Method L was followed except that two equivalents of N-butyllithium and two equivalents of aldehyde were used.

### General Method N

The benzodiazepine in 3-7 ml THF/mmol benzodiazepine was stirred at -78°C while 1.1 equivalents butyllithium was added under nitrogen. The solution was stirred for one hour at -78°C, 1.1-1.3 equivalents of hexachloroethane were added, and the reaction was stirred for one-half hour at -78°C. The reaction was poured into 1N HCl, washed three times with ether, made basic with 35% sodium hydroxide, extracted into ether, dried over potassium carbonate, filtered and stripped. The resulting brown oil was filtered through silica with ethyl acetate, stripped and taken directly to the next step. The 3-chloromethyl benzodiazepine was either dissolved in chloroform and treated with 3-5 equivalents of the appropriate amine or it was dissolved directly in a large excess of the amine. The solution was refluxed from 1-20 hours. The solvent was removed and the product was crystallized as shown in Table E.

### General Method O

The procedure was substantially similar to General Method L except that inverse addition of the lithium salt of the benzodiazepine was made to 1.5 equivalents of the chloroester.

### General Method P

The procedure was substantially similar to General Method L except that lithium diisopropylamide, generated from butyllithium and diisopropylamine, was used as the base and the reaction was run at 0°.

### General Method Q

The procedure was substantially similar to General Method L except that the reaction was quenched after stirring one hour at -55° by adding a slight excess of acetic acid in THF.

### General Method R

The benzodiazepine-3-one was dissolved in 13-14 equivalents phosphorus oxychloride and one-quarter equivalent of phosphorus pentoxide was added. The mixture was stirred at room temperature under nitrogen briefly, then heated at 90°C for 18 hours. The solution was stripped in vacuo, and the residue treated with 4-9 equivalents of the appropriate amine and stirred for two hours at room temperature. The excess amine was stripped in vacuo, and the residue was crystallized as shown in Table F.

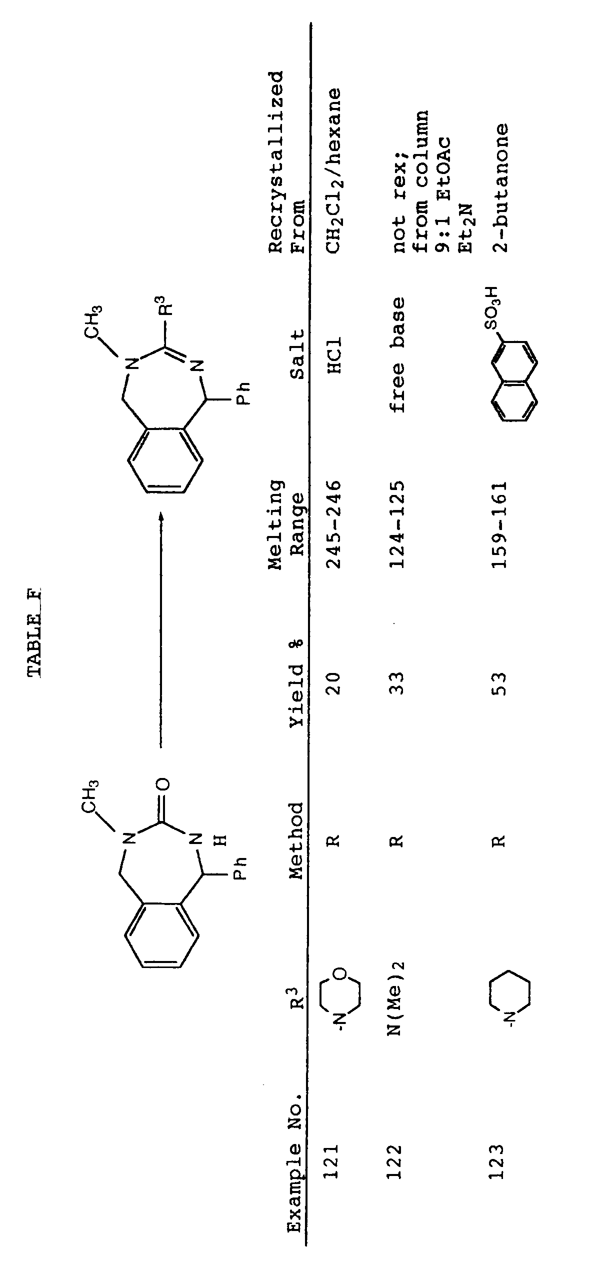

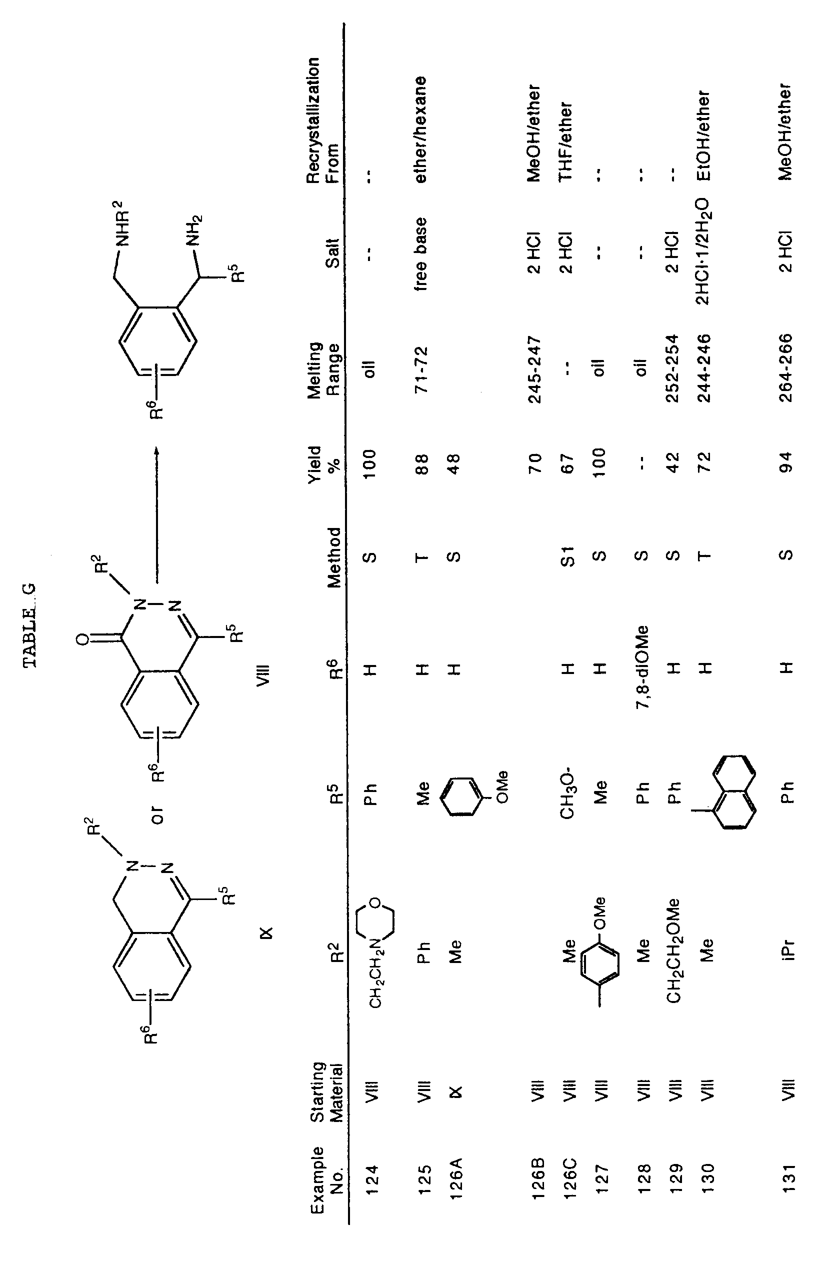

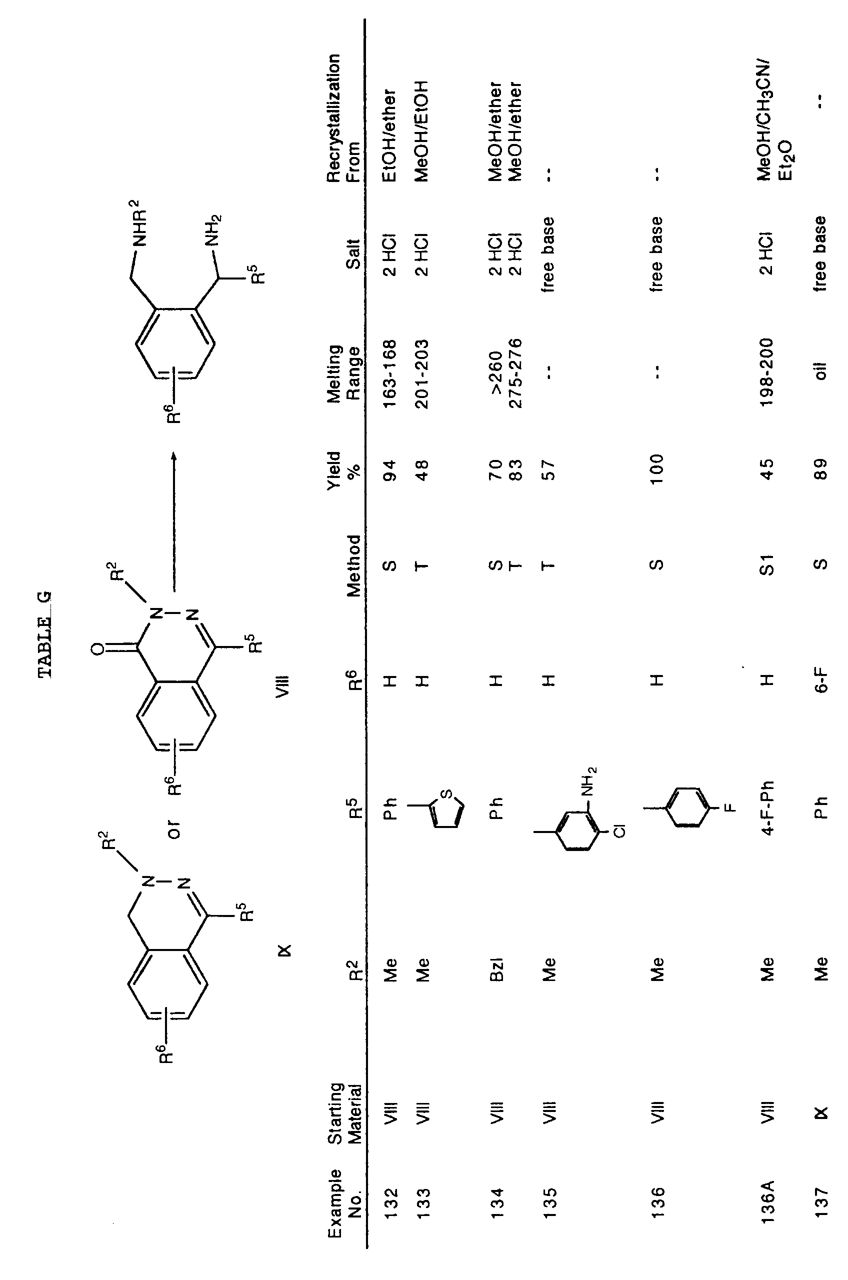

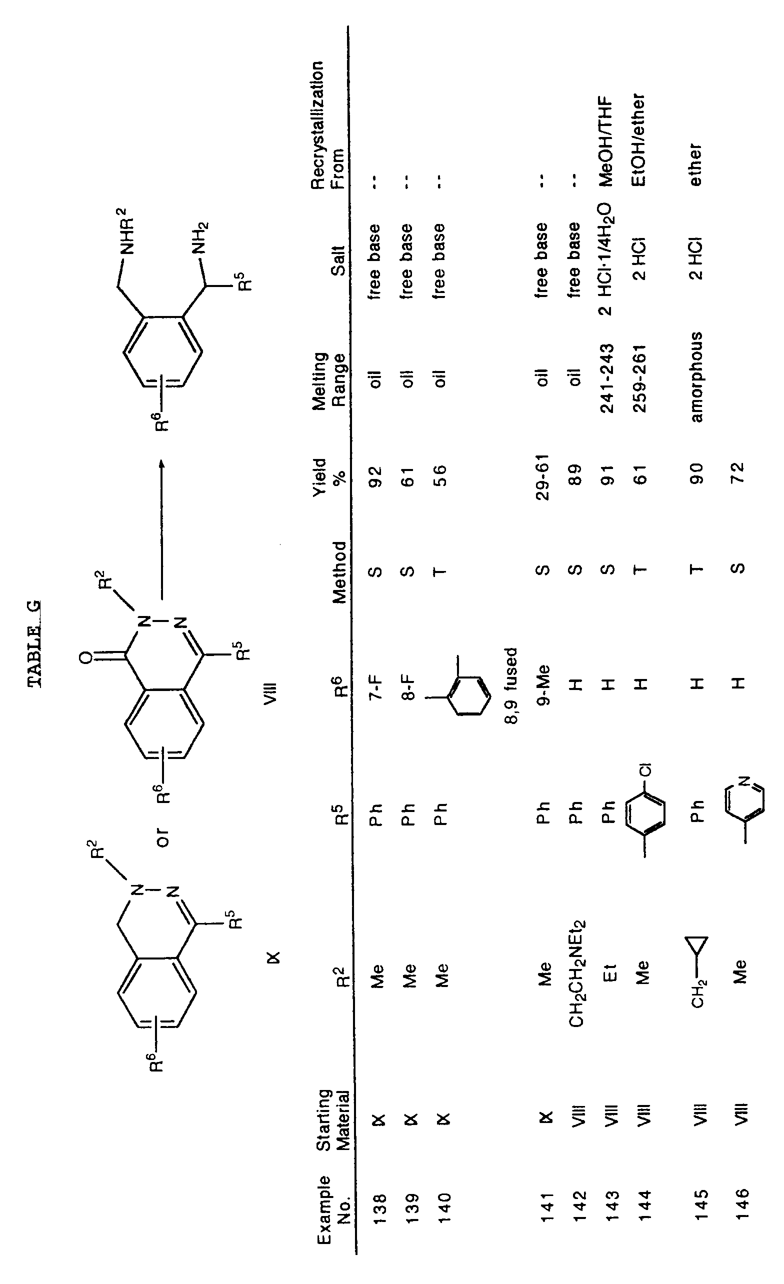

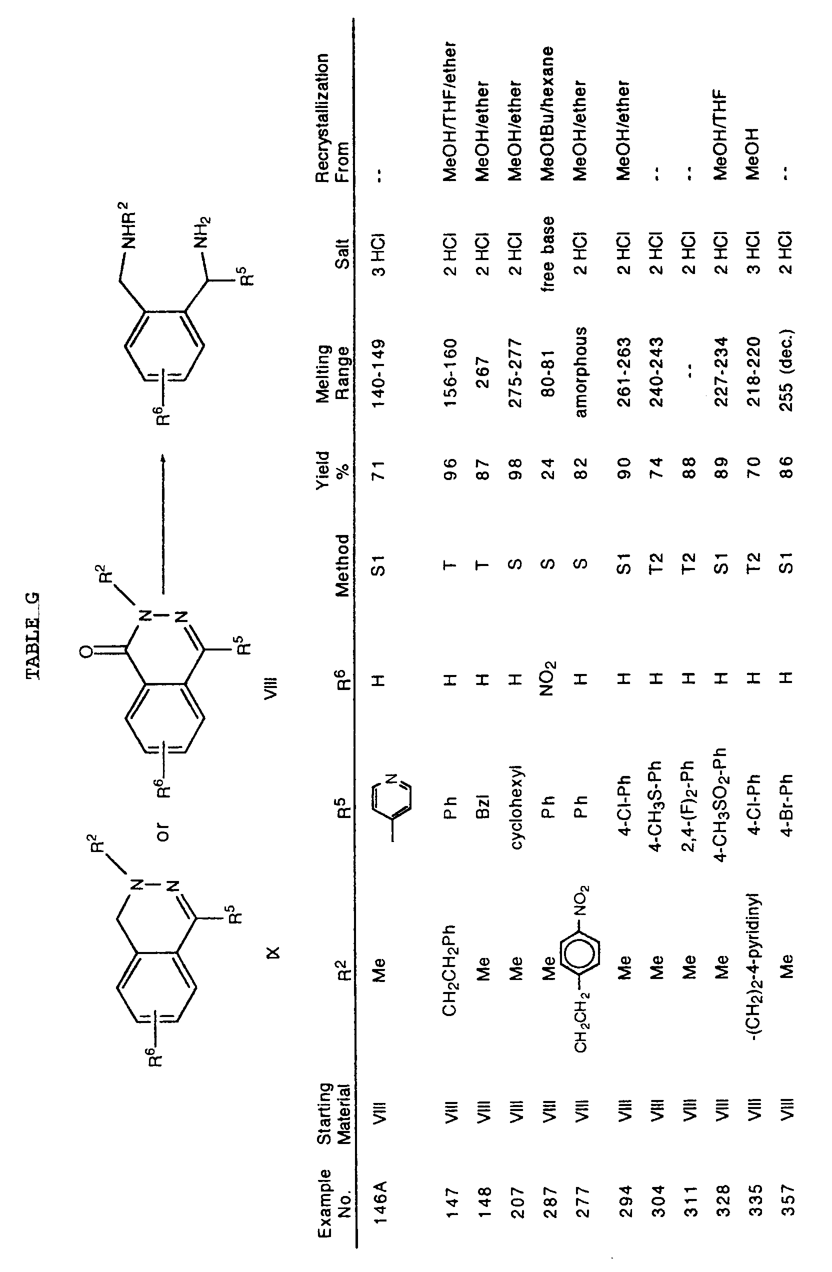

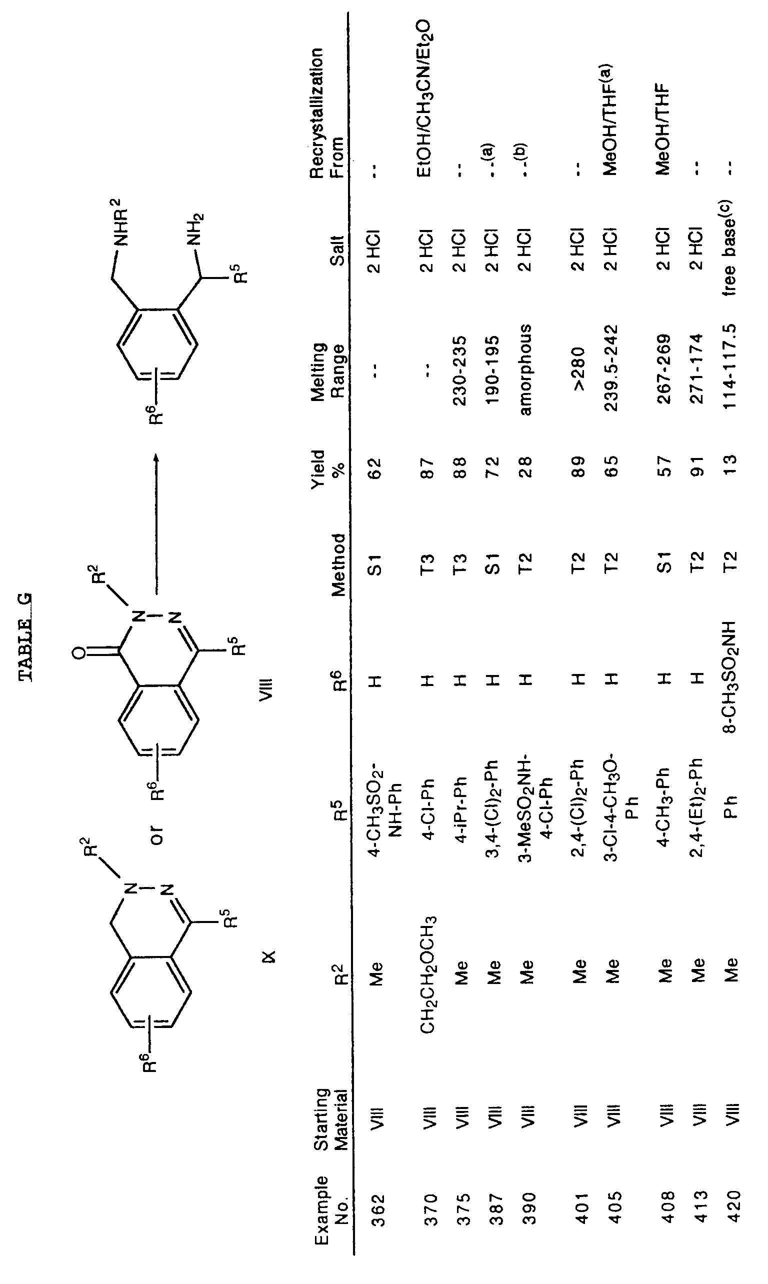

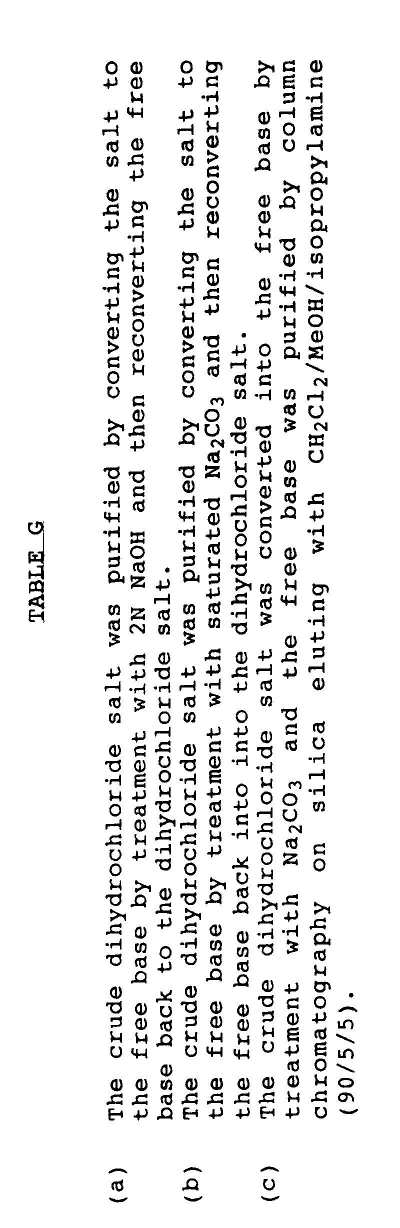

### General Method S

The appropriate phthalazine or phthalazinone was treated with 4-8 equivalents diborane in THF and the mixture was refluxed for 2-5 days under nitrogen. Usually the 4-8 equivalents of diborane were added in two or three portions over the course of the reaction. The reaction was allowed to cool to room temperature and excess aqueous or alcoholic hydrochloric acid was added carefully under nitrogen. The reaction was refluxed, the THF was removed in vacuo and the residue was made basic with 35% aqueous sodium hydroxide. The product was extracted into ethyl acetate, dried over sodium sulfate, concentrated in vacuo, and either purified as the hydrochloride salt as shown in Table G or, more commonly, used without further purification as the free base. In Table G, the Roman numeral IX indicates that the starting material was the corresponding phthalazine; the Roman numeral VIII indicates that the starting material was the corresponding phthalazinone.

### General Method S1

The appropriate phthalazinone in THF under N₂ was treated with 4-7.5 equivalents of a 1M borane THF solution and the mixture was refluxed for 2-9 days. Usually the 4-7.5 equivalents of borane were added in two portions over the course of the reaction. The reaction mixture was cooled to room temperature, quenched with methanol and refluxed for about 0-4 hours. The mixture was again cooled and saturated methanolic·HCl was added and the mixture was brought back to reflux for about 0-1.5 hours. The mixture was filtered and filtrate was concentrated in vacuo to afford the dihydrochloride salt which was purified by recrystallization as shown in Table G.

### General Method T

The procedure was substantially similar to General Method S except that 0.1-0.5 equivalent sodium borohydride and 0.7 to 1.5 ml diglyme/mmol of phthalazinone were added.

### General Method T2

The procedure was substantially similar to that described in General Method S1 except that 0.1-0.7 equivalents sodium borohydride and 0.2-7 ml diglyme/mmol of phthalazinone were added.

### General Method T3

The procedure was similar to that described in General Method S1 except that 0.05-0.08 equivalents of sodium borohydride and 0.5-0.6 ml diglyme/mmol of phthalazinone were added, and the mixture was refluxed for 0-4 hours after the addition of the methanolic·HCl.

### Example 149

### 4,5-Dihydro-3-ethyl-4-methyl-1-phenylmethyl-1H-2,4-benzodiazepine (Formula I: R¹, R⁴, R⁶ = H; R² = Me; R³ = Et; R⁵ = Bzl)

A solution of 12.5 g (50 mmol) of 2-[(1-amino-2-phenyl)ethyl]-N-methylbenzenemethanamine in 150 ml isopropyl acetate was treated with 4.1 g (50 mmol) sodium acetate and 30 ml(150 mmol) triethylorthopropionate and 5 ml (87 mmol) acetic acid. The mixture was refluxed for three hours and poured into 1.5l ice water containing 200 ml 2N sodium hydroxide. The product was extracted into ethyl acetate, dried over sodium sulfate and stripped. The residue was recrystallized from isopropyl alcohol/ether to yield 7.5 g of the free base. The free base in ethanol was treated with 4.6 g cyclohexane sulfamic acid and the solvent removed in vacuo. The residue was recrystallized from isopropyl alcohol/ether to provide 5.8 g of product as the cyclohexane sulfamic acid salt, mp 137-138.

### Example 150

### 4,5-Dihydro 3-ethyl-1-phenyl-1H-2,4-benzodiazepine (Formula I: R¹, R², R⁴, R⁶ = H; R³ = Et; R⁵ = Ph)

A mixture of 1.36 g (3.6 mmol) 4-benzyl-4,5-dihydro-3-ethyl-1-phenyl-1H-2,4-benzodiazepine, 136 mg 10% palladium on carbon, and 257 mg (4.0 mmol) ammonium formate in 50 ml methanol was refluxed under nitrogen for three hours. Four more 230-mg portions of ammonium acetate were added every two hours during reflux until TLC on silica gel with 5% diethylamine in ethyl acetate showed a complete conversion. The reaction was cooled, filtered and stripped. The residue was distributed between aqueous sodium hydroxide and ether. The ether extract was dried over sodium sulfate, treated with decolorizing carbon, filtered and stripped. The residue was taken up in 60:40 ethyl acetate/ether and acidified with dilute ethereal HCl. The resulting precipitate was filtered off and recrystallized from isopropanol/ether to yield 0.61 g (61%) of the hydrochloride salt of the product, mp 203-204.

### Example 151

### 4,5-Dihydro-4-methyl-1-phenyl-1H-2,4-benzodiazepin-3-amine monohydrochloride (Formula I: R¹, R⁴, R⁶ = H; R² = Me; R³ = NH₂; R⁵ = Ph)

A solution of 15 g (66 mmol) 2-[(methylamino)methyl]-α-phenylbenzenemethanamine in 85 ml methanol was treated with 7.2 g (68 mmol) cyanogen bromide at room temperature. The solution was stirred at room temperature for 18 hours and stripped. The residue was dissolved in ethanol and the ethanol stripped off. The residue was recrystallized from methanol/isopropyl acetate to yield 4.55 g of the free base, mp 156-159. The mother liquors were dissolved in ethanol, treated with a slight excess of ethanolic HCl and recrystallized from ethanol to yield 1.3 g of the hydrochloride salt, mp 259-261.

### Example 152

### 1,2,4,5-Tetrahydro-4-methyl-1-phenyl-3H-2,4-benzodiazepin-3-thione

To a suspension of 15 g (50 mmol) 2-[(methylamino)methyl]-α-phenylbenzenemethanamine dihydrochoride in 100 ml isopropyl alcohol was added 10 g (100 mmol) potassium acetate followed by 3.3 ml (55 mmol) carbon disulfide in 35 ml isopropyl alcohol. The suspension was stirred at room temperature for one and one-half hours and then refluxed for 30 minutes. The reaction was chilled in ice and the internal salt of the carbamodithioic acid, contaminated with two equivalents of of potassium chloride, was filtered off. The carbamodithioic acid was suspended in 125 ml 95% ethanol, and 1.3 ml 12N hydrochloric acid was added. The suspension was refluxed for three days, cooled, and 15.3 g (114%) of the crude benzodiazepin-3-thione was filtered off. A 6-g portion of the crude product was recrystallized from 2-ethoxy ethanol to yield 2.0 g (38%) of product, mp 208-209.

### Example 153

### 3-[[2-(Diethylamino)ethyl]amino]-4,5-dihydro-4-methyl-1-phenyl-1H-2,4-benzodiazepine [Formula I: R¹, R⁴, R⁶ = H; R² = Me; R³ = NH(CH₂)₂N(C₂H₅)₂]

A slurry of 11.7 g (44 mmol) 4-methyl 1-phenyl-1,2,4,5-tetrahydro-3H-2,4-benzodiazepin-3-thione of Example 152 in 146 ml ethanol was treated with 4.2 ml (67 mmol) iodomethane in 30 ml ethanol added dropwise at 50°. The reaction was stirred at ambient temperature for 18 hours and 13.48 g (75%) of 4-methyl-1-phenyl-3-methylthio-4,5-dihydro-1H-2,4-benzodiazepine was collected, mp 201-205, as the hydriodide salt.

A solution of 22.7 g (55 mmol) of the 3-methylthiobenzodiazepine in 285 ml methanol was refluxed with 7.8 ml (55 mmol) N,N-diethylethylenediamine for 18 hours. The reaction was filtered hot to remove a small amount of insoluble impurity, cooled, stripped, and distributed between methylene chloride and aqueous sodium hydroxide. The organic extracts were dried over magnesium sulfate and stripped. The residue was recrystallized with great difficulty as the fumarate salt from isopropanol. After multiple recrystallizations 1.5 g of the product was obtained as the difumarate hemihydrate, mp 160-162.

### Example 154

### 4,5-Dihydro-4-methyl-1-phenyl-1H-2,4-benzodiazepin-3-sulfonic acid (Formula I: R¹, R⁴, R⁶ = H; R² = Me; R³ = SO₃H; R⁵ = Ph)

Twenty-nine grams (108 mmol) 1,2,4,5-tetrahydro-4-methyl-1-phenyl-3H-2,4-benzodiazepin-3-thione of Example 152 was treated with 2.4 g sodium chloride, 420 mg sodium molybdate dihydrate and 35 ml 30% hydrogen peroxide in 50 ml water and 10 ml t-butanol according to the procedure of Maryanoff et al., J.O.C. 51, 1882 (1986). The reaction remained a suspension at all times, and after heating at 70-80° for two hours, the product was filtered off from the chilled suspension to yield 30.6 g (90%) of the sulfonic acid requiring no further purification, mp 188-190.

### Example 155

### 4,5-Dihydro-4-methyl-1-phenyl-3(1-pyrrolidino)-1H-2,4-benzodiazepine (Formula I: R¹, R⁴, R⁶ = H; R² = Me; R³ = C₄H₈N; R⁵ = Ph)

A mixture of 4.75 g (15 mmol) of the sulfonic acid of Example 154 and 20 ml pyrrolidine was refluxed for 18 hours. The pyrrolidine was stripped off and the residue was chromatographed on 340 g of silica gel, eluting with 95:5 ethyl acetate/diethylamine to yield 3.12 g of residue which was recrystallized from 40 ml hexane to yield 2.14 g (47%) of product, mp 118-119.

### Example 156

### 3-[(4,5-Dihydro-4-methyl-1-phenyl-1H-2,4-benzodiazepin-3-yl)thio]-N,N-diethylpropaneamine (Formula I: R¹, R⁴, R⁶ = H; R² = Me; R³ = S(CH₂)₃N(C₂H₅)₂; R⁵ = Ph)

A solution of of 12 g (45 mmol) 1,2,4,5-tetrahydro-4-methyl-1-phenyl-3H-2,4-benzodiazepin-3-thione of Example 152 in 100 ml DMF was treated with 1.24 g (50 mmol) sodium hydride at 70° and 7.5 g (50 mmol) 3-diethylaminopropyl chloride was added dropwise at 70°. The reaction was stirred at 70° for five hours and then at room temperature for two days. The reaction was poured into 250 ml ice water and extracted twice into ethyl acetate. The product was extracted into 150 ml 2N HCl, washed with ethyl acetate, made basic, and extracted back into ethyl acetate. The ethyl acetate solution was dried over magnesium sulfate, stripped, and the residue was dissolved in acetone. Two equivalents of maleic acid in 40 ml acetone was added, followed by a small amount of ether. The resulting precipitate was recrystallized from acetone/ether to provide 13.2 g of product as the dimaleate salt, mp 95-97.

### Example 157

### 4,5-Dihydro-4-methyl-3-methylthio-1-phenyl-1H-2,4-benzodiazepine (Formula I: R¹, R⁴, R⁶ = H; R² = Me; R³ = SMe; R⁵ = Ph)

A solution of 8 g (30 mmol) of the thione of Example 152 and 2.7 ml (44 mmol) methyl iodide in 100 ml ethanol was refluxed two hours, cooled, and the hydriodide of the product filtered off. The salt was partitioned between methylene chloride and aqueous sodium bicarbonate, the organic layer dried over magnesium sulfate, and stripped. The residue was dissolved in ethanol and 2.7 g methanesulfonic acid was added followed by ether. The resulting precipitate was filtered off and recrystallized from ethanol to yield 5.2 g of product as the methanesulfonate salt, mp 195-196.

### Example 158

### 1,2,4,5-Tetrahydro-4-methyl-1-phenyl-3H-2,4-benzodiazepin-3-one

A solution of 32.8 g (145 mmol) 2-[(methylamino)methyl]-α-phenylbenzenemethanamine in 215 ml chloroform was treated with 25.9 g (159 mmol) carbonyldiimidazole. The reaction was stirred at room temperature for 19 hours, washed four times with water, dried over sodium sulfate and stripped in vacuo. The gummy residue was triturated in and recrystallized from ethyl acetate to yield 26 g (71%) of product, mp 198-199.

### Example 159

### 5-Butyl-4,5-dihydro-3-ethyl-4-methyl-1-phenyl-1H-2,4-benzodiazepine (Formula I: R¹ = nBu; R² = Me; R³ = Et; R⁴ and R⁶ = H, R⁵ =Ph)

A suspension of 14.16 g (60 mmol) 2-methyl-4-phenyl-1(2H)-phthalazinone in 340 ml THF was cooled to -65° under nitrogen and treated with 24.8 ml (62 mmol) 2.5N n-butyllithium in hexane. The mixture was stirred for 20 minutes at -65°, and 240 ml (240 mmol) 1N borane-THF complex was added. The solution was allowed come to room temperature and 340 mg (9 mmol) sodium borohydride were added. The reaction was refluxed for 20 hours, another 340 mg sodium borohydride was added, the reaction refluxed another 24 hours. The reaction was cooled and quenched with 100 ml methanol. 80ml of 3.5N HCl in methanol was added, the reaction was refluxed two hours and 19.9 g (93%) of the dihydrochloride of 2-[1-(methylamino)pentyl]-α-phenyl-benzenemethanamine was isolated by filtration. The benzenemethanamine was treated with triethylorthopropionate and sodium acetate in isopropyl acetate according to General Method E to yield 9.48 g of the free base of the product, mp 102-114 after recrystallization from methyl t-butyl ether/hexane. Seven grams of the free base was converted to the hydrochoride salt and recrystallized from acetone ether to yield 5.08 g of product as the monohydrochloride salt, mp 209-211.

### Example 160

### 4,5-Dihydro-1,5-diphenyl-3-ethyl-4-methyl-1H-2,4-benzodiazepine (Formula I: R¹, R⁵ = Ph; R² = Me; R³ = Et; R⁴, R⁶ = H)

The procedure of Example 159 was used, substituting phenyllithium for butyllithium. The intermediate 2-[(methylamino)phenylmethyl]-α-phenylbenzenemethanamine was crystallized as the dihydrochloride salt containing 0.6 moles water of hydration, mp 202-216. It was cyclized with triethylorthopropionate as in Example 158 to yield 32% of the product as the hydrochloride salt, mp 275-276, from acetone/ether.

### Example 266

### 4,5-Dihydro-1-(4-hydroxyphenyl)-4-methyl-3-(2-phenylethyl)-1H-2,4-benzodiazepine

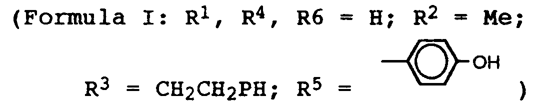

A solution of 6.78 g (17 mmol) of the methoxy compound of example 60 in 70 ml methylene chloride was treated with 32 ml 1M boron tribromide in methylene chloride (32 mmol) at 0° under nitrogen for 2 hours. The reaction was poured into 2N aqueous HCl, stirred 1 hour, filtered free of boron salts and extracted into methylene chloride with a trace of methanol after making basic with Na₂CO₃. The organic layer was dried, stripped and the residue taken up in methanol. Methanolic HCl was added and the salt crystallized by the addition of ether. The hydrochloride was recrystallized from methanol, mp 245-247, yield 90%.

### Example 267

### 4,5-Dihydro-3-[2-(4-hydroxyphenyl)ethyl]-4-methyl-1-phenyl-1H-2,4-benzodiazepine

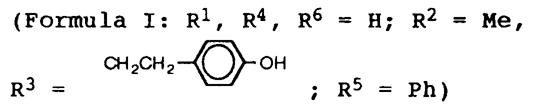

By a process analogous to that of Example 266, 1.14 g of 4,5-dihydro-3-[2-(4-hydroxyphenyl)ethyl]-4-methyl-1-phenyl-1H-2,4-benzodiazepine was obtained as the hydrochloride salt from 2.47g (6.1 mmol) of the methoxy compound of Example 35, mp 160-162 from methanol/ether.

### Example 268

### 4,5-Dihydro-1-(4-hydroxyphenyl)-3-[2-(4-hydroxyphenyl)ethyl]-4-methyl-1H-2,4-benzodiazepine

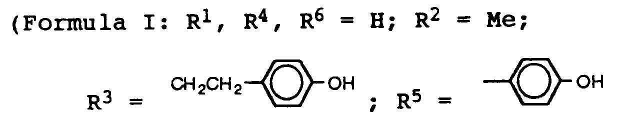

By a process analogous to that of Example 266, 1.80 g of 4,5-dihydro-1-(4-hydroxyphenyl)-3-[2-(4-hydroxyphenyl)ethyl]-4-methyl-1H-2,4-benzodiazepine was obtained from 4.5 g (8.7 mmol) of the dimethoxy compound of example 229 using 3.5 equivalents of boron tribromide. The free base was insoluble in methylene chloride. The hydrochloride hemihydrate was obtained by recrystallization from MeCN/MeOH, mp 266-228.

### Resolution of enantiomers

### Example 167

### (R)-(+)-4,5-Dihydro-4-methyl-1-phenyl-3-(2-phenylethyl)-1H-2,4-benzodiazepine

To a 1 l Erlenmeyer flask was added 100 ml methanol, 200 ml water and 49.8 g (0.133 mol) of the racemic hydrochloride salt of Example 25. The solution was stirred for ten minutes, then 200 ml t-butylmethyl ether (TBME) added to the homogeneous solution followed by 220 ml (0.66 mol, 5.0 eq) 3N sodium hydroxide. The mixture was stirred for 10 minutes. The layers were separated and the aqueous layer extracted with 100 ml saturated sodium chloride. The organic layer was dried over magnesium sulfate, filtered and the solvent removed under reduced pressure at 30°C to give a quantitative yield of free base. The viscous golden-brown oil was placed on a vacuum pump at 0.5 mmHg for 1 hour.

The free base was dissolved into 40 ml methanol with slight warming. The solution was transferred to a 500 ml 3 neck flask equipped with mechanical stirrer and condenser. The transfer was completed by rinsing with an additional 20 ml methanol. The methanol solution was warmed to 45°C with an external temperature-controlled water bath.

To a 250 ml beaker was added 40.4 g (0.113 mol, 0.85 eq) D-O,O'-dibenzoyltartaric acid and 40 ml methanol. (Slight warming may be necessary to get chiral acid into solution.) The methanol solution of the chiral acid was added slowly with constant stirring to the solution of free base. The resulting mixture became a very light green color. An additional 20 ml methanol was used for rinsing to complete the transfer. After stirring 5 minutes, the solution was seeded. The product began to precipitate immediately. The solution was stirred overnight at 45°C. The granular, white precipitate was collected on a Buchner funnel, washed 3 x 25 ml with cold methanol (5°C) and dried overnight at 60°C under reduced pressure.

The dried dibenzoyltartrate salt weighed 40.9 g (88%) after correcting for the quantity of seed crystal; [α]_{D}²⁵ = +192.(c=1, methanol); mp 143-145°C dec.

The hydrochloride salt may be made by the following procedure:
The free base from 100 g (0.143 mol) dibenzoyltartrate salt was prepared as above. The free base was dissolved into 300 ml ethyl acetate. The solution was transferred to a 2-l, 3-neck flask equipped with mechanical stirrer, condenser and additional funnel. The transfer was completed by rinsing with an additional 300 ml ethyl acetate. The ethyl acetate solution was warmed to 45°C with a temperature-controlled water bath.

To the warmed ethyl acetate solution of the free base was slowly added 69 ml 2.3N hydrogen chloride/ethyl acetate solution. The addition of the acid was completed over a 0.5 h period, followed by stirring at 45°C for 1 hour. The ethyl acetate suspension of the resulting hydrochloride salt was refluxed for 1 hour to eliminate the excess hydrogen chloride present in the solvent and cooled to ambient temperature. The flocculent white precipitate was collected on a Buchner funnel and washed 3 x 150 ml with ethyl acetate. The product was dried overnight at 80°C under reduced pressure.

The dried hydrochloride salt weighed 50.9 g, [α]_{D}²⁵ = +234° (c=1, methanol); mp 197-199°C.

### Example 168

### (S)-(-)-4,5-Dihydro-4-methyl-1-phenyl-3-(2-phenylethyl)-1H-2,4-benzodiazepine

The mother liquors from cyrstallization in Example 167 were stripped and the free base liberated as before using tBuOMe and aqueous NaOH. The free base was dissolved in 100 ml methanol, treated with 34.3 g dibenzoyl-L-tartaric acid and seeded. There was obtained 37.2 g of the diastereomeric salt of the (-) isomer, mp 160-170, [α]_{D}²⁵ -198°(C=1, MeOH). The free base was generated as above, and the HCl salt was formed and recrystallized from acetonitrile/ether, mp 198-199, [α]_{D}²⁵ = -249° (C=1, CHCl₃).

### Example 169

### (+)-4,5-Dihydro-1-phenyl-1,3,4-trimethyl-1H-2,4-benzodiazepine

By a process analogous to that of Example 167 involving multiple recrystallizations, 1.4 g of (+)-4,5-dihydro-1-phenyl-1,3,4-trimethyl-1H-2,4-benzodiazepine was obtained from 8.9 g (33.7 mmol) of the racemic product of Example 96 and 12.7 g (33.7 mmol) of dibenzoyl-L-tartaric acid hydrate. The free base was obtained, without recrystallization, by stripping the tBuOMe, mp 115-116, [α]_{D}²⁵ = +101° (C=1, MeOH).

### Example 170

### (-)-4,5-Dihydro-1-phenyl-1,3,4-trimethyl-1H-2,4-benzodiazepine

By a process analogous to that of Example 168, 1.9 g (-)-4,5-dihydro-1-phenyl-1,3,4-trimethyl-1H-2,4-benzodiazepine was obtained from the mother liquors of Example 169, mp 116-117, [α]_{D}²⁵ = -93° (C=1, MeOH).

### Example 171

### (R)-(+)-4,5-Dihydro-3-ethyl-4-methyl-1-phenyl-1H-2,4-benzodiazepine

By a process analogous to that of Example 167, 7.5 g R-(+)-4,5-dihydro-3-ethyl-4-methyl-1-phenyl-1H-2,4-benzodiazepine was obtained from 92 g of the free base of the racemic product of Example 8, after repeated crystallization. The hydrochloride salt was obtained from ethanol/ether, mp 244-247, [α]_{D}²⁵ = +347° (C=1, CHCl₃).

The d-10-camphorsulfonic acid salt was obtained from acetonitrile, mp 215-218, [α]_{D}²⁵ = + 203° (C=1, MeOH), [α]_{D}²⁵ = +242° (C=1, CHCl₃).

### Example 172

### (S-)(-)-4,5-Dihydro-3-ethyl-4-methyl-1-phenyl-1H-2,4-benzodiazepine

By a process analogous to that of Example 168, 15 g of the levo enantiomer was obtained from the mother liquors of Example 171. The product was crystallized as the hydrochloride from ethanol/ether, mp 247-249, [α]_{D}²⁵ = -343° (C=1, CHCl₃).

### Example 173

### (S)-(-)4,5-Dihydro-3-ethyl-4-methyl-1-phenyl-1H-2,4-benzodiazepine

The following procedure describes an alternate synthesis of the compound of Example 172:
Two grams (6.3 mmol) of the monohydrochloride of (S)-N-[[[2-(methylamino)methyl]phenyl]phenylmethyl]propanamide (Example 181) was stirred in 15 ml toluene under nitrogen and 3.45 ml 2M trimethyl aluminum in toluene was added at 0°. The mixture was stirred two hours at room temperature, then 1.5 hours at reflux. The reaction was cooled and quenched with 0.31 ml water followed by 0.93 ml 30% aqueous NaOH. Methylene chloride, a small amount of methanol and some sodium sulfate were added, the mixture was filtered, stripped and the residue recrystallized from MeOH/ether as the hydrochloride, mp 247-248.

### Example 296

### (+)-4,5-Dihydro-4-methyl-3-(4-chlorophenyl)-3-methyl-1H-2,4-benzodiazepine

A solution of the racemic free base of Example 295 (14.007 g) in ethanol was heated on a steam bath and a solution of dibenzoyl-L-tartaric acid (17.80 g) in hot ethanol (500 ml) was added. The mixture was cooled to room temperature and the crystals that formed were collected by filtration (mother liquor used in Example 297) and dried at 80°C in vacuo. The salt was recrystallized from methanol and dried at 60°C in vacuo to afford 13.53 g of the dibenzoyl-L-tartaric acid salt, m.p. 160.5-161.5°C, [α]_{D}²⁵= +94.7° (C=0.61, MeOH). The salt was then converted into the free base of the (+)-isomer by dissolving the salt in a mixture of 1N NaOH, ether, CH₂Cl₂ and methanol, separating the organic layer, washing the organic layer with brine, drying the organic layer over Na₂SO₄ and removing the solvent in vacuo. The free base was purified by Kugelrohr distillation, followed by recrystallization from ethyl acetate/hexane to afford 5.35 g of the free base of the (+)-isomer, m.p. 138-139°C, [α]_{D}²⁵= +341° (C=1.0, EtOH).

### Example 297

### (-)-4,5-Dihydro-4-methyl-1-(4-chlorophenyl)-3-methyl-1H-2,4-benzodiazepine

The mother liquor from the initial crystallization of the dibenzoyl-L-tartaric acid salt of Example 296 was concentrated in vacuo and the residue was taken up in ethanol/t-butylmethyl ether and treated with 1N NaOH. The organic layer was separated, washed with brine, dried over Na₂SO₄ and concentrated in vacuo to regenerate the free base. The free base was dissolved in ethanol (300 ml), heated on a steam bath, and treated with dibenzoyl-D-tartaric acid monohydrate (12.75 g) in hot ethanol (200 ml). The crystals which formed were collected by filtration and dried at 60°C in vacuo to afford 15.03 g of the dibenzoyl-D-tartaric acid salt, m.p. 162-163°C, [α]_{D}²⁵= -87.3° (C=0.565, MeOH). The dibenzoyl-D-tartaric acid salt was then converted to the free base of the (-)-isomer as described above in Example 296 to afford 6.085 g free base of the (-)-isomer, m.p. 137-138.5°C, [α]_{D}²⁵= -335° (C=1.00, EtOH).

### Example 307

### (+)-4,5-Dihydro-3,4-dimethyl-1-(4-methylthiophenyl)-1H-2,4-benzodiazepine

The racemic free base of Example 306 (7.45 g, 25 mmol) was dissolved in ethanol (50 ml) and treated with a solution of dibenzoyl-L-tartaric acid (9.28 g, 25.9 mmol) in hot ethanol (100 ml). The precipitate which formed was collected by filtration, washed with ethanol and then t-butylmethyl ether (combined filtrates used in Example 308) and the salt was recrystallized from methanol to afford 4.60 g of the dibenzoyl-L-tartaric acid salt, m.p. 164.5-165.5°C, [α]_{D}²⁵= +101° (C=0.385, MeOH). The dibenzoyl-L-tartaric acid salt (3.92 g, 6 mmol) was then mixed with 2N NaOH (7 ml, 14 mmol), water (10 ml) and CH₂Cl₂ (20 ml) to afford a two-phase mixture. The organic phase was separated, the aqueous phase was extracted with CH₂Cl₂ and the combined organic extracts were washed with water containing a small amount of a saturated Na₂CO₃ solution. The organic layer was dried over Na₂SO₄ and the solvent was removed in vacuo to afford an oil which was crystallized from t-butylmethyl ether to afford 1.57 g of (+)-4,5-dihydro-3,4-dimethyl-1-(4-methylthiophenyl)-1H-2,4-benzodiazepine, m.p. 133-134°C, [α]_{D}²⁵= +352.3° (C=0.01095, EtOH).

### Example 308

### (-)-4,5-Dihydro-3,4-dimethyl-1-(4-methylthiophenyl-1H-2,4-benzodiazepine

The mother liquors from the initial crystallization of the dibenzoyl-L-tartaric acid salt were concentrated in vacuo, dissolved in CH₂Cl₂, and basified with 1N NaOH (50 ml). The aqueous phase was extracted with CH₂Cl₂ and the combined organic layers were washed with water containing 2N NaOH (1 ml) and dried over Na₂SO₄. The solvent was removed in vacuo to afford 4.1 g of the free base. The free base (3.4 g, 11.5 mmol) was dissolved in ethanol (25 ml), and added to a solution of dibenzoyl-D-tartaric acid in hot ethanol (25 ml). The precipitate which formed was collected by filtration, washed with ethanol and then t-butylmethyl ether and dried in vacuo at 80°C. The salt was then recrystallized from methanol to afford 3.13 g of the dibenzoyl-D-tartaric acid salt, m.p. 169.5-170°C, [α]_{D}²⁵= -100.5° (C=0.0439, MeOH). The dibenzoyl-D-tartaric acid salt was then converted into the free base as described hereinabove in Example 307 to afford (-)-4,5-dihydro-3,4-dimethyl-1-(4-methylthiophenyl)-1H-2,4-benzodiazepine, m.p. 134-135.5°C, [α]_{D}²⁵= -341.1° (C=0.00455, EtOH).

### Example 314

### (+)-4,5-Dihydro-3,4-dimethyl-1-(2,4-difluorophenyl)-1H-2,4-benzodiazepine

Following a procedure substantially similar to that described in Example 307 except that methanol was used as the solvent rather than ethanol, there was obtained 26.5 g of the dibenzoyl-L-tartaric acid salt, [α]_{D}²⁵= +32.8° (C=0.119, MeOH), after recrystallization of the salt from methanol, from the racemic free base of Example 313 (25.70 g, 0.0898 mol) and dibenzoyl-L-tartaric acid (33.46 g, 0.0924 mol). The benzoyl -L-tartaric acid salt (18.0 g) was converted into the free base as described in Example 307 to afford 7.94 g of (+)-4,5-dihydro-3,4-dimethyl-1-(2,4-difluorophenyl)-1H-2,4-benzodiazepine, m.p. 137-138°C, [α]_{D}²⁵= +236° (C=1.025, EtOH).

### Example 315

### (-)-4,5-Dihydro-3,4-dimethyl-1-(2,4-difluorophenyl)-1H-2,4-benzodiazepine

The mother liquors from Example 314 were concentrated in vacuo and the residue was treated with CH₂Cl₂ (400 ml), H₂O (300 ml) and 2N NaOH (45 ml). The organic layer was separated, washed with water and dried over Na₂SO₄. Removal of the solvent in vacuo and purification of the residue by recrystallization from ethyl acetate, dissolving the solid in ether and washing with 0.5N HCl, and recrystallization from tert-butylmethyl ether (3X) affords the free base (10.5 g) of racemic 4,5-dihydro-3,4-dimethyl-1-(2,4-difluorophenyl)-1H-2,4-benzodiazepine. The racemic free base (10.5 g, 39.1 mmol) was mixed with dibenzoyl-D-tartaric acid (14.4 g, 40.3 mmol) and hot methanol (2.5 l). The precipitate which formed was collected by filtration and was recrystallized from methanol (6X) to afford 5.45 g of the dibenzoyl-D-tartaric acid salt, [α]_{D}²⁵= -25° (C=0.138, MeOH). An additional 2.90 g of the dibenzoyl-D-tartaric acid salt was also obtained from the methanol filtrate by repeated recrystallization from methanol to afford a total of 8.35 g of the salt. The salt (8.0 g) was suspended in CH₂Cl₂ (200 ml)/water (200 ml), cooled to 0°C and treated with 2N NaOH (12.4 ml, 24.8 mmol). After stirring for 15 minutes the aqueous phase was separated, extracted with CH₂Cl₂ and the combined organic layers were dried over Na₂SO₄. Removal of the solvent in vacuo and azeotroping the residue with toluene (2 x 200 ml) afforded 3.8 g of (-)-4,5-dihydro-3,4-dimethyl-1-(2,4-difluorophenyl)-1H-2,4-benzodiazepine.

### Example 174

### 2-[[(1,1-Dimethylethyl)amino]methyl]-α-phenylbenzenemethanamine

A solution of 15.9 g (90 mmol) N-t-butylbenzamide in 390 ml THF was cooled to -15° under nitrogen and 77 ml (193 mmol) 2.5N n-butyllithium in hexane was added. The mixture was stirred at -5 ± 3° for one hour and 17.5 g (99 mmol) of the trimethylsilylimine of benzaldehyde [prepared according to the procedure of Hart et al., J. Org. Chem. 48, 289-294 (1983)] was added over ten minutes at -10°. The reaction was stirred at 0° for one hour, then 5° for 45 minutes. It was poured into 400 ml ice water containing 225 ml 2N HCl and washed twice with ether. The aqueous layer was made basic with sodium hydroxide and extracted into ether. The ether extracts were dried over sodium sulfate and stripped to yield 25.4 g of 2-[(amino)(phenyl)methyl]-N-(1,1-dimethylethyl)benzamide.

The entire portion of aminoamide in 50 ml THF was combined with 450 ml (450 mmol) 1N borane-THF complex and the mixture stirred at reflux for 18 hours. The reaction was cooled, 225 ml methanol was added, and the solution was refluxed for one hour. It was recooled and 200 ml half-saturated methanolic HCl was added. The solution was again refluxed for one hour, evaporated in vacuo and the residue recrystallized from chloroform/ether to yield 21.3 g (70%) of product as the dihydrochloride, mp 222-231.

### Example 175

### 2-(Aminomethyl)-N-methyl-α-phenylbenzenemethanamine

Seventy-five grams (0.36 mole) 2-benzoylbenzaldehyde was dissolved in 70 ml THF and 18.5 g (0.39 moles) of methylhydrazine was added over 30 minutes at 0°. The suspension became a homogeneous solution which was allowed to stand for four days. A first portion comprising 8.5 g of product was obtained by addition of hexane and filtration. A second portion of 27.5 g of product was obtained by chromatography on silica gel with 85:15 methylene chloride/ethyl acetate. The mp of the hydrazone after recrystallization from methylene chloride-hexane was 164-165.

A solution of 44.5 g of the methylhydrazone in 80 ml THF was treated with 374 ml 1 M borane-THF and stirred at reflux. After 24 hours and 72 hours, additional 187 ml portions of 1 M borane were added. After six days the reaction was worked up as described in Example 164 and the dihydrochloride recrystallized from methanol ether, mp 224-226.

### Example 176

### 2-(Aminomethyl)-α-phenyl-N-(phenylmethyl)benzenemethanamine

Sixty-two grams (0.30 mole) 2-benzoylbenzaldehyde in 140 ml THF was treated with 81.5 g (0.59 mole) potassium carbonate and 64 g (0.32 mole) benzylhydrazine dihydrochloride. The mixture was stirred 30 minutes at 0° and 40 ml methylene dichloride was added. The mixture was stirred at room temperature for one day, filtered and stripped in vacuo to provide 131 g of 2-benzyl-1-phenyl phthalazinium chloride.

The crude phthalazinium chloride in 175 ml THF was treated with 1.325 l of 1 M borane-THF at reflux under nitrogen. After 24 hours the reaction was worked up as described in Example 175 and the dihydrochloride salt was formed by precipitation from ether with ethereal HCl to provide 36.8 g of dihydrochloride of the product, mp 175-178.

### Example 270

### N-Methyl-α'-phenyl-2,3-thiophenedimethanamine

Following the procedure of General Method T, 19 g N-methyl-α'-phenyl-2,3-thiophenedimethanamine was prepared from 32.6 g (0.135 mol) of 6,7-dihydro-6-methyl-7-oxo-4-phenylthieno[2,3-d]pyridazine. The product was recrystallized as its dihydrochloride from ethanol-water, mp 290-292.

### Example 279

### 2-(Amino)phenylmethyl-N-methylbenzeneethanamine (Formula XXXII: R^{2b} = Me, R^{5d} = Ph, R^{6a} = H)

By General Method T, 25.8 g (0.103 mol) 4,5-dihydro-3-methyl-1-phenyl-2,3-benzodiazepin-4-one was reduced to yield 18.6 g of 2-(amino)phenylmethyl-N-methylbenzeneethanamine as its dihydrochloride salt, mp 257-258 from methanol-ether.

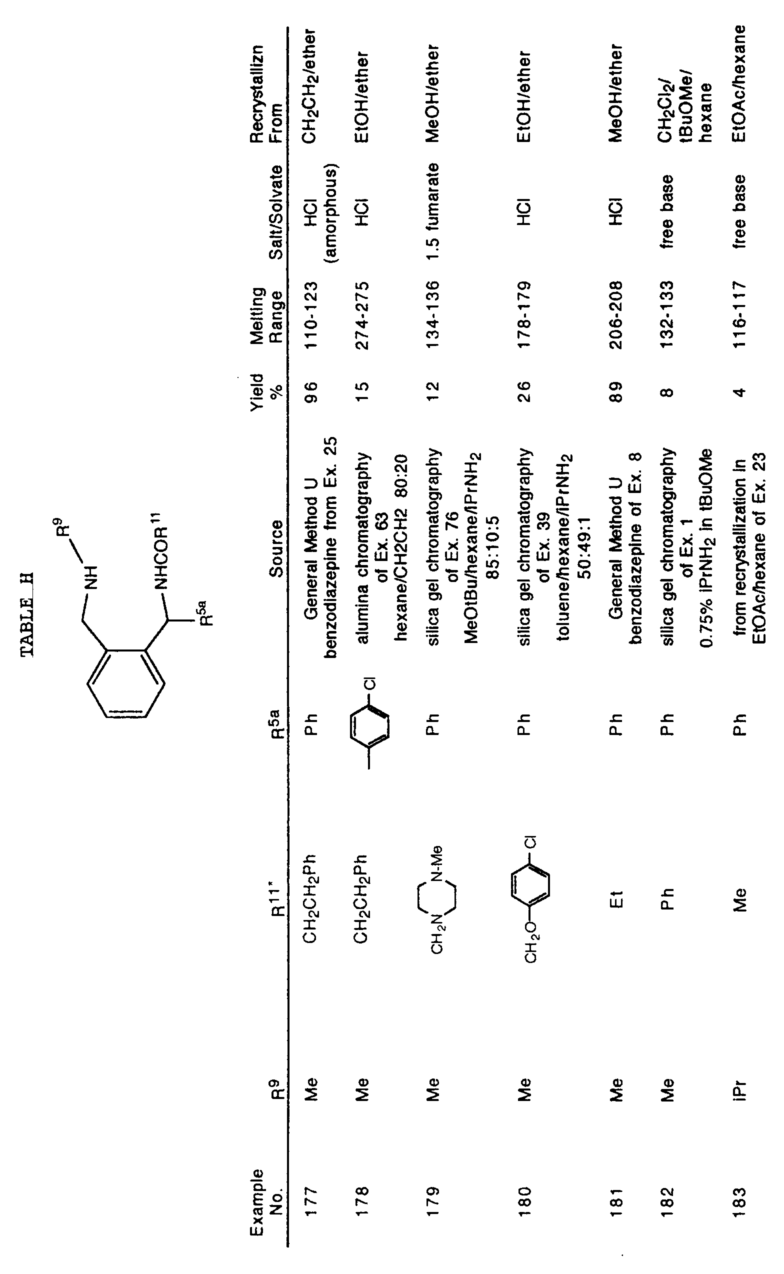

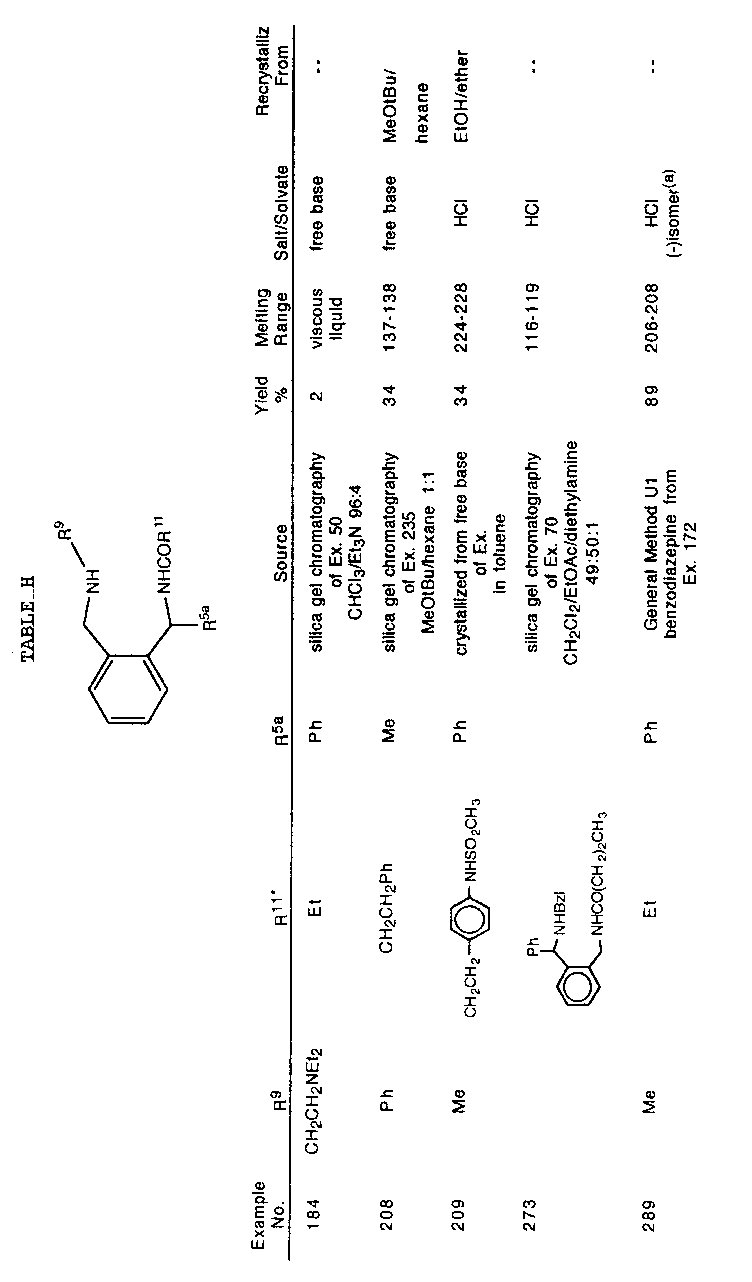

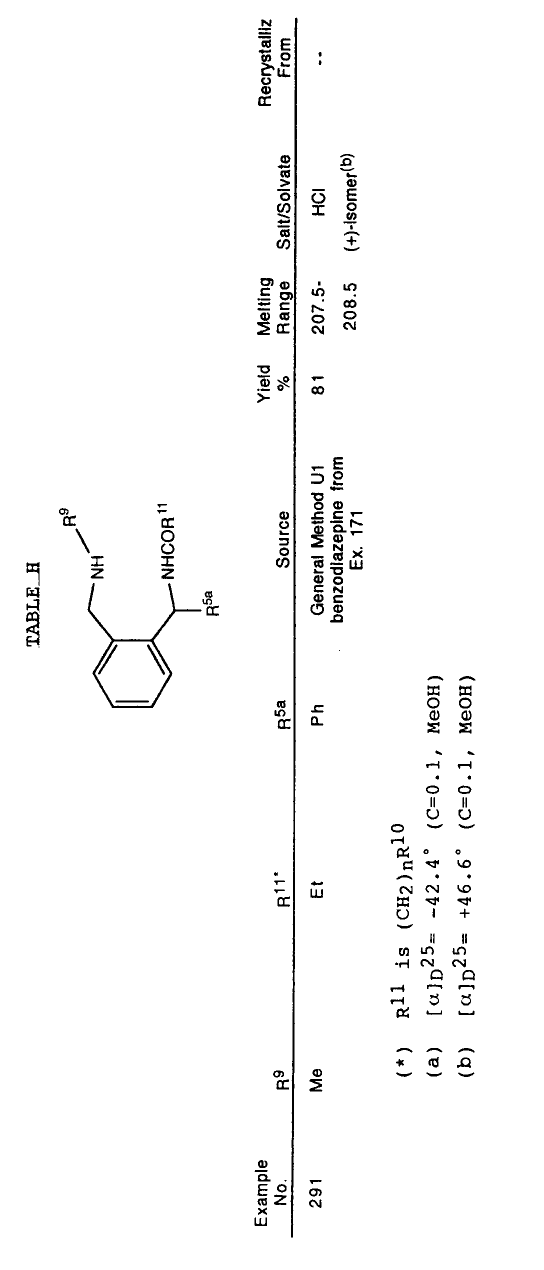

### Examples 177-184, 208, 209, 273

Aminoamides of formula III 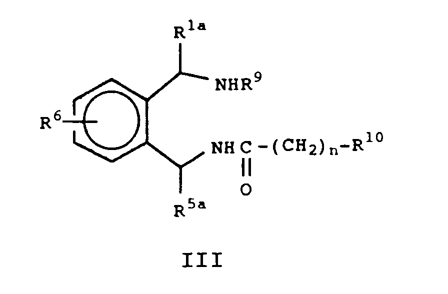
were obtained as by-products of the synthesis of the corresponding benzodiazepines and were usually isolated via chromatography on silica gel using basic elution solvents. They were also obtained by hydrolysis of the corresponding benzodiazepines as described in General Method U. Examples are given in Table H

### General Method U

A solution of the appropriate benzodiazepine in 3 to 5 ml methanol/mmol diazepine was stirred at room temperature for 1 to 4 days with 3 to 5 equivalents of potassium hydroxide in 1 to 2 ml water/mmol diazepine. Aqueous sodium chloride was added and the aminoamide was extracted into ether. The ether layer was dried over MgSO₄, filtered, stripped and the residue was treated as shown in Table H. It is anticipated that any species described in Tables A through E could be converted to the corresponding aminoamide by this procedure.

### General Method U1

The procedure described under General Method U was followed except that 5.6-5.7 equivalents of potassium hydroxide was used and the aminoamide was extracted with a CH₂Cl₂/ether mixture.

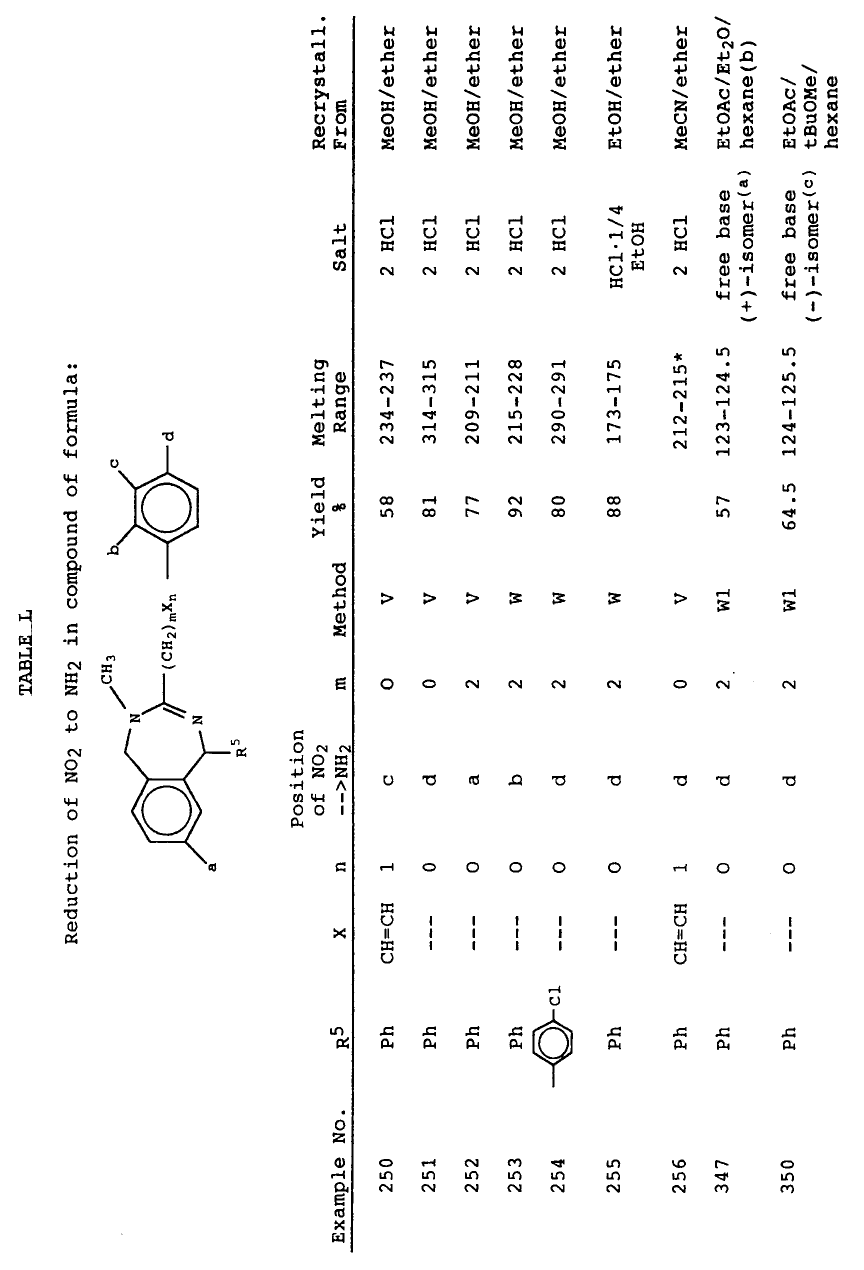

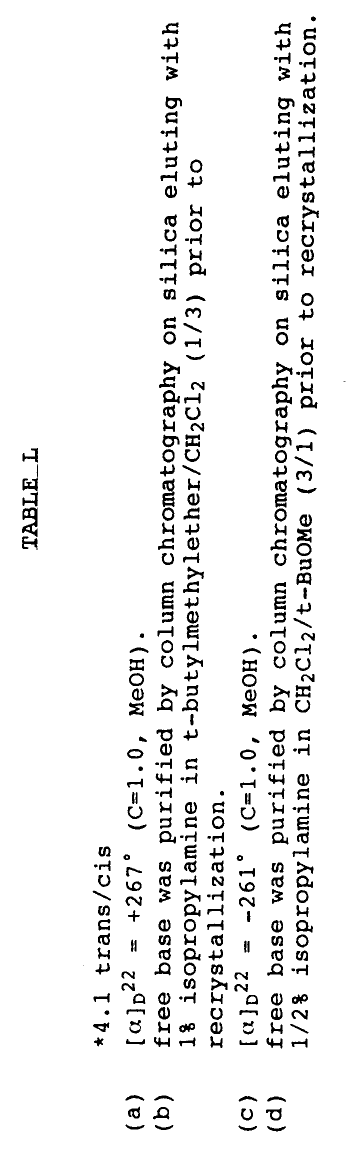

### General Method V

The appropriate nitro compound, as its salt, usually the fumarate salt, was dissolved in about 10 ml dry methanol/mmol nitro compound and 0.1 to 0.15 g 10% Pd on carbon was added/mmol nitro compound. The reaction was stirred at 18-24° and 7.5 to 8.0 equivalents of ammonium formate was added. After 1-2 hours the reaction was filtered, stripped, distributed between methylene chloride and 2N NaOH, separated, dried and stripped. The residue was crystallized as shown in Table L.

### General Method W

The appropriate nitro compound, as its hydrochloride salt or the free base plus one equivalent of methanolic HCl, was dissolved in about 20 ml methanol or ethanol/mmol nitro compound and about 0.05 to 0.1 g of 10% Pd on carbon was added/ mmol of nitro compound. The mixture was hydrogenated at 3.5 to 1.4 atm on a Parr shaker. When the calculated amount of hydrogen had been consumed, the reaction was filtered, excess ethereal HCl was added, and the solution was stripped. The residue was crystallized as shown in Table L.

### General Method W1

The procedure was similar to that described in General method W, except that about 4 ml methanol rather than 20 ml methanol or ethanol/mmol nitro compound was used and the reaction mixture was worked up as follows. The catalyst was removed by filtration, the filtrate was concentrated in vacuo and the residue was dissolved in CH₂Cl₂ and basified with 1N-2N NaOH. The CH₂Cl₂ layer was separated dried over Na₂SO₄, and concentrated in vacuo and the residue thus obtained was purified as the free base as illustrated in Table L.

### Example 274

### 4-[2-(4,5-Dihydro-3-ethyl-1H-2,4-benzodiazepin-4-yl) ethyl]benzeneamine

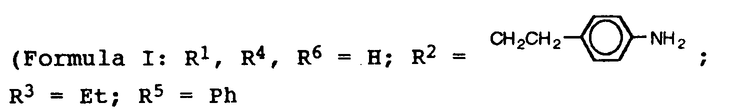

By General Method W, 4.50 g (10.3 mmol) 4,5-dihydro-3-ethyl-4-[2-(4-nitrophenyl)ethyl]-1H-2,4-benzodiazepine hydrochloride of Example 276 was reduced to 3.33 g 4[2-(4,5-dihydro-3-ethyl-1H-2,4-benzodiazepin-4-yl)ethyl]benzeneamine as its monohydrochloride, mp 149-151 from EtOH-ether.

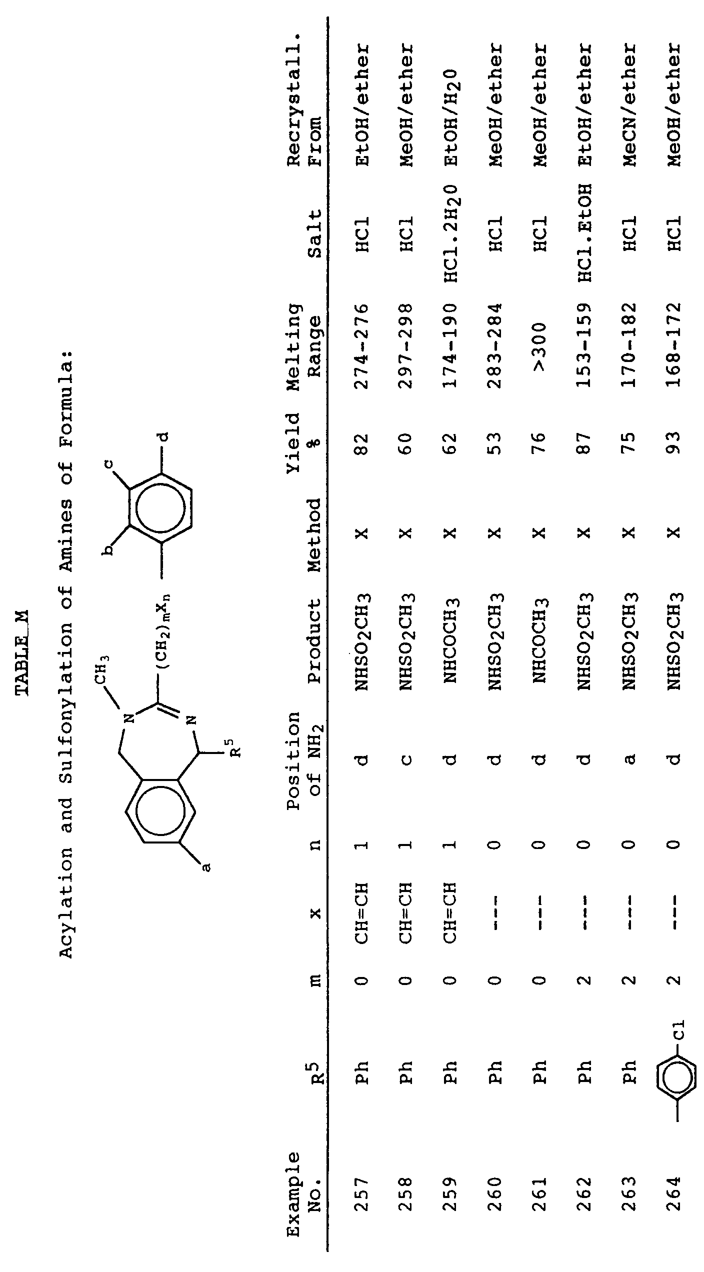

### General Method X

A solution of the appropriate amine as its dihydrochloride and from 3 equivalents of pyridine to 30 equivalents of pyridine were stirred at 0° in about 10 ml methylene chloride/mmol amine under a nitrogen atmosphere while 1.1 to 1.5 equivalents of methanesulfonyl chloride or acetyl chloride was added dropwise. The reaction was stirred at 0° for 1-2 hours and one volume of saturated aqueous Na₂CO₃ was added. In a few cases where TLC showed incomplete reaction, an additional 1 to 3 equivalents of chloride was added before the Na₂CO₃ solution. The layers were separated and the organic layer was stripped. The residue was flash chromatographed, if necessary, on silica gel eluting with MeOH/MeOtBu/isopropylamine 49:49:2. The product was recrystallized as shown in Table M.

### Example 275

### N-[4-[2-(4,5-Dihydro-3-ethyl-1H-2,4-benzodiazepin-4-yl) ethyl]phenyl]methanesulfonamide

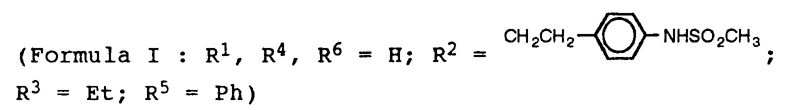

By General Method X, 3.25 g 4-[2-(4,5-dihydro-3-ethyl-1H-2,4-benzodiazepin-4-yl)-ethyl]benzeneamine of Example 274 was converted to 3.49 g N-[4-[2-(4,5-dihydro-3-ethyl-1H-2,4-benzodiazepin-4-yl)-ethyl]phenyl]methanesulfonamide, mp 129-142 as the free base from EtOH-ether-methylene chloride.

### Starting Materials

The phthalazinones, which are the starting materials for the synthesis of diamines described in Table G, are generally available by methods known in the literature. They are most commonly synthesized by condensation of the corresponding γ-ketoacids with the appropriate hydrazine. For example,

### Example 185

### 2-Methyl-4-phenyl-1(2H)-phthalazinone

A 100 gallon stainless steel unit was charged with 40.0 kg 2-benzoylbenzoic acid and 87.5 kg toluene. Methylhydrazine was added over about 45 minutes with the internal temperature rising to 34°.

The resulting thin slurry was warmed at reflux (95-118°) for 4 1/2 hours while collecting about 7.5 l water.

The reaction mixture was cooled slowly with initial precipitation evident at 88°. The resulting slurry was cooled to 0 to -5° before collecting the beige colored crystals. The cake was washed with 2 x 20 l cold toluene and dried in vacuo at 45-50° overnight to afford 38.0 kg (91.0% yield) 2-methyl-4-phenyl-1(2H)-phthalazinone, mp 166-168.

### Example 271

### 6,7-Dihydro-6-methyl-7-oxo-4-phenylthieno-[2,3-d] pyridazine

A solution of 31.2 g (0.134 mol) 3-benzoyl-2-thiophenecarboxylic acid in 400 ml ethanol was treated with 9.3 (0.2 mol) methylhydrazine at room temperature for 18 hours, refluxed 3 hours, cooled and 30.7 g of the product filtered off, mp 174-175.

The 3-benzoyl-2-thiophene carboxylic acid was obtained from 3-bromothiophene by the method of MacDowell and Ballas [J. Org. Chem. 42, 3717 (1977)]

In the cases where the appropriate alkylhydrazine for the condensation to the phthalazinone is not readily available, the γ-ketoacid is condensed with hydrazine and the resulting 2-unsubstituted 1-phthalazinone is alkylated. For example,

### Example 186

### 4-Phenyl-2-(2-phenylethyl)-1(2H)-phthalazinone

85 g (0.38 moles) of 4-phenyl-1(2H)-phthalazinone was added to 18.4 g (0.47 moles) sodium hydride in 1l DMSO in four portions. The mixture was stirred for two hours at room temperature until evolution of hydrogen had ceased, and 95.5 g (0.52 moles) of 2-bromoethylbenzene was added. The mixture was stirred 1.5 hours at room temperature, 1 l 2N NaOH was added and the slurry was poured into 1 l water. The product was filtered off and dried to yield 118 g (95%) of 4-phenyl-2-(2-phenylethyl)-1(2H)-phthalazinone, mp 135-138.

### Example 278

### 2-[2-(4-Nitrophenyl)ethyl]-4-phenyl-1(2H)-phthalazinone

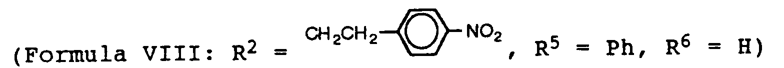

By a procedure analogous to that of Example 186, 11.8 g 2-[2-(4-nitrophenyl)ethyl]-4-phenyl-1(2H)-phthalazinone was prepared from 10.0 g (45 mmol) 4-phenyl-1(2H)-phthalazinone and 11.6 g (50 mmol) 4-nitrophenethyl bromide. The product was recrystallized from EtOAc-ether-hexane, mp 152-155°.

### Synthesis of Esters of the formula R⁸CH=CH-COOEt and R⁸CH₂CH₂COOEt wherein R⁸is heteroaryl

In those cases where the appropriate propanoate and propenoate esters were not commercially available, the propenoate was synthesized by condensation of ethyl acetate with the appropriate aldehyde in the presence of one equivalent of sodium metal. The unsaturated esters were reduced with a large excess of magnesium metal in methanol to provide the propanoates.

Miscellaneous syntheses of diamines, phthalazines, and precursors are shown below:

### Example 187

### 4-Benzyl-2-methyl-1(2H)-phthalazinone

A solution of 30 g potassium hydroxide and 31.3 g (140 mmol) benzylidenephthalide in 100 ml water was heated to homogeneity and poured into a solution of 40 ml H₂SO₄ in 250 ml water. After cooling, the resulting solid was collected and dissolved in aqueous sodium bicarbonate. 2N HCl was added until the first signs of precipitation, the aqueous solution was washed four times with chloroform and then acidified with excess 2N HCl. A white precipitate of 20.3 g of 2-(1-oxo-2-phenylethyl)benzoic acid was filtered off and dried, mp 74-75, after recrystallization from ethanol water. This γ-ketoacid was treated with methylhydrazine according to the method of Example 185 to yield 17.3 g of the phthalazinone product, mp 144-146.

### Example 188

### 2-Methyl-4-(2-thienyl-1(2H)-phthalazinone

A solution of 74.1 g (0.5 mole) of phthalic anhydride in 300 ml nitrobenzene was treated with 147 g (1.1 mole) aluminum chloride. The solution was stirred for two hours and 42.1 g (0.5 mole) thiophene was added dropwise over 80 minutes at 40-45°. The reaction was stirred at 50-55° for two hours and then let sit at room temperature overnight. The reaction was poured into 2.8 l cold water, stirred, separated, and the nitrobenzene was removed from the nitrobenzene layer by steam distillation. The residue was recrystallized from toluene to provide 31.1 g (27%) of 2-thienoylbenzoic acid, mp 141-143. The thienoylbenzoic acid was treated with methylhydrazine as described in Example 185 to provide 24.4 g (75%) of the phthalazinone product, mp 143-144, after recrystallization from ethyl acetate.

### Example 189

### 5-Fluoro-3,4-dihydro-3-methyl-1-phenylphthalazine

To a solution of 20 g (0.14 mole) 2-fluoro-6-chlorotoluene in 125 ml THF was added 6.9 g (0.28 mole) magnesium turnings. The mixture was refluxed while 12 ml 1,2-dibromoethane in 50 ml benzene was added dropwise over three hours. The reaction was refluxed a further hour and 15 ml benzonitrile was added. The reaction was refluxed a further two hours, cooled and quenched with 50 ml water added dropwise. The mixture was extracted into ethyl acetate, dried over sodium sulfate and stripped. The residue was dissolved in 50 ml ethanol, 25 ml 1N HCl was added, and the mixture was refluxed for three hours. The ethanol was stripped, the product was extracted into ethyl acetate, the ethyl acetate dried over sodium sulfate and stripped. The residue of 3-fluoro-2-methylbenzophenone was chromatographed on silica gel with 20% ether in hexane.

A solution of 1.69 g (7.89 mmol) of the benzophenone in 40 ml carbon tetrachloride was treated with 100 mg benzoyl peroxide and 1.5 g (8.43 mmol) N-bromosuccinimide. The reaction was stirred at room temperature for two hours and then refluxed an additional four hours during which a second portion of 50 mg benzoyl peroxide and 400 mg N-bromosuccinimide were added. The reaction was cooled, a small amount of impurity filtered off, and the filtrate concentrated in vacuo to provide 2.5 g of 2-bromo-methyl-3-fluorobenzophenone, presumably containing trapped carbon tetrachloride.

The residue of α-bromomethylketone was dissolved in 40 ml chloroform and a mixture of 1.5 ml triethylamine and one equivalent of methylhydrazine was added dropwise. The reaction was stirred one hour, washed with 20 ml aqueous sodium bicarbonate and filtered directly through silica gel eluting with 25% ethyl acetate in hexane. Concentration in vacuo gave 1.86 g (98%) of product which was reduced immediately as shown in Table G.

### Example 190

### 2-Benzoyl-5-fluorobenzoic acid

Following the procedure of Example 189, 4.0 g (21.2 mmol) of 2-bromo-5-fluoro toluene was treated with 2.4 ml (23.5 mmol) benzonitrile. The imine resulting from the condensation was not hydrolyzed. Instead 28.6 g (0.13 mole) 4-fluoro-2-methylbenzophenoneimine in 200 ml water and 100 ml pyridine was refluxed for eight hours and treated with four portions of potassium permanganate at roughly two-hour intervals. The portions were 53 g, 28 g, 20 g, and 10 g. The reaction was cooled, filtered through diatomaceous earth and concentrated in vacuo. The residue was distributed between aqueous acetic acid and ethyl acetate, the ethyl acetate was dried over magnesium sulfate, and the ethyl acetate was removed in vacuo to provide 16.2 g (51%) of a yellow gum which was used as is.

### Example 191

### 2-Benzoyl-4-fluorobenzoic acid

The procedure of Example 190 was used to provide 14.2 g (53%) of product from 21.3 g 2-bromo-4-fluorotoluene.

### Example 192

### 3,4-dihydro-3-methyl-1-phenylbenzo[f]phthalazine

A solution of 10 g (45 mmol) 1-bromo-2-methylnaphthalene in 75 ml THF was refluxed with 1.2 g (50 mmol) magnesium turnings for three hours. The reaction was cooled on ice and 4.8 ml (43 mmol) benzaldehyde was added. The ice was removed, the reaction was stirred 45 minutes and quenched with 5 ml 1N HCl followed by 50 ml water. The reaction was extracted into ethyl acetate, dried over sodium sulfate and flash chromatographed through silica gel with 5-10% ethyl acetate in hexane to provide 8.4 g (75%) of 2-methyl-α-phenyl-1-naphthalenemethanol as a pale yellow gum.

A solution of 10.0 g (40 mmol) of the secondary alcohol in dichloromethane was treated with 12.4 g (58 mmol) pyridine chlorochromate, refluxed briefly, and stirred at room temperature for one hour. The reaction was diluted with 150 ml ether and filtered through florisil. Concentration of the filtrate in vacuo afforded 7.92 g (80%) of 1-benzoyl-2-methylnaphthalene as a bright orange gum which slowly crystallized on standing.

By the procedure described in Example 189, 4.4 g (18 mmol) of the benzoylnaphthalene was converted to 1.73 g phthalazine product, which was reduced immediately as shown in Table G.

### Example 193

### 3,4-Dihydro-3,8-dimethyl-1-phenylphthalazine

By a procedure exactly analagous to that of Example 192, the phthalazine was synthesized from 2-bromo-m-xylene.

### Example 194

### 2-Aminomethyl-α-phenylbenzenemethanamine

To a slurry of 3.8 g (100 mmol) lithium aluminium hydride in 120 ml THF was added 11.1 g (50 mmol) 4-phenyl-1(2H)phthalazinone. The mixture was refluxed one hour, cooled, diluted with 100 ml ether and sequentially treated with 3.8 ml water, 3.8 ml 15% aqueous sodium hydroxide and 11.4 ml water. The mixture was stirred for 30 minutes and the granular precipitate filtered off. The filtrate was diluted with a little toluene, dried over sodium sulfate and stripped to provide 14.1 g of an oil which was dissolved in 180 ml ethanol and hydrogenated at 50 psi in the presence of 20 ml ethanolic HCl and 1.5g of 10% palladium on carbon. After 24 hours a precipitate had formed. The reaction was filtered, the precipitate was slurried in 250 ml hot methanol and filtered again. The combined filtrates were stripped to about 100 ml and diluted with ether. On cooling, 7.3 g of 1,2,3,4-tetrahydro-1-phenylphthalazine as the monohydrochloride salt was filtered off. It was recrystallized from methanol/ether to provide 6.93 g (56%) of product, mp 251-253.

The tetrahydrophthalazine was redissolved in 200 ml methanol by warming and hydrogenated at 50 psi at 66° for 20 hours in the presence of 3.5 g Raney nickel catalyst. The catalyst was filtered off and the filtrate stripped. The residue was recrystallized from methanol/ether to provide 99% yield of the diamine dihydrochloride salt, mp 270-273.

### Example 195

### 2-(4-Methoxybenzoyl)benzoic acid

A mixture of 57 g (0.4 mole) phthalic anhydride and 43 ml (0.4 mole) anisole in 400 ml benzene was treated with 105 g (0.8 mole) aluminum chloride at 5°. The reaction was kept for five days at 5°, poured into 600 ml 2N aqueous HCl and ice and filtered. The residue was triturated in aqueous sodium carbonate and filtered repeatedly until the solid no longer contained product. The sodium carbonate extracts were combined, washed with ether, and acidified with 2N aqueous HCl. The product was extracted into ether, dried over sodium sulfate and stripped. It was recrystallized from toluene to provide 80% yield of product, mp 145-147.

### Example 196

### 2-(2-Methoxyethyl)-4-phenyl-1(2H)-phthalazinone

85 g (0.38 mole) 2-benzoylbenzoic acid and 28.6 g (0.38 mole) hydroxyethylhydrazine were reacted according to the procedure of Example 185. The resulting hydroxyethylphthalazinone was suspended in 300 ml DMF and 200 ml THF and 12.3 g 60% sodium hydride in oil was added in portions over 40 minutes under nitrogen. The reaction was stirred an additional 45 minutes at room temperature and the evolution of hydrogen ceased. 31 ml methyl iodide was added over 1.5 hours and the reaction was stirred at gentle reflux for 16 hours. It was poured into water and extracted into ether. The ether layers were dried over sodium sulfate and stripped. The residue was chromatographed on silica with 5% ethylamine in ethyl acetate to provide 39 g (47%) of product, mp 115-118 after recrystallization from cyclohexane.

### Example 197

### 2-Benzoyl-4,5-dimethoxybenzoic acid

500 ml 37% formalin solution was saturated with hydrogen chloride gas at 15-20°C and 70 g (0.38 mole) veratric acid was added in one portion. The mixture was heated at 60-70° for seven hours and allowed to sit at room temperature for 14 hours. The solution was concentrated in vacuo, dissolved in about 300 ml water, cooled and made basic with ammonium hydroxide. The resulting solid was collected by filtration and dried to provide a 65% yield of dimethoxyphthalide.

108 g (0.56 mole) of the phthalide was oxidized with 258 g (1.64 moles) potassium permanganate according to the procedure of Example 190.

81 g of the dimethoxyphthalic acid was converted to 72 g of the corresponding dimethoxyphthalic anhydride by heating briefly in 200 ml acetic anhydride.

30 g (0.14 mole) of 4,5-dimethoxyphthalic anhydride was suspended in 300 ml THF and 87 ml (0.17 mole) phenylmagnesium chloride in THF was added over two hours. The reaction was stirred at room temperature for 14 hours, refluxed for two hours, cooled and poured into saturated ammonium chloride. The mixture was made acidic with 6N HCl, extracted into chloroform, dried over magnesium sulfate, concentrated to provide 30 g of 2-benzoyl-4,5-dimethoxybenzoic acid.

### Example 272

### Methyl 4-(Diethylaminosulfonyl)benzenepropanoate

To 14.5 g N,N-diethyl-4-bromobenzenesulfonamide (50 mmol) (prepared by reaction of 4-bromobenzenesulfonyl chloride with diethylamine) was added 13.8 g tetrabutylammonium chloride (50 mmol), 10.3 g NaHCO₃ (124 mmol), 266 mg palladium (II) acetate (1.07 mmol) and 100 ml DMF in the order given. To this suspension was added 8.8 ml methyl acrylate (98 mmol) and the reaction was stirred 1 hour at 80°. The reaction was cooled, 500 ml water and 900 ml ether were added, the layers were separated and the ether layer was filtered to remove Pd (0) and combined with 2 further ether washes of the aqueous phase. The combined ether solutions were dried over MgSO₄, filtered, stripped and recrystallized from methanol/ether to yield 9.4 g methyl 4-(diethylaminosulfonyl) benzene-2-propenoate. 6 g of the propenoate was reduced in ethanol at 3.5 atm over 10% Pd on carbon in a Parr Shaker to produce 5.9 g of product as a yellow oil. It was used in that form in Example 269.

### Example 198

### 1[4-(Diethylamino)phenyl]-3-ethyl-4-methyl-1H-2,4-benzodiazepine

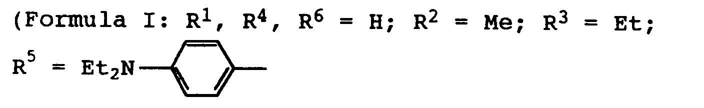

By a procedure analogous to that of Example 41, it is contemplated that 1-[4-(diethylamino)phenyl]-3-ethyl-4-methyl-1H-2,4-benzodiazepine can be synthesized from 2-[4-(diethylamino)benzoyl]benzoic acid (see U.S. Pat. 4,106,174), methylhydrazine, and triethylorthopropionate.

### Example 199

### 3-Methyl-1-[2-[(1-oxopropyl)amino]phenyl]-4-(3-phenylpropyl)1H-2,4-benzodiazepine

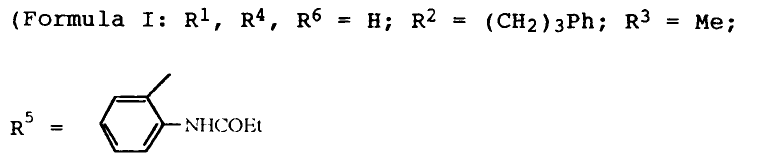

By a procedure analogous to that of Example 41, it is contemplated that 1-(2-aminophenyl)-4-(3-phenylpropyl)-3-methyl-1H-2,4-benzodiazepine can be synthesized from 2-(2-aminobenzoyl) benzoic acid, hydrazine, bromobenzenepropane and triethylorthoacetate. It is further contemplated that this product may be acylated by treatment with propionic anhydride at room temperature to produce 3-methyl-1-[2-[(1-oxopropyl)amino]phenyl]-4-(3-phenylpropyl)-1H-2,4-benzodiazepine.

### Example 290

### (-)-2-[[(1-Methyl)amino]methyl]-α-phenylbenzenemethanamine dihydrochloride (Formula VI: R¹ = R⁶ = H, R² = Me, R⁵ = Ph)

A mixture of the aminoamide of Example 288 (41.35 g, 0.1297 mol) in concentrated hydrochloric acid (500 ml) was heated to reflux and concentrated sulfuric acid (5 ml) was added and the mixture was refluxed for 23 hours. Additional concentrated sulfuric acid (5 ml) was added and the mixture was refluxed for 13 hours. The reaction mixture was cooled, neutralized with concentrated ammonium hydroxide, poured into a mixture of ether/CH₂Cl₂ (500 ml), and basified with 35% NaOH until the aqueous layer was at a pH ≧10. The organic layer was separated, and the aqueous layer was extracted with ether. The organic layers were combined, washed with saturated sodium chloride, dried over Na₂SO₄ and the solvent was concentrated in vacuo. The residue was purified by column chromatography on silica eluting with MeOtBu and 1-5% isopropylamine to afford 18.76 g of the product as the free base which converted into 23.95 g of the dihydrochloride salt after recrystallization of the salt from Et₂O/acetone/methanol.

### Example 292

### (a) (+)-2-[[(1-Methyl)amino]methyl]-α-phenylbenzenemethanamine dihydrochloride (Formula VI: R¹ = R⁶ = H, R² = Me, R⁵ = Ph)

A mixture of the aminoamide of Example 291 (128.07 g) and a 50% by weight solution of H₂SO₄/water was heated to reflux for 4 days. The reaction mixture was cooled, neutralized with ammonium hydroxide and treated with 35% NaOH. The mixture was extracted with an ether/CH₂Cl₂ mixture, and the organic layer was washed with brine, and a small amount of concentrated NaOH. The organic layer was dried over Na₂SO₄, the solvent was removed in vacuo, and the residue was purified by column chromatography on silica eluting with MeOtBu/1-5% isopropylamine to afford 39.0 g of the product as the free base. The free base was then converted into 42.26 g of the dihydrochloride salt, m.p. 180-200°C, after recrystallization from MeOH/acetone.

### (b)

Alternatively, the desired product may be prepared by treatment of the free base of (R)-(+)-4,5-dihydro-3-ethyl-4-methyl-1-phenyl-1H-2,4-benzodiazepine of Example 171 (3.26 g, 1.2 mol) in toluene (2.75 l) under N₂ with ethylenediamine and heating the reaction mixture to reflux for 65 hours. The solvent was removed in vacuo, and the residue was diluted with t-butylmethyl ether and washed with 0.1 N NaOH. The aqueous layer was separated, extracted with t-butylmethyl ether and the combined organic layers were washed with brine and dried over Na₂SO₄. The solvent was removed in vacuo to afford 270.1 g (99.4%) of the product as the free base. The free base (268 g) was dissolved in t-butylmethyl ether and treated with ethereal·HCl to afford 335 g of the dihydrochloride salt as a blue solid.

Following a procedure substantially similar to that described in Example 292(b) but substituting an appropriately substituted benzodiazepine for (R)-(+)-4,5-dihydro-3-ethyl-4-methyl-1-phenyl-1H-2,4-benzodiazepine, there was prepared the following compounds illustrated in Table AA.

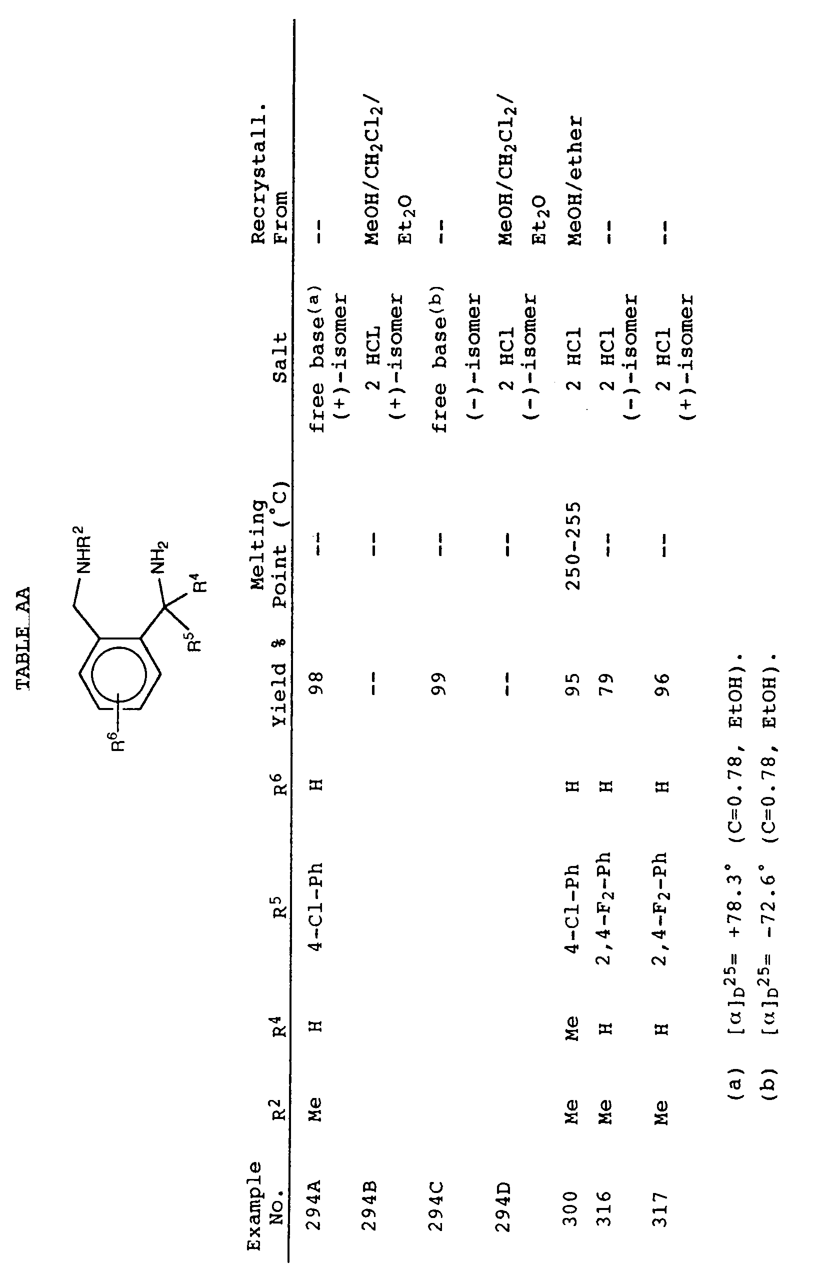

### Example 301

### N-[4-[1-(4,5-Dihydro-1-(4-chlorophenyl)-1,4-dimethyl-1H-2,4-benzodiazepin-3-yl)methoxy]phenyl] methanesulfonamide hydrochloride

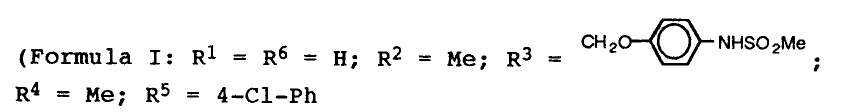

Following a procedure substantially similar to that described in General Method F2, there was obtained 3.15 g (84%) N-[4-[1-(4,5-dihydro-1-(4-chlorophenyl)-1,4-dimethyl-1H-2,4-benzodiazepin-3-yl)methoxy]phenyl]methanesulfonamide hydrochloride, m.p. 263-264.5 when recrystallized from ethanol/t-butylmethyl ether, from 2-[[(1-methyl)amino]methyl]-α-phenyl-α-methylbenzenemethanamine dihydrochloride (2.5 g, 7.18 mmol), trimethylaluminum (12.58 ml, 25.16 mmol), sulfolane (36 ml) and N-[4-(ethoxycarbonylmethoxy)phenyl]methanesulfonamide (2.16 g, 7.91 mmol).

### Example 303

### N-[4-[(4,5-Dihydro-1-(4-chlorophenyl)-4-methyl-1H-2,4-benzodiazepin-3-yl)methylsulfonyl]phenyl]methanesulfonamide hydrochloride

The sulfide of Example 302 (1.88 g, 3.6 mmol) was dissolved in hot acetic acid (42 ml), the solution was cooled to room temperature and 30% hydrogen peroxide (1.0 ml) was added. The mixture was heated to 55°C for 20 hours, additional 30% H₂O₂ (0.3 ml) was added and the mixture was heated for another 7 hours. Cyclohexane (1.2 g) was added and the reaction mixture was allowed to stand overnight at room temperature. The solvent was removed in vacuo , and the residue was treated with ethanol, and diluted with ether. A precipitate formed, which was collected and purified by column chromatography on silica eluting with CHCl₃/ethanol/trifluoroacetic acid (87/10/3). The residue thus obtained was treated with ethanolic·HCl and the hydrochloride salt was recrystallized from isopropanol to afford 0.78 g of N-[4-[(4,5-dihydro-1-(4-chlorophenyl)-4-methyl-1H-2,4-benzodiazepin-3-yl)methylsulfonyl]phenyl]methanesulfonamide hydrochloride, m.p. 185-220°C.

### Example 303A

### (+)-N-[4,5-Dihydro-1-(4-chlorophenyl)-4-methyl-1H-2,4-benzodiazepin-3-yl)methylsulfonyl]phenyl] methanesulfonamide hydrochloride

Following a procedure substantially similar to that described in Example 303, there was obtained 0.31 g (56%) of (+)-N-[4,5-dihydro-1-(4-chlorophenyl)-4-methyl-1H-2,4-benzodiazepin-3-yl)methylsulfonyl]phenyl]methanesulfonamide hydrochloride, m.p. 270-272°C when recrystallized from acetonitrile, [α]_{D}²⁵= +180.6° (C=0.615, EtOH); from the sulfide of Example 302A (0.5 g, 1 mmol), acetic acid (12 ml) and 30% H₂O₂ (0.37 ml), followed by conversion of the free base thus obtained into the hydrochloride salt and purification of the salt by column chromatography on silica eluting with CHCl₃/10% EtOH/3% trifluoroacetic acid.

### Example 303B

### (-)-N-[4,5-Dihydro-1-(4-chlorophenyl)-4-methyl-1H-2,4-benzodiazepin-3-yl)methylsulfonyl]phenyl] methanesulfonamide hydrochloride·1/2 ethanol

Following a procedure substantially similar to that described in Example 303, there was obtained 0.213 g (38%) (-)-N-[4,5-dihydro-1-(4-chlorophenyl)-4-methyl-1H-2,4-benzodiazepin-3-yl)methylsulfonyl]phenyl]methanesulfonamide hydrochloride·1/2 ethanol, m.p. <160°C, [α]_{D}²⁵= -123.2° (C=0.615, EtOH); from the sulfide of Example 302B (0.5 g, 1 mmol), acetic acid (12 ml) and 30% H₂O₂ (0.37 ml), followed by conversion of the free base into the hydrochloride salt and purification of the salt by column chromatography on silica (1X) eluting with CHCl₃/10% ethanol/3% trifluoroacetic acid and then a second silica column eluting with CHCl₃/10% isopropanol/3% trifluoroacetic acid.

### Example 309

### (+)-2-[[(Methyl)amino]methyl]-α-(4-methylthiophenyl) benzenemethanamine dihydrochloride (Formula VI: R¹ = R⁶ = H, R² = CH₃, R⁵ = 4-CH₃S-Ph, (+)-isomer)

A mixture of (+)-4,5-dihydro-3,4-dimethyl-1-(4-methylthiophenyl)-1H-2,4-benzodiazepine of Example 307 (1.18 g, 4 mmol), ethylene diamine (0.24 g, 4.04 mmol) and toluene (2 ml) was refluxed for 18 hours. The reaction mixture was cooled in an ice-bath, diluted with t-butylmethyl ether (7 ml) and the precipitate which formed was collected by filtration and washed with t-butylmethyl ether. The filtrate was washed with water containing a small amount of 2N NaOH, then brine, and finally 1N HCl (2X). The aqueous acidic solution was washed with t-butylmethyl ether and then was basified with 2N NaOH and extracted with t-butylmethyl ether. The ether extracts were combined, washed with brine, dried over Na₂SO₄ and the solvent was removed in vacuo. The residue was dissolved in ethanol and treated with ethanolic·HCl to afford 0.92 g (67%) of the product as the dihydrochloride salt, m.p. 256-257°C.

### Example 310

### (-)-2-[[(Methyl)amino]methyl]-α-(4-methylthiophenyl) benzenemethanamine dihydrochloride (Formula VI: R¹ = R⁶ = H, R² = CH₃, R⁵ = 4-CH₃S-Ph, (-)-isomer)

Following a procedure substantially similar to that described in Example 309, there was obtained (-)-2-[[(methyl)amino]methyl]-α-(4-methylthiophenyl)benzenemethanamine dihydrochloride, from (-)-4,5-dihydro-3,4-dimethyl-1-(4-methylthiophenyl)-1H-2,4-benzodiazepine of Example 308 (2.96 g, 10 mmol), ethylene diamine (0.69 ml 10.3 mmol) and toluene (6 ml), followed by treatment of the free base with ethanolic·HCl.

### Example 318

### 2-(4-Methylthiobenzoyl)benzoic acid

A mixture of phthalic anhydride (37 g, 0.25 mol), thioanisole (31 g, 0.25 mol) and tetrachloroethane (300 ml) was cooled to 3°C and anhydrous AlCl₃ (66.5 g, 0.5 mol) was added while maintaining the reaction temperature at 3-5°C. After stirring for 10 minutes, the mixture was warmed to room temperature and stirred for 3 hours, then the mixture was stirred at 35-42°C for 3 hours and then at room temperature overnight. The mixture was poured into ice water/concentrated HCl, and the aqueous layer was separated and extracted with CHCl₃. The organic layers were combined, washed with dilute HCl and filtered through solka Folc. The filtrate was extracted with 1N NaOH and the basic solution was washed with CH₂Cl₂ and then was acidified with concentrated HCl. A crystalline product formed, which was collected by filtration, washed with water and recrystallized from ethanol/water to afford 38.3 g (56.3%) of 2-(4-methylthiobenzoyl)benzoic acid, m.p. 151-155°C.

### Example 319

### 2-(2,4-Difluorobenzoyl)benzoic acid

To a mixture of phthalic anhydride (64.8 g, 438 mmol), tetrachloroethane (600 ml) and 1,3-difluorobenzene (100 g, 877 mmol) was added anhydrous AlCl₃ (141 g, 964 mmol) over 20 minutes while maintaining the reaction temperature below 40°C. The temperature was increased to 90°C for 2 hours, the reaction mixture was cooled and poured into ice/6N HCl. The mixture was extracted with ether (2 x 1.2 l) and the combined ether extracts were dried over Na₂SO₄. The solvent was removed in vacuo and the residue was recrystallized from hot ethyl acetate to afford 86.5 g (75%) of 2-(2,4-difluorobenzoyl)benzoic acid, m.p. 129-131°C.

### Example 320

### 2-Methyl-4-(4-chlorophenyl)-1-(2H)-phthalazinone

Following a procedure substantially similar to that described in Example 185, there was obtained 92.22 g (87.4%) 2-methyl-4-(4-chlorophenyl)-1-(2H)-phthalazinone, m.p. 151-152.5°C, from methylhydrazine (19.95 g, 0.4287 mol), toluene (260 ml) and 2-(4-chlorobenzoyl)benzoic acid (103.66 g, 0.3897 mol).

### Example 321

### 2-Methyl-4-(4-methoxyphenyl)-1-(2H)-phthalazinone

Following a procedure substantially similar to that described in Example 185, there was obtained 39.65 g (78%) 2-methyl-4-(4-methoxyphenyl)-1-(2H)-phthalazinone, from 2-(4-methoxybenzoyl)benzoic acid (49.65 g, 0.19 mol) methylhydrazine (10.3 ml) and toluene (150 ml).

### Example 322

### 2-Methyl-4-(4-methylthiophenyl)-1-(2H)-phthalazinone

Following a procedure substantially similar to that described in Example 185, there was obtained 37.42 g (78%) 2-methyl-4-(4-methylthiophenyl)-1-(2H)-phthalazinone, m.p. 166.5-168.5°C, from 2-(4-methylthiobenzoyl)benzoic acid (46.2 g, 0.17 mol), methyl hydrazine (9.2 g, 0.2 mol) and toluene (400 ml).

### Example 323

### 2-Methyl-4-(2,4-difluorophenyl)-1-(2H)-phthalazinone

Following a procedure substantially similar to that described in Example 185, there was obtained 20.0 g 2-methyl-4-(2,4-difluorophenyl)-1-(2H)-phthalazinone, as a yellow solid, from 2-(2,4-difluorobenzoyl)benzoic acid (52.4 g, 0.2 mol), methyl hydrazine (11.2 ml, 0.21 mol) and toluene (500 ml).

### Example 324

### N-[4-(Ethoxycarbonylmethoxy)phenyl]methanesulfonamide

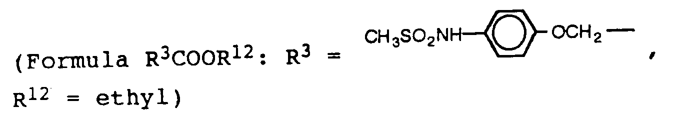

The product was made by procedures which are well known in the art, that is, by alkylation of 4-nitrophenol (29.2 g, 0.21 mol) with ethyl chloroacetate (24.5 g, 0.20 mole) in the presence of potassium carbonate (30.4 g, 0.22 mol), potassium iodide (0.5 g) and acetonitrile (150 ml) to afford ethyl 4-nitrophenoxyacetate (33 g, 73.3%), m.p. 75-76°C after recrystallization from t-butylmethyl ether; hydrogenation of the latter (22.5 g) at 50 psi in ethyl acetate (200 ml) in the presence of 10% palladium on Carbon (0.3 g) to afford 22.5 g (97%) of ethyl 4-aminophenoxyacetate, m.p. 157-158°C; and treatment of the latter (18.52 g, 0.08 mol) with methanesulfonyl chloride (12.6 g, 0.11 mol) in CH₂Cl₂ (250 ml) in the presence of pyridine (35 ml) to afford N-[4-(ethoxycarbonylmethoxy)phenyl]methanesulfonamide, 20.06 g (92%), m.p. 77.3-78.5°C after recrystallization from ethanol.

### Example 325

### N-[4-Ethoxycarbonylmethylthio)phenyl]methanesulfonamide

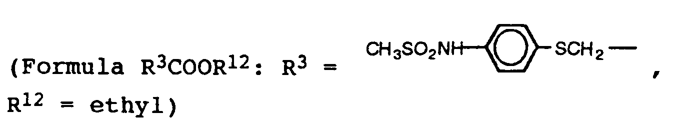

The product was made by procedures which are well known in the art, that is, by esterification of 4-aminothiophenoxy acetic acid (5.49 g, 0.03 mol) with ethanol (50 ml) in sulfuric acid (2 ml) to afford 6.95 g (93.5%) ethyl 4-aminophenylthioacetate hydrochloride, m.p. 154-155.5°C, after formation of the hydrochloride salt by treatment of the free base with ethanolic·HCl; and treatment of the latter (6.43 g, 0.026 mol) with methanesulfonyl chloride (3.44 g, 0.03 mol) in CH₂Cl₂ (60 ml) in the presence of pyridine (6 ml) to afford 6.9 g (95.4%) of N-[4-(ethoxycarbonylmethylthio)phenyl]methanesulfonamide, m.p. 46-48°C after recrystallization from t-butylmethyl ether/hexane.

### Example 326

### N-[4-(Ethoxycarbonylethyl)phenyl]methanesulfonamide

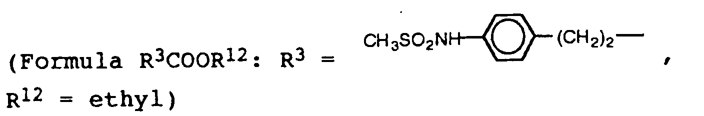

The product was made by reducing ethyl 4-nitrocinnamate (110.6 g, 0.5 mol) with 10% palladium on Carbon (4.0 g) in 95% ethanol (2 l) in the presence of ammonium formate (126 g) to afford ethyl 3-(4-aminophenyl)propionate which was sulfonated with methanesulfonyl chloride in CH₂Cl₂ in the presence of pyridine to produce 102 g (75.2%) of N-[4-(ethoxycarbonylethyl)phenyl]methanesulfonamide.

### Example 327

### 2-Methyl-4-(4-methylsulfonylphenyl)-1-(2H)-phthalazinone

To a solution of 2-methyl-4-(4-methylthiophenyl)-1-(2H)-phthalazinone (14.15 g, 0.05 mol) in acetic acid (330 ml) was added 30% H₂O₂ (22 ml). The mixture was heated to 35°C overnight, additional 30% H₂O₂ (10 ml) was added and the mixture was stirred at 35°C for 2 hours and finally one final portion of 30% H₂O₂ (10 ml) was added and the mixture was stirred for another 2 hours at 35°C. The reaction mixture was poured into water (1 l/ice and the solid thus obtained was collected by filtration, washed with water, then ether, and then was dried to afford 14.6 g (93%) 2-methyl-4-(4-methylsulfonylphenyl)-1-(2H)-phthalazinone as a pale yellow solid, m.p. 218-220°C when recrystallized from ethanol/chloroform (2X).

### Example 331

### (a) N-Methyl-4-bromophenylsulfonamide

A mixture of 4-bromophenylsulfonyl chloride (25.5 g, 100 mmol), CH₂Cl₂ (400 ml) and pyridine (1.6 g, 110 mol) was cooled to 0°C and methylamine gas was added over 45 minutes. The mixture was warmed to room temperature, the solvent was removed in vacuo and the residue was dissolved in CH₂Cl₂ and washed with 2N HCl (2 X 100 ml), water (1 X 200 ml), saturated NaHCO₃ (1 X 200 ml) and then brine (1 X 200 ml). The solvent was dried over MgSO₄ and the solvent was removed in vacuo to afford 23 g of N-methyl-4-bromophenylsulfonamide.

### (b) N-Methyl-N-(4-methoxybenzyl)-4-bromophenylsulfonamide

To a solution of N-methyl-4-bromophenylsulfonamide (2.5 g, 10 mmol) in DMF (20 ml) at room temperature was added in portions NaH (264 mg, 11 mmol). The mixture was stirred for 45 minutes and then p-methoxybenzylchloride (1.49 ml, 11 mmol) was added dropwise. The mixture was stirred for 1 hour, quenched with water and the precipitate which formed was collected by filtration and dried in vacuo at 60°C to afford 3.6 g (97%) of N-methyl-N-(4-methoxybenzyl)-4-bromophenylsulfonamide.

### (c) N-Methyl-N-(4-methoxybenzyl)-4-(2-(methoxycarbonyl)ethenyl)phenylsulfonamide

A mixture of N-methyl-N-(4-methoxybenzyl)-4-bromophenylsulfonamide (1.68 g, 4.54 mmol), Pd(OAc)₂ (20 mg, 0.091 mmol), tri-o-tolylphosphine (55 mg, 0.18 mmol), triethylamine (1.25 ml), acetonitrile (2.5 ml) and methyl acrylate (0.83 ml, 9.08 mmol) were placed in a stainless steel bomb and heated to about 120°C for 4 hours. The mixture was cooled to 0°C, the bomb was vented, and the solid reaction mixture was collected, washed with ether and dried to afford 2.1 g of crude product which was used directly in the next step without further purification.

### (d) N-Methyl-N-(4-methoxybenzyl)-4-(2-(methoxycarbonyl)ethyl)phenylsulfonamide

To a suspension of the sulfonamide of Example 331(c) (2.1 g, 4.54 mmol) in ethanol (125 ml) under nitrogen was added 10% Pd/C (0.6 g). The mixture was placed on a Parr hydrogenator at 50 psi hydrogen for 2 hours, the catalyst was removed by filtration and the solvent was removed in vacuo. The residue was recrystallized from Et₂O/MeOH, extracted with CH₂Cl₂, washed with water, then brine and was dried over MgSO₄. The solvent was removed in vacuo to afford 1.41 g (82%) of N-methyl-N-(4-methoxybenzyl)-4-(2-(methoxycarbonyl)ethyl)phenylsulfonamide as a colorless oil.

### (e) 4,5-Dihydro-4-methyl-1-phenyl-3-[2-(4-(N-methyl-N-4-methoxybenzylaminosulfonyl)phenyl)ethyl]-1H-2,4-benzodiazepine

To a suspension of 2-[[(1-methyl)amino]methyl]-α-phenylbenzenemethanamine dihydrochloride (2.59 g, 8.59 mmol) in toluene (75 ml) at 0°C under nitrogen was added 2M trimethylaluminum (9.0 ml, 18 mmol) over 10 minutes. The mixture was warmed to room temperature and after 2 hours N-methyl-N-(4-methoxybenzyl)-4-(2-(methoxycarbonyl)ethyl)phenylsulfonamide (3.4 g, 9.02 mmol) was added in one portion. The reaction mixture was heated to reflux for 2 hours, cooled to room temperature, partitioned between saturated Na₂CO₃/CH₂Cl₂ and filtered through solka floc. The organic layer was separated, dried over Na₂SO₄ and the solvent was removed in vacuo. The residue was azeotroped with ethanol (2X), decolorized with charcoal and the solvent was removed in vacuo to afford 3.6 g of 4,5-dihydro-4-methyl-1-phenyl-3-[2-(4-(N-methyl-N-4-methoxybenzylaminosulfonyl)phenyl)ethyl]-1H-2,4-benzodiazepine.

### (f) 4,5-Dihydro-4-methyl-1-phenyl-3-[2-(4-(N-methylaminosulfonyl)phenyl)ethyl]-1H-2,4-benzodiazepine

To a solution of the benzodiazepine of Example 331E (2.0 g, 3.67 mmol) in CH₂Cl₂ (14.6 ml) was added trifluoroacetic acid (5.84 mL, 77 mmol) dropwise over 10 minutes. The mixture was stirred for 4 hours, and then was added to a solution of saturated Na₂CO₃ (75 ml) at 0°C. The mixture was extracted with CH₂Cl₂, the aqueous layer was back extracted with additional CH₂Cl₂ and the organic layers were combined, dried over MgSO₄ and the solvent was removed in vacuo to afford 2.5 g 4,5-dihydro-4-methyl-1-phenyl-3-[2-(4-(N-methylaminosulfonyl)phenyl)ethyl]-1H-2,4-benzodiazepine. The free base was treated with ethanolic·HCl to afford the hydrochloride salt, and the salt was purified by column chromatography on neutral Al₂O₃ eluting with 5% MeOH/CH₂Cl₂. The residue was recrystallized from CH₂Cl₂/ether to afford 1.16 g (68%) of the product as the hydrochloride salt, m.p. 101(dec.).

### Example 332

### N-[4-(Ethoxycarbonylethyl)phenyl]trifluoromethanesulfonamide

To a solution of ethyl 3-(4-aminophenyl)propionate (7.87 g, 40.7 mmol) in CH₂Cl₂ (75 ml under N₂ was added triethylamine (5.96 ml, 42.76 mmol). The mixture was cooled to -78°C and trifluoromethanesulfonic anhydride (7.19 ml, 42.76 mmol) in CH₂Cl₂ (20 ml) was added via syringe. The reaction mixture was stirred at -78°C for 15 minutes, then at room temperature for 15 minutes and was then shaken with water (100 ml) and 2N HCl (5 drops). The organic layer was separated, dried over MgSO₄ and concentrated in vacuo. The residue was dissolved in Et₂O (100 ml), washed with water/2N HCl (5 drops) (3X), then brine, and was dried over MgSO₄ and concentrated in vacuo. The residue was taken back up in ether and washed with water and 2N NaOH. The aqueous phase was separated, acidified with concentrated HCl, saturated with sodium chloride and extracted with ether. The combined ether extracts were washed with brine, dried over Na₂SO₄ and concentrated in vacuo to afford 11.894 g of N-[4-ethoxycarbonylethyl)phenyl]trifluoromethanesulfonamide as an oil.

### Example 334

### 2-(2-(4-Pyridinyl)ethyl)-4-(4-chlorophenyl)-1(2H)-phthalazinone

A mixture of 2-(4-chlorobenzoyl)benzoic acid (65.13 g, 0.25 mol), toluene (500 ml) and pyridylethyl hydrazine (37.0 g, 0.27 mol) was refluxed for 1 hour while removing water with a Dean-Stark trap. The mixture was concentrated in vacuo, and the residue was diluted with t-butylmethyl ether (200 ml). A precipitate formed, which was collected by filtration, dissolved in CH₂Cl₂, filtered through solka floc and the filtrate was diluted with hexane. The precipitate which formed was collected by filtration, and recrystallized from hot ethanol to afford 46.5 g (51.4%) of 2-(2-(4-pyridinyl)ethyl)-4-(4-chlorophenyl)-1(2H)-phthalazinone, m.p. 142-144°C. 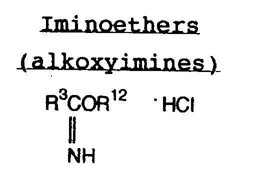
The ethoxy and methoxy imines used for condensation with the diamines were obtained from the corresponding nitriles by methods well known in the art. In general, the nitrile was dissolved in ether, chloroform or a mixture thereof, 1.1 equivalents of alkanol was added, 1.1 equivalents of dry HCl gas was bubbled in, and the mixture was held at 5° for 24-48 hours; the hydrochloride salt of the iminoether was recovered by simple filtration.

The trialkylorthoesters were obtained by treating the corresponding iminoether with the appropriate alkanol under conditions known in the art.

### Example 337

### N-[4-(Ethoxycarbonylmethoxy)-3-(methyl)phenyl]methanesulfonamide

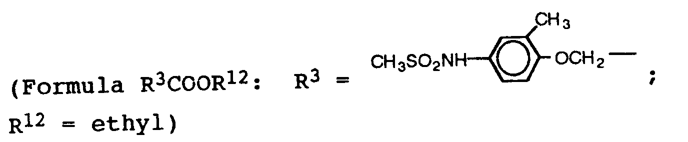

The product was prepared by procedures which are well known in the art, that is, by the alkylation of 4-nitro-2-methylphenol (2.508 g, 0.0164 mol) with ethyl bromoacetate (3.00 g, 0.018 mol) in acetonitrile (50 ml) in the presence of potassium carbonate (2.76 g) to afford ethyl 4-nitro-2-methylphenoxyacetate (4.0 g, 100%); hydrogenation of the latter (4.0 g, 0.0164 mol) in ethanol (250 ml) in the presence of 10% palladium on carbon (1.0 g) to afford ethyl 4-amino-2-methylphenoxyacetate; which was then dissolved in CH₂Cl₂ (50 ml) and treated with pyridine (4.0 ml), followed by methanesulfonyl chloride (1.88 g) in CH₂Cl₂ (5.0 ml) to afford 3.27 g (69.4%) of N-[4-(ethoxycarbonylmethoxy)-3-(methyl)phenyl]methanesulfonamide, m.p. 83-84°C, after purification by column chromatography on silica.

### Example 338

### N-[4-(Ethoxycarbonylmethoxy)-2-(methyl)phenyl] methanesulfonamide

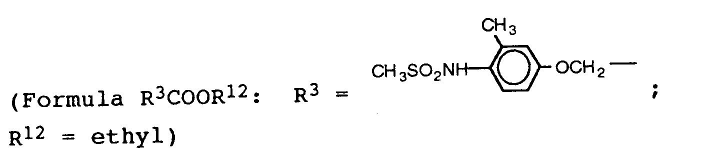

The product was prepared by the alkylation of 4-nitro-3-methylphenol (15.3 g) with ethyl bromoacetate (16.7 g) in DMF (150 ml) in the presence of potassium carbonate (13.8 g) to afford ethyl 4-nitro-3-methylphenoxyacetate; hydrogenation of the latter (7.18 g, 0.03 mol) in ethanol (250 ml) in the presence of 5% palladium on carbon (2.1 g) to afford 6.20 g (99%) of ethyl 4-amino-3-methylphenoxyacetate as a brown oil; and treatment of the latter (6.2 g, 0.0296 mol) in CH₂Cl₂ (100 ml) with pyridine (10 ml), followed by methanesulfonylchloride (3.73 g, 0.033 mol) to afford 6.54 g (76.9%) of N-[4-(ethoxycarbonylmethoxy)-2-(methyl)phenyl]methanesulfonamide, m.p. 93-94°C, after recrystallization from ethanol.

### Example 341

### N-[4-(Ethoxycarbonylmethoxy)-3-(methoxy)phenyl] methanesulfonamide

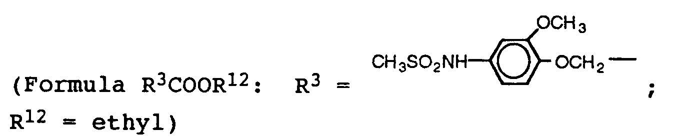

The product was prepared by the alkylation of 4-nitro-2-methoxyphenol (10.0 g, 0.059 mol) with ethyl bromoacetate (10.0 g, 0.06 mol) in acetonitrile (50 ml) in the presence of potassium carbonate (8.0 g) to afford 14.33 g (95%) of ethyl 4-nitro-2-methoxyphenoxyacetate as a yellow solid, m.p. 87-88°C, after recrystallization from t-butylmethyl ether; hydrogenation of the latter (13.08 g, 0.051 mol) in THF (125 ml)/ethanol (125 ml) in the presence of 10% Pd/C (1.0 g) to afford 11.5 g (100%) of ethyl 4-amino-2-methoxyphenoxyacetate as a pink oil; and then treatment of the latter (11.5 g, 0.051 mol) in CH₂Cl₂ (100 ml) with pyridine (10 ml), followed by methanesulfonyl chloride (6.43 g, 0.056 mol) to afford 12.328 g (79.6%) of N-[4-(ethoxycarbonylmethoxy)-3-(methoxy)phenyl]methanesulfonamide, m.p. 118-119°C, after purification by treatment with charcoal and filtration through silica eluting with ethyl acetate.

### Example 343

### 4,5-Dihydro-1-(4-chlorophenyl)-4-methyl-3-(4-pyridylmethyl)1H-2,4-benzodiazepine dihydrochloride

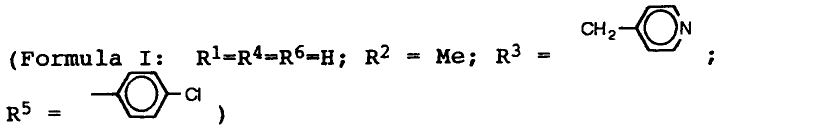

To a mixture of 2-[[(N-methyl)amino]methyl]-α-phenylbenzenemethanamine dihydrochloride (Formula VI: R¹ = H; R² = Me; R⁵ = 4-Cl-Ph; R⁶ = H) (8.34 g, 0.025 mol) in sulfolane (75 ml) and toluene (25 ml) under nitrogen was added triisobutyl aluminum (38.0 ml, 0.076 mol). The mixture was stirred for 30 minutes, and then ethyl 4-pyridylacetate (5.36 g, 0.0325 mol) was added. The mixture was heated to 110°C for 2 hours, the solution was cooled, poured into a saturated Rochelle salt solution (200 ml) and the mixture was diluted with water (200 ml) and extracted with CH₂Cl₂ (3X). The organic extracts were combined, washed with water, and the solvent was removed in vacuo to afford an oil. The oil was treated with ethyl acetate/HCl to afford the hydrochloride salt which was recrystallized from ethanol/acetonitrile, then isopropanol/acetonitrile to afford 4,5-dihydro-1-(4-chlorophenyl)-4-methyl-3-(4-pyridylmethyl)-1H-2,4-benzodiazepine dihydrochloride as a white solid, m.p. 185-190°C (dec.).

### Example 344

### N-[4-(Ethoxycarbonylmethoxy)-3-chlorophenyl] methanesulfonamide

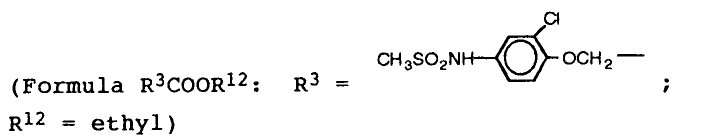

The product was prepared by the alkylation of 2-chloro-4-nitrophenol (17.35 g) in DMF (150 ml) with ethyl bromoacetate (16.7 g) in the presence of potassium carbonate (13.8 g) to afford 19.6 g (75.6%) of ethyl 4-nitro-2-chlorophenoxyacetate as a white solid, m.p. 73-74°C; hydrogenation of the latter (10.0 g, 38.5 mmol) in ethanol (200 ml) on a Parr hydrogenator in the presence of 5% platinum on carbon (sulfided) (200 mg) afforded 8.1 g (92%) of ethyl 4-amino-2-chlorophenoxyacetate, m.p. 59-62°C, after recrystallization from t-butylmethylether, and finally, treatment of the latter (7.3 g, 31.8 mmol) in CH₂Cl₂ (75 ml) at 5°C with pyridine (3.0 mL, 38.7 mmol) and methanesulfonyl chloride (2.29 ml) to afford N-[4-(ethoxycarbonylmethoxy)-3-chlorophenyl]methanesulfonamide, m.p. 87-88°C after recrystallization from tert-butylmethylether.

### Example 348

### (+)-4,5-Dihydro-4-methyl-1-phenyl-3-[2-[(4-methylsulfonylamino)phenyl]ethyl]-1H-2,4-benzodiazepine· 1/2 hydrate

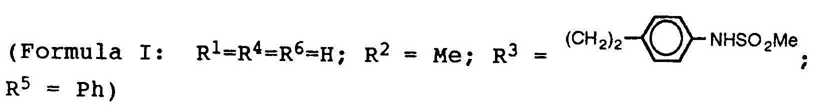

A solution of (+)-4,5-dihydro-4-methyl-1-phenyl-3-[2-[(4-amino)phenyl]ethyl]-1H-2,4-benzodiazepine (3.00 g, 8.44 mmol) in CH₂Cl₂ (50 ml) was treated with an excess of ethereal·HCl, and the dihydrochloride salt which formed was isolated by removing the solvent in vacuo. The dihydrochloride salt was treated with CH₂Cl₂ (60 ml) and pyridine (25 ml) and the solution was cooled to 0°C. Methanesulfonyl chloride (1.00 ml) in CH₂Cl₂ (4.0 ml) was added and the reaction mixture was stirred at 0°C for 30 minutes, then at room temperature for 2.5 hours. The reaction mixture was diluted with CH₂Cl₂ (50 ml), water (100 ml) and a saturated Na₂CO₃ solution (75 ml) and the organic layer was separated and dried over Na₂SO₄. The solvent was removed in vacuo and the residue was recrystallized from ethanol/ether to afford 3.15 g of (+)-4,5-dihydro-4-methyl-1-phenyl-3-[2-[(4-methylsulfonylamino)phenyl]ethyl]-1H-2,4-benzodiazepine·1/2 hydrate, m.p. 210-211°C, [α]_{D}²² = +204° (C = 0.5, MeOH). An additional 0.44 g of product was also obtained by recrystallization of the mother liquor from ethanol/ether.

### Example 351

### (-)-4,5-Dihydro-4-methyl-1-phenyl-3-[2-[(4-methylsulfonylamino)phenyl]ethyl]-1H-2,4-benzodiazepine· 1/2 hydrate

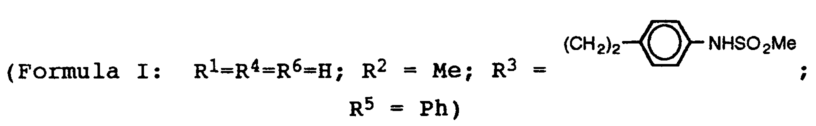

Following a procedure similar to that described in Example 348, there was obtained 2.15 g (71%) of (-)-4,5-dihydro-4-methyl-1-phenyl-3-[2-[(4-methylsulfonylamino)phenyl]ethyl]-1H-2,4-benzodiazepine·1/2 hydrate, m.p. 211.5-212.5 after recrystallization from ethanol/ether (2X); from the dihydrochloride salt of (-)-4,5-dihydro-4-methyl-1-phenyl-3-[2-[(4-amino)phenyl]ethyl]-1H-2,4-benzodiazepine (2.49 g, 7 mmol of free base), CH₂Cl₂ (50 ml), pyridine (20 ml) and methanesulfonyl chloride (0.83 ml, 10.5 mmol).

### Example 353

### 4,5-Dihydro-4-methyl-1-phenyl-3-[2-[(4-amino) phenyl]ethyl]-1H-2,4-benzodiazepine monohydrochloride

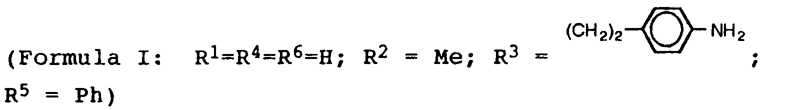

To a solution of 4,5-dihydro-4-methyl-1-phenyl-3-[2-[(4-nitro)phenyl]ethyl]-1H-2,4-benzo diazepine (12.70 g, 0.0278 mol) in ethanol (150 ml) under nitrogen was added 10% palladium on carbon (1.91 g). The reaction mixture was hydrogenated on a Parr hydrogenator at 50 psi for 85 minutes. The catalyst was removed by filtration, the filtrate was concentrated in vacuo and the residue was recrystallized from ethanol/ether and then methanol/t-BuOMe to afford 9.86 (90.5%) of 4,5-dihydro-4-methyl-1-phenyl-3-[2-[(4-amino)phenyl]ethyl]-1H-2,4-benzodiazepine monohydrochloride, m.p. 158-160.5°C.

### Example 354

### 4,5-Dihydro-4-methyl-1-phenyl-3-[2-[(4-ethylsulfonylamino)phenyl]ethyl]-1H-2,4-benzodiazepine monohydrochloride

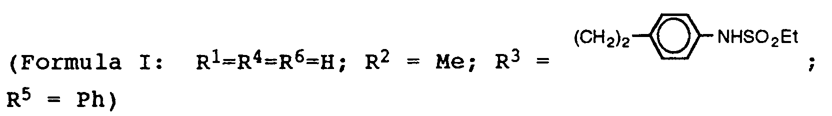

To a cooled solution of 4,5-dihydro-4-methyl-1-phenyl-3-(2-[(4-amino)phenyl]ethyl]-1H-2,4-benzodiazepine monohydrochloride (3.00 g, 7.65 mmol) in CH₂Cl₂ (75 ml) under nitrogen was added pyridine (25 ml), followed by ethanesulfonyl chloride (0.87 ml, 9.18 mmol) in CH₂Cl₂ (5.0 ml). The reaction mixture was stirred at 0°C for 1 hour, and then at room temperature for 1 hour. The mixture was treated with water (50 ml) and saturated Na₂CO₃ (25 ml), and the organic layer was separated. The aqueous layer was extracted with CH₂Cl₂ (100 ml) and the combined organic layers were washed with water (1X) and dried over Na₂SO₄. The solvent was removed in vacuo and the residue was treated with ethanolic·HCl and the hydrochloride salt thus formed was recrystallized from methanol/t-BuOMe to afford 2.915 g of 4,5-dihydro-4-methyl-1-phenyl-3-[2-[(4-ethylsulfonylamino)phenyl]ethyl]-1H-2,4-benzo diazepine monohydrochloride as an orange powder, m.p. 240-241°C.

### Example 356

### 2-Methyl-4-(4-bromophenyl)-1-(2H)-phthalazinone (Formula VIII: R² = Me; R⁵ = 4-Br-Ph; R⁶ = H)

To a suspension of 2-(4-bromobenzoyl)benzoic acid (15.0 g, 49.2 mmol) in toluene (75 ml) at room temperature was added methylhydrazine (2.88 ml, 54.1 mmol). The reaction mixture was heated to reflux while collecting water in a Dean Stark trap for 3 hours, additional methylhydrazine (2.0 ml) was added and refluxing was continued for 15 minutes. The reaction mixture was cooled to room temperature and the product was collected by filtration and dried in vacuo to afford 13.4 g (87%) of 2-methyl-4-(4-bromophenyl)-1-(2H)-phthalazinone, m.p. 168-170°C.

### Example 359

### 2-Methyl-4-(4-nitrophenyl)-1-(2H)-phthalazinone (Formula VIII: R² = Me; R⁵ = 4-NO₂-Ph; R⁶ = H)

Following a procedure similar to that described in Example 356, there was obtained 15.3 g (54%) of 2-methyl-4-(4-nitrophenyl)-1-(2H)-phthalazinone, m.p. 200-202°C, after recrystallization from hot methanol; from 2-(4-nitrobenzyl)benzoic acid (27.1 g, 100 mmol), toluene (500 ml) and methyl hydrazine (6.38 ml, 120 mmol).

### Example 360

### 2-Methyl-4-(4-aminophenyl)-1-(2H)-phthalazinone (Formula VIII: R² = Me; R⁵ = 4-NH₂-Ph; R⁶ = H)

To a suspension of 2-methyl-4-(4-nitrophenyl)-1-(2H)-phthalazinone (3.0 g, 10.7 mmol) in ethanol (150 ml) under N₂ was added 10% Pd/C (0.75 g). The mixture was hydrogenated on a Parr hydrogenator at 50 psi for 2 hours, the catalyst was removed by filtration and the solvent was removed in vacuo to afford 2.67 g (97%) of 2-methyl-4-(4-aminophenyl)-1-(2H)-phthalazinone as a yellow solid.

### Examples 361

### 2-Methyl-4-(4-methylsulfonylaminophenyl)-1-(2H)-phthalazinone (Formula VIII: R² = Me; R⁵ = 4-CH₃SO₂NH-Ph; R⁶ = H)

To a mixture of 2-methyl-4-(4-aminophenyl)-1-(2H)-phthalazinone (8.77 g, 34.9 mmol), CH₂Cl₂ (300 ml) and pyridine (3.11 ml, 38.4 mmol) at 0°C was added dropwise methanesulfonyl chloride (2.97 ml, 38.4 mmol) in CH₂Cl₂ (20 ml) over 15 minutes. The reaction mixture was warmed to room temperature and stirred for 12 hours. Additional pyridine (0.5 ml) and methanesulfonyl chloride (0.5 ml) was added and the mixture was stirred as such for 2 hours. The reaction mixture was poured into 2N HCl, the organic layer was separated and then washed with water and dried over Na₂SO₄. The solvent was removed in vacuo and the residue was azeotroped with toluene and CH₂Cl₂ to afford 9.0 g (78%) of 2-methyl-4-(4-methylsulfonylaminophenyl)-1-(2H)-phthalazinone as a yellow solid.

### Example 365

### 2-(Isonicotinoyl)benzoic acid (Formula XIII: R⁵ = 4-pyridyl; R⁶ = H)

To a warmed mixture of ether (500 ml) and magnesium turnings (14.9 g, 0.614 mol) was added dropwise 2-bromotoluene (105 g, 0.614 mol) and then a crystal of iodine. The reaction mixture was refluxed for 1 hour, then 4-cyanopyridine (30.2 g, 0.29 mol) in THF (130 ml) was added dropwise over 45 minutes. The mixture was refluxed for 1.5 hours, cooled to room temperature and quenched with 6N HCl (200 ml). The organic phase was separated, the aqueous phase was extracted with Et₂O, and the combined ether phases were extracted with 2N HCl. The aqueous acidic phases were combined, basified with concentrated NH₄OH, extracted with Et₂O (3 x 750 ml) and the combined organic layers were dried over Na₂SO₄ and the solvent was removed in vacuo to afford 54 g of crude 2-isonicotinoyltoluene.

The latter (54 g, 274 mmol) was mixed with water (3 l), and MgSO₄ (33 g, 274 mmol) and the mixture was heated to reflux. Potassium permanganate (188 g, 1.99 mol) was then added in portions over 15 minutes. The mixture was refluxed for 6 hours, cooled and filtered through celite. The filtrate was concentrated to 0.5 l, diluted with ethanol (500 ml) and the precipitate which formed was filtered and washed with ethanol. The filtrate was concentrated in vacuo and the residue was treated with acetic acid (50 ml) to give a white solid which was collected by filtration. The solid was triturated with ether and filtered to afford 16.79 g (27%) of 2-(isonicotinoyl)benzoic acid, m.p. 191-193°C.

### Example 366

### 2-Methyl-4-(4-pyridyl)-1-(2H)-phthalazinone (Formula VIII: R² = Me; R⁵ = 4-pyridyl; R⁶ = H)

To a suspension of 2-(isonicotinoyl)benzoic acid (10.0 g, 44.1 mmol) in toluene (50 ml) was added methyl hydrazine (2.59 ml, 48.6 mmol). The mixture was heated to reflux while collecting water in a Dean-Stark trap for 6 hours. Additional methylhydrazine (2.59 ml) was added and the mixture was heated to reflux for another 5 hours. The reaction mixture was cooled to room temperature and the product was collected by filtration. The product was recrystallized from hot methanol to afford 8.5 g (81%) of 2-methyl-4-(4-pyridyl)-1-(2H)-phthalazinone, m.p. 173-174°C.

### Example 367

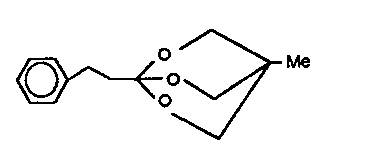

### Orthoester of Formula:

To a solution of hydrocinnamic acid (12.0 g, 79.8 mmol) in ether (400 ml) at room temperature under nitrogen was added dimethylamino pyridine (0.951 g, 7.8 mmol), oxetane (7.89 ml, 87 mmol) and dicyclohexylcarbodiimide (17.9 g, 87 mmol). The mixture was stirred at room temperature for 2 hours, the mixture was filtered and the filtrate was concentrated in vacuo. The residue was distilled under reduced pressure, and then passed through a silica column eluting with ethyl acetate to afford 12.8 g (69%) of an oxetane ester of the formula: 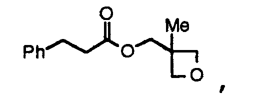
as an oil.

The latter (12.8 g, 54.7 mmol) was dissolved in CH₂Cl₂ (55 ml), cooled to -10°C under nitrogen and then BF₃·Et₂O (1.69 ml, 13.7 mmol) was added via syringe over one minute. The mixture was stirred at -10°C for 2 hours, then at 0°C for 1 hour and the mixture was then warmed to room temperature and quenched with triethylamine. The solvent was removed in vacuo to afford 10.5 g (87%) of the desired orthoester.

### Example 368

### 4,5-Dihydro-4-methyl-1-(4-pyridyl)-3-[2-phenylethyl]-1H-2,4-benzodiazepine fumarate (Formula I: R¹=R⁴=R⁶=H; R² = Me; R³ = CH₂CH₂Ph; R⁵ = 4-pyridyl)

To a mixture of 2-(N-methylaminomethyl)-α-(4-pyridyl)benzenemethanamine trihydrochloride of Example 146A (1.50 g, 4.46 mmol) in methanol (40 ml) at room temperature was added sodium acetate (0.92 g, 11.2 mmol) and the orthoester of Example 367 (1.31 g, 5.58 mmol). The mixture was heated to reflux for 5 hours, additional orthoester (1.31 g) was added and the mixture was refluxed for another 12 hours. The mixture was cooled to room temperature, the solvent was removed in vacuo and the residue was partitioned between half-saturated Na₂CO₃ and t-BuOMe. The aqueous layer was extracted with additional t-BuOMe and the organic layers were combined, dried over Na₂SO₄ and concentrated in vacuo. The residue was treated with fumaric acid and the mixture was dissolved in hot ethanol and diluted with ether. The fumarate salt was collected by filtration to afford 0.9 g (57%) of 4,5-dihydro-4-methyl-1-(4-pyridyl)-3-[2-phenylethyl]-1H-2,4-benzodiazepine fumarate, m.p. 173-176°C.

### Example 369

### 2-(2-Methoxyethyl)-4-(4-chlorophenyl)-1-(2H)-phthalazinone (Formula VIII: R² = CH₂CH₂OMe; R⁵ = 4-Cl-Ph; R⁶ = H)

A slurry of 2-(4-chlorobenzoyl) benzoic acid (104 g, 0.4 mol) in toluene (400 ml) was treated with hydrazine hydrate (22.5 g, 0.45 mol) and the mixture was heated to reflux for 1.5 hours with removal of water by a Dean Stark trap. The reaction mixture was cooled and 101.28 g (98.7%) of 4-(4-chlorophenyl)-1-(2H)-phthalazinone, m.p. 270-272°C, was collected.

The latter (25.6 g, 0.1 mol) was then added over 2 hours to a suspension of sodium hydride (4.8 g, 0.12 mol, prewashed with hexane) in DMSO (250 ml) under nitrogen. The mixture was stirred for 3/4 hour and then 2-chloroethylmethyl ether (12.29 g, 0.13 mol) in DMSO (25 ml) was added over 5 minutes. The reaction mixture was stirred overnight. The mixture was heated to 40°C for 1.5 hours, additional 2-chloromethylmethyl ether (2.5 g) was added and the mixture was heated at 40°C for 4 hours The reaction mixture was cooled, poured into water (1 l) and the precipitate which formed was collected by filtration and washed with water, then hexane. The solid was recrystallized from hot ethanol to afford 26.8 g (85%) of 2-(2-methoxyethyl)-4-(4-chlorophenyl)-1-(2H)-phthalazinone, m.p. 116.5-117.5°C.

### Example 374

### 2-Methyl-4-(4-isopropylphenyl)-1-(2H)-phthalazinone (Formula VIII: R² = Me; R⁵ = 4-iPr-Ph; R⁶ = H)

Methyl hydrazine (8.05 g, 0.175 mol) was added to a mixture of 2-(4-isopropylbenzoyl)benzoic acid (40.2 g, 0.15 mol) in toluene (350 ml) and the mixture was heated to reflux for 3 hours with the removal of water with a Dean Stark trap. The reaction mixture was hot filtered through celite, the filtrate was concentrated to 125 ml and the filtrate was diluted with hexane (100 ml) and cooled. A solid was collected by filtration to afford 27.97 g (67%) of 2-methyl-4-(4-isopropylphenyl)-1-(2H)-phthalazinone, m.p. 107-107.5°C.

### Example 378

### (a) 4,5-Dihydro-4-methyl-1-phenyl-3-[1-(4-nitrophenyl) methyl]-1H-2,4-benzodiazepine fumarate

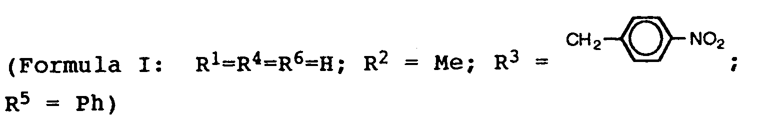

Anhydrous sodium acetate (2.62 g, 32 mmol) was added to a mixture of 2-(N-methylaminomethyl)-α-phenylbenzenemethanamine of Example 132 (4.78 g, 16 mmol), the imino ester hydrochloride of formula PhCH₂C(NH)OCH₃·HCl (11.06 g, 48 mmol) and methanol (80 ml) and the mixture was stirred at room temperature for 64 hours. The reaction mixture was filtered, the filtrate was concentrated in vacuo, and the residue was dissolved in methanol and diluted with ether. The solvent was decanted, the residue was washed with additional ether, and then was treated with CH₂Cl₂ and a water/saturated Na₂CO₃ solution. The layers were separated, the aqueous layer was extracted with CH₂Cl₂ and the combined organic layers were washed with water containing saturated Na₃CO₃ (3 drops), dried over Na₂SO₄ and concentrated in vacuo. The residue was purified by column chromatography on silica eluting with 3% isopropylamine/CH₂Cl₂ to afford an oil which was crystallized by the addition of ether. The solid product was dissolved in ethanol, treated with fumaric acid (2.4 g) and diluted with ether to afford the fumarate salt which was collected by filtration. The fumarate salt was recrystallized from methanol/ether to afford 2.78 g (36%) of 4,5-dihydro-4-methyl-1-phenyl-3-[1-(4-nitrophenyl)methyl]-1H-2,4-benzodiazepine fumarate, m.p. 205.5-207°C.

### (b) 4,5-Dihydro-4-methyl-1-phenyl-2-[1-(4-aminophenyl) methyl]-1H-2,4-benzodiazepine dihydrochloride

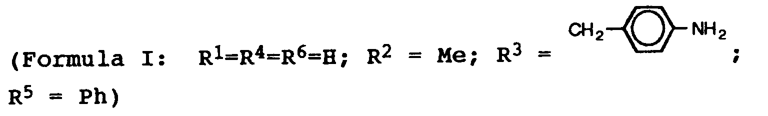

To a solution of the 2,4-benzodiazepine of Example 378(a) (0.49 g, 1 mmol) in methanol (50 ml) was added 10% Pd/C (0.07 g) and the mixture was hydrogenated on a Parr hydrogenator at 54 psi for 25 minutes. The catalyst was removed by filtration through solka floc and the filtrate was concentrated in vacuo and the residue was combined with the crude product obtained from a similar experimental run starting with 1.8 g of the 2,4-benzodiazepine of Example 378(a). The combined residue was dissolved in CH₂Cl₂, basified with saturated Na₂CO₃ and the layers were separated. The aqueous phase was extracted with CH₂Cl₂ and the organic layers were combined, dried over Na₂SO₄ and acidified with ethereal HCl. The solvent was removed in vacuo and the residue was recrystallized from methanol/ether to afford 1.49 g (76%) of 4,5-dihydro-4-methyl-1-phenyl-3-[1-(4-aminophenyl)methyl]-1H-2,4-benzodiazepine dihydrochloride.

### (c) 4,5-Dihydro-4-methyl-1-phenyl-3-[1-(4-methylsulfonyl-aminophenyl)methyl]-1H-2,4-benzodiazepine

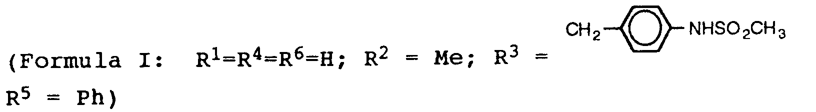

To a cooled mixture of the diamine dihydrochloride of Example 378(b) (1.16 g, 28 mmol), CH₂Cl₂ (25 ml) and pyridine (6 ml) under nitrogen was added methanesulfonyl chloride (0.64 g, 56 mmol). The reaction mixture was stirred, with cooling in ice-bath, for 1 3/4 hours and then was poured into a solution of water/saturated Na₂CO₃. The layers were separated, the aqueous layer was extracted with CH₂Cl₂, and the organic layers were combined and washed with water/saturated Na₂CO₃. The organic layer was dried over Na₂SO₄, the solvent was removed in vacuo, and the residue was diluted with hexane. A precipitate formed which was slurried with t-BuOMe and collected by filtration. The solid was recrystallized from hot CH₂Cl₂/hexane to afford 0.85 g (73%) of 4,5-dihydro-4-methyl-1-phenyl-3-[1-(4-methylsulfonylaminophenyl)methyl]-1H-2,4-benzodiazepine as an off-white solid, m.p. 182-185°C.

### Example 379

### 4,5-Dihydro-4-methyl-1-phenyl-3-[2-(2-methylsulfonyl-aminophenyl)ethyl]-1H-2,4-benzodiazepine hydrochloride

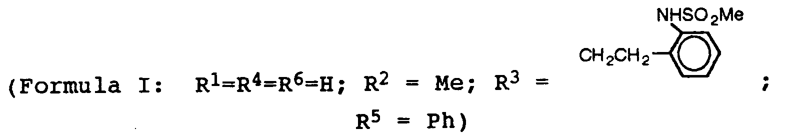

To a mixture of 4,5-dihydro-4-methyl-1-phenyl-3-[2-(2-aminophenyl)ethyl-1H-2,4-benzodiazepine dihydrochloride of Example 253 (3.7 g, 8.6 mmol), CH₂Cl₂ (65 ml), and pyridine (17 ml, 0.21 mol) at 0°C under nitrogen was added methanesulfonyl chloride (1.67 g, 14.6 mol) in CH₂Cl₂ (2.0 ml). The mixture was stirred as such for 2 hours, diluted with water (100 ml) and basified with a saturated Na₂CO₃ solution. The layers were separated, the aqueous layer was extracted with CH₂Cl₂ and the combined organic extracts were washed with water containing 1 ml saturated Na₂CO₃. The solvent was dried over Na₂SO₄, and concentrated in vacuo to afford a residual oil. The oil was crystallized from t-BuOMe (50 ml) and hexane (100 ml) and the crystalline product was recrystallized from CH₂Cl₂ (10 ml)/t-BuOMe (10 ml)/hexane (40 ml) to afford 3.34 g (90%) of the product as the free base, m.p. 208-212°C. The free base was slurried with methanol (20 ml), acidified with ethereal HCl, and diluted with ether to afford a precipitate which was collected by filtration to afford 3.62 g (90%) of 4,5-dihydro-4-methyl-1-phenyl-3-[2-(2-methylsulfonylaminophenyl)ethyl]-1H-2,4-benzodiazepine hydrochloride as a white solid, m.p. 245-246°C.

### Example 380

### Methyl 3-(2-pyridyl)propionate

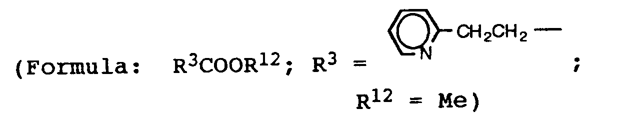

To a solution of methyl triphenylphosphoranylideneacetate (31.2 g) in CH₂Cl₂ (120 ml) was added dropwise over 15 minutes 2-pyridine carboxaldehyde (8.9 ml, 0.093 mol) in CH₂Cl₂ (40 ml). The mixture was heated to reflux for 8 hours, additional ylide (3.1 g) was added and the mixture was refluxed overnight. The reaction mixture was cooled, concentrated to 1/2 value and allowed to stand for 2 hours. A solid was collected by filtration and was washed with CH₂Cl₂/hexane. The filtrate was concentrated in vacuo and the residue was vacuum distilled at 80-107°C and 1.5 mm Hg to afford 12.2 g (80%) of methyl 3-(2-pyridyl)-2-propenoate.

A mixture of the latter (12.2 g), ethanol (150 ml) and 10% Pd/C (1.22 g) was hydrogenated on a Parr hydrogenator for about 15 hours, the catalyst was removed by filtration and the filtrate was concentrated in vacuo to afford 12.26 g of methyl 3-(2-pyridyl)propionate.

### Example 382

### Methyl-3-(3-pyridyl)propionate

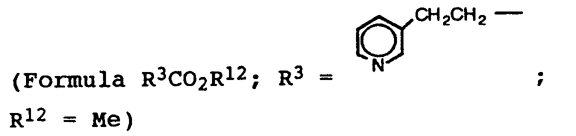

A mixture of 3-bromopyridine (4.82 ml, 0.05 mol), palladium acetate (0.22 g, 0.02 equiv.), tri-(o-tolyl)phosphine (0.61 g, 0.04 equiv.), methyl acrylate (9.01 ml, 2 equiv.), triethylamine (12.5 ml) and CH₃CN (25 ml) was sealed in a bomb and heated to 100°C for 3.5 hours. The mixture was cooled in an ice-bath, the bomb was vented and the reaction mixture was concentrated in vacuo. The residue was extracted with 0.5N HCl/Et₂O (3X), the organic layer was backwashed with additional 0.5N HCl and the aqueous layers were combined, basified with saturated NaHCO₃ and extracted with ether. The ether layer was washed with brine, dried over Na₂SO₄ and concentrated in vacuo to afford 7.89 g (97%) of methyl 3-(3-pyridyl)-2-propenoate.

A mixture of the latter (9.34 g, 0.057 mol), ethanol (100 ml) and 10% Pd/C was hydrogenated on a Parr hydrogenator for 3 hours. The catalyst was removed by filtration and the filtrate was concentrated in vacuo to afford 8.75 g (93%) of methyl 3-(3-pyridyl)propionate.

### Example 384

### (a) 4,5-Dihydro-4-methyl-1-(4-methoxyphenyl)-3-[2-(4-nitrophenyl)ethyl]-1H-2,4-benzodiazepine hydrochloride

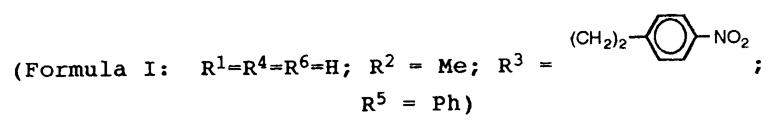

A mixture of the diamine dihydrochloride of Example 126C (7.15 g, 0.022 mol), the ortho ester of the formula (MeO)₃C-(CH₂)₂-4-NO₂-Ph (15 g), sodium acetate (4.3 g) and isopropyl acetate (150 ml) was refluxed for 2.5 hours. The mixture was filtered while hot, and the filtrate was extracted with 2N NaOH/CH₂Cl₂. The mixture was filtered through solka floc, the pad was washed with brine, 2N NaOH and water. The filtrate was dried over Na₂SO₄ and concentrated in vacuo. The residue was recrystallized from methanol/ether to afford 4.7 g of the free base which was converted into the hydrochloride salt for use in the next step.

### (b) 4,5-Dihydro-4-methyl-1-(4-methoxyphenyl)-3-[2-(4-aminophenyl)ethyl]-1H-2,4-benzodiazepine dihydrochloride

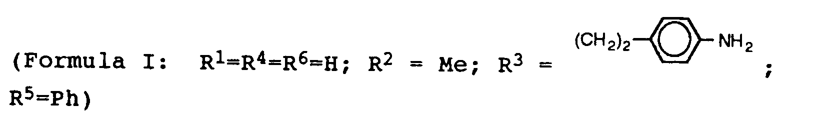

A mixture of the benzodiazepine of Example 384(a) (4.5 g, 0.01 mol), ethanol (120 ml) and 10% Pd/C was hydrogenated in a Parr hydrogenator for 1.5 hours. The catalyst was removed by filtration and the filtrate was concentrated in vacuo. The residue was converted into the dihydrochloride salt, and the salt was recrystallized from EtOH/CH₃CN/Et₂O then MeOH/CH₃CN/Et₂O. The mother liquor was concentrated in vacuo, the residue converted back into the free base, and the free base was purified by column chromatography on silica eluting with t-BuOMe/2-4% isopropylamine. The residue was converted into the dihydrochloride salt and the salt was combined with that obtained above to afford 4.82 g (54%) of 4,5-dihydro-1-(4-methoxyphenyl)-3-[2-(4-aminophenyl)ethyl]-1H-2,4-benzodiazepine dihydrochloride.

### (c) 4,5-Dihydro-4-methyl-1-(4-methoxyphenyl)-3-[2-(4-methylsulfonylaminophenyl)ethyl]-1H-2,4-benzodiazepine hydrochloride

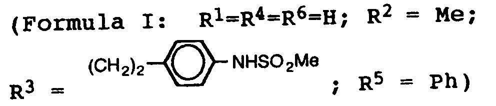

To an ice-cooled solution of the amine of Example 384(b) (3.33 g, 7.3 mmol) in CH₂Cl₂ (35 ml) was added pyridine (17 ml), followed by methanesulfonyl chloride (0.84 ml) in CH₂Cl₂ (10 ml). The mixture was warmed to room temperature, stirred for 1.5 hours, and poured into saturated K₂CO₃ (50 ml). The organic layer was separated and the precipitate which formed over 2 hours was collected by filtration. The solid was converted into the fumarate salt, which was purified by column chromatography on neutral alumina eluting with 5-6% methanol in CH₂Cl₂. The purified salt was then converted back into the free base and then into the hydrochloride salt to afford 1.224 g of 4,5-dihydro-4-methyl-1-(4-methoxyphenyl)-3-[2-(4-methylsulfonylaminophenyl)ethyl]-1H-2,4-benzodiazepine hydrochloride as a light yellow powder, m.p. 149°C (dec.).

### Example 386

### 2-Methyl-4-(3,4-dichlorophenyl)-1-(2H)-phthalazinone (Formula VIII: R² = Me, R⁵ = 3,4-(Cl)₂-Ph; R⁶ = H)

To a solution of 2-(3,4-dichlorobenzoyl)benzoic acid (29.4 g, 0.1 mol) in toluene (100 ml) was added methyl hydrazine (5.8 ml). The mixture was brought to reflux for 3 hours, with the removal of water via a Dean Stark trap. The mixture was cooled, and the product was collected by filtration to afford 25.54 g (84%) of 2-methyl-4-(3,4-dichlorophenyl)-1-(2H)-phthalazinone, m.p. 179-181°C.

### Example 389

### 2-Methyl-4-(3-methylsulfonylamino-4-chlorophenyl)-1-(2H)-phthalazinone (Formula VIII: R² = Me; R⁵ = 3-CH₃SO₂NH-4-Cl-Ph; R⁶ = H)

To a mixture of 2-methyl-4-(3-amino-4-chlorophenyl)-1-(2H)-phthalazinone (38.72 g, 0.136 mol), CH₂Cl₂ (380 ml) and pyridine (75 ml) at 0°C was added methanesulfonyl chloride (15.75 ml) in CH₂Cl₂ (75 ml). The reaction mixture was stirred overnight, poured into 1N HCl (400 ml) and stirred for 45 minutes. The mixture was filtered and the filtrate layers were separated. The solid thus obtained was treated with saturated NaHCO₃, filtered, stirred with hexane, then filtered, stirred with hot methanol, then filtered and finally stirred with hot ethyl acetate and filtered to afford 38.8 g (78%) of 2-methyl-4-(3-methylsulfonylamino-4-chlorophenyl)-1-(2H)-phthalazinone, m.p. 234-236°C.

### Example 394

### Methyl 3-(4-pyridyl)propionate

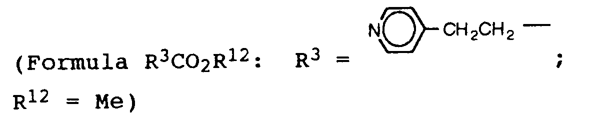

A mixture of 4-bromopyridine (40.6 g, 0.25 mmol), palladium acetate (1.16 g, 5.14 mmol), tri-(O-tolyl)phosphine (3.14 g, 10.3 mmol), CH₃CN (129 ml), triethylamine (64.3 ml, 0.46 mmol) and methyl acrylate (46.8 ml, 0.514 mmol) was sealed in a bomb and placed in an oil bath at 120°C for 4 hours. The mixture was cooled to 0°C, the bomb was vented, and the slurry thus obtained was partitioned between CH₂Cl₂/water. The aqueous phase was separated, extracted with CH₂Cl₂ (2 X 200 ml) and the combined organic layers were dried over MgSO₄. The solvent was removed in vacuo to afford 38 g of methyl 3-(4-pyridyl)propenoate.

To a solution of the latter (10.07 g, 0.06 mol) in ethanol (200 ml) was added 10% Pd/C (1.1 g). The mixture was hydrogenated on a Parr hydrogenator for 2 hours, then the catalyst was removed by filtration and the filtrate was concentrated in vacuo to afford 10.09 g (100%) of methyl 3-(4-pyridyl)propionate.

### Example 400

### 1-Methyl-4-(2,4-dichlorophenyl)-1-(2H)-phthalazinone Formula VIII: R² = Me; R⁵ = 2,4-(Cl)₂-Ph; R⁶ = H)

A mixture of 2-(2,4-dichlorobenzoyl)benzoic acid (31.37 g, 0.106 mol), toluene (100 ml) and methyl hydrazine (6.2 ml) was refluxed for 3 hours with the removal of water via a Dean Stark trap. The mixture was cooled, and the product was collected by filtration and dried to afford 20.74 g of 1-methyl-4-(2,4-dichlorophenyl)-1-(2H)-phthalazinone as an off-white powder, m.p. 145-146°C.

### Example 404

### 2-Methyl-4-(3-chloro-4-methoxyphenyl)-1-(2H)-phthalazinone (Formula VIII: R² = Me; R⁵ = 3-Cl-4-CH₃O-Ph; R⁶ = H)

A mixture of 2-(3-chloro-4-methoxybenzoyl)benzoic acid (10.46 g, 0.036 mol), toluene (50 ml) and methyl hydrazine (2.1 ml) was refluxed in the presence of a Dean Stark trap until the theoretical amount of water had been collected. The reaction mixture was cooled, and the product was collected by filtration to afford 7.42 g (68.7%) of 2-methyl-4-(3-chloro-4-methoxyphenyl)-1-(2H)-phthalazinone, m.p. 195-195°C. An additional 1.89 g of product was also collected from the filtrate for a total of 9.31 g (86.2%).

### Example 407

### 2-Methyl-4-(4-methylphenyl)-1-(2H)-phthalazinone (Formula VIII: R² = Me; R⁵ = 4-CH₃-Ph; R⁶ = H)

Following a procedure similar to that described above for Example 404, there was prepared 25.78 g (82.4%) of 2-methyl-4-(4-methylphenyl)-1-(2H)-phthalazinone, m.p. 150-152°C, from 2-(p-tolyl)benzoic acid (30.0 g, 0.125 mol), toluene (100 ml) and methyl hydrazine (7.3 ml).

### Example 411

### 2-(2,4-diethylbenzoyl)benzoic acid (Formula XIII: R⁵ = 2,4-(Et)₂-Ph; R⁶ = H)

To a cooled mixture of phthalic anhydride (50 g, 0.34 mol) and aluminum chloride (99 g) in tetrachloroethane (300 ml) was added dropwise 1,3-diethylbenzene (50 g) over 1 1/4 hours. The mixture was stirred in an ice bath for 30 minutes, additional tetrachloroethane was added, and the mixture was stirred for 2 hours. The reaction mixture was poured into concentrated HCl/ice (1 l), ether (500 ml) was added and the mixture was filtered. The organic layer was separated, washed with saturated Na₂CO₃ (6X), the basic layer was extracted with CH₂Cl₂ and the CH₂Cl₂ layer was extracted with 2N NaOH and the 2N NaOH layer was acidified. The basic layer was acidified with aqueous HCl, this acidic layer was combined with the above acidified 2N NaOH layer and the mixture was extracted with ether. The CH₂Cl₂ layer and the ether layer were combined and concentrated in vacuo. The residue was slurried with water, filtered and rinsed with hexane to afford 88.8 g (93%) of 2-(2,4-diethylbenzoyl)benzoic acid, m.p. 115-118°C.

### Example 412

### 2-Methyl-4-(2,4-diethylphenyl)-1-(2H)-phthalazinone (Formula VIII: R² = Me; R⁵ = 2,4-(Et)₂-Ph; R⁶ = H)

A mixture of 2-(2,4-diethylbenzoyl)benzoic acid (40 g, 0.14 mol), toluene (120 ml) and methyl hydrazine (7.5 ml) were refluxed for 3 hours with removal of water via a Dean Stark trap. Additional toluene (100 ml) and methyl hydrazine (4.0 ml) were added and the mixture was refluxed until 2.8 ml of water was collected. The mixture was cooled, extracted with CH₂Cl₂/2N NaOH, then 1N HCl and the organic layer was separated, dried over Na₂SO₄ and concentrated in vacuo. The residue was crystallized from ethanol/water and recrystallized from ethanol/water to afford 22.86 g (55.9%) of 2-methyl-4-(2,4-diethylphenyl)-1-(2H)-phthalazinone, m.p. 94.5-96.5°C.

### Example 416

### 4,5-Dihydro-1-(4-hydroxyphenyl)-4-methyl-3-[2-(4-pyridyl)-ethyl]-1H-2,4-benzodiazepine dihydrochloride

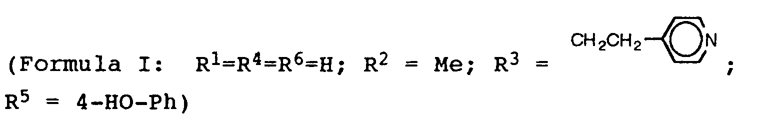

To a mixture of 4,5-dihydro-1-(4-methoxyphenyl)-4-methyl-3-[2-(4-pyridyl)ethyl]-1H-2,4-benzodiazepine dihydrochloride (3.81 g, 8.6 mmol, prepared from the fumarate of Example 398 by standard procedures) in CH₂Cl₂ (40 ml) in an ice-bath under N₂ was added boron tribromide (17.2 ml, 1M in CH₂Cl₂). The mixture was stirred for 3 hours, 2N HCl was added and the mixture was stirred for 1/2 hour. The layers were separated, the aqueous layer was extracted with CH₂Cl₂ and the combined organic layers were washed with 2N HCl. The organic layer was treated with saturated Na₂CO₃ and the product which precipitated was collected by filtration to afford 3.06 g (83%) of 4,5-dihydro-1-(4-hydroxyphenyl)-4-methyl-3-[2-(4-pyridyl)ethyl]-1H-2,4-benzodiazepine dihydrochloride, m.p. 258-259°C after recrystallization from methanol/ether.

### Example 417

### 4,5-Dihydro-1-phenyl-2-methyl-3-[4-methylsulfonylaminophenyl]-1H-2,4-benzodiazepine·ethanol

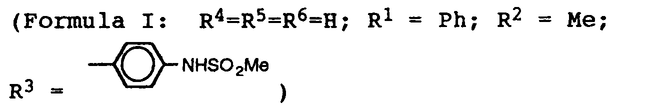

Following a procedure similar to that described in General Method F2, but refluxing the material for 6 hours rather than for 50 minutes to 2.5 hours, there was obtained 3.863 g (57.8%) of 4,5-dihydro-1-phenyl-2-methyl-3-[4-methylsulfonylaminophenyl]-1H-2,4-benzodiazepine·ethanol, m.p. 123.5-126°C after recrystallization for ethanol/ether then ethanol; from 2-aminomethyl-N-methyl-α-phenylbenzenemethanamine of Example 175 (5.0 g, 0.0167 mol), trimethyl aluminum (29.24 ml, 0.05848 mol), sulfolane (50 ml) and N-[4-(ethoxycarbonyl)phenyl]methanesulfonamide (4.27 g, 0.01754 mol).

### Example 418

### 4,5-Dihydro-1-(4-methylsulfonylaminophenyl)-4-methyl-3-[2-(4-methylsulfonylaminophenyl)ethyl]-1H-2,4-benzodiazepine hydrochloride

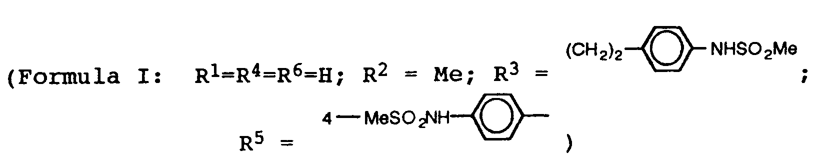

To a solution of the diamine dihydrochloride of Example 362 (1.96 g, 5.00 mmol) in sulfolane (30 ml) at room temperature was added 2M trimethylaluminum (8.74 ml, 17.5 mmol) over 10 minutes. The mixture was stirred for 30 minutes then N-[4-(2-(ethoxycarbonyl)ethyl)phenyl]methanesulfonamide of Example 326 (1.42 g, 5.25 mmol) was added. The mixture was heated to 100-120°C for 2 hours, cooled to room temperature and quenched with a Rochelle salt solution (50 ml). CH₂Cl₂ (100 ml) and water (50 ml) were added, followed by a half saturated solution of Na₂CO₃ (25 ml) and then additional CH₂Cl₂ (100 ml). The organic layer was separated, and the aqueous layer was acidified with 2N HCl, filtered and then extracted with CH₂Cl₂. The combined organic layers were dried over Na₂SO₄ and concentrated in vacuo. The residue was azeotroped with ethanol, the solvent was removed in vacuo. The residue was purified by column chromatography on silica eluting with CHCl₃/iPrOH/CF₃CO₂H (67/30/3) then CHCl₃/EtOH/CF₃CO₂H (67/30/3) to afford the product as the CF₃CO₂H salt. The CF₃CO₂H salt was treated with ethanolic HCl and the HCl salt thus obtained was recrystallized from hot ethanol to afford 1.07 g (41%) of 4,5-dihydro-1-(4-methylsulfonylaminophenyl)-4-methyl-3-[2-(4-methylsulfonylaminophenyl)ethyl]-1H-2,4-benzodiazepine hydrochloride, m.p. 274-275°C.

### Example 419

### (a) 2-Methyl-4-phenyl-8-amino-1-(2H)-phthalazinone hydrochloride (Formula VIII: R² = Me; R⁵ = Ph; R⁶ = 8-NH₂)

A mixture of 2-benzoyl-6-aminobenzoic acid (5.4 g, 22.4 mmol), methyl hydrazine (2.30 g, 50 mmol), ethanol (50 ml) and toluene (25 ml) was heated at 70°C for 2 hours and then at reflux for 22 hours with removal of water via a Dean Stark trap. Additional methyl hydrazine (0.6 g) was added and the mixture was refluxed for 5 hours. The solvent was removed in vacuo, the residue was dissolved in CH₂Cl₂, additional crude product (0.4 g) was added from a similar experimental run, and the mixture was diluted with water and 2N NaOH. The aqueous layer was separated, and extracted with CH₂Cl₂, and the combined organic layers were washed with water containing 2N NaOH (1 ml), dried over Na₂SO₄ and concentrated in vacuo. The residue was treated with ethanolic HCl and the mixture was diluted with ether to afford the HCl salt which was collected by filtration and dried at 85°C in vacuo to afford 5.40 g (79%) of 2-methyl-4-phenyl-8-amino-1-(2H)-phthalazinone hydrochloride, m.p. 199-213°C.

### (b) 2-Methyl-4-phenyl-8-methylsulfonylamino-1-(2H)-phthalazinone (Formula VIII: R² = Me; R⁵ = Ph; R⁶ = 8-NHSO₂Me)

A mixture of the phthalazinone of Example 419(a) (5.2 g, 17 mmol) CH₂Cl₂ (90 ml), and pyridine (23 ml) was cooled in an ice-bath and treated with methanesulfonyl chloride (6.18 g, 54 mmol) in CH₂Cl₂ (5 ml). The mixture was warmed to room temperature over 1.5 hours and then was stirred for 4 hours. The solvent was removed in vacuo, the residue was diluted with water (60 ml) and concentrated HCl (15 ml). The mixture was filtered, and the collected precipitate was slurried with water and saturated Na₂CO₃ (5 ml), and this mixture was filtered and washed with water. The product was then slurried with acetone/hexane (1/1, 40 ml) and collected by filtration to afford 5.73 g (100%) of 2-methyl-4-phenyl-8-methylsulfonylamino-1-(2H)-phthalazinone, m.p. 217-218°C.

### Example 421

### 4,5-Dihydro-1-phenyl-3-[2-phenylethyl]-2-methyl-6-methylsulfonylamino-1H-2,4-benzodiazepine hydrochloride (Formula I: R¹=R⁴=H; R² = Me; R³ = (CH₂)₂Ph; R⁵ = Ph; R⁶ = 6-CH₃SO₂NH)

A mixture of 2-(N-methylaminoethyl)-3-methylsulfonylamino-α-phenylbenzenemethanamine of Example 420 (0.70 g, 2.6 mmol), the ortho ester of formula Ph(CH₂)₂C(OMe)₃ (2.18 g, 10.4 mol), methanol (8 ml), acetic acid (0.62 g, 10.4 mol) and methanolic HCl (2 drops) was stirred at room temperature for 1 day, then additional methanolic HCl (2 drops) was added and the mixture was stirred for 24 hours. Additional methanolic HCl was added and the reaction was stirred for another four days. The reaction mixture was acidified with methanolic HCl, diluted with ether, and cooled in an ice-bath and the precipitate thus obtained was collected by filtration and washed with methanol/ether to afford 0.75 g (68%) of 4,5-dihydro-1-phenyl-3-[2-phenylethyl]-2-methyl-6-methylsulfonyl-amino-1H-2,4-benzodiazepine hydrochloride, m.p. 270-270.5°C.

### BIOLOGICAL TEST RESULTS

The compounds of this invention having formula XXXVI have antiarrhythmic activity as shown by the results of standard pharmacological tests carried out on representative examples as described below.

Antiarrhythmic activity was demonstrated by a procedure, which is a modification of standard programmed electrophysiological techniques utilized in large animals and in clinical studies in humans. Male Duncan-Hartley guinea pigs (600-800 grams) were anesthetized with sodium pentobarbital (30 mg/kg, i.p.) and artificially ventilated with a Harvard small-animal respirator. A left thoracotomy was performed and a fluid-filled catheter and transducer (Millar Micro-tip, Model 4F, Millar Inst. Inc., Houston, Texas) were inserted through the anterior wall of the left ventricle to monitor left ventricular pressure (LVP). The first derivative of the LVP (dP/dt) was obtained from a Grass differentiator (Model 7P20B) and used as an index of contractile function. A lead II EKG along with LVP and dP/dt were continuously recorded on a Grass polygraph (Model 7B). Rate pressure product (RPP), an index of cardiac work, was calculated using peak systolic LVP and heart rate (HR).

Effective refractory periods (ERP) were evaluated during left ventricular pacing. Grass subcutaneous electrodes were implanted as bipolar ventricular electrodes to deliver stimuli from a Bloom DTU-2 stimulator (Bloom Electronics, Inc., Reading, Pennsylvania) and stimulus isolation unit. Hearts were stimulated at the slowest frequency allowing consistent pacing (S1, 240-300 bpm) using 2 ms pulses at twice diastolic threshold. Threshold was determined by increasing the stimulation voltage until a 1:1 capture of the ventricular response with the stimulus was observed. A train of 8 normal pulses was delivered followed by a premature (S2) pulse. The interval between the last S1 and the premature S2 pulse was reduced in 10-ms increments until a ventricular response was not initiated. The longest S1-S2 interval that failed to produce a ventricular response was defined as the ERP. Pacing stimuli and the EKG were displayed at a sampling frequency of 92 Hz on an Apple IIe microcomputer using a two-channel 8-bit A/D converter (R.C. Electronics, Compu-Scope APL-D2, Santa Barbara, California).

Baseline hemodynamic function was evaluated followed by ventricular pacing to determine ERP. Pacing was discontinued prior to drug administration and resumed at set intervals during the protocol to evaluate ERP. Test compounds were administered (1 ml/kg) via the left ventricular catheter over a 15-second interval for doses less than 10 mg/kg. Higher doses (>10 mg/kg) were slowly infused over a 1-minute interval. Doses were cumulatively increased every 15 minutes until a maximally tolerated dose which reduced dP/dt by 50% was noted. Ten minutes after each dose, hemodynamics and ERP were reevaluated.

Data were analyzed using an analysis of variance for repeated measures of raw data and are expressed as means. An effective dose to increase ERP by a minimum of 20 msecs (ED₂₀), which was consistently a statistically significant increase, was derived for each animal from a linear regression of the data and expressed as a mean for the treated population. Biological significance was established at a probability of error less than 0.05. The results are presented in Table N.

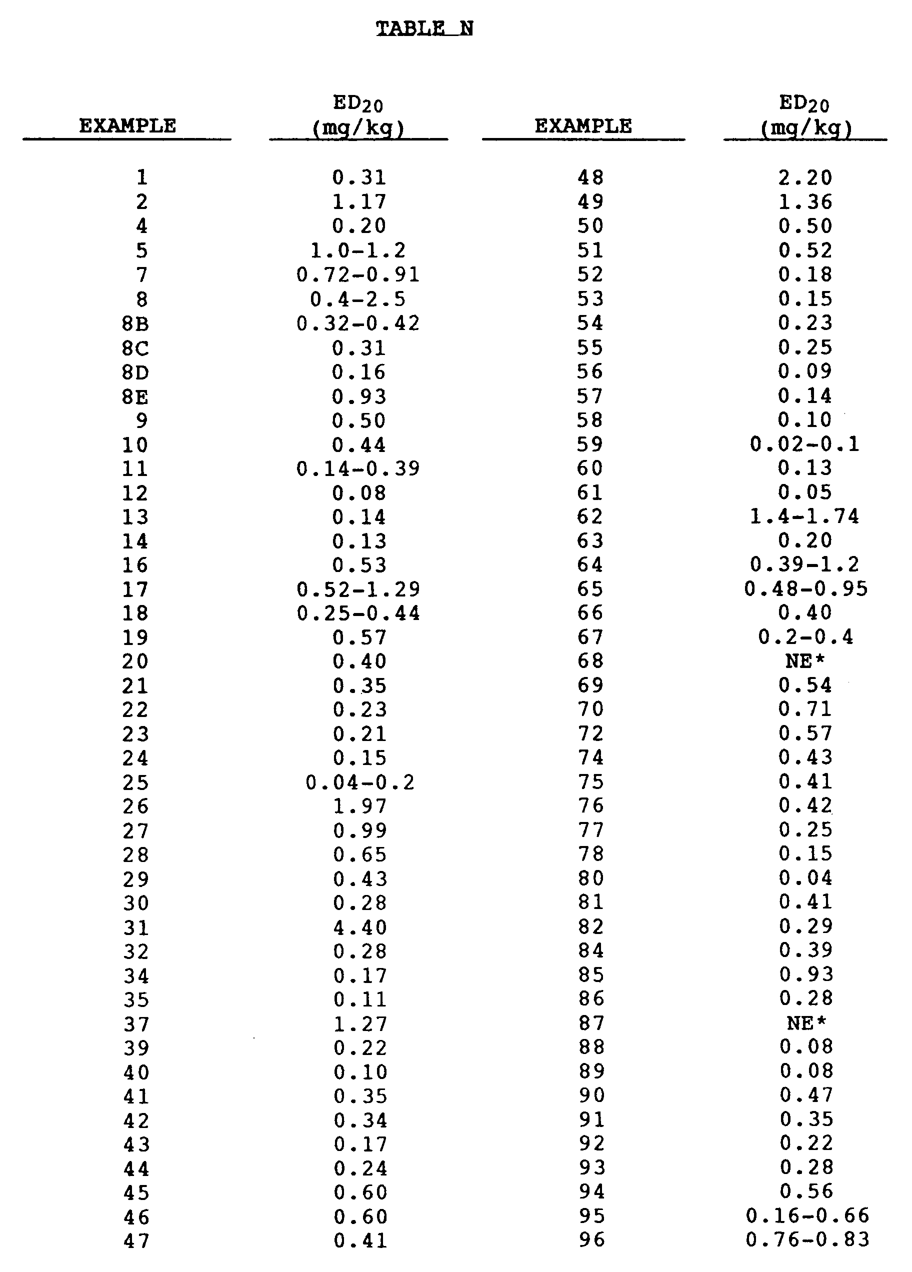

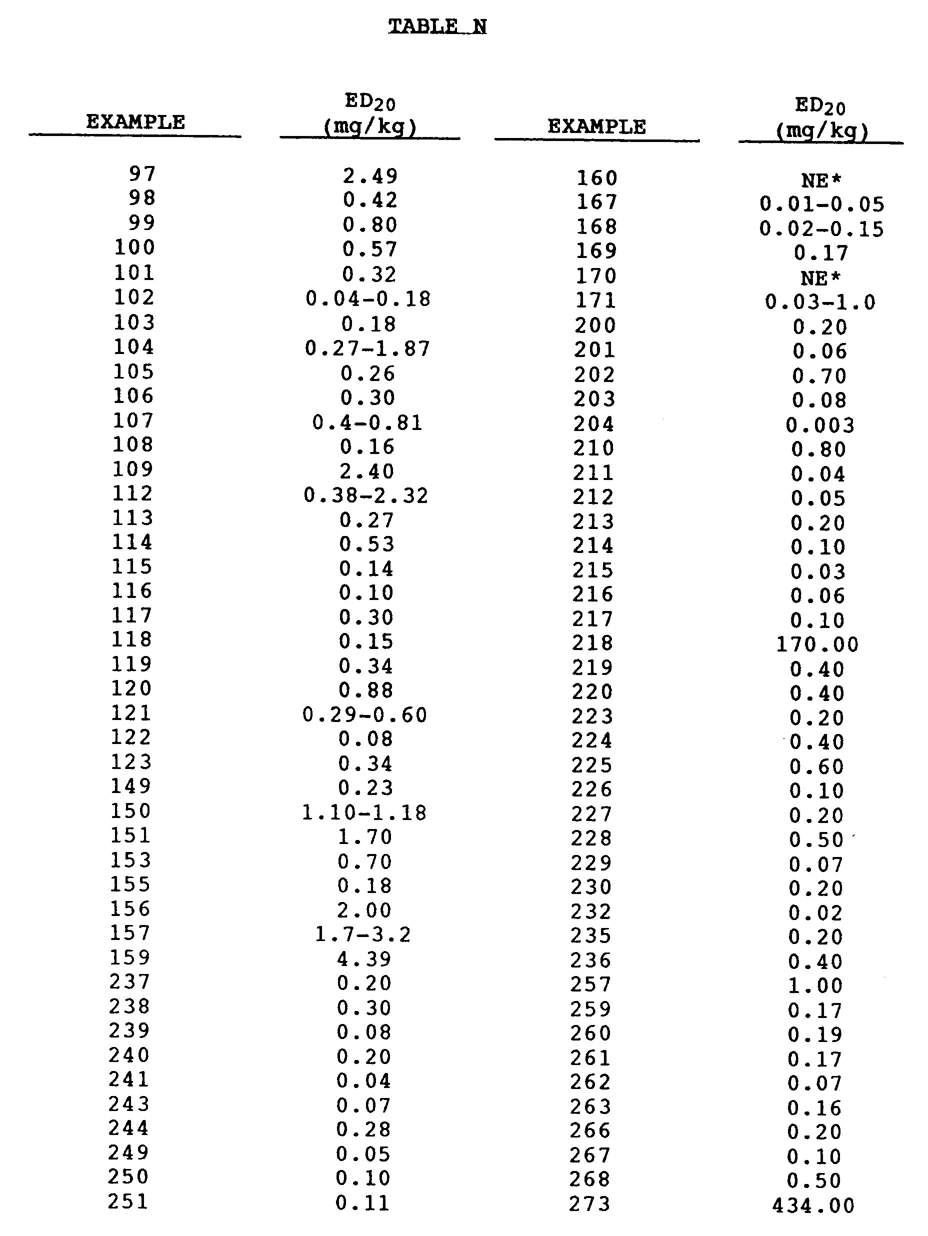

The antiarrhythmic activity of representative compounds of the invention was also demonstrated by the following procedure:

### Rabbit Langendorff Model:

New Zealand White male rabbits (2.5 - 3.5 kg, Hazelton Farms, N.Y.) were anesthetized with 35 mg/kg sodium pentobarbital intravenously via an ear vein. Heparin (1000 U) was subsequently injected. A tracheotomy was performed and the animal was intubated and ventilated with room air. A medialsternal thoracotomy was performed. The pericardium was removed and the aorta was dissected free. The inferior vena cava was occluded and the pulmonary vein was severed. The aorta was cannulated and retrograde perfusion (20 ml/min) with 37°C Krebs solution was begun. The heart was then excised and transferred directly to the Langendorff apparatus and perfused at 30 ml/min. All experimental procedures were performed in accord with the guidelines for the Care and Use of Animals (NIH Publication No. 86-23, 1985), and the Animal Welfare Act (P.L. 89-544, as amended).

A latex balloon (size 12 Hugo Sachs Eletronik Hugstetten, Germany) was inserted into the left ventricle via the left atria. The balloon was connected to a pressure transducer (Gould p23ID, Cleveland, OH). The balloon was inflated with Krebs solution until a stable end diastolic pressure of approximately 5 mm Hg was established. The right atrium was removed and the AV node crushed. Bipolar stimulating electrodes (Grass platinum, Quincy, MA) were placed in the free wall of the right ventricle. The heart was stimulated at a base frequency (S1) of 120 stimuli/min with 2 ms constant current pulses at twice threshold with a 1 mA minimum intensity. Stimulation parameters were controlled by a Bloom Associates (Reading, PA) model DTU 215 stimulator with isolation unit. Two other electrodes were placed at the extremes of the ventricular longitudinal axis to monitor the electrocardiogram (Grass model 7P6C preamplifier and polygraph). A pressure transducer (Gould p23ID) was connected, at the level of the heart, to the perfusion line to monitor perfusion pressure.

The Krebs solution was composed of (in mM): 118.0 NaCl, 4.5 KCl, 1.3 mM CaCl₂, 1.16 MgSO₄, 11 dextrose, 25.0 NaHCO₃. The solution was equilibrated with 95% O₂ and 5% CO₂ and maintained at 37°C.

Measured ventricular function parameters included end diastolic ventricular pressure (EDLVP), systolic ventricular pressure (SLVP), developed pressure (SLVP-EDLVP) and perfusion pressure in mm Hg. The first derivative (dP/dt) of the ventricular pressure was determined electronically. Data was collected by a Buxco Electronics logging analyzer system (LS-14, Sharron, CT). The heart was allowed to equilibrate for 45 minutes prior to determining control (drug free) effective refractory period (ERP). Ventricular function parameters were determined at -15, -10, -5 and -1 min prior to determining ERP. Each concentration of the test agent was perfused for 15 minutes with function parameters recorded every 5 minutes. At the end of each 15 min exposure ERP was determined. Cumulative concentration responses were recorded for 5 concentrations of each test agent.

Effective refractory period was determined by inserting a premature stimuli (S2) into the base frequency of stimulation (S1). The interval between S1 and S2 was reduced by 5 ms until there was no contraction associated with S2. The last interval from S1 to S2 which resulted in a contraction was reported as the ERP. Stimulation intensity was determined for each concentration of test agent. A minimum of 20 S1 stimuli were allowed between ERP interrogation.

A change (delta) in ERP ≧20 ms (approx. 20%) or a change in dp/dt (+ or -) of a magnitude ≧220 mm Hg (approx. 20%) were considered to be physiologically important. A four parameter logistic curve was fit to the ERP and dp/dt concentration response for each heart using Sigmaplot (4.0) software (Jandel Scientific, Corte Madera, CA). The concentration (in nM) at which there was a change in ERP of 20 ms (EC₂₀ₘₛ) was determined from the curve. The concentration (in nM) at which there was a change in dp/dt of ± 220 mm Hg was determined from the curve and was termed the EC_{dp/dt}. A positive (+) number for EC_{dp/dt} (nM) indicates that the compounds are mild positive ionotropes which do not reduce (depress) cardiac function. An entry of NE* for EC_{dp/dt} (nM) indicates that no estimate was obtained as the compound failed to show a concentration related effect on contraction. Compounds which have a positive (+) value or an entry of NE* for EC_{dp/dt} (nM) are thus useful in the treatment of cardiac arrhythmias in patients with impaired ventricular function or congestive heart failure. A negative (-) number for EC_{dp/dt} (nM) indicates that the compounds reduce (depress) cardiac function and, although they are useful as antiarrhythmic agents, their use in the treatment of cardiac arrhythmias in patients with impaired ventricular function or congestive heart failure would be contraindicated.

Data were analyzed using an analysis of variance for repeated measures of raw data relative to time course control experiments and expressed as mean change (delta) from baseline. Biological significance was established at a probability of error less than 0.05.

The following table summarises the results obtained from the testing of representative compounds of the invention in the Rabbit Langendorff Model.

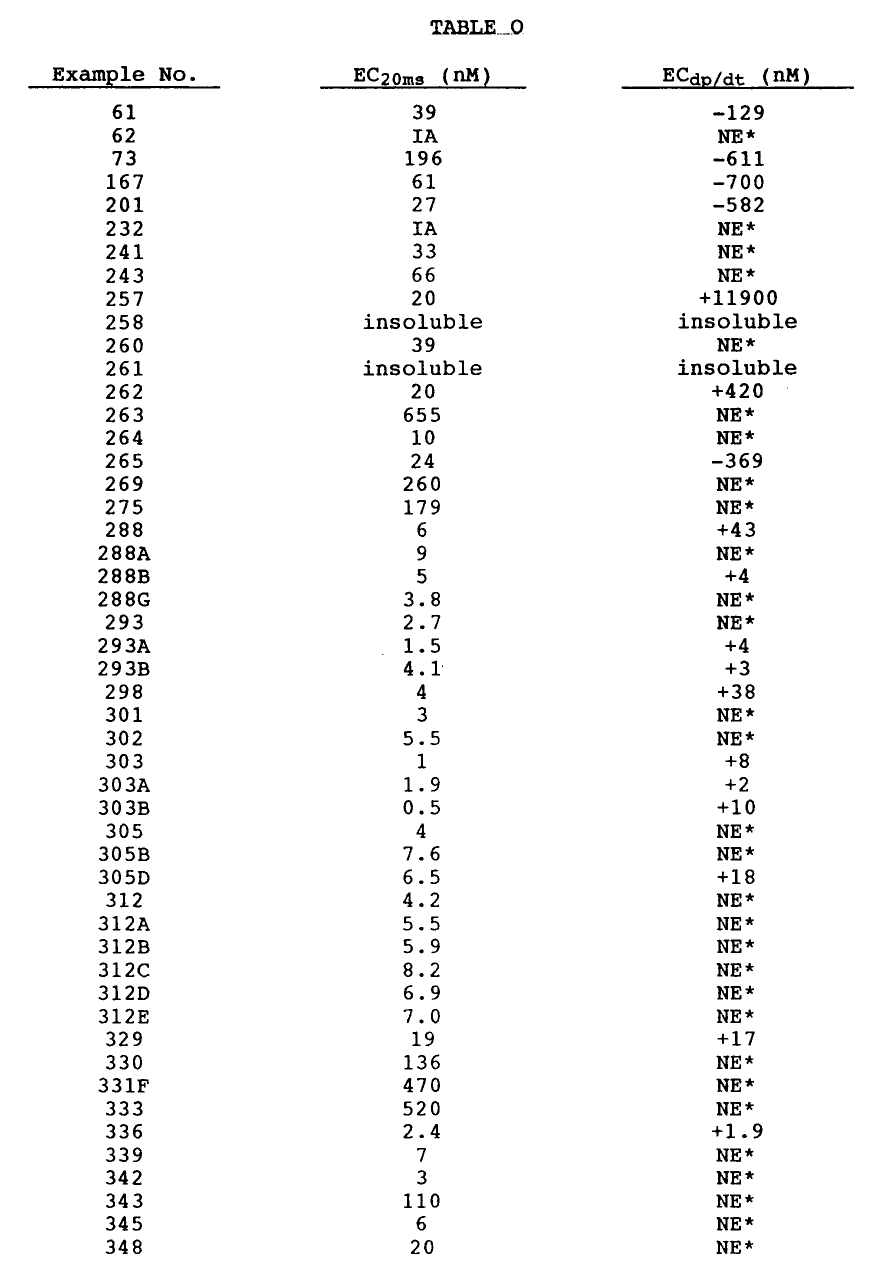

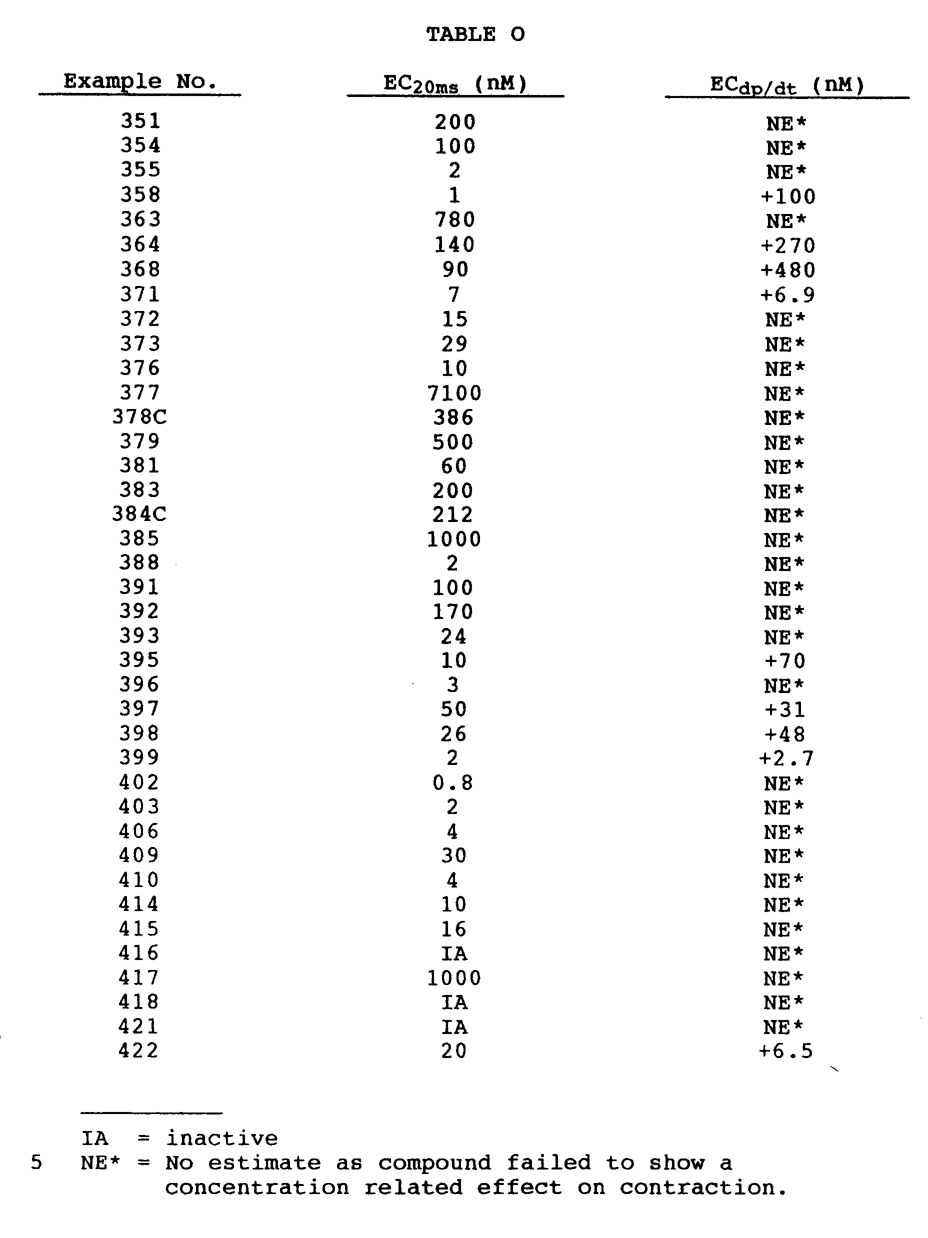

The compounds of the invention can be prepared for use by conventional pharmaceutical procedures: i.e. by dissolving or suspending them or their pharmaceutically acceptable salts in a pharmaceutically acceptable vehicle, e.g. water, aqueous alcohol, glycol, oil solution or oil-water emulsion, for parenteral or oral administration; or by incorporating them in unit dosage form as capsules or tablets for oral administration either alone or in combination with conventional adjuvants or excipients, e.g. calcium carbonate, starch, lactose, talc, magnesium stearate, gum acacia, and the like.

The percentage of active component in the composition and method for treating or preventing arrhythmia can be varied so that a suitable dosage is obtained. The dosage administered to a particular patient is variable depending upon the clinician's judgement using as the criteria: the route of administration, the duration of treatment, the size and condition of the patient, the potency of the active component, and the patient's response thereto. An effective dosage amount of active component can thus be determined by the clinician considering all criteria and utilizing his best judgement on the patient's behalf.

## Claims

1. A compound of formula: 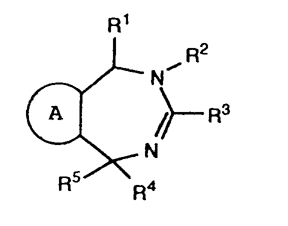 wherein
1) A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
R¹ is hydrogen, lower-alkyl, benzyl, naphthyl, thienyl, pyridinyl, phenyl, or phenyl having one or two substituents chosen from the group consisting of lower-alkyl and lower-alkoxy;
R² is -CH₂CH₂R⁷ where R⁷ is pyridinyl;
R³ is Yₚ-(CH₂)ₘ-Xₙ-R⁸ wherein
Y is -NH-, -O-, -S-, or 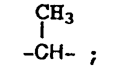 p is zero or one;
m is an integer from zero to seven;
X is -S-, -O-, -SO₂-, 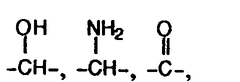 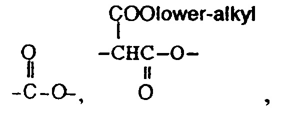 -CH=CH-, 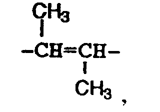 -C≡C-, 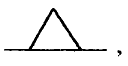 or -NHSO₂-;
n is zero or one; and
R⁸ is phenyl having one or two substituents chosen independently from the group consisting of lower-alkylsulfonamido, polyfluorolower-alkylsulfonamido, and lower-alkylaminosulfonyl ;
R⁴ is hydrogen; lower-alkyl; allyl; lower-alkoxy-lower-alkyl; acetyl; lower-alkylaceto; lower-alkyl carboxyl; or α-hydroxy-lower-alkyl; and
R⁵ is hydrogen; lower-alkyl; naphthyl; thienyl; pyridinyl; benzyl; phenyl; or phenyl having one or two substituents chosen independently from the group consisting of lower-alkyl, lower-alkoxy, halogen, hydroxyl, amino, di(lower-alkyl)amino, lower-alkyl-sulfonamido, lower-acylamino, lower-alkylthio, and lower-alkylsulfonyl;
or an acid-addition salt thereof; with the proviso that the total number of carbon atoms in R¹ plus R² plus R⁴ plus R⁵ must be five or greater; or
2) R² is hydrogen; lower-alkyl; benzyl; phenyl; phenyl substituted with halogen, lower-alkyl or lower-alkoxy; or
R² is -CH₂CH₂R⁷ where R⁷ is lower-alkoxy; benzyl; di(lower-alkyl)amino, pyrrolidino; piperidino; morpholino; pyridinyl; phenyl; or phenyl substituted with amino, nitro or lower-alkyl sulfonamido;
n is one;
X is -NHSO₂-; and
A, R¹, R³, Y, p, m, R⁸, R⁴ and R⁵ are as defined hereinabove in part 1); or
3) R⁸ is phenyl having one or two substituents chosen independently from the group consisting of polyfluoro-loweralkylsulfonamido and lower-alkylaminosulfonyl;
R² is as defined in part 2) above; and A, R¹, R³, Y, p, m, X, n, R⁴ and R⁵ are defined in part 1) above; or
4) R⁵ is phenyl having one or two substituents chosen independently from the group consisting of lower-alkylthio and lower-alkylsulfonyl;
R² is as defined in part 2) above; and A, R¹, R³, Y, p, m, X, n, R⁸, and R⁴ are as defined in part 1) above ; it being understood that lower-alkyl describes linear, branched, or cyclic saturated carbon chains of eight or fewer carbon atoms ; and lower-alkoxy describes linear or branched alkyloxy substituents containing eight or fewer carbon atoms ;

2. A compound as claimed in claim 1 wherein:
A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
R¹ is hydrogen, lower-alkyl, benzyl, naphthyl, thienyl, pyridinyl, phenyl, or phenyl having one or two substituents chosen from the group consisting of lower-alkyl and lower-alkoxy;
R² is -CH₂CH₂R⁷ where R⁷ is pyridinyl;
R³ is Yₚ-(CH₂)ₘ-Xₙ-R⁸ wherein
Y is -NH-, -O-, -S-, or 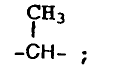 p is zero or one;
m is an integer from zero to seven;
X is -S-, -O-, -SO₂-, 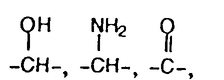 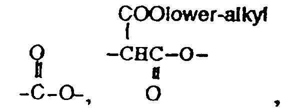 -CH=CH-, 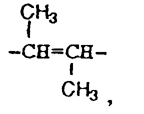 -C≡C-, 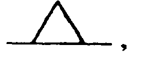 or -NHSO₂-;
n is zero or one; and
R⁸ is phenyl having one or two substituents chosen independently from the group consisting of lower-alkylsulfonamido, polyfluorolower-alkylsulfonamido and lower-alkylamino-sulfonyl.
R⁴ is hydrogen; lower-alkyl; allyl; lower-alkoxy-lower-alkyl; acetyl; lower-alkylaceto; lower-alkyl carboxyl; or α-hydroxy-lower-alkyl; and
R⁵ is hydrogen; lower-alkyl; naphthyl; thienyl; pyridinyl; benzyl; phenyl; or phenyl having one or two substituents chosen independently from the group consisting of lower-alkyl, lower-alkoxy, halogen, hydroxyl, amino, di(lower-alkyl)amino, lower-alkylsulfonamido, lower-acylamino, lower-alkylthio, and lower-alkylsulfonyl;
or an acid-addition salt thereof;
with the proviso that the total number of carbon atoms in R¹ plus R² plus R⁴ plug R⁵ must be five or greater.

3. A compound as claimed in claim 2 wherein A is phenyl; R¹ is hydrogen or lower-alkyl; R³ is Yₚ-(CH₂)ₘ-Xₙ-R⁸ wherein p is zero, m is an integer from one to three, X is -O-, n is one, and R⁸ is phenyl having one or two lower-alkylsulfonamido substituents; R⁴ is hydrogen or lower-alkyl; and R⁵ is phenyl having one or two halogen substituents.

4. A compound as claimed in claim 3 wherein: R¹ is hydrogen; R³ is Yₚ-(CH₂)ₘ-Xₙ-R⁸ wherein m is one, R⁸ is phenyl having one lower-alkylsulfonamido substituent; R⁴ is hydrogen; and R⁵ is phenyl having one halogen substituent.

5. 4,5-Dihydro-3-[(4-methanesulfonamidophenoxy)methyl]-4-(4-pyridinylethyl)-1-(4-chlorophenyl)-1H-2,4-benzodiazepine.

6. A compound as claimed in claim 1: wherein A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
R¹ is hydrogen, lower-alkyl, benzyl, naphthyl, thienyl, pyridinyl, phenyl, or phenyl having one or two substituents chosen from the group consisting of lower-alkyl and lower-alkoxy;
R² is hydrogen; lower-alkyl; benzyl; phenyl; phenyl substituted with halogen, lower-alkyl or lower-alkoxy; or
R² is -CH₂CH₂R⁷ where R⁷ is lower-alkoxy; benzyl; di(lower-alkyl)amino, pyrrolidino; piperidino; morpholino; pyridinyl; phenyl; or phenyl substituted with amino, nitro or lower-alkyl sulfonamido;
R³ is Yₚ-(CH₂)ₘ-Xₙ-R⁸ wherein
Y is -NH-, -O-, -S-, or 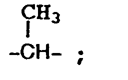 p is zero or one;
m is an integer from zero to seven;
X is -NHSO₂-;
n is one; and
R⁸ is phenyl having one or two substituents chosen independently from the group consisting of lower-alkylsulfonamido, polyfluorolower-alkylsulfonamido and lower-alkylamino-sulfonyl.
R⁴ is hydrogen; lower-alkyl; allyl; lower-alkoxy-lower-alkyl; acetyl; lower-alkylaceto; lower-alkyl carboxyl; or α-hydroxy-lower-alkyl; and
R⁵ is hydrogen; lower-alkyl; naphthyl; thienyl; pyridinyl; benzyl; phenyl; or phenyl having one or two substituents chosen independently from the group consisting of lower-alkyl, lower-alkoxy, halogen, hydroxyl, amino, di(lower-alkyl)amino, lower-alkylsulfonamido, lower-acylamino, lower-alkylthio, and lower-alkylsulfonyl;
or an acid-addition salt thereof;
with the proviso that the total number of carbon atoms in R¹ plus R² plus R⁴ plus R⁵ must be five or greater.

7. A compound as claimed in claim 1 wherein A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
R¹ is hydrogen, lower-alkyl, benzyl, naphthyl, thienyl, pyridinyl, phenyl, or phenyl having one or-two substituents chosen from the group consisting of lower-alkyl and lower-alkoxy;
R² is hydrogen; lower-alkyl; benzyl; phenyl; phenyl substituted with halogen, lower-alkyl or lower-alkoxy; or
R² is -CH₂CH₂R⁷ where R⁷ is lower-alkoxy; benzyl; di(lower-alkyl)amino, pyrrolidino; piperidino; morpholino; pyridinyl; phenyl; or phenyl substituted with amino, nitro or lower-alkyl sulfonamido;
R³ is Yₚ-(CH₂)ₘ-Xₙ-R⁸ wherein
Y is -NH-, -O-, -S-, or 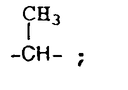 p is zero or one;
m is an integer from zero to seven;
X is -S-, -O-, -SO₂-, 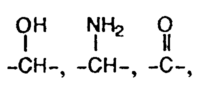 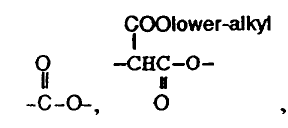 -CH=CH-, 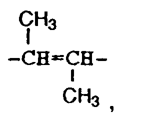 -C≡C-, 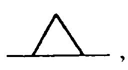 or -NHSO₂-;
n is zero or one; and
R⁸ is phenyl having one or two substituents chosen independently from the group consisting of polyfluorolower-alkylsulfonamido, and lower-alkylaminosulfonyl;
R⁴ is hydrogen; lower-alkyl; allyl; lower-alkoxy-lower-alkyl; acetyl; lower-alkylaceto; lower-alkyl carboxyl; or α-hydroxy-lower-alkyl; and
R⁵ is hydrogen; lower-alkyl; naphthyl; thienyl; pyridinyl; benzyl; phenyl; or phenyl having one or two substituents chosen independently from the group consisting of lower-alkyl, lower-alkoxy, halogen, hydroxyl, amino, di(lower-alkyl)amino, lower-alkyl-sulfonamido, lower-acylamino, lower-alkylthio, and lower-alkylsulfonyl;
or an acid-addition salt thereof; with the proviso that the total number of carbon atoms in R¹ plus R² plus R⁴ plus R⁵ must be five or greater.

8. A compound as claimed in claim 7 wherein A is phenyl; R¹ is hydrogen or lower-alkyl; R² is hydrogen or lower-alkyl, R³ is Yₚ-(CH₂)ₘ-Xₙ-R⁸ wherein p is zero, m is an integer from one to three, n is zero and R⁸ is phenyl having one polyfluorolower-alkylsulfonamido or lower-alkylaminosulfonyl substituent; R⁴ is hydrogen or lower-alkyl; and R⁵ is phenyl.

9. A compound as claimed in claim 8 wherein R¹ is hydrogen; R² is lower-alkyl; R³ is Yₚ-(CH₂)ₘ-Xₙ-R⁸ wherein m is two; and R⁴ is hydrogen.

10. A compound as claimed in claim 1 wherein A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;
R¹ is hydrogen, lower-alkyl, benzyl, naphthyl, thienyl, pyridinyl, phenyl, or phenyl having one or two substituents chosen from the group consisting of lower-alkyl and lower-alkoxy;
R² is hydrogen; lower-alkyl; benzyl; phenyl; phenyl substituted with halogen, lower-alkyl or lower-alkoxy; or
R² is -CH₂CH₂R⁷ where R⁷ is lower-alkoxy; benzyl; di(lower-alkyl)amino, pyrrolidino; piperidino; morpholino; pyridinyl; phenyl; or phenyl substituted with amino, nitro or lower-alkyl sulfonamido;
R³ is Yₚ-(CH₂)ₘ-Xₙ-R⁸ wherein
Y is -NH-, -O-, -S-, or 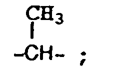 p is zero or one;
m is an integer from zero to seven;
X is -S-, -O-, -SO₂-, 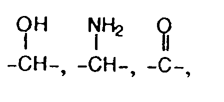 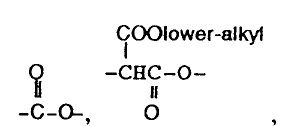 -CH=CH-, 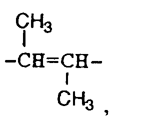 -C≡C-, 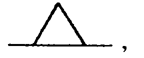 or -NHSO₂-;
n is zero or one; and
R⁸ is phenyl having one or two substituents chosen independently from the group consisting of lower-alkylsulfonamido, polyfluorolower-alkylsulfonamido and lower-alkylamino-sulfonyl.
R⁴ is hydrogen; lower-alkyl; allyl; lower-alkoxy-lower-alkyl; acetyl; lower-alkylaceto; lower-alkyl carboxyl; or α-hydroxy-lower-alkyl; and
R⁵ is phenyl having one or two substituents chosen independently from the group consisting of lower-alkylthio, and lower-alkylsulfonyl;
or an acid-addition salt thereof;
with the proviso that the total number of carbon atoms in R¹ plus R² plus R⁴ plus R⁵ must be five or greater.

11. A compound as claimed in claim 10 wherein A is phenyl; R¹ is hydrogen or lower-alkyl; R² is hydrogen or lower-alkyl; R³ is Yₚ-(CH₂)ₘ-Xₙ-R⁸ wherein p is zero, m is an integer from one to three, X is -O-, n is one, and R⁸ is phenyl having one lower-alkylsulfonamido substituent; and R⁴ is hydrogen or lower-alkyl.

12. A compound as claimed in claim 11 wherein R¹ is hydrogen; R² is lower-alkyl; R³ is Yₚ-(CH₂)ₘ-Xₙ-R⁸ wherein m is one; and R⁴ is hydrogen.

13. A compound as claimed in claim 12 selected from 4,5-dihydro-3-[(4-methanesulfonamodophenoxy)methyl]-4-methyl-1-(4-methylthiophenyl)-1H-2,4-benzodiazepine or 4,5-dihydro-3-[(4-methanesulfonamidophenoxy)methyl]-4-methyl-1-(4-methylsulfonylphenyl)-1H-2,4-benzodiazepine.

14. The use of a compound as claimed in any one of claims 1 to 13 for the preparation of a medicament for the treatment of cardiac arrhythmia in a patient in need of such treatment.

15. A composition for the treatment of cardiac arrhythmia comprising an antiarrhythmically effective amount of a compound as claimed in any one of claims 1 to 13.

16. The use of a compound of the formula: 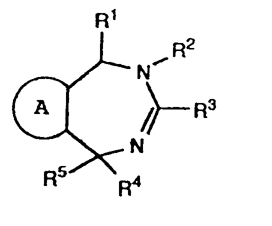 wherein:
A is a ring chosen from the group consisting of phenyl, thienyl and phenyl substituted at any available carbon atom by one or two lower-alkylsulfonamido groups;
R¹ is hydrogen or phenyl;
R² is lower-alkyl or -CH₂CH₂R^{7'} wherein R^{7'} is lower-alkoxy, pyridinyl, phenyl or phenyl having one or two lower-alkylsulfonamido substituents;
R³ is -(CH₂)ₘ-Xₙ-R⁸ wherein
m is an integer from zero to seven;
Xₙ is -S-, -O-, -SO₂-, -CH=CH-, or -NHSO₂-;
n is zero or one;
R⁸ is phenyl having one or two substituents chosen independently from the group consisting of lower-alkylsulfonamido and polyfluorolower-alkylsulfonamido.
R⁴ is hydrogen or lower-alkyl; and
R⁵ is hydrogen, pyridinyl, benzyl, phenyl or phenyl having one or two substituents chosen independently from the group consisting of halogen, lower-alkyl, lower-alkoxy, lower-alkylthio, lower-alkylsulfonyl, lower-alkylsulfonamido, and hydroxy;
or an acid-addition salt thereof; with the proviso that the total number of carbon atoms in R¹ plus R² plus R⁴ plus R⁵ must be five or greater; further provided that at least one of R², R³, R⁵ or ring A must contain a pyridinyl group, an -NHSO₂- group, or a lower-alkylsulfonamido, dilower-alkylaminosulfonyl, or polyfluorolower-alkylsulfonamido substituent; for the preparation of a medicament for the treatment of cardiac arrhythmia in a patient with impaired ventricular function or congestive heart failure.

17. A use as claimed in claim 16 wherein the compound is of the formula: 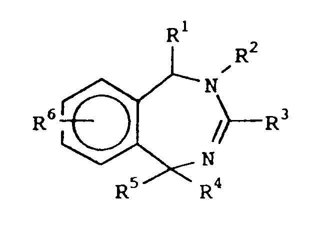 wherein R⁶ is hydrogen, or one or two lower-alkylsulfonamido groups substituted at any available phenyl ring carbon atom.

18. A use as claimed in Claim 17 wherein:
R² is lower-alkyl, or -CH₂CH₂R^{7'} wherein R^{7'} is lower-alkoxy, pyridinyl, or phenyl having one lower-alkylsulfonamido substituent;
R³ is -(CH₂)ₘ-Xₙ-R⁸ wherein
m is an integer from zero to three;
R⁸ is phenyl having one or two substituents chosen independently from the group consisting of lower-alkylsulfonamido and trifluoromethylsulfonamido; and
R⁶ is hydrogen, or a lower-alkylsulfonamido group substituted at any available phenyl ring carbon atom.

19. A use as claimed in claim 18 wherein:
R² is methyl, or -CH₂CH₂R^{7'} wherein R^{7'} is methoxy, 4-pyridinyl, or phenyl having one methylsulfonamido substituent;
R⁸ is phenyl having one or two substituents chosen independently from the group consisting of lower-alkylsulfonamido and trifluoromethyl-sulfonamido ;
R⁴ is hydrogen or methyl;
R⁵ is hydrogen, 4-pyridinyl, benzyl, phenyl or phenyl having one or two substituents chosen independently from the group consisting of halogen, lower-alkyl, lower-alkoxy, lower-alkylthio, lower-alkylsulfonyl, lower-alkylsulfonamido, and hydroxy; and
R⁶ is hydrogen, or a 6- or 8-methylsulfonamido group.

20. A use as claimed in claim 19 wherein the compound is selected from the group consisting of:
4,5-dihydro-3-[(4-methanesulfonamidophenoxy)methyl]-4-methyl-1-phenyl-1H-2,4-benzodiazepine;
4,5-dihydro-3-[(4-methanesulfonamidophenoxy)methyl]-4-methyl-1-(4-chlorophenyl)-1H-2,4-benzodiazepine;
4,5-dihydro-3-[(4-methanesulfonamidophenoxy)methyl]-4-methyl-1-(4-methoxyphenyl)-1H-2,4-benzodiazepine;
4,5-dihydro-3-[(4-methanesulfonamidophenylsulfonyl)methyl]-4-methyl-1-(4-chlorophenyl)- 1H-2,4-benzodiazepine;
4,5-dihydro-3-[(4-methanesulfonamidophenoxy)methyl]-4-methyl-1-(4-methylthiophenyl)-1H-2,4-benzodiazepine; and
4,5-dihydro-4-methyl-1-(2,4-difluorophenyl)-3-[2-(4-methanesulfonamidophenyl)ethyl]-1H-2,4-benzodiazepine.

21. A use as claimed in claim 19 wherein the compound is (+)-4,5-Dihydro-3-[(4-methanesulfonamidophenoxy)methyl]-4-methyl-1-phenyl-1H-2,4-benzodiazepine.

22. A use as claimed in claim 16 wherein the compound is of the formula:

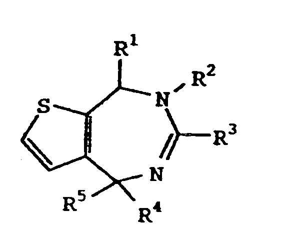

23. A use as claimed in claim 22 wherein R¹ is hydrogen; R² is lower-alkyl; R³ is -(CH₂)ₘ-Xₙ-R⁸ wherein m is one or two; n is zero; R⁸ is phenyl having one lower-alkylsulfonamido substituent; R⁴ is hydrogen; and R⁵ is phenyl

24. A use as claimed in claim 23 wherein R² is methyl; and R⁸ is 4-methylsulfonamidophenyl.

25. A use as claimed in claim 24 wherein the compound is selected from the group consisting of:
4,5-dihydro-4-methyl-1-phenyl-3-[(4-methanesulfonamidophenoxy)methyl]-1H-thieno [2,3-e]-2,4-diazepine and
4,5-dihydro-4-methyl-1-phenyl-3-[2-(4-methanesulfonamidophenyl)ethyl]-1H-thieno [2,3-e]-2,4-diazepine.

26. A composition for the treatment of cardiac arrhythmia in a patient with impaired ventricular function or congestive heart failure which comprises an antiarrhythmically effective amount of a compound of the formula as defined in any one of claims 16 to 25,
together with a pharmaceutically acceptable vehicle, adjuvant or excipient.

## Patentansprüche

1. Verbindung der Formel 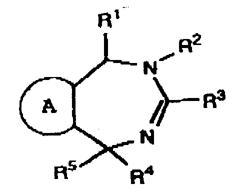 worin bedeuten:
1) A ein Ring, gewählt aus der Phenyl, Thienyl, Furanyl, Naphthyl, Pyridinyl, Cyclohexyl und Phenyl mit einem oder zwei Substituenten, welche aus der Amino, Niederalkyl, Niederalkoxy, Halogen, Nitro und Niederalkylsulfonamido umfassenden Gruppe gewählt sind, umfassenden Gruppe;
R¹ Wasserstoff, Niederalkyl, Benzyl, Naphthyl, Thienyl. Pyridinyl. Phenyl oder Phenyl mit einem oder zwei Substituenten, gewählt aus der Niederalkyl und Niederalkoxy umfassenden Gruppe;
R² -CH₂CH₂R⁷, worin R⁷ Pyridinyl ist;
R³ Yₚ-(CH₂)ₘ-Xₙ-R⁸, worin
Y -NH-, -O-, -S- oder 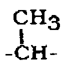 ist;
p Null oder Eins ist;
m eine ganze Zahl von Null bis Sieben ist;
X -S-, -O-, -SO₂-, 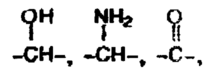 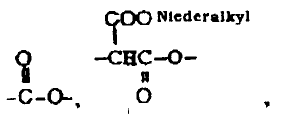 -CH=CH-, 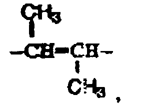 -C≡C-, oder 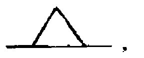 -NHSO₂- ist;
n Null oder Eins ist; und
R⁸ Phenyl mit einem oder zwei Substituenten ist welche unabhängig aus der Niederalkylsulfonamido, Polyfluorniederalkylsulfonamido und Niederalkyl-aminosulfonyl umfassenden Gruppe gewählt sind;
R⁴ Wasserstoff, Niederalkyl, Allyl, Niederalkoxyniederalkyl, Acetyl, Niederalkylaceto, Niederalkylcarboxyl oder α-Hydroxy-Niederalkyl; und
R⁵ Wasserstoff, Niederalkyl, Naphthyl, Thienyl, Pyridinyl, Benzyl, Phenyl oder Phenyl mit einem oder zwei Substituenten, welche unabhängig aus der Niederalkyl, Niederalkoxy, Halogen, Hydroxyl, Amino, Di(niederalkyl)amino, Niederalkylsulfonamido, Niederacylamino, Niederalkylthio und Niederalkylsulfonyl umfassenden Gruppe gewählt sind;
oder ein Säureadditionssalz hiervon;
mit der Maßgabe, daß die Gesamtanzahl der Kohlenstoffatome in R¹ plus R² plus R⁴ plus R⁵ fünf oder größer sein muß;
oder
2) R² Wasserstoff, Niederalkyl, Benzyl, Phenyl, mit Halogen, Niederalkyl oder Niederalkoxy substituiertes Phenyl; oder
R² -CH₂CH₂R⁷, worin R⁷ Niederalkoxy, Benzyl, Di(niederalkyl)amino, Pyrrolidino, Piperidino, Morpholino, Pyridinyl, Phenyl oder mit Amino, Nitro oder Niederalkylsulfonamido substituiertes Phenyl ist;
n Eins;
X -NHSO₂-; und
A, R¹, R³, Y, p, m, R⁸, R⁴ und R⁵ wie oben in Teil 1) definiert sind; oder
3) R⁸ Phenyl mit einem oder zwei Substituenten, welche unabhängig aus der Polyfluorniederalkylsulfonamido und Niederalkylaminosulfonyl umfassenden Gruppe gewählt sind;
R² wie oben in Teil 2) definiert; und
A, R¹, R³, Y, p, m, X, n, R⁴ und R⁵ wie oben in Teil 1) definiert; oder
4) R⁵ Phenyl mit einem oder zwei Substituenten, welche unabhängig aus der Niederalkylthio und Niederalkylsulfonyl umfassenden Gruppe gewählt sind;
R² wie oben in Teil 2) definiert; und
A, R¹, R³, Y, p, m, X, n, R⁸ und R⁴ wie oben in Teil 1) definiert: wobei Niederalkyl für lineare, verzweigte oder cyclische Kohlenstoffketten mit 8 oder weniger Kohlenstoffatomen steht; und Niederalkoxy für lineare oder verzweigte Alkoxysubstituenten mit 8 oder weniger Kohlenstoffatomen steht.

2. Verbindung nach Anspruch 1, worin bedeuten;
A ein Ring, gewählt aus der Phenyl, Thienyl. Furanyl, Naphthyl, Pyridinyl, Cyclohexyl und Phenyl mit einem oder zwei Substituenten, welche aus der Amino, Niederalkyl, Niederalkoxy, Halogen, Nitro und Niederalkylsulfonamido umfassenden Gruppe gewählt sind, umfassenden Gruppe:
R¹ Wasserstoff, Niederalkyl, Benzyl, Naphthyl, Thienyl, Pyridinyl, Phenyl oder Phenyl mit einem oder zwei Substituenten, gewählt aus der Niederalkyl und Niederalkoxy umfassenden Gruppe:
R² -CH₂CH₂R⁷, worin R⁷ Pyridinyl ist;
R³ Yₚ-(CH₂)ₘ-Xₙ-R⁸, worin
Y -NH-, -O-, -S-, oder 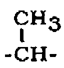 ist:
p Null oder Eins ist:
m eine ganze Zahl von Null bis Sieben ist:
X -S-, -O-, -SO₂-, 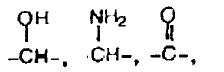 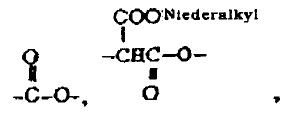 -CH=CH-, 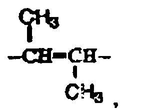 -C≡C-, 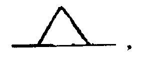 oder -NHSO₂- ist;
n Null oder Eins ist: und
R⁸ Phenyl mit einem oder zwei Substituenten ist, welche unabhängig aus der Niederalkylsulfonamido, Polyfluorniederalkylsulfonamido und Niederalkylaminosulfonyl umfassenden Gruppe gewählt sind;
R⁴ Wasserstoff, Niederalkyl, Allyl, Niederalkoxyniederalkyl, Acetyl, Niederalkylaceto, Niederalkylcarboxyl oder α-Hydroxy-Niederalkyl: und
R⁵ Wasserstoff, Niederalkyl, Naphthyl, Thienyl, Pyridinyl, Benzyl, Phenyl oder Phenyl mit einem oder zwei Substituenten, welche unabhängig aus der Niederalkyl, Niederalkoxy, Halogen, Hydroxyl, Amino, Di(niederalkyl)amino, Niederalkylsulfonamido, Niederacylamino, Niederalkylthio und Niederalkylsulfonyl umfassenden Gruppe gewählt sind:
oder ein Säureadditionssalz hiervon;
mit der Maßgabe, daß die Gesamtanzahl der Kohlenstoffatome in R¹ plus R² plus R⁴ plus R⁵ fünf oder größer sein muß.

3. Verbindung nach Anspruch 2, worin A Phenyl ist; R¹ Wsaserstoff oder Niederalkyl ist; R³ Yₚ-(CH₂)ₘ-Xₙ-R⁸ ist, worin p 0 ist, m eine ganze Zahl von Eins bis Drei ist, X -O- ist, n Eins ist und R⁸ Phenyl mit einem oder zwei Niederalkylsulfonamido-Substituienten ist; R⁴ Wasserstoff oder Niederalkyl ist; und R⁵ Phenyl mit einem oder zwei Halogensubstituenten ist.

4. Verbindung nach Anspruch 3, worin R¹ Wasserstoff ist; R³ Yₚ-(CH₂)ₘ-Xₙ-R⁸ ist, worin m Eins ist, R⁸ Phenyl mit einem Niederalkylsulfonamido-Substituenten ist; R⁴ Wasserstoff ist; und R⁵ Phenyl mit einen Halogen-Substituenten ist.

5. 4,5-Dihydro-3-[(4-methansulfonamidophenoxy)methyl]-4-(4-pyridinylethyl)-1-(4-chlorphenyl)-1H-2,4-benzodiazepin.

6. Verbindung nach Anspruch 1, worin bedeuten;
A ein Ring, gewählt aus der Phenyl, Thienyl, Furanyl, Naphthyl, Pyridinyl, Cyclohexyl und Phenyl mit einem oder zwei Substituenten, welche aus der Amino, Niederalkyl, Niederalkoxy, Halogen, Nitro und Niederalkylsulfonamido umfassenden Gruppe gewählt sind, umfassenden Gruppe:
R¹ Wasserstoff, Niederalkyl, Benzyl, Naphthyl, Thienyl, Pyridinyl, Phenyl oder Phenyl mit einem oder zwei Substituenten, welche aus der Niederalkyl und Niederalkoxy umfassenden Gruppe gewählt:
R² Wasserstoff, Niederalkyl, Benzyl, Phenyl, mit Halogen, Niederalkyl oder Niederalkoxy substituiertes Phenyl; oder
R² -CH₂CH₂R⁷, worin R⁷ Niederalkoxy, Benzyl, Di-(niederalkyl)amino, Pyrrolidino Piperidino, Morpholino, Pyridinyl, Phenyl oder Phenyl ist, welches mit Amino, Nitro oder Niederalkylsulfonamido substituiert ist;
R³ Yₚ-(CH₂)ₘ-Xₙ-R⁸, worin
Y -NH-, -O-, -S-, oder 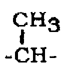 ist:
p Null oder Eins ist;
m eine ganze Zahl von Null bis Sieben ist;
X -NHSO₂- ist;
n Eins ist; und
R⁸ Phenyl mit einem oder zwei Substituenten ist, welche unabhängig aus der Niederalkylsulfonamido, Polyfluorniederalkylsulfonamido und Niederalkylaminosulfonyl umfassenden Gruppe gewählt sind;
R⁴ Wasserstoff, Niederalkyl, Allyl, Niederalkoxyniederalkyl, Acetyl, Niederalkylaceto, Niederalkylcarboxyl oder α-Hydroxy-Niederalkyl; und
R⁵ Wasserstoff, Niederalkyl, Naphthyl, Thienyl, Pyridinyl, Benzyl, Phenyl oder Phenyl mit einem oder zwei Substituenten, welche unabhängig aus der Niederalkyl, Niederalkoxy, Halogen, Hydroxyl, Amino, Di-(niederalkyl)amino, Niederalkylsulfonamido, Niederacylamino, Niederalkylthio und Niederalkylsulfonyl umfassenden Gruppe gewählt sind;
oder ein Saureadditionssalz hiervon;
mit der Maßgabe, daß die Gesamtanzahl der Kohlenstoffatome in R¹ plus R² plus R⁴ plus R⁵ fünf oder größer sein muß.

7. Verbindung nach Anspruch 1, worin bedeuten:
A ein Ring, gewählt aus der Phenyl, Thienyl. Furanyl, Naphthyl, Pyridinyl, Cyclohexyl und Phenyl mit einem oder zwei Substituenten, welche aus der Amino, Niederalkyl, Niederalkoxy, Halogen, Nitro und Niederalkylsulfonamido umfassenden Gruppe gewählt sind, umfassenden Gruppe;
R¹ Wasserstoff, Niederalkyl, Benzyl, Naphthyl, Thienyl, Pyridinyl, Phenyl oder Phenyl mit einem oder zwei Substituenten, gewählt aus der Niederalkyl und Niederalkoxy umfassenden Gruppe;
R² Wasserstoff Niederalkyl, Benzyl, Phenyl, mit Halogen, Niederalkyl oder Niederalkoxy substituiertes Phenyl; oder
R² -CH₂CH₂R⁷, worin R⁷ Niederalkoxy, Benzyl, Di-(niederalkyl)amino, Pyrrolidino, Piperidino, Morpholino, Pyridinyl, Phenyl oder Phenyl ist, das mit Amino, Nitro oder Niederalkylsulfonamido substituiert ist;
R³ Yₚ-(CH₂)ₘ-Xₙ-R⁸, worin
Y -NH-, -O-, -S-, oder 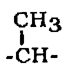 ist;
p Null oder Eins ist;
m eine ganze Zahl von Null bis Sieben ist;
X -S-, -O-, -SO₂-, 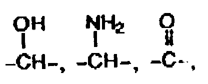 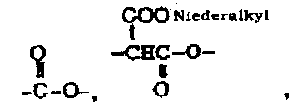 -CH=CH-, 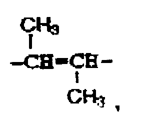 -C≡C-, 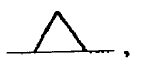 oder -NHSO₂- ist;
n Null oder Eins ist; und
R⁸ Phenyl mit einem oder zwei Substituenten ist, welche unabhängig aus der Polyfluorniederalkylsulfonamido und Niederalkylaminosulfonyl umfassenden Gruppe gewählt sind:
R⁴ Wasserstoff, Niederalkyl, Allyl, Niederalkoxyniederalkyl, Acetyl, Niederalkylaceto, Niederalkylcarboxyl oder α-Hydroxy-Niederalkyl; und
R⁵ Wasserstoff, Niederalkyl, Naphthyl, Thienyl, Pyridinyl, Benzyl, Phenyl oder Phenyl mit einem oder zwei Substituenten, welche unabhängig aus der Niederalkyl, Niederalkoxy, Halogen, Hydroxyl, Amino, Di-(niederalkyl)amino, Niederalkylsulfonamido, Niederacylamino, Niederalkylthio und Niederalkylsulfonyl umfassenden Gruppe gewählt sind;
oder ein Säureadditionssalz hiervon;
mit der Maßgabe, daß die Gesamtanzahl der Kohlenstoffatome in R¹ plus R² plus R⁴ plus R⁵ fünf oder größer sein muß.

8. Verbindung nach Anspruch 7, worin A Phenyl ist; R¹ Wasserstoff oder Niederalkyl ist; R² Wasserstoff oder Niederalkyl ist; R³ Yₚ-(CH₂)ₘ-Xₙ-R⁸ ist. worin p Null ist, m eine ganze Zahl von Eins bis Drei ist, n Null ist und R⁸ Phenyl mit einem Polyfluorniederalkylsulfonamido, oder Niederalkylaminosulfonyl-Substituenten ist; R⁴ Wasserstoff oder Niederalkyl ist; und R⁵ Phenyl ist.

9. Verbindung nach Anspruch 8, worin R¹ Wasserstoff ist; R² Niederalkyl ist; R³ Yₚ-(CH₂)ₘ-Xₙ-R⁸ ist, worin m Zwei ist; und R⁴ Wasserstoff ist.

10. Verbindung nach Anspruch 1, worin bedeuten:
A ein Ring, gewählt aus der Phenyl, Thienyl, Furanyl, Naphthyl, Pyridinyl, Cyclohexyl und Phenyl mit einem oder zwei Substituenten, welche aus der Amino, Niederalkyl, Niederalkoxy, Halogen, Nitro und Niederalkylsulfonamido umfassenden Gruppe gewählt sind, umfassenden Gruppe;
R¹ Wasserstoff, Niederalkyl, Benzyl, Naphthyl, Thienyl, Pyridinyl, Phenyl oder Phenyl mit einem oder zwei Substituenten, welche aus der Niederalkyl und Niederalkoxy umfassenden Gruppe gewählt sind;
R² wasserstoff, Niederalkyl, Benzyl, Phenyl, mit Halogen, Niederalkyl oder Niederalkoxy substituiertes Phenyl; oder
R² CH₂CH₂R⁷, worin R⁷ Niederalkoxy, Benzyl, Di(niederalkyl)amino. Pyrrolidino, Piperidino, Morpholino, Pyridinyl, Phenyl oder Phenyl ist, das mit Amino, Nitro oder Niederalkylsulfonamido substituiert ist;
R³ Yₚ-(CH₂)ₘ-Xₙ-R⁸, worin
Y -NH, -O-, -S- oder 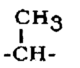 ist
p Null oder Eins ist;
m eine ganze Zahl von Null bis Sieben ist:
X -S-, -O-, -SO₂-, 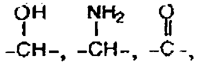 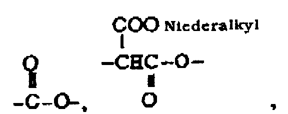 -CH=CH-, 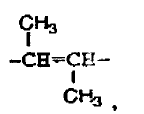 -C≡C-, 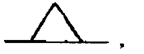 oder -NHSO₂- ist;
n Null oder Eins ist; und
R⁸ Phenyl mit einem oder zwei Substituenten ist, welche unabhängig aus der Niederalkylsulfonamido, Polyfluorniederalkylsulfonamido und Niederalkylaminosulfonyl umfassenden Gruppe gewählt sind;
R⁴ Wasserstoff, Niederalkyl, Allyl, Niederalkoxyniederalkyl, Acetyl, Niederalkylaceto, Niederalkylcarboxyl oder α-Hydroxy-Niederalkyl; und
R⁵ Phenyl mit einem oder zwei Substituenten, welche unabhängig aus der Niederalkylthio oder Niederalkylsulfonyl umfassenden Gruppe gewählt sind;
oder ein Säureadditionssalz hiervon:
mit der Maßgabe, daß die Gesamtanzahl der Kohlenstoffatome in R¹ plus R² plus R⁴ plus R⁵ fünf oder größer sein muß.

11. Verbindung nach Anspruch 10, worin A Phenyl ist: R¹ Wasserstoff oder Niederalkyl ist; R² Wasserstoff oder Niederalkyl ist; R³ Yₚ-(CH₂)ₘ-Xₙ-R⁸ ist. worin p Null ist, m eine ganze Zahl von Eins bis Drei ist, X -O- ist, n Eins ist und R⁸ Phenyl mit einem Niederalkylsulfonylamido-Substituenten ist; und R⁴ Wasserstoff oder Niederalkyl ist.

12. Verbindung nach Anspruch 11, wobei R¹ Wasserstoff ist, R² Niederalkyl ist; R³ Yₚ-(CH₂)ₘ-Xₙ-R⁸ ist, worin m Eins ist und R⁴ Wasserstoff Ist

13. Verbindung nach Anspruch 12, gewählt aus 4,5-Dihydro-3-[(4-methansulfonamidophenoxy)methyl]-4-methyl-1-(4-methylthiophenyl]-1H-2,4-benzodiazepin oder 4,5-Dihydro-3[(4-methansulfonamidophenoxylmethyl]-4-methyl-1-(4-methylsulfonylphenyl)-1H-2,4-benzodiazepin.

14. Verwendung einer Verbindung nach mindestens einem der Ansprüche 1 bis 13 zur Herstellung eines Arzneimittels zur Behandlung von Pulsarrythmie bei einem Patienten, welcher einer solchen Behandlung bedarf.

15. Zusammensetzung zur Behandlung von Pulsarrythmie, umfassend eine antiarrythmisch wirksame Menge einer Verbindung nach mindestens einem der Ansprüche 1 bis 13.

16. Verwendung einer Verbindung der Formel: 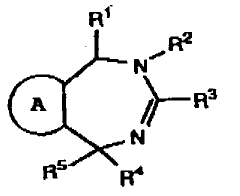 worin bedeuten:
A ein Ring, gewählt aus der Phenyl, Thienyl und Phenyl, das an irgendeinem verfügbaren Kohlenstoffatom durch eine oder zwei Niederalkylsulfonamidogruppen substituiert ist, umfassenden Gruppe;
R¹ Wasserstoff oder Phenyl;
R² Niederalkyl oder -CH₂CH₂R^{7'}, worin R^{7'} Niederalkoxy, Pyridinyl, Phenyl oder Phenyl mit einem oder zwei Niederalkylsulfonamido-Substituenten ist;
R³ -(CH₂)ₘ-Xₙ-R⁸, worin
m eine ganze Zahl von Null bis Sieben ist;
Xₙ -S-, -O-, -SO₂-, -CH=CH- oder -NHSO₂- ist;
n Null oder Eins ist;
R⁸ Phenyl mit einem oder zwei Substituenten ist, welche unabhängig aus der Niederalkylsulfonamido und Polyfluorniederalkylsulfonamido umfassenden Gruppe gewählt sind;
R⁴ Wasserstoff oder Niederalkyl; und
R⁵ Wasserstoff, Pyridinyl, Benzyl, Phenyl oder Phenyl mit einem oder zwei Substituenten, welche unabhängig aus der Halogen Niederalkyl, Niederalkoxy, Niederalkylthio, Niederalkylsulfonyl, Niederalkylsulfonamido und Hydroxy umfassenden Gruppe gewählt sind;
oder ein Säureadditionssalz hiervon;
mit der Maßgabe, daß die Gesamtanzahl der Kohlenstoffatome in R¹ plus R² plus R⁴ plus R⁵ fünf oder größer sein muß;
weiterhin mit der Maßgabe, daß mindestens eines von R², R³, R⁵ oder der Ring A eine Pyridinylgruppe, eine -NHSO₂-Gruppe oder einen Niederalkylsulfonamido-, Diniederalkylaminosulfonyl- oder Polyfluorniederalkylsulfonamido-Substituenten enthalten muß;
zur Herstellung eines Arzneimittels zur Behandlung von Pulsarrythmie bei einem Patienten mit beeinträchtigter Ventrikelfunktion oder Blutandrang erzeugendem Herzversagen.

17. Verwendung nach Anspruch 16, wobei die Verbindung der Formel 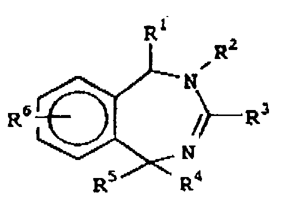 entspricht, worin R⁶ Wasserstoff oder eine oder zwei Niederalkylsulfonamidogruppen bedeutet, welche an irgendeinem verfügbaren Phenylring-Kohlenstoffatom substituiert sind.

18. Verwendung nach Anspruch 17, worin bedeuten:
R² Niederalkyl oder -CH₂CH₂R^{7'}, worin R^{7'} Niederalkoxy, Pyridinyl oder Phenyl mit einem Niederlkylsulfonamido-Substituenten ist;
R³ -(CH₂)ₘ-Xₙ-R⁸, worin
m eine ganze Zahl von Null bis Drei ist:
R⁸ Phenyl mit einem oder zwei Substituente ist, welche unabhängig aus der Niederalkylsulfonamido und Trifluormethylsulfonamido umfassenden Gruppe gewählt sind; und
R⁶ Wasserstoff oder eine Niederalkylsulfonamidogruppe, welche an irgendeinem verfügbaren Phenylring-Kohlenstoffatom substituiert ist.

19. Verwendung nach Anspruch 18, wobei bedeuten:
R² Methyl oder -CH₂CH₂R^{7'}, worin R^{7'} Methoxy, 4-Pyridinyl oder Phenyl mit einem Methylsulfonamido-Substituenien ist;
R⁸ Phenyl mit einem oder zwei Substituenten, welche unabhängig aus der Niederalkylsulfonamido und Trifluormethylsulfonamido umfassenden Gruppe gewählt sind:
R⁴ Wasserstoff oder Methyl;
R⁵ Wasserstoff, 4-Pyridinyl, Benzyl, Phenyl oder Phenyl mit einem oder zwei Substituenten, welche unabhängig aus der Halogen, Niederalkyl, Niederalkoxy, Niederalkylthio, Niederalkylsulfonyl, Niederalkylsulionamido und Hydroxy umfassenden Gruppe gewählt sind: und
R⁶ Wasserstoff oder eine 6- oder 8-Methylsulfonamidogruppe.

20. Verwendung nach Anspruch 19, wobei die Verbindung aus der
4,5-Dihydro-3-[(4-methansulfonamidophenoxy)methyl]-4-methyl-1-phenyl-1H-2,4-benzodiazepin;
4,5-Dihydro-3-[(4-methansulfonamidophenoxy)methyl]-4-methyl-1-(4-chlorphenyl)-1H-2,4-benzodiazepin:
4,5-Dihydro-3-[(4-methansulfonamidophenoxy)methyl]-4-methyl-1-(4-methoxyphenyl)-1H-2,4-benzodiazepin;
4,5-Dihydro-3-[(4-methansulfonamidophenylsulfonyl)methyl]-4-methyl-1-(4-chlorophenyl)-1H-2,4-benzodiazepin;
4,5-Dihydro-3-[(4-methansulfonamidophenoxy)methyl]-4-methyl-1-(4-methylthiophenyl)-1H-2,4-benzodiazepin; und
4,5-Dihydro-4-methyl-1-(2,4-difluorphenyl)-3-[2-(4-methansulfonamidophenyl)ethyl]-1H-2,4-benzodiazepin
umfassenden Gruppe gewählt ist.

21. Verwendung nach Anspruch 19, wobei die Verbindung (+)-4,5-Dihydro-3-[(4-methansulfonamidophenoxy)methyl]-4-methyl-1-phenyl-1H-2,4-benzodiazepin ist.

22. Verwendung nach Anspruch 16, wobei die Verbindung der Formel 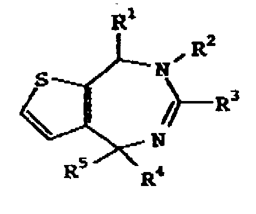 entspricht.

23. Verwendung nach Anspruch 22, wobei R¹ Wasserstoff ist, R² Niederalkyl ist; R³ -(CH₂)ₘ-Xₙ-R⁸ ist, worin m Eins oder Zwei ist; n Null ist; R⁸ Phenyl mit einem Niederalkylsulfonamido-Substituienten ist; R⁴ Wasserstoff ist; und R⁵ Phenyl ist.

24. Verwendung nach Anspruch 23, wobei R² Methyl ist; und R⁸ 4-Methylsulfonamidophenyl ist.

25. Verwendung nach Anspruch 24, wobei die Verbindung aus der
4,5-Dihydro-4-methyl-1-phenyl-3-[(4-methansulfonamidophenoxy)methyl]-1H-thieno[2,3-e]-2,4-diazepin und
4,5-Dihydro-4-methyl-1-phenyl-3-[2-(4-methansulfonamidophenyl) ethyl]-1H-thieno[2,3-e]-2,4-diazepin umfassenden Gruppe gewählt ist.

26. Zusammensetzung zur Behandlung von Pulsarrythmie bei einem Patienten mit beeinträchtigter Ventrikelfunktion oder Blutandrang erzeugendem Herzversagen, umfassend eine antiarriythmisch wirksame Menge einer Verbindung der Formel, wie in mindestens einem der Ansprüche 16 bis 25 definiert, zusammen mit einem pharmazeutisch annehmbaren Träger, Adjuvans oder Corrigens.

## Revendications

1. Composé de formule : 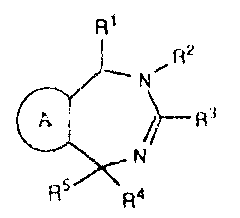 dans laquelle
1) A est un noyau choisi parmi phényle, thiényle, furannyle, naphtyle, pyridinyle, cyclohexyle et phényle ayant un ou deux substituants choisis parmi les groupes amino, alkyle inférieurs, alcoxy inférieurs, halogéno, nitro et alkylsulfonamido inférieurs ;
R¹ est un atome d'hydrogène, un groupe alkyle inférieur, un groupe benzyle, un groupe naphtyle, un groupe thiényle, un groupe pyridinyle, un groupe phényle ou un groupe phényle ayant un ou deux substituants choisis parmi un groupe alkyle inférieur et un groupe alcoxy inférieur ;
R² est -CH₂CH₂R⁷ où R⁷ est un groupe pyridinyle ;
R³ est Yₚ-(CH₂)ₘ-Xₙ-R⁸ où
Y est -NH-, -O-, -S- ou 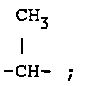 p est zéro ou un ;
m est un entier de zéro à sept ;
X est -S-, -O-, -SO₂-, 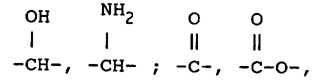 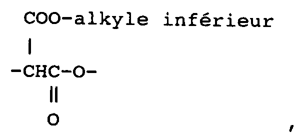 -CH=CH-, 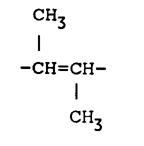 -C≡C-, 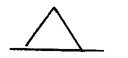 ou -NHSO₂- ;
n est zéro ou un ; et
R⁸ est un groupe phényle ayant un ou deux substituants choisis indépendamment parmi les groupes alkylsulfonamido inférieurs, polyfluoroalkylsulfonamido inférieurs et alkylaminosulfonyle inférieurs ;
R⁴ est un atome d'hydrogène ; un groupe alkyle inférieur ; un groupe allyle ; un groupe alcoxy inférieur-alkyle inférieur ; un groupe acétyle ; un groupe (alkyl inférieur)acéto ; un groupe (alkyl inférieur)carboxy ; ou un groupe α-hydroxyalkyle inférieur ; et
R⁵ est un atome d'hydrogène ; un groupe alkyle inférieur ; un groupe naphtyle ; un groupe thiényle ; un groupe pyridinyle ; un groupe benzyle ; un groupe phényle ; ou un groupe phényle ayant un ou deux substituants choisis indépendamment parmi les groupes alkyle inférieurs, alcoxy inférieurs, halogéno, hydroxy, amino, di(alkyl inférieur)amino, alkylsulfonamido inférieurs, acylamino inférieurs, alkylthio inférieurs et alkylsulfonyle inférieurs ;
ou un de ses sels d'addition d'acides ;
sous réserve que le nombre total des atomes de carbone dans R¹ plus R² plus R⁴ plus R⁵ soit de cinq ou plus ; ou
2) R² est un atome d'hydrogène ; un groupe alkyle inférieur ; un groupe benzyle ; un groupe phényle ; un groupe phényle substitué par un groupe halogéno, alkyle inférieur ou alcoxy inférieur ; ou
R² est -CH₂CH₂R⁷ où R⁷ est un groupe alcoxy inférieur ; un groupe benzyle : un groupe di(alkyl inférieur)amino ; un groupe pyrrolidino ; un groupe pipéridino ; un groupe morpholino ; un groupe pyridinyle ; un groupe phényle ; ou un groupe phényle substitué avec un groupe amino, nitro ou alkylsulfonamido inférieur ;
n est un ;
X est -NHSO₂- ; et
A, R¹, R³, Y, p, m, R⁸, R⁴ et R⁵ sont tels que précédemment définis dans la partie 1) ; ou
3) R⁸ est un groupe phényle ayant un ou deux substituants choisis indépendamment parmi les groupes polyfluoroalkylsulfonamido inférieurs et alkylaminosulfonyle inférieurs ;
R² est tel que défini dans la partie 2) ci-dessus ; et A, R¹, R³, Y, p, m, X, n, R⁴ et R⁵ sont tels que définis dans la partie 1) ci-dessus ; ou
4) R⁵ est un groupe phényle ayant un ou deux substituants choisis indépendamment parmi les groupes alkylthio inférieurs et alkylsulfonyle inférieurs ;
R² est tel que défini dans la partie 2) ci-dessus : et A, R¹, R³, Y, p, m, X, n, R⁸ et R⁴ sont tels que définis dans la partie 1) ci-dessus ; étant entendu qu'un groupe alkyle inférieur est constitué d'une chaîne carbonée saturée linéaire, ramifiée ou cyclique de huit atomes de carbone ou moins ; et un groupe alcoxy inférieur est un substituant alkyloxy linéaire ou ramifié contenant huit atomes de carbone ou moins.

2. Composé selon la revendication 1, où
A est un noyau choisi parmi phényle, thiényle, furannyle, naphtyle, pyridinyle, cyclohexyle et phényle ayant un ou deux substituants choisis parmi les groupes amino, alkyle inférieurs, alcoxy inférieurs, halogéno, nitro et alkylsulfonamido inférieurs ;
R¹ est un atome d'hydrogène, un groupe alkyle inférieur, un groupe benzyle, un groupe naphtyle, un groupe thiényle, un groupe pyridinyle, un groupe phényle ou un groupe phényle ayant un ou deux substituants choisis parmi un groupe alkyle inférieur et un groupe alcoxy inférieur ;
R² est -CH₂CH₂R⁷ où R⁷ est un groupe pyridinyle ;
R³ est Yₚ-(CH₂)ₘ-Xₙ-R⁸ où
Y est -NH-, -O-, -S- ou 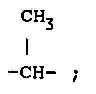 p est zéro ou un ;
m est un entier de zéro à sept ;
X est -S-, -O-, -SO₂-, 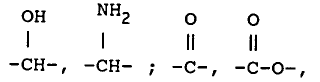 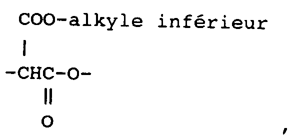 -CH=CH-, 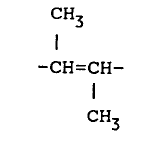 -C≡C-, 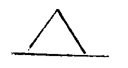 ou -NHSO₂ - ;
n est zéro ou un ; et
R⁸ est un groupe phényle ayant un ou deux substituants choisis indépendamment parmi les groupes alkylsulfonamido inférieurs, polyfluoroalkylsulfonamido inférieurs et alkylaminosulfonyle inférieurs ;
R⁴ est un atome d'hydrogène ; un groupe alkyle inférieur ; un groupe allyle ; un groupe alcoxy inférieur-alkyle inférieur ; un groupe acétyle ; un groupe (alkyl inférieur)acéto ; un groupe (alkyl inférieur)carboxy ; ou un groupe α-hydroxyalkyle inférieur ; et
R⁵ est un atome d'hydrogène ; un groupe alkyle inférieur ; un groupe naphtyle ; un groupe thiényle ; un groupe pyridinyle ; un groupe benzyle ; un groupe phényle ; ou un groupe phényle ayant un ou deux substituants choisis indépendamment parmi les groupes alkyle inférieurs, alcoxy inférieurs, halogéno, hydroxy, amino, di(alkyl inférieur)amino, alkylsulfonamido inférieurs, acylamino inférieurs, alkylthio inférieurs et alkylsulfonyle inférieurs ;
ou un de ses sels d'addition d'acides ;
sous réserve que le nombre total des atomes de carbone dans R¹ plus R² plus R⁴ plus R⁵ soit de cinq ou plus.

3. Composé selon la revendication 2, où A est un groupe phényle ; R¹ est un atome d'hydrogène ou un groupe alkyle inférieur ; R³ est Yₚ-(CH₂)ₘ-Xₙ-R⁸ où p est zéro, m est un entier de un à trois, X est -O-, n est un et R⁸ est un groupe phényle ayant un ou deux substituants alkylsulfonamido inférieurs ; R⁴ est un atome d'hydrogène ou un groupe alkyle inférieur ; et R⁵ est un groupe phényle ayant un ou deux substituants halogéno.

4. Composé selon la revendication 3, où : R¹ est un atome d'hydrogène ; R³ est Yₚ-(CH₂)ₘ-Xₙ-R⁸ où m est un, R⁸ est un groupe phényle ayant un substituant alkylsulfonamido inférieur ; R⁴ est un atome d'hydrogène et R⁵ est un groupe phényle ayant un substituant halogéno.

5. 4,5-dihydro-3-[(4-méthanesulfonamidophénoxy)méthyl]-4-(4-pyridinyléthyl)-1-(4-chlorophényl)-1H-2,4-benzodiazépine.

6. Composé selon la revendication 1, où A est un noyau choisi parmi phényle, thiényle, furannyle, naphtyle, pyridinyle, cyclohexyle et phényle ayant un ou deux substituants choisis parmi les groupes amino, alkyle inférieurs, alcoxy inférieurs, halogéno, nitro et alkylsulfonamido inférieurs ;
R¹ est un atome d'hydrogène, un groupe alkyle inférieur, un groupe benzyle, un groupe naphtyle, un groupe thiényle, un groupe pyridinyle, un groupe phényle ou un groupe phényle ayant un ou deux substituants choisis parmi un groupe alkyle inférieur et un groupe alcoxy inférieur ;
R² est un atome d'hydrogène ; un groupe alkyle inférieur ; un groupe benzyle ; un groupe phényle ; un groupe phényle substitué avec un groupe halogéno, alkyle inférieur ou alcoxy inférieur ; ou
R² est -CH₂CH₂R⁷ où R⁷ est un groupe alcoxy inférieur ; un groupe benzyle ; un groupe di(alkyl inférieur)amino ; un groupe pyrrolidino ; un groupe pipéridino ; un groupe morpholino ; un groupe pyridinyle ; un groupe phényle ; ou un groupe phényle substitué avec un groupe amino, nitro ou alkylsulfonamido inférieur ;
R³ est Yₚ-(CH₂)ₘ-Xₙ-R⁸ où
Y est -NH-, -O-, -S- ou 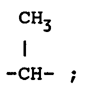
p est zéro ou un ;
m est un entier de zéro à sept ;
X est -NHSO₂- ;
n est un ; et
R⁸ est un groupe phényle ayant un ou deux substituants choisis indépendamment parmi les groupes alkylsulfonamido inférieurs, polyfluoroalkylsulfonamido inférieurs et alkylaminosulfonyle inférieurs ;
R⁴ est un atome d'hydrogène ; un groupe alkyle inférieur ; un groupe allyle ; un groupe alcoxy inférieur-alkyle inférieur ; un groupe acétyle ; un groupe (alkyl inférieur)acéto ; un groupe (alkyl inférieur)carboxy ; ou un groupe α-hydroxyalkyle inférieur ; et
R⁵ est un atome d'hydrogène ; un groupe alkyle inférieur ; un groupe naphtyle ; un groupe thiényle ; un groupe pyridinyle ; un groupe benzyle ; un groupe phényle ; ou un groupe phényle ayant un ou deux substituants choisis indépendamment parmi les groupes alkyle inférieurs, alcoxy inférieurs, halogéno, hydroxy, amino, di(alkyl inférieur)amino, alkylsulfonamido inférieurs, acylamino inférieurs, alkylthio inférieurs et alkylsulfonyle inférieurs ;
ou un de ses sels d'addition d'acides ;
sous réserve que le nombre total des atomes de carbone dans R¹ plus R² plus R⁴ plus R⁵ soit de cinq ou plus.

7. Composé selon la revendication 1, où A est un noyau choisi parmi phényle, thiényle, furannyle, naphtyle, pyridinyle, cyclohexyle et phényle ayant un ou deux substituants choisis parmi les groupes amino, alkyle inférieurs, alcoxy inférieurs, halogéno, nitro et alkylsulfonamido inférieurs ;
R¹ est un atome d'hydrogène, un groupe alkyle inférieur, un groupe benzyle, un groupe naphtyle, un groupe thiényle, un groupe pyridinyle, un groupe phényle ou un groupe phényle ayant un ou deux substituants choisis parmi un groupe alkyle inférieur et un groupe alcoxy inférieur ;
R² est un atome d'hydrogène ; un groupe alkyle inférieur ; un groupe benzyle ; un groupe phényle ; un groupe phényle substitué avec un groupe halogéno, alkyle inférieur ou alcoxy inférieur ; ou
R² est -CH₂CH₂R⁷ où R⁷ est un groupe alcoxy inférieur ; un groupe benzyle ; un groupe di(alkyl inférieur)amino ; un groupe pyrrolidino ; un groupe pipéridino ; un groupe morpholino ; un groupe pyridinyle ; un groupe phényle ; ou un groupe phényle substitué avec un groupe amino, nitro ou alkylsulfonamido inférieur ;
R³ est Yₚ-(CH₂)ₘ-Xₙ-R⁸ où
Y est -NH-, -O-, -S- ou 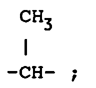 p est zéro ou un ;
m est un entier de zéro à sept ;
X est -S-, -O-, -SO₂-, 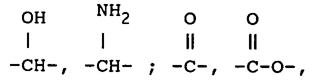 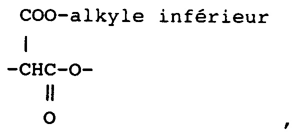 -CH=CH-, 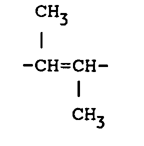 -C≡C-, 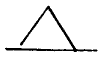 ou -NHSO₂- ;
n est zéro ou un ; et
R⁸ est un groupe phényle ayant un ou deux substituants choisis indépendamment parmi les groupes polyfluoroalkylsulfonamido inférieurs et alkylaminosulfonyle inférieurs ;
R⁴ est un atome d'hydrogène ; un groupe alkyle inférieur ; un groupe allyle ; un groupe alcoxy inférieur-alkyle inférieur ; un groupe acétyle ; un groupe (alkyl inférieur)acéto ; un groupe (alkyl inférieur)carboxy ; ou un groupe α-hydroxyalkyle inférieur ; et
R⁵ est un atome d'hydrogène ; un groupe alkyle inférieur ; un groupe naphtyle ; un groupe thiényle ; un groupe pyridinyle ; un groupe benzyle ; un groupe phényle ; ou un groupe phényle ayant un ou deux substituants choisis indépendamment parmi les groupes alkyle inférieurs, alcoxy inférieurs, halogéno, hydroxy, amino, di(alkyl inférieur)amino, alkylsulfonamido inférieurs, acylamino inférieurs, alkylthio inférieurs et alkylsulfonyle inférieurs ;
ou un de ses sels d'addition d'acides ;
sous réserve que le nombre total des atomes de carbone dans R¹ plus R² plus R⁴ plus R⁵ soit de cinq ou plus.

8. Composé selon la revendication 7 où A est un phényle ; R¹ est un atome d'hydrogène ou un groupe alkyle inférieur ; R² est un atome d'hydrogène ou un groupe alkyle inférieur ; R³ est Yₚ-(CH₂)ₘ-Xₙ-R⁸ dans laquelle p est zéro, m est un entier de un à trois, n est zéro et R⁸ est un groupe phényle ayant un substituant polyfluoroalkylsulfonamido inférieur ou alkylaminosulfonyle inférieur ; R⁴ est un atome d'hydrogène ou un groupe alkyle inférieur ; et R⁵ est un groupe phényle,

9. Composé selon la revendication 8, où R¹ est un atome d'hydrogène ; R² est un groupe alkyle inférieur ; R³ est Yₚ-(CH₂)ₘ-Xₙ-R⁸ où m est 2 ; et R⁴ est un atome d'hydrogène.

10. Composé selon la revendication 1, où A est un noyau choisi parmi phényle, thiényle, furannyle, naphtyle, pyridinyle, cyclohexyle et phényle ayant un ou deux substituants choisis parmi les groupes amino, alkyle inférieurs, alcoxy inférieurs, halogéno, nitro et alkylsulfonamido inférieurs ;
R¹ est un atome d'hydrogène, un groupe alkyle inférieur, un groupe benzyle, un groupe naphtyle, un groupe thiényle, un groupe pyridinyle, un groupe phényle ou un groupe phényle ayant un ou deux substituants choisis parmi un groupe alkyle inférieur et un groupe alcoxy inférieur ;
R² est un atome d'hydrogène ; un groupe alkyle inférieur ; un groupe benzyle ; un groupe phényle ; un groupe phényle substitué avec un groupe halogéno, alkyle inférieur ou alcoxy inférieur ; ou
R² est -CH₂CH₂R⁷ où R⁷ est un groupe alcoxy inférieur ; un groupe benzyle ; un groupe di(alkyl inférieur)amino ; un groupe pyrrolidino ; un groupe pipéridino ; un groupe morpholino ; un groupe pyridinyle ; un groupe phényle ; ou un groupe phényle substitué avec un groupe amino, nitro ou alkylsulfonamido inférieur ;
R³ est Yₚ-(CH₂)ₘ-Xₙ-R⁸ où
Y est -NH-, -O-, -S- ou 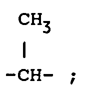 p est zéro ou un ;
m est un entier de zéro à sept ;
X est -S-, -O-, -SO₂-, 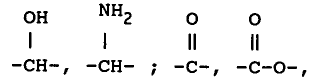 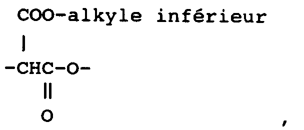 -CH=CH-, 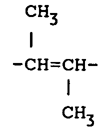 -C≡C-, 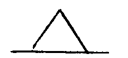 ou -NHSO₂- ;
n est zéro ou un ; et
R⁸ est un groupe phényle ayant un ou deux substituants choisis indépendamment parmi les groupes alkylsulfonamido inférieurs, polyfluoroalkylsulfonamido inférieurs et alkylaminosulfonyle inférieurs ;
R⁴ est un atome d'hydrogène ; un groupe alkyle inférieur ; un groupe allyle ; un groupe alcoxy inférieur-alkyle inférieur ; un groupe acétyle ; un groupe (alkyl inférieur)acéto ; un groupe (alkyl inférieur)carboxy ; ou un groupe α-hydroxyalkyle inférieur ; et
R⁵ est un groupe phényle ayant un ou deux substituants choisis indépendamment parmi les groupes alkylthio inférieurs et alkylsulfonyle inférieurs ;
ou un de ses sels d'addition d'acides ;
sous réserve que le nombre total des atomes de carbone dans R¹ plus R² plus R⁴ plus R⁵ soit de cinq ou plus ;

11. Composé selon la revendication 10 où A est un groupe phényle ; R¹ est un atome d'hydrogène ou un groupe alkyle inférieur ; R² est un atome d'hydrogène ou un groupe alkyle inférieur ; R³ est Yₚ-(CH₂)ₘ-Xₙ-R⁸ dans laquelle p est zéro, m est un entier de un à trois, X est -O-, n est un et R⁸ est un groupe phényle ayant un substituant alkylsulfonamido inférieur ; et R⁴ est un atome d'hydrogène ou un groupe alkyle inférieur,

12. Composé selon la revendication 11, où R¹ est un atome d'hydrogène ; R² est un groupe alkyle inférieur ; R³ est Yₚ-(CH₂)ₘ-Xₙ-R⁸ où m est 1 ; et R⁴ est un atome d'hydrogène.

13. Composé selon la revendication 12 choisi parmi la 4,5-dihydro-3[(4-méthanesulfonamidophénoxy)méthyl]-4-méthyl-1-(4-méthylthiophényl)-1H-2,4-benzodiazépine ou la 4,5-dihydro-3-[(4-méthanesulfonamidophénoxy)méthyl]-4-méthyl-1-(4-méthylsulfonylphényl)-1H-2,4-benzodiazépine,

14. Utilisation d'un composé selon l'une quelconque des revendications 1 à 13, pour la préparation d'un médicament pour le traitement de l'arythmie cardiaque chez un patient nécessitant un tel traitement.

15. Composition pour le traitement de l'arythmie cardiaque comprenant une quantité efficace contre l'arythmie d'un composé selon l'une quelconque des revendications 1 à 13.

16. Utilisation d'un composé de formule : 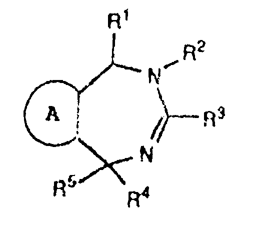 dans laquelle :
A est un noyau choisi dans le groupe constitué par phényle, thiényle et phényle substitué sur un atome de carbone disponible quelconque par un ou deux groupes alkylsulfonamido inférieurs ;
R¹ est un atome d'hydrogène ou un groupe phényle ;
R² est un groupe alkyle inférieur ou -CH₂CH₂R^{7'} où R^{7'} est un groupe alcoxy inférieur, pyridinyle, phényle ou phényle ayant un ou deux substituants alkylsulfonamido inférieurs ;
R³ est -(CH₂)ₘ-Xₙ-R⁸ où
m est un entier de zéro à sept ;
X est -S-, -O-, -SO₂-, -CH=CH- ou -NHSO₂- ;
n est zéro ou un ;
R⁸ est un groupe phényle ayant un ou deux substituants choisis indépendamment parmi alkylsulfonamido inférieurs et polyfluoroalkylsulfonamido inférieurs ;
R⁴ est un atome d'hydrogène ou un groupe alkyle inférieur ; et
R⁵ est un atome d'hydrogène, un groupe pyridinyle, un groupe benzyle, un groupe phényle ou un groupe phényle ayant ou deux substituants choisis indépendamment parmi les groupes halogéno, alkyle inférieurs, alcoxy inférieurs, alkylthio inférieurs, alkylsulfonyle inférieurs, alkylsulfonamido inférieurs et hydroxy ;
ou un sel d'addition d'acide de celui-ci ; sous réserve que le nombre total des atomes de carbone de R¹ plus R² plus R⁴ plus R⁵ soit de cinq ou plus ; et sous réserve de plus qu'au moins un de R², R³, R⁵ ou du noyau A contienne un groupe pyridinyle, un groupe -NHSO₂- ou un substituant alkylsulfonamido inférieur, di(alkyl inférieur)aminosulfonyle ou polyfluoroalkylsulfonamido inférieur ; pour la préparation d'un médicament pour le traitement de l'arythmie cardiaque chez un patient présentant une altération de la fonction ventriculaire ou une insuffisance cardiaque.

17. Utilisation selon la revendication 16, où le composé répond à la formule : 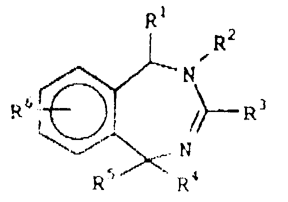 dans laquelle R⁶ est un atome d'hydrogène ou un ou deux groupes alkylsulfonamido inférieurs substituant un atome de carbone disponible quelconque du cycle phényle.

18. Utilisation selon la revendication 17, où :
R² est un groupe alkyle inférieur ou -CH₂CH₂R^{7'} où R^{7'} est un groupe alcoxy inférieur, pyridinyle ou phényle ayant un substituant alkylsulfonamido inférieur ;
R³ est -(CH₂)ₘ-Xₙ-R⁸ où
m est un entier de zéro à trois ;
R⁸ est un groupe phényle ayant un ou deux substituants choisis indépendamment parmi les groupes alkylsulfonamido inférieurs et trifluorométhylsulfonamido ; et
R⁶ est un atome d'hydrogène ou un groupe alkylsulfonamido inférieur substituant l'un quelconque des atomes de carbone disponibles du cycle phényle.

19. Utilisation selon la revendication 18, où :
R² est un groupe méthyle ou -CH₂CH₂R^{7'} où R^{7'} est un groupe méthoxy, un groupe 4-pyridinyle ou un groupe phényle ayant un substituant méthylsulfonamido ;
R⁸ est un groupe phényle ayant un ou deux substituants choisis indépendamment parmi les groupes alkylsulfonamido inférieurs et trifluorométhylsulfonamido ;
R⁴ est un atome d'hydrogène ou un groupe méthyle ;
R⁵ est un atome d'hydrogène, un groupe 4-pyridinyle, un groupe benzyle, un groupe phényle ou un groupe phényle ayant un ou deux substituants choisis indépendamment parmi les groupes halogéno, alkyle inférieurs, alcoxy inférieurs, alkylthio inférieurs, alkylsulfonyle inférieurs, alkylsulfonamido inférieurs et hydroxy ; et
R⁶ est un atome d'hydrogène ou un groupe 6- ou 8-méthylsulfonamido.

20. Utilisation selon la revendication 19, dans laquelle le composé est choisi parmi :
la 4,5-dihydro-3-[(4-méthanesulfonamidophénoxy)méthyl]-4-méthyl-1-phényl-1H-2,4-benzodiazépine ;
la 4,5-dihydro-3-[(4-méthanesulfonamidophénoxy)méthyl]-4-méthyl-1-(4-chlorophényl)-1H-2,4-benzodiazépine ;
la 4,5-dihydro-3-[(4-méthanesulfonamidophénoxy)méthyl)-4-méthyl-1-(4-méthoxyphényl)-1H-2,4-benzodiazépine ;
la 4,5-dihydro-3-[(4-méthanesulfonamidophénylsulfonyl) méthyl]-4-méthyl-1-(4-chlorophényl)-1H-2,4-benzodiazépine ;
la 4,5-dihydro-3-[(4-méthanesulfonamidophénoxy)méthyl]-4-méthyl-1-(4-méthylthiophényl)-1H-2,4-benzodiazépine ; et
la 4,5-dihydro-4-méthyl-1-(2,4-difluorophényl)-3-(2-(4-méthanesulfonamidophényl)éthyl]-1H-2,4-benzodiazépine.

21. Utilisation selon la revendication 18 où le composé est la (+)-4,5-dihydro-3-[(4-méthanesulfonamidophénoxy)méthyl]-4-méthyl-1-phényl-1H-2,4-benzodiazépine.

22. Utilisation selon la revendication 16 où le compose répond à la formule :

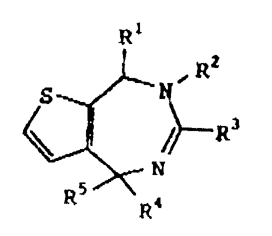

23. Utilisation selon la revendication 22 où R¹ est un atome d'hydrogène ; R² est un groupe alkyle inférieur ; R³ est -(CH₂)ₘ-Xₙ-R⁸ où m est un ou deux ; n est zéro ; R⁸ est un groupe phényle ayant un substituant alkylsulfonamido inférieur ; R⁴ est un atome d'hydrogène ; et R⁵ est un groupe phényle.

24. Utilisation selon la revendication 23 où R² est un groupe méthyle et R⁸ est un groupe 4-méthylsulfonamidophényle.

25. Utilisation selon la revendication 24 où le composé est choisi parmi :
la 4,5-dihydro-4-méthyl-1-phényl-3-[(4-méthanesulfonamidophénoxy)méthyl]-1H-thiéno[2,3-e]-2,4-diazépine et
la 4,5-dihydro-4-méthyl-1-phényl-3-[2-(4-méthanesulfonamidophényl)éthyl]-1H-thiéno[2,3-e]-2,4-diazépine.

26. Composition pour le traitement de l'arythmie cardiaque chez un patient présentant une altération de la fonction ventriculaire ou une insuffisance cardiaque, qui comprend une quantité efficace contre l'arythmie d'un composé de formule définie dans l'une quelconque des revendications 16 à 25, avec un véhicule, adjuvant ou excipient pharmaceutiquement acceptables.
